# EUROPEAN PATENT APPLICATION

(11) **EP 2 272 858 A2**
(43) Date of publication of application: **12.01.2011**
(21) Application number: 10161798.3
(22) Date of filing: 09.04.2007
(51) Int. Cl.: C07K 5/06, C07K 5/08, C07K 5/10, A61K 38/00, A61P 31/14

(54) **HCV inhibitors comprising beta amino acids and their uses**

(30) Priority: 11.04.2006 US 791611 P
(62) Divisional of application: 07781534.8
(71) Applicant: Novartis AG, 4056 Basel (CH)
(72) Inventor: Brandl, Trixi, 4056, Basel (CH); Britt, Shawn D., Andover, MA 01840 (US); Cottens, Sylvain, 4108, Witterswil (CH); Ehrhardt, Claus, 79541, Lörrach (DE); Fu, Jiping, Arlington, MA 02474 (US); Karur, Subramanian, Arlington, MA 02476 (US); Li, Hongju, Edison, NJ 08820 (US); Lu, Peichao, Malden, MA 02148 (US); Parker, David Thomas, Lexington, MA 02420 (US); Patane, Michael A., Andover, MA 01810 (US); Radetich, Branko, Boston, MA 02116 (US); Raman, Prakash, Acton, MA 01720 (US); Randl, Stefan Andreas, 63457, Hanau (DE); Rigollier, Pascal, 68100, Mulhouse (FR); Seepersaud, Mohindra, Acton, MA 01720 (US); Simic, Oliver, 4054, Basel (CH); Tichkule, Ritesh Bhanudasji, Cambridge, MA 02141 (US); Zhu, Yanyi, Acton, MA 01720 (US)
(74) Representative: Strang, Andrea Josephine

(57) **Abstract**

The present application describes organic compounds that are useful for the treatment, prevention and/or amelioration of human diseases.

## Description

### Background

Hepatitis C virus (HCV) is a (+)-sense single-stranded RNA virus that has been implicated as the major causative agent in non-A, non-B hepatitis (NANBH), particularly in blood-associated NANBH (BB-NANBH). NANBH is to be distinguished from other types of virai-induced liver disease, such as hepatitis A virus (HAV), hepatitis B virus (HBV), delta hepatitis virus (HDV), cytomegalovirus (CMV) and Epstein-Barr virus (EBV), as well as from other forms of liver disease such as alcoholism and primary biliar cirrhosis.

Recently, an HCV protease necessary for polypeptide processing and viral replication has been identified, cloned and expressed. (See, *e.g*., U.S. Pat. No. 5,712,145). This approximately 3000 amino acid polyprotein contains, from the amino terminus to the carboxy terminus, a nucleocapsid protein (C), envelope proteins (El and E2) and several non-structural proteins (NS1, 2, 3, 4a, 5a and 5b). NS3 is an approximately 68 kda protein, encoded by approximately 1893 nucleotides of the HCV genome, and has two distinct domains: (a) a serine protease domain consisting of approximately 200 of the N-terninal amino acids; and (b) an RNA-dependent ATPase domain at the C-terminus of the protein. The NS3 protease is considered a member of the chymotrypsin family because of similarities in protein sequence, overall three-dimensional structure and mechanism of catalysis. The HCV NS3 serine protease is responsible for proteolysis of the polypeptide (polyprotein) at the NS3/NS4a, NS4a/NS4b, NS4b/NS5a and NS5a/NS5b junctions and is thus responsible for generating four viral proteins during viral replication. This has made the HCV NS3 serine protease an attractive target for antiviral chemotherapy.

It has been determined that the NS4a protein, an approximately 6 kda polypeptide, is a co-factor for the serine protease activity of NS3. Autocleavage of the NS3/NS4a junction by the NS3/NS4a serine protease occurs intramolecularly (*i.e*., cis) while the other cleavage sites are processed intermolecularly (*i.e*., trans).

HCV has been implicated in cirrhosis of the liver and in induction of hepatocellular carcinoma. The prognosis for patients suffering from HCV infection is currently poor. HCV infection is more difficult to treat than other forms of hepatitis due to the lack of immunity or remission associated with HCV infection. Current data indicates a less than 50% survival rate at four years post cirrhosis diagnosis. Patients diagnosed with localized resectable hepatocellular carcinoma have a five-year survival rate of 10-30%, whereas those with localized unresectable hepatocellular carcinoma have a five-year survival rate of less than 1%.

Current therapies for hepatitis C include interferon-α (INF_{α}) and combination therapy with ribavirin and interferon. See, *e.g*., Beremguer et al. (1998) Proc. Assoc. Am. Physicians 110(2):98-112. These therapies suffer from a low sustained response rate and frequent side effects. See, *e,g*., Hoofnagle et al. (1997) N. Engl. J. Med. 336:347. Currently, no vaccine is available for HCV infection.

### Summary of the Invention

There remains a need for new treatments and therapies for HCV infection, as well as HCV-associated disorders. There is also a need for compounds useful in the treatment or prevention or amelioration of one or more symptoms of HCV, as well as a need for methods of treatment or prevention or amelioration of one or more symptoms of HCV. Furthermore, there is a need for methods for modulating the activity of HCV-serine proteases, particularly the HCV NS3/NS4a serine protease, using the compounds provided herein.

In one aspect, the invention provides compounds of the formula I: 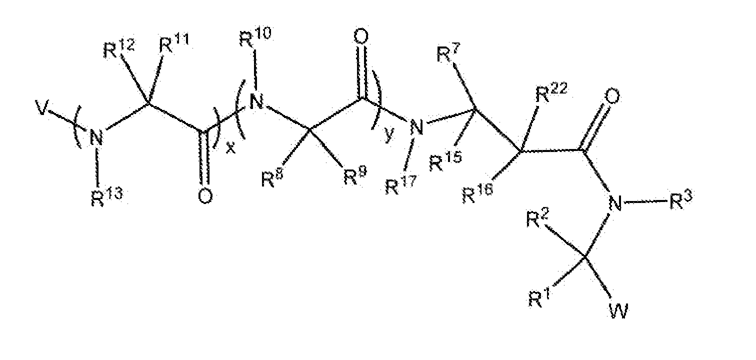 and pharmaceutically acceptable salts and stereoisomers thereof.

In another aspect, the invention provides compounds of the formula II: 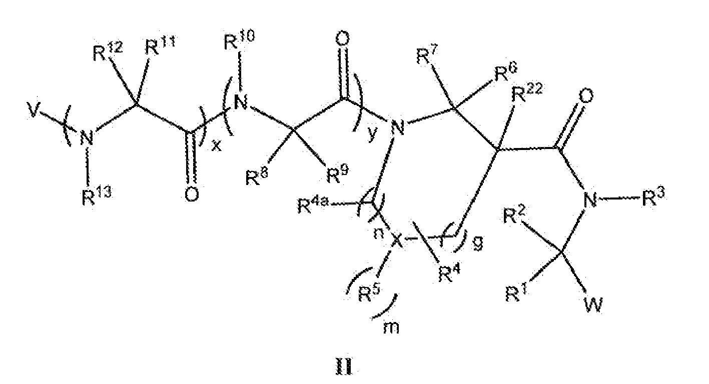 and pharmaceutically acceptable salts and stereoisomers thereof.

In one embodiment, the invention provides a method of treating an HCV-associated disorder comprising administering to a subject in need thereof a pharmaceutically acceptable amount of a compound of the invention, such that the HCV-associated disorder is treated.

In another embodiment, the invention provides a method of treating an HIV infection comprising administering to a subject in need thereof a pharmaceutically acceptable amount of a compound of the invention.

In still another embodiment, the invention provides a method of treating, inhibiting or preventing the activity of HCV in a subject in need thereof, comprising administering to the subject a pharmaceutically acceptable amount of a compound of the invention. In one embodiment, the compounds of the invention inhibit the activity of the NS2 protease, the NS3 protease, the NS3 helicase, the NS5a protein, and/or the NS5b polymerase. In another embodiment, the interaction between the NS3 protease and NS4A cofactor is disrupted. In yet another embodiment, the compounds of the invention prevent or alter the severing of one or more of the NS4A-NS4B, NS4B-NS5A and NS5A-NS5B junctions of the HCV. In another embodiment, the invention provides a method of inhibiting the activity of a serine protease, comprising the step of contacting said serine protease with a compound of the invention. In another embodiment, the invention provides a method of treating, inhibiting or preventing the activity of HCV in a subject in need thereof, comprising administering to the subject a pharmaceutically acceptable amount of a compound of the invention, wherein the compound interacts with any target in the HCV life cycle. In one embodiment, the target of the HCV life cycle is selected from the group consisting of NS2 protease, NS3 protease, NS3 helicase, NS5a protein andNS5b polymerase.

In another embodiment, the invention provides a method of decreasing the HCV RNA load in a subject in need thereof comprising administering to the subject a pharmaceutically acceptable amount of a compound of the invention.

In another embodiment, the compounds of the invention exhibit HCV protease activity. In one embodiment, the compounds are an HCV NS3-4A protease inhibitor.

In another embodiment, the invention provides a method of treating an HCV-associated disorder in a subject, comprising administering to a subject in need thereof a pharmaceutically acceptable amount of a compound of the invention, and a pharmaceutically acceptable carrier, such that the HCV-associated disorder is treated.

In still another embodiment, the invention provides a method of treating an HCV-associated disorder comprising administering to a subject in need thereof a pharmaceutically effective amount of a compound of the invention, in combination with a pharmaceutically effective amount of an additional HCV-modulating compound, such as interferon or derivatized interferon, or a cytochrome P450 monooxygenase inhibitor, such that the HCV-associated disorder is treated. In one embodiment, the additional HCV-modulating compound is selected from the group consisting of Sch 503034 and VX-950.

In another embodiment, the invention provides a method of inhibiting hepatitis C virus replication in a cell, comprising contacting said cell with a compound of the invention.

In yet another embodiment, the invention provides a packaged HCV-associated disorder treatment, comprising an HCV-modulating compound of the invention, packaged with instructions for using an effective amount of the HCV-modulating compound to treat an HCV-associated disorder.

In certain embodiments, the HCV-associated disorder is selected from the group consisting of HCV infection, liver cirrhosis, chronic liver disease, hepatocellular carcinoma, cryoglobulinaemia, non-Hodgkin's lymphoma, and a suppressed innate intracellular immune response.

In another embodiment, the invention provides a method of treating HCV infection, liver cirrhosis, chronic liver disease, hepatecollular carcinoma, cryoglobulinaemia, non-Hodgkin's lymphoma, and/or a suppressed innate intracellular immune response in subject in need thereof comprising administering to the subject a pharmaceutically acceptable amount of a compound of the invention.

In one embodiment, the HCV to be treated is selected of any HCV genotype. In another embodiment, the HCV is selected from HCV genotype 1, 2 and/or 3.

### Detailed Description of the Invention

This invention is directed to compounds, *e.g*., peptide compounds, and intermediates thereto, as well as pharmaceutical compositions containing the compounds for use in treatment of HCV infection. This invention is also directed to the compounds of the invention or compositions thereof as protease inhibitors, particularly as serine protease inhibitors, and more particularly as HCV NS3 protease inhibitors. The compounds are particularly useful in interfering with the life cycle of the hepatitis C virus and in treating or preventing an HCV infection or physiological conditions associated therewith. The present invention is also directed to methods of combination therapy for inhibiting HCV replication in cells, or for treating or preventing an HCV infection in patients using the compounds of the invention or pharmaceutical compositions, or kits thereof.

In one aspect, the invention provides a compound of the Formula 1: 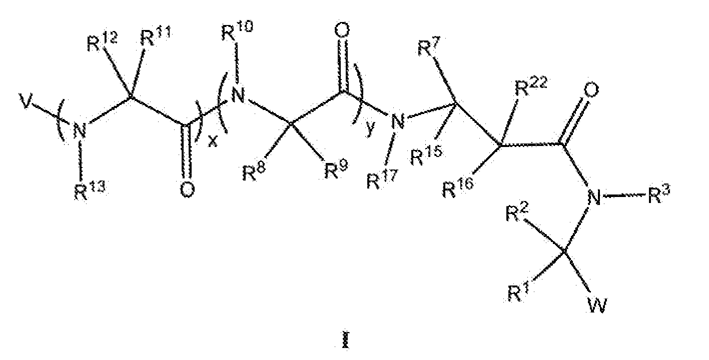 and pharmaceutically acceptable salts, enantiomers, stereoisomers, rotamers, tautomers, diastereomers, or racemates thereof;
wherein
x is 0 or 1;
y is 0, 1 or 2;
R¹, R², R³, R⁷,R⁸, R⁹, R¹⁰, R¹¹, R¹², R¹³ R¹⁶ R¹⁵, R¹⁷, R²², V and W are each, independently, selected from hydrogen or from the group consisting of alkyl, alkyl-aryl, heteroalkyl, heterocyclyl, heteroaryl, aryl-heteroaryl, alkyl-heteroaryl, cycloalkyl, alkyloxy, alkyl-aryloxy, aryloxy, heteroaryloxy, heterocyclyloxy, cycloalkyloxy, amino, alkylamino, arylamino, alkyl-arylamino, arylamino, heteroarylamino, cycloalkylamino, carboxyalkylamino, arlylalkyloxy and heterocyclylamino; each of which may be further independently substituted one or more times with X¹ and X²; wherein X¹ is alkyl,alkenyl, alkynyl, cycloalkyl, cycloalkyl-alkyl, heterocyclyl, heterocyclylalkyl, aryl, alkylaryl, aralkyl, aryloxy, arylthio, arylheteroaryl, heteroaryl, heterocyclylamino, alkylheteroaryl, or heteroaralkyl; wherein X¹ can be independently substituted with one or more of X² moieties which can be the same or different and are independently selected; wherein X² is hydroxy, oxo, alkyl, cycloalkyl, heterocycloalkyl, aryl, heteroaryl, alkoxy, aryloxy, thio, alkylthio, amino, mono- and di-alkylamino, arylamino, alkylsulfonyl, arylsulfonyl, alkylsulfonamido, arylsulfonamido, carboxy, carbalkoxy, carboxamido, alkoxycarbonylamino, alkoxycarbonyl, alkoxycarbonyloxy, alkylureido, arylureido, halogen, cyano, or nitro; wherein each X₂ residue selected to be alkyl, alkoxy, and aryl can be unsubstituted or optionally independently substituted with one or more moieties which can be the same or different and are independently selected from alkyl, alkenyl, alkynyl, cycloalkyl, cycloalkyl-alkyl, heterocyclyl, heterocyclylalkyl, aryl, alkylaryl, aralkyl, arylheteroaryl, heteroaryl, heterocyclylamino, alkylheteroaryl and heteroaralkyl;
W is also selected from the group consisting of C(O)OH, C(O)OR²⁴, C(O)-amine, C(O)-C(O)OH, C(=N-O-R²⁴)-C(O)-amine, C(O)N(H)S(O)₂R²⁴, C(O)-C(O)-amine, CON(H)SO₂-amine and C(O)-[C(O)]₂-heterocycle, wherein the heterocycle may be substituted or unsubstituted, wherein a is 0 or 1, wherein each R²⁴ is independently selected from hydrogen or from the group consisting of C₁₋₄-alkyl, C₃₋₆-cycloalkylC₀₋₄alkyl, substituted or unsubstituted aryl and substituted or unsubstituted heterocycle, each of which may be independently substituted one or more times with a halogen atom or C₁₋₄-alkyl;
V is also selected from the group consisting of -Q¹-Q², wherein Q¹ is absent, C(O), N(H), N(C₁₋₄-alkyl),C=N(CN), C=N(SO₂CH₃), or C=N-COH, and Q² is H or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
or R²² and R¹⁶ may together form a 3, 4, 5, 6 or 7-membered ring that is aromatic or non-aromatic and may contain one or more heteroatoms, wherein the ring may be further substituted one or more times;
or R⁷ and R¹⁵ may together form a 3, 4, 5, 6 or 7-membered ring that is aromatic or non-aromatic and may contain one or more heteroatoms, wherein the ring may be further substituted one or more times;
or R¹⁵ and R¹⁷ may together form a 3, 4, 5, 6 or 7-membered ring that is aromatic or non-aromatic and may contain one or more heteroatoms, wherein the ring may be further substituted one or more times;
or R¹⁵ and R¹⁶may together form a 4, 5, 6 or 7-membered ring that is aromatic or non-aromatic and may contain one or more heteroatoms, wherein the ring may be further substituted one or more times;
or R¹ and R² may together form a 3, 4, 5, 6 or 7-membered ring that is aromatic or non-aromatic and may contain one or more heteroatoms, wherein the ring may be further substituted one or more times;
or R¹⁷ and R¹⁶ may together form a 4, 5, 6, 7 or 8-membered ring of the formula III: 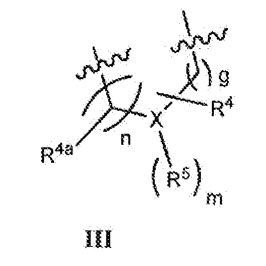 wherein
n and g are each, independently, 0, 1 or 2;
m is 0 or 1;
X is O, N or C;
R⁵, R⁴ and R^{4a} are each, independently, selected from hydrogen or oxo or are selected from the group consisting of hydroxyl, C₁₋₈alkyl, C₂₋₈-alkenyl, C₂₋₈-alkynyl, C₃₋₈-cycloalkyl-C₀₋₄-alkyl, aryl-C₀₋₄-alkyl, heterocycle-C₀₋₄-aryl, heteroaryl-C₀₋₄-alkyl, C₃₋₈-cycloalkyloxy, aryloxy, N(R₂₃)_{2 ,} NR₂₃COR₂₃, CONR₂₃R₂₃, NR₂₃CONHR₂₃, OCONR₂₃R₂₃, NR₂₃COOR₂₃, OCOR₂₃, COOR₂₃, aryl-C(O)O, aryl-C(O)NR₂₃, heteroaryloxy, heteroaryl-C(O)O, heteroaryl-C(O)NR₂₃, each of which may be independently substituted one or more times with a halogen atom, aryl, heteroaryl, trihalomethyl, C₁₋₄-alkyl, or C₁₋₄-alkoxy;
or R⁴ and R⁵ may together form a 4, 5, 6 or 7-membered ring that is aromatic or non-aromatic and may contain one or more heteroatoms, wherein the ring may be further substituted one or more times; and
R₂₃ is independently selected at each occurrence from hydrogen or the group consisting of alkyl, alkenyl, alkynyl, cycloalkyl, cycloalkylalkyl, aryl, heteroaryl, heteroaralkyl and aralkyl, each of which is substituted with 0-2 substituents independently selected from halogen, alkyl, and alkoxy.

In one embodiment of Formula I, R¹⁵ and R¹⁶ together form a ring of the formula IV: 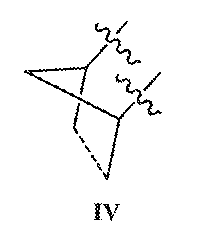 wherein
the dashed line represents a single or double bond, wherein formula IV may be further substituted one or more times.

In another embodiment of Formula I, R¹⁵ and R¹⁶ together form a ring of the formula V: 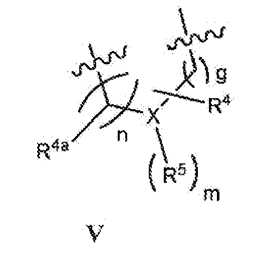 wherein
n and g are each, independently, 0, 1, 2 or 3 (such that the sum of n an g is less than 5);
m is 0 or 1;
X is O, N or C;
R⁵, R⁴ and R^{4a} are each, independently, selected from hydrogen or oxo or are selected from the group consisting of hydroxyl, C₁₋₈-alkyl, C₂₋₈-alkenyl, C₂₋₈-alkynyl, C₃₋₈-cycloalkyl-C₀₋₄-alkyl, aryl-C₀₋₄-alkyl, heterocycle-C₀₋₄-alkyl, heteroaryl-C₀₋₄-alkyl, C₃₋₈-cycloalkyloxy, aryloxy, N(R₂₃)_{2 ,} NR₂₃COR₂₃, CONR₂₃R₂₃, NR₂₃CONHR₂₃, OCONR₂₃R₂₃, NR₂₃COOR₂₃, OCOR₂₃, COOR₂₃, aryl-C(O)O, aryl-C(O)NR₂₃, heteroaryloxy, heteroaryl-C(O)O, heteroaryl-C(O)NR₂₃, each of which may be independently substituted one or more times with a halogen atom, aryl, heteroaryl, trihalomethyl, C₁₋₄-alkyl, or C₁₋₄-alkoxy;
or R⁴ and R⁵ may together form a 4, 5, 6 or 7-membered ring that is aromatic or non-aromatic and may contain one or more heteroatoms, wherein the ring may be further substituted one or more times;
R₂₃ is independently selected at each occurrence from hydrogen or the group consisting of alkyl, alkenyl, alkynyl, cycloalkyl, cycloalkylalkyl, aryl, heteroaryl, heteroaralkyl and aralkyl, each of which is substituted with 0-2 substituents independently selected from halogen, alkyl, and alkoxy;
or R¹⁵ and R¹⁶ may together form a 4, 5, 6 or 7-membered ring that is aromatic or non-aromatic and may contain one or more heteroatoms, wherein the ring may be further substituted one or more times;
or R¹ and R² may together form a 3, 4, 5, 6 or 7-membered ring that is aromatic or non-aromatic and may contain one or more heteroatoms, wherein the ring may be further substituted one or more times.

In yet another embodiment of Formula I, R³ is selected from the group consisting of H, C₁₋₄-alkyl, and C₃₋₆-cycloalkylC₀₋₄alkyl;
R⁸, R¹¹, R¹⁵ and R²² are selected from the group consisting of H, alkyl-aryl, C₁₋₄-alkyl, O-C₁₋₄-alkyl, N(H)-C₁₋₄-alkyl, and C₃₋₆-cycloalkylC₀₋₄alkyl;
R¹⁰ and R¹⁷ are each, independently, selected from the group consisting of H, C₁₋₄-alkyl and C₃₋₆-cycloalkylC₀₋₄alkyl; and
R¹³ is selected from the group consisting of -Q¹-Q², wherein Q¹ is absent, C(O), N(H), N(C₁₋₄-alkyl), C=N(CN), C=N(SO₂CH₃), or C=N-COH, and Q² is hydrogen or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl.

In still another embodiment of Formula I, y is 0, 1 or 2;
R¹ and R² are each, independently, selected from the group consisting of H, C₁₋₄-alkyl, O-C₁₋₄-alkyl, N(H)-C₁₋₄-alkyl, and C₃₋₆-cycloalkylC₀₋₄alkyl;
W is also selected from the group consisting of C(O)OH, C(O)OR²⁴, C(O)-amine, C(O)-C(O)H, C(=N-O-R²⁴)-C(O)-amine, C(O)-C(O)-amine and C(O)-[C(O)]ₐ-heterocycle, wherein the heterocycle may be independently substituted one or more times with aryl, C₁₋₄-alkyl, C₁₋₄-alkyl substituted by one or more halogen atoms, and C₃₋₆-cycloalkyl, wherein a is 0 or 1, wherein each R²⁴ is independently selected at each occurrence from hydrogen or from the group consisting of C₁₋₄-alkyl, C₃₋₆-cycloalkylC₀₋₄alkyl, substituted or unsubstituted aryl and substituted or unsubstituted heterocycle, each of which may be independently substituted one or more times with a halogen atom or C₁₋₄-alkyl;
R³ is selected from the group consisting of H, C₁₋₄-alkyl, and C₃₋₆-cycloalkylC₀₋₄alkyl;
R⁷ is selected from the group consisting of H, C₁₋₄-alkyl, O-C₁₋₄-alkyl, N(H)-C₁₋₄-alkyl, C₃₋₆-cycloalkylC₀₋₄alkyl, aryl, CON(H)SO₂-amine and heterocycle, each of which may be independently substituted one or more times with a halogen atom, C₁₋₄-alkyl, C₁₋₄-alkyl substituted by one or more halogen atoms, or C₃₋₆-cycloalkyl;
R⁸, R⁹, R¹¹, R¹², R¹⁵ and R¹⁶ are selected from the group consisting of H, C₁₋₄-alkyl, O-C₁₋₄-alkyl, N(H)-C₁₋₄-alkyl, and C₃₋₆-cycloalkylC₀₋₄alkyl;
R¹⁰ and R¹⁷ are each, independently, selected from the group consisting of H, C₁₋₄-alkyl and C₃₋₆-cycloalkylC₀₋₄alkyl;
R¹³ is selected from the group consisting of Q¹-Q², wherein Q¹ is absent, C(O), S(O)₂, N(H), N(C₁₋₄-alkyl), C=N(CN), C=N(SO₂CH₃), C=N-COH, or C=N-COC₁₋₄alkyl, and Q² is hydrogen or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-allkyl, C₃₋₆-cycloalkyl-C₀₋₄-alkyl, aryl, heteroaryl and heterocycle, each of which may be independently substituted one or more times with a halogen atoms, C₁₋₄-alkyl, C₁₋₄-alkyl substituted by one or more halogen atoms, or C₃₋₆-cycloalkyl;
V is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-COC₁₋₄alkyl, and Q² is hydrogen or is selected from the group consisting of C₁₋₄-alkyl, O-C₁₋₄-alkyl, NH₂, N(H)-C₁₋₄-alkyl, N(C₁₋4-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
or R¹⁷ and R¹⁶ may together form a 5- or 6-membered ring of the formula III': 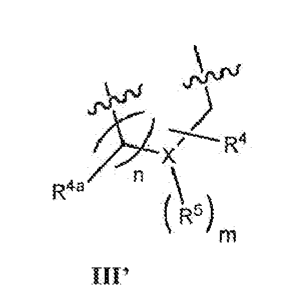 wherein
   m and n are each, independently, 0, 1 or 2;
   X is O, N or C;
   R⁵, R⁴ and R^{4a} are each, independently, selected from hydrogen or oxo or is selected from the group consisting of hydroxyl, C₁₋₈-alkyl, C₂₋₈-alkenyl, C₂₋₈-alkynyl, C₃₋₈-cycloalkyl-C₀₋₄-alkyl, aryl-C₀₋₄-alkyl, heterocycle-C₀₋₄-alkyl, heteroaryl-C₀₋₄-alkyl, C₃₋₈-cycloalkyloxy, aryloxy, N(R₂₃)₂, NR₂₃COR₂₃, CONR₂₃R₂₃, NR₂₃CONHR₂₃, OCONR₂₃R₂₃, NR₂₃COOR₂₃, OCOR₂₃, COOR₂₃, aryl-C(O)O, aryl-C(O)NR₂₃, heteroaryloxy, heteroaryl-C(O)O, heteroaryl-C(O)NR₂₃, each of which may be independently substituted one or more times with a halogen atom, aryl, heteroaryl, trihalomethyl, C₁₋₄-alkyl, or C₁₋₄-alkoxy;
   or R⁴ and R⁵ may together form a 4, 5, 6 or 7-membered ring that is aromatic or non-aroniatic and may contain one or more heteroatoms, wherein the ring may be further substituted one or more times;
   R₂₃ is independently selected at each occurrence from hydrogen or the group consisting of alkyl, alkenyl, alkynyl, cycloalkyl, cycloalkylalkyl, aryl, heteroaryl, heteroaralkyl and aralkyl, each of winch is substituted with 0-2 substituents independently selected from halogen, alkyl, and alkoxy;
   R⁴ and R^{4a} are each, independently, selected from hydrogen or from the group consisting of C₁₋₄-alkyl, O-C₁₋₄-alkyl, N(H)-C₁₋₄-alkyl, C₃₋₆-cycloalkylC₀-₄alkyl, aryl and heterocycle, each of which may be independently substituted one or more times with a halogen atom or C₁₋₄-alkyl;
   R⁵ is selected from the group consisting of H, hydroxyl, oxo, C₁₋₄-alkyl, C₁₋₄-alkoxy, mono- and di-C₁₋₄alkylamino, C₃₋₆-cycloalkyl-C₀₋₄-alkyl, aryl-C₀₋₄-alkyl, heterocycle-C₀₋₄-alkyl, each of which may be independently substituted one or more times with a halogen atom, aryl, trihalomethyl, or C₁₋₄-alkyl;
   or R⁴ and R⁵ may together form a cycloalkyl or phenyl ring, either of which may be substituted with a halogen atom, aryl, trihalomethyl, or C₁₋₄-alkyl, or a dimethyl cyclopropyl ring such that formula III is a fused ring system;
or R¹⁵ and R¹⁶ may together form a ring of the formula IV: 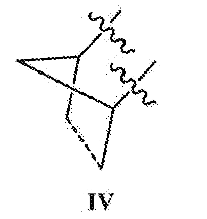 wherein
   the dashed line represents a single or double bond.
   In another embodiment of Formula I, R¹ is selected from the group consisting of H and C₁₋₄-alkyl;
   R² is selected the group consisting of C₁₋₄-alkyl and C₃₋₆-cycloalkylC₀₋₄alkyl;
   W is selected from the group consisting of C(O)-C(O)-amine and C(O)-[C(O)]ₐ-heterocycle, wherein the heterocycle may be independently substituted one or more times with aryl, C₁₋₄-alkyl, C₁₋₄-alkyl substituted by one of more halogen atoms, or C₁₋₆-cycloalkyl, wherein a is 0 or 1;
   R³ is selected from the group consisting of H and C₁₋₄-alkyl;
   R¹³ is H;
   R⁸, R¹⁰ and, R¹¹ are each, independently, selected from the group consisting of H and C₁₋₄-alkyl;
   R⁹ and R¹² are each, independently, selected from the group consisting of H, C₁₋₄-alkyl and C₃₋₆-cycloalkylC₀₋₄-alkyl; and
   V is selected from the group consisting of-Q¹-Q², wherein Q¹ is absent, C(O), N(H), N(C₁₋₄-alkyl), C=N(CN), C=N(SO₂CH₃), or C=N-COH, and Q² is hydrogen or is selected from the group consisting of C₁₋₄-alkyl, O-C₁₋₄-alkyl, NH₂, N(H)-C₁₋₄-alkyl, N(C₁₋₄-alkyl)₂, C₃₋₆-cycloalky-C₀₋₄-alkyl, aryl, and heterocycle, each of which may be independently substituted one or more times with a halogen atom, C₁₋₄-alkyl, C₁₋₄-alkyl substituted by one or more halogen atoms, or C₃₋₆-cycloalkyl.

In yet another embodiment of Formula I, any of the C₃₋₆-cycloalkyl groups may be independently substituted one or more times with a halogen atom, aryl, trihalomethyl, or C₁₋₄-alkyl.

In still another embodiment of Formula I, R¹⁷ is H and R¹⁵ and R¹⁶ together form the ring of formula IV, wherein the dashed line represents a double bond.

In another embodiment of Formula I, R¹⁷ and R¹⁶ together form a 5- or 6-membered ring of the formula III, wherein formula III is represented by the substituents selected from the group consisting of: 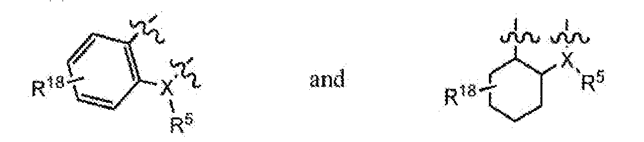 wherein R⁵ is (CH₂)₀₋₃-aryl or (CH₂)₀₋₃-heterocycle, wherein aryl and heterocycle may be independently substituted one or more times with a halogen atom, aryl, trihalomethyl, C₃₋₆-cycloalkyl of C₁₋₄-alkyl; and each R¹⁸ is independently selected from the group consisting of hydrogen, a halogen atom, aryl, trihalomethyl, or C₁₋₄-alkyl,

In another embodiment, Formula I is represented by a compound of the Formula II: 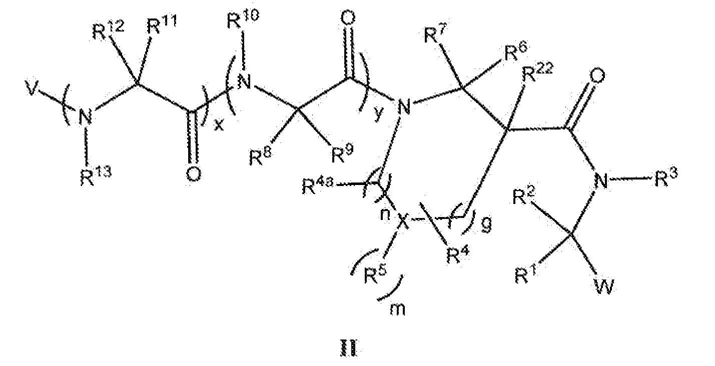 and pharmaceutically acceptable salts, enantiomers, stereoisomers, rotamers, tautomers, diastereomers, or racemates thereof;
wherein
x is 0 or 1;
y is 0, 1 or 2,
R¹ and R² are each, independently, selected from the group consisting of H, C₁₋₄-alkyl, O-C₁₋₄-alkyl, N(H)-C₁₋₄-alkyl, and (CH₂)₀₋₄C₃₋₆-cycloalkyl;
W is selected from the group consisting of C(O)OH, C(O)OR²⁴, C(O)-amine, C(O)-C(O)OH, C(=N-O-R²⁴)-C(O)-amine, C(O)N(H)S(O)₂R²⁴, C(O)-C(O)-amine, SO₂-N(R²⁴)₂ and C(O)-[C(O)]ₐ-heteroeycle, wherein the heterocycle may be substituted or unsubstituted, wherein a is 0 or 1, wherein each R²⁴ is independently selected from hydrogen or halogen or is selected from the group consisting of hydroxyl, formyl, carboxylate, amide, amino, substituted or unsubstituted-C₁₋₄-alkyl, substituted or unsubstituted-C₁₋₄-alkoxy, substituted or unsubstituted-C₁₋₄-alkanoyl, substituted or unsubstituted-C₁₋₄-alkycarbonyl, substituted or unsubsttiuted-C₁₋₄-alkanoyloxy, substituted or unsubstituted mono- and di-C₁₋₄-alkylamino, substituted or unsubstituted-C₃₋₆cyctoalkyl-C₀₋₄alkyl, substituted or unsubstituted aryl-C₀₋₄alkyl, and substituted or unsubstituted heterocycle-C₀₋₄alkyl;
R³ is selected from the group consisting of H, C₁₋₄-alkyl and (CH₂)₀₋₄C₃₋₆-cycloalkyl;
R²² and R⁷ are each, independently, selected from hydrogen or from the group consisting of C₁₋₄-alkyl, O-C₁₋₄-alkyl, N(H)-C₁₋₄-alkyl, (CH₂)₀₋₄C₃₋₆-cycloalkyl, aryl and heterocycle, each of which may be independently substituted one or more times;
n and g are each, independently, 0, 1 or 2;
m is 0 or 1;
X is O, N or C;
R⁴ and R^{4a} are each, independently, selected from hydrogen or from the group consisting of C₁₋₄-alkyl, O-C₁₋₄-alkyl, N(H)-C₁₋₄-alkyl, (CH₂)₀₋₄-C₃₋₆-cycloalkyl, aryl, O-aryl and heterocycle, each of which may be further independently substituted;
R⁵ is selected from hydrogen or oxo or is selected from the group consisting of hydroxyl, C₁₋₄-alkyl, C₁₋₄-alkoxy, mono- and di-C₁₋₄alkylamino, C₃₋₆-cycloalkyl-C₀₋₄-alkyl, aryl-C₀₋₄-alkyl, heterocycle-C₀₋₄-alkyl, each of which may be further independently substituted;
R⁶, R⁸, R⁹, R¹¹ and R¹² are each, independently, selected from the group consisting of H, C₁₋₄-alkyl, O-C₁₋₄-alkyl, N(H)-C₁₋₄-alkyl, and (CH₂)₀₋₄-C₃₋₆-cycloalkyl;
R¹⁰ is selected from the group consisting of H, C₁₋₄-alkyl and (CH₂)₀₋₄-C₃₋₆-cycloalkyl;
R¹³ is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-CO-C₁₋₄alkyl, and Q² is hydrogen or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl; and
V is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-COC₁₋₄alkyl, and Q² is hydrogen or is selected from the group consisting Of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
or R⁴ and R⁵ may together form a 4, 5, 6 or 7-membered ring that is aromatic or non-aromatic and may contain one or more heteroatoms, wherein the ring may be further substituted one or more times.

In another embodiment of Formula I, R⁴ and R⁵ together form a phenyl ring, which may be substituted with a halogen atom, aryl, trihalomethyl, or C₁₋₄-alkyl, or a dimethyl cyclopropyl ring such that a fused ring system is formed.

In yet another embodiment of Formula I, one of g and n is 0.

In still another embodiment of Formula I, R¹ is selected from the group consisting of H and C₁₋₄-alkyl;
R² is selected from the group consisting of C₁₋₄-alkyl and (CH₂)₀₋₄-C₃₋₆-cycloalkyl;
W is selected from the group consisting of C(O)-C(O)-amine and C(O)-[C(O)]ₐ-heterocycle, wherein the heterocycle may be independently substituted one or more times with acryl, C₁₋₄-alkyl, C₁₋₄₀-alkyl substituted by one or more halogen atoms, and C₃₋₆-cycloalkyl, wherein a is 0 or 1, wherein R²⁴ is selected from hydrogen or is selected from the group consisting or C₁₋₄-alkyl, (CH₂)₀₋₄-C₃₋₆-cycloalkyl, aryl and heterocycle, each of which may be independently substituted one or more times with a halogen atom or C₁₋₄-alkyl;
R³ is selected from the group consisting of H and C₁₋₄-alkyl;
R⁷ is hydrogen or is selected from the group consisting of C₁₋₄-alkyl, C₃₋₆-cycloalkyl, aryl and heterocycle, each of which may be independently substituted one or more times with a halogen atom, C₁₋₄-alkyl, C₁₋₄-alkyl substituted by one or more halogen atoms, or C₃₋₆-cyclOalkyl;
R⁴ and R^{4a} are each, independently, selected from hydrogen or from the group consisting of C₁₋₄-alkyl, C₃₋₆-cycloalkyl, aryl and heterocycle, each of which may be independently substituted one or more times with a halogen atom or C₁₋₄-alkyl;
R⁵ is hydrogen or oxo or is selected from the group consisting of hydroxyl, C₁₋₄-alkyl, C₁₋₄-alkoxy, mono- and di-C₁₋₄alkylamino, C₃₋₆-cycloalkyl-C₀₋₄-alkyl, aryl-C₀₋₄-alkyl, heterocyle-C0-4-alkyl, each of which may be independently substituted one or more times with a halogen atom, aryl, trihalomethyl, or C₁₋₄-alkyl;
R¹³ and R⁶ are H;
R⁸, R¹⁰ and R¹¹ are each, independently, selected from the group consisting of H and C₁₋₄-alkyl;
R⁹ and R¹² are each, independently, selected from the group consisting of H, C₁₋₄-alkyl and C₃₋₆-cycloalkyl; and
V is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-COC₁₋₄alkyl, and Q² is hydrogen or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
or R⁴ and R⁵ may together form a phenyl ring, which may be substituted with a halogen atom, aryl, trihalomethyl, or C₁₋₄-alkyl, or a dimethyl cyclopropyl ring such that a fused ring system is formed.

In one embodiment of Formula II, R⁴ is H and R⁵ is (CH₂)₀₋₃-aryl, -O-heterocycle, or (CH₂)₀₋₃-heterocycle, wherein aryl and heterocycle may be independently substituted one or more times with a halogen atom, aryl, trihalomethyl, C₃₋₆-cycloalkyl or C₁₋₄-alkyl.

In yet another embodiment of Formula II, n is I, and R⁴ and R⁵ together form the following fused ring systems: 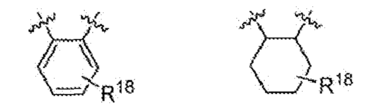 wherein each R¹⁸ is independently selected from the group consisting of hydrogen, a halogen atom, aryl, trihalomethyl, and C₁₋₄-alkyl.

In still another embodiment, Formula I is represented by a compound of the Formula VI: 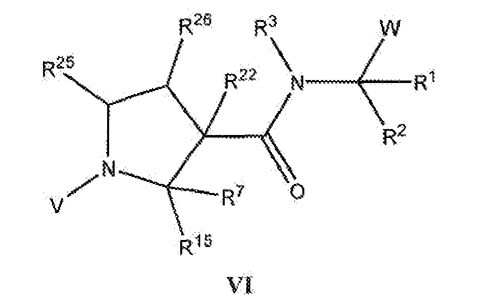 and pharmaceutically acceptable salts, enantiomers, stereoisomers, rotamers, tautomers, diastereomers, or racemates thereof;
wherein
R¹, R², R³, R⁷, R¹⁵, R²², V and W have the meanings set forth for claim 1; and
R²⁵ and R²⁶ are each, independently, selected from hydrogen or from the group consisting of C₁₋₄-alkyl, O-C₁₋₄-alkyl, N(R²⁴)₂, (CH₂)₀₋₄-C₃₋₆-cycloalkyl, substituted or unsubstituted aryl and substituted or unsubstituted heterocycle, wherein each R²⁴ is independently selected from hydrogen or halogen or from the group consisting or hydroxy, COOH, CONH₂, amino, mono- and di-C₁₋₄alkylamino, C₁₋₄-alkyl, C₁₋₄alkoxy, C₁₋₄alkanoyl, C₃₋₆-cycloalkylC₀₋₄alkyl, C₃₋₆-cycloalkylC₀₋₄alkoxy, aryl and heterocycle, each of which may be independently substituted one or more times with a halogen atom. C₁₋₄-alkyl, C₁₋₄-alkyl substituted by one or more halogen atoms, or C₃₋₆-cycloalkyl;
or R²² or R²⁶ may together form a 3-membered ring that may or may not be substituted.

In another embodiment of Formula VI, R²⁵ is H and R²⁶ is amine, substituted or unsubstituted phenyl, or substituted or un substituted benzyl.

In another embodiment, Formula I is represented by a compound of the Formula VII: 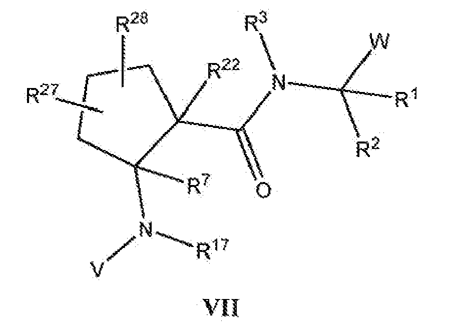 and pharmaceutically acceptable salts, enantiomers, stereoisomers, rotamers, tautomers, diastereomers, or racemates thereof;
wherein
R¹, R², R³, R⁷, R¹⁷, R²², V and W have the meanings set forth for claim 1; and
R²⁷ and R²⁸ are each, independently, hydrogen or are selected from the group consisting of C₁₋₄alkyl, C₁₋₄-alkoxy, N(R²⁴)₂, C₃₋₆-cycloalkylC₀₋₄alkyl, aryl, aryloxy, and heterocycle, each of which is substituted 0 to 5 times with halogen atom, C₁₋₄-alkyl, C₁₋₄-alkyl substituted by one or more halogen atoms, or C₃₋₆-cycloalkyl; wherein R²⁴ is independently selected from hydrogen or from the group consisting of hydroxy, C(O)NH₂, substituted or unsubstituted-C₁₋₄-alkyl, C₃₋₆-cycloalkylC₀₋₄alkyl, aryl and heterocycle, each of which is substituted 0 to 5 times with halogen atom, C₁₋₄-alkyl, C₁₋₄-alkyl substituted by one or more halogen atoms, or C₃₋₆-cycloalkyl.

In another embodiment, Formula VII is represented by a compound of the formula: 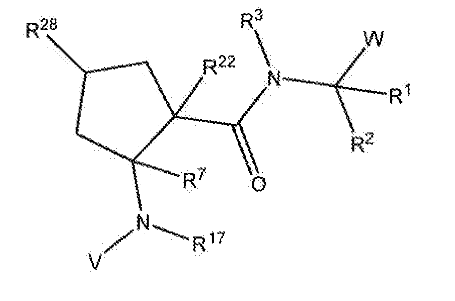 and pharmaceutically acceptable salts, enantiomers, stereoisomers, rotamers, tautomers, diastereomers, or racemates thereof;
wherein
R¹, R², R³, R⁷_{,} R¹⁷, R²², V and W have the meanings set forth for claim 1; and
R²⁸ is hydrogen or is selected from the group consisting or C₁₋₄alkyl, C₁₋₄-alkoxy, N(R²⁴)₂, C₃₋₆-cycloalkylC₀₋₄alkyl, aryl, aryloxy, and heterocycle, each of which is substituted 0 to 5 times with halogen atom, C₁₋₄-alkyl, C₁₋₄-alkyl substituted by one or more halogen atoms, or C₃₋₆-cycloalkyl; wherein R²⁴ is independently selected at each occurrence from hydrogen or from the group consisting of hydroxy, C(O)NH₂, substituted or unsubsdtuted-C₁₋₄-alkyl, C₃₋₆-cycloalkylC₀₋₄alkyl, aryl and heterocycle, each of which is substituted 0 to 5 times with halogen atom, C₁₋₄-alkyl, C₁₋₄-alkyl substituted by one or more halogen atoms, or C₃₋₆-cycloalkyl. In one embodiment of Formula VIII, R²⁸ is quinoline, C₁₋₄-alkyl, O-C₁₋₄-alkyl, or O-quinoline, wherein the quinoline and O-quinoline substituents may be independently substituted one or more times with halogen, amino, O-C₁₋₄-alkyl, substituted or unsubstituted-C₁₋₄-alkyl, substituted or unsubstituted-(CH₂)₀₋₄-C₃₋₆-cycloalkyl, substituted or unsubstituted aryl, substituted or unsubstituted O-aryl, and substituted or unsubstituted heterocycle.

In another embodiment, Formula I is represented by a compound of the Formula VIII: 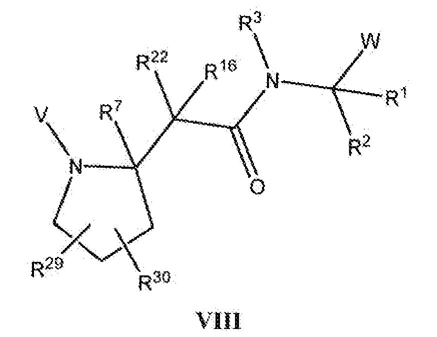 and pharmaceutically acceptable salts, enantiomers, stereoisomers, rotamers, tautomers, diastereomers, or racemates thereof;
wherein
R¹, R², R³, R⁷, R¹⁶, R²², V and W have the meanings set forth for claim 1; and
R²⁹ and R³⁰ are hydrogen or are selected from the group consisting of C₁₋₄alkyl, C₁₋₄-alkoxy, N(R²⁴)₂, C₃₋₆-cycloalkylC₀₋₄alkyl, aryl, aryloxy, and heterocycle, each of which is substituted 0 to 5 times with halogen atom, C₁₄-alkyl, C₁₋₄-alkyl substituted by one or more halogen atoms, or C₃₋₆-cycloalkyl; wherein R²⁴ is independently selected from hydrogen or from the group consisting of hydroxy, C(O)NH₂, substituted or unsubstituted-C₁₋₄-alkyl, C₃₋₆-cycloalkylC₀₋₄alkyl, aryl and heterocycle, each of which is substituted 0 to 5 times with halogen atom, C₁₋₄-alkyl, C₁₋₄-alkyl substituted by one or more halogen atoms, or C₃₋₆-cycloalky. In one embodiment, Formula VII, is represented by a compound of the Formula IX: 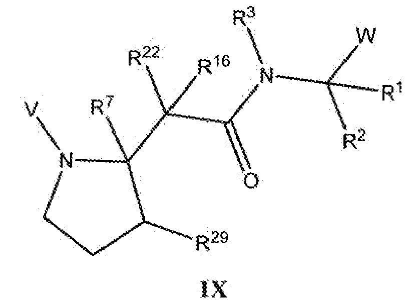 and pharmaceutically acceptable salts, enantiomers, stereoisomers, rotamers, tautomers, diastereomers, or racemates thereof;
   wherein R¹, R², R³, R⁷, R¹⁶, R²², R²⁹, V and W have the meanings set forth for claim 21.

In one embodiment of Formula IX, R²⁹ is selected from the group consisting of O-phenyl and O-benzyl.

In another embodiment, Formula I is represented by a compound of the Formula X: 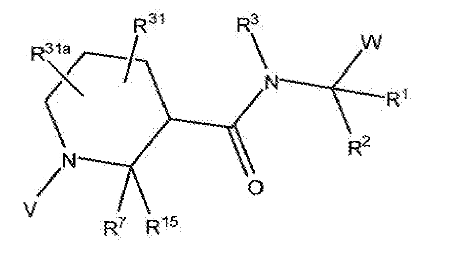 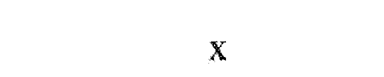 and pharmaceutically acceptable salts, enantiomers, stereoisomers, rotamers, tautomers, diastereomers, or racemates thereof;
wherein
R¹, R², R³, R⁷, R¹⁵, V and W have the meanings set forth for claim 1; and
R³¹ and R^{31a} are hydrogen or are independently selected from the group consisting of C₁₋₄alkyl, C₁₋₄-alkoxy, N(R²⁴)₂, C₃₋₆-cycloalkylC₀₋₄alkyl, aryl, aryloxy, and heterocycle, each of which is substituted 0 to 5 times with halogen atom, C₁₋₄-alkyl, C₁₋₄-alkyl substituted by one or more halogen atoms, or C₃₋₆-cycloalkyl; wherein R²⁴ is independently selected from hydrogen or the group consisting of hydroxy, C(O)NH₂, substituted or unsubstituted-C₁₋₄-alkyl, C₃₋₆-cycloalkylC₀₋₄alkyl, aryl and heterocycle, each of which is substituted 0 to 5 times with halogen atom, C₁₋₄-alkyl, C₁₋₄-alkyl substituted by one or more halogen atoms, or C₃₋₆-cycloalkyl;
or R³³¹ and R^{31a} may together form a 3, 4, 5, 6 or 7-membered ring that is aromatic or non-aromatic and may contain one or more heteroatoms, wherein the ring may be further substituted one or more times.

In another embodiment, Formula X is represented by a compound of the Formula XI: 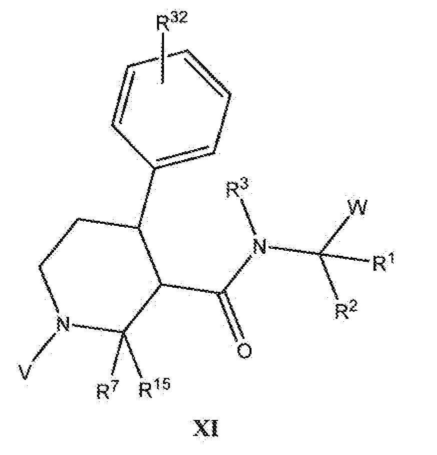 and pharmaceutically acceptable salts, enantiomers, stereoisomers, rotamers, tautomers, diastereomers, or racemates thereof;
wherein
R¹, R², R³, R⁷, R¹⁵, V and W have the meanings set forth for claim 19; and
R³² is H of halogen or is selected from the group consisting of hydroxy, amino, C₁₋₄-alkyl, C₁₋₄alkoxy, mono- and di-C₁₋₄alkylamino, C₃₋₆-cycloalkylC₀₋₄alkyl, C₃₋₆-cycloalkylC₀₋₄alkoxy, aryl, aralkyl, heterocycleC₀₋₄alkyl, and heterocycleC₀₋₄alkoxy, each of which is substituted with 0 to 5 resides independently selected from halogen, hydroxy, amino, oxo, C₁₋₄-alkyl, C₁₋₄alkoxy, mono- and di-C₁₋₄alkylamino, C₃₋₆-cycloalkyl, aryl, and heterocycle. In another embodiment, Formula X is represented by a compound of the Formula XII: 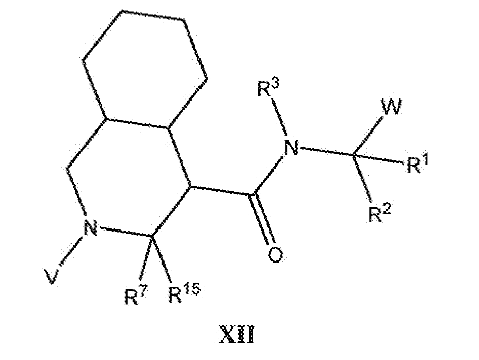 and pharmaceutically acceptable salts, enantiomers, stereoisomers, rotamers, tautomers, diastereomers, or racemates thereof;
   wherein
   R¹, R², R³, R⁷, R¹⁵, V and W have the meanings set forth for claim 24.

In another embodiment, Formula I is represented by a compound of the Formula XIII: 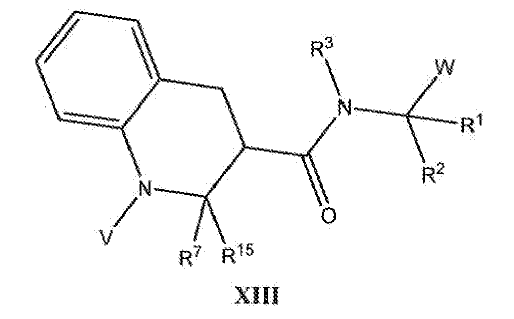 and pharmaceutically acceptable salts, enantiomers, stereoisomers, rotamers, tautomers, diastereomers, or racemates thereof;
wherein
R¹, R², R³, R⁷, R¹⁵, V and W have the meanings set forth for claim 1.

In yet another embodiment, Formula I is represented by a compound of the Formula XIV: 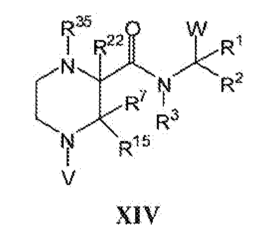 and pharmaceutically acceptable salts, enantiomers, stereoisomers, rotamers, tautomers, diastereomers, or racemates thereof;
wherein
R¹, R², R³, R⁷, R¹⁵, R²², V and W have the meanings set forth for claim 1; and
R³⁵ is hydrogen or halogen or is selected from the group consisting of hydroxy, amino, C₁₋₄-alkyl, C₁₋₄alkoxy, mono- and di-C₁₋₄alkylamino, C₃₋₆-cycloalkylC₀₋₄alkyl, C₃₋₆-cycloalkylC₀₋₄alkoxy, aryl, aralkyl, heterocycleC₀₋₄alkyl, and heterocycleC₀₋₄alkoxy, each of which is substituted with 0 to 5 residues independently selected from halogen, hydroxy, amino, oxo, C₁₋₄-alkyl, C₁₋₄alkoxy, mono- and di-C₁₋₄alkylamino, C₃₋₆-cycloalkyl, aryl, and heterocycle.

In one embodiment of Formula XIV, R²⁵ is phenyl, optionally substituted with chloro.

In another embodiment of the invention, W, R¹ and R² form a substituent of the following formulas: 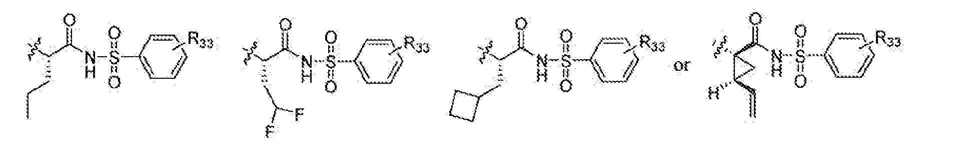 wherein R³³ is selected from the group consisting of H, phenyl, methyl CF₃, tBu, NO₂, Cl, CN, NH₂, OH, NHCH₃, OCH₃, NHPh, OPh, NHCOCH₃, NHCOPh, OCH2Ph, COCH₃, CO₂Et, CO₂CH₃, CONHPh and CONHCH₃, or R¹³ can be fused with the phenyl ring to form a naphthyl ring.

In still another embodiment of the invention, W, R¹ and R² form substituents selected from the group consisting of

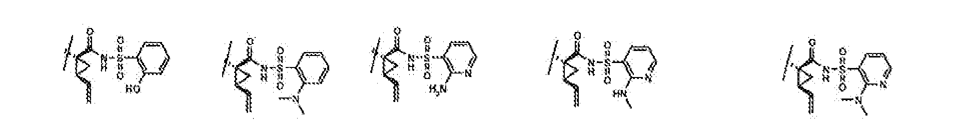

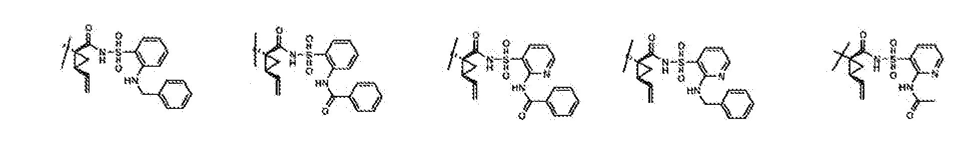

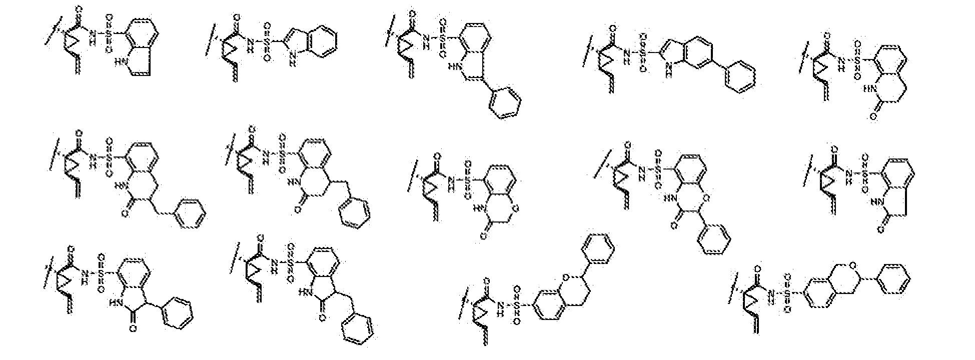

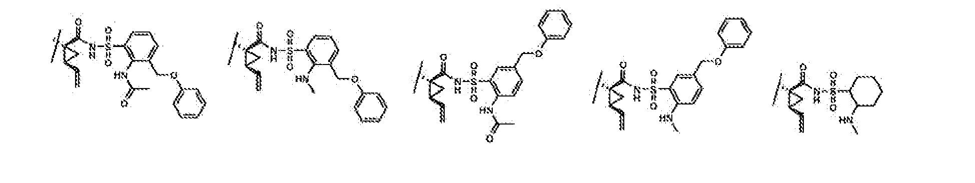

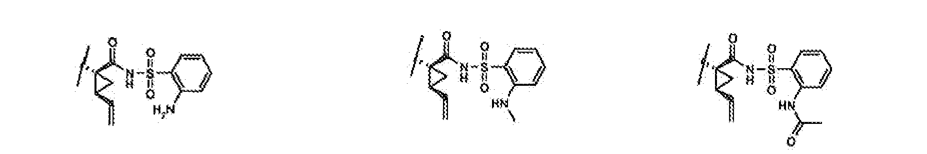

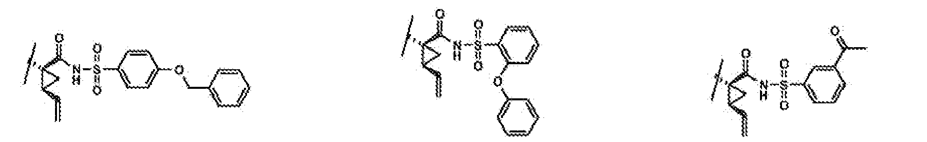

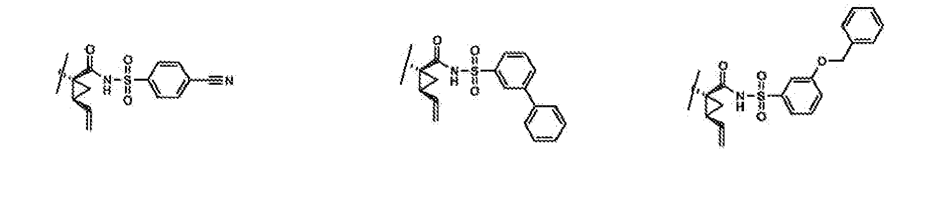

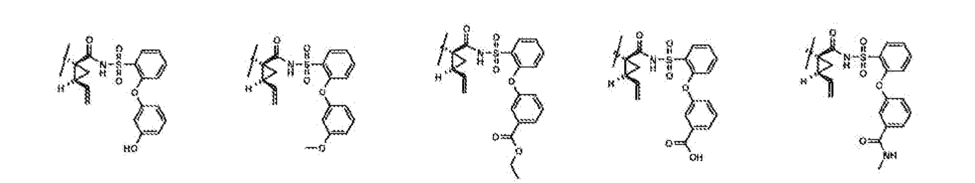

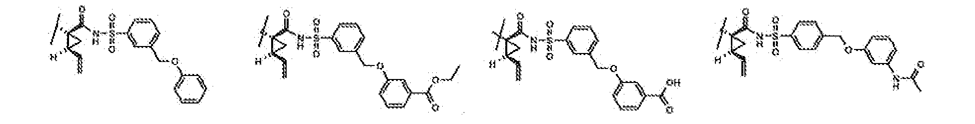

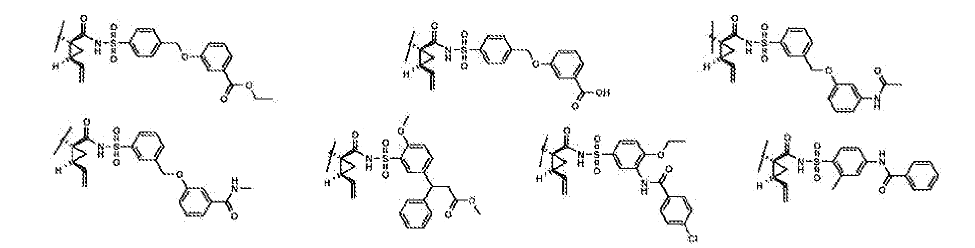

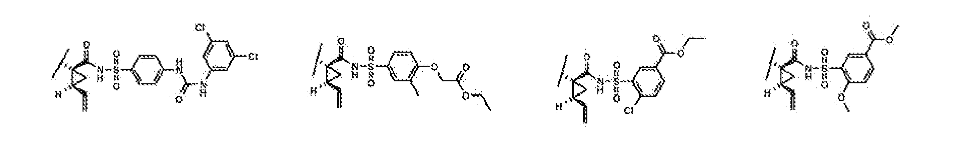

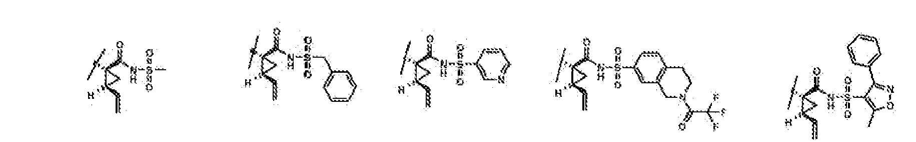

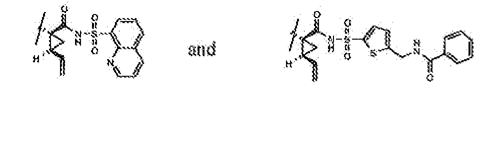

In another embodiment of the invention, any of the heterocycle groups are independently selected from the group consisting of acridinyl, carbazolyl, cinnolinyl, quinoxalinyl, pyrrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, letrahydroquinoline, benzoimidazolyl, benzofuranyl, benzofurazanyl, benzopyrazolyl, benzotriazolyl, benzothiophenyl, benzoxazolyl, carbazolyl, carbolinyl, cinnolinyl, furanyl, imidazolyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, isoxazoline, isoxazoline, oxetanyl, pyranyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrahydropyranyl, tetrazolyl, tetrazolopyridyl, thiadiazolyl, thiazolyl, thienyl, triazolyl, azetidinyl, 1,4-dioxanyl, hexahydroazepinyl, piperazinyl, piperidinyl, pyridin-2-onyl, pyrrolidinyl, morpholinyl, thiomorphotinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenxoyl, tetrahydrofuranyl, and tetrahydrothienyl, and N-oxides thereof, each of which may be independently further substituted one or more times with a halogen atom, C₁₋₄-alkyl, C₁₋₄-alkyl substituted by one or more halogen atoms, or C₃₋₆-cycloalkyl.

In another embodiment of the invention, W is C(O)-C(O)-N(H)-cyclopropyl.

In still another embodiment of the invention, V is selected from the group consisting of C(O)R²⁴, C(O)N(H)R²⁴ and C(O)OR²⁴, wherein each R²⁴ is independently selected from hydrogen or halogen or the group consisting of C₁₋₄-alkyl, amino, mono-and di-C₁₋₄alkylamino, C₁₋₄alkoxy, C₃₋₆-cycloalkylC₀₋₄alkyl, C₃₋₆-cycloalkylC₀₋₄alkoxy, aryl, aralkyl and heterocyeleC₀₋₄alkyl, wherein each R²⁴ residue is further substituted with 0 to 5 groups selected from halogen, hydroxy, oxo, amino, C₁₋₄-alkyl, amino, mono- and di-C₁₋₄alkylamino, C₁₋₄alkoxy, C₃₋₆cycloalkyl, aryl, and heterocycle.

In yet another embodiment of the invention, V is selected from the group consisting of benzyl, substituted benzyl, naphthyl, C₁₋₄-alkyl, and

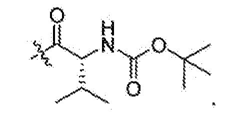

In another embodiment of the invention, any of the C₃₋₆-cycloalkyl groups may be independently substituted one or more times with a halogen atom, aryl, trihalomethyl, or C₁₋₄-alkyl.

In yet another embodiment of the invention, R⁵ is selected from the group consisting of piperidine, phenyl, -O-pyridinyl and CH₂-pyridinyl, wherein the phenyl and pyridinyl groups may be independently substituted one or more times with a halogen atom or C₁₋₄-alkyl. In another embodiment, R⁵ is 5-chloro-pyridin-2-yl or 5-chloro-pyridin-2-yloxy.

In another embodiment of the invention, W is selected from the group consisting of C(O)-C(O)N(R²³)₂, wherein R²³ is independently selected from hydrogen or the group consisting of C₁₋₄-alkyl, (CH₂)₀₋₄C₃₋₆-cycloalkyl, aryl and heterocycle, each of which may be independently substituted one or more times with a halogen atom or C₁₋₄-alkyl.

In still another embodiment of the invention, W is selected from the group consisting of C(O)-C(O)NH₂, C(O)-C(O)N(H)-cyclopropyl, C(O)-benzothiazole, C(O)-benzoimidazole, C(O)-oxazole, C(O)-imidazole, and C(O)-oxadiazole, wherein the benzothiazole, benzoimidazole, oxazole and oxadiazole groups may be independently substituted one or more times with a halogen atom, aryl trihalomethyl, (CH₂₎₀₋₄-C₃₋₆-cycloalkyl or C₁₋₄-alkyl.

In yet another embodiment of the invention, W is selected from the group consisting of 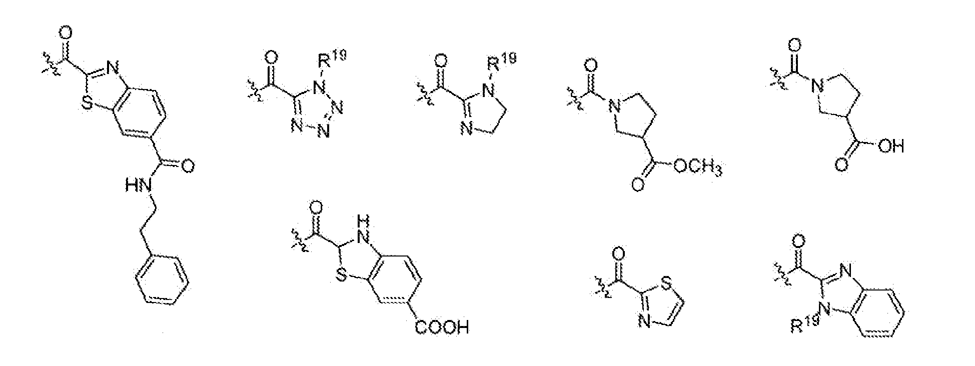 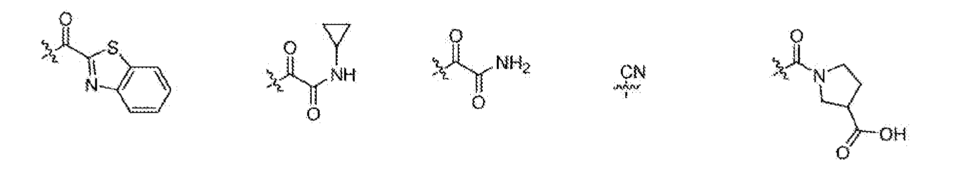 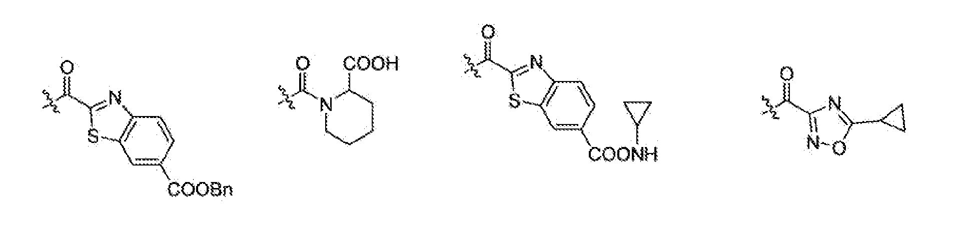 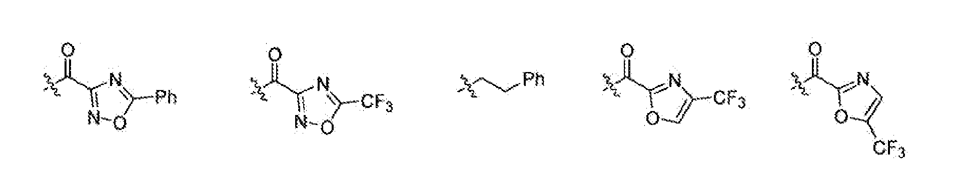 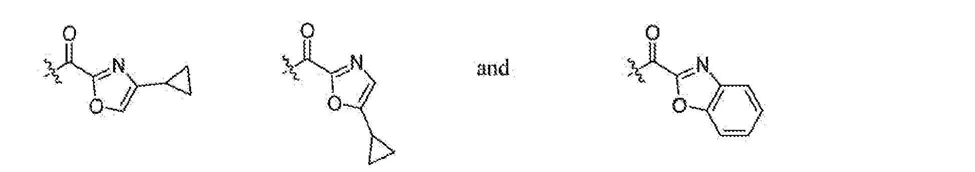 wherein R¹⁹ is selected from the group consisting of hydrogen, a halogen atom, aryl, trihalomethyl, and C₁₋₄-alkyl.

In another embodiment of the invention, R² is selected from the group consisting of propyl, CH₂-cyclobutyl and (CH₂)₂-cyclobutyl. In another embodiment of the invention, R¹¹ is H and R¹² is C₃₋₆-cycloalkyl. In another embodiment, R¹² is cyclohexyl.

In another embodiment of the invention, V is selected from the group consisting of C(O)-N(H)-/-butyl. In still another embodiment of the invention, V is C(O)-R²⁰, wherein R²⁰ is selected from the group consisting of C₃₋₆-cycloalkyl, phenyl, pyrazine, benzooxazole, 4,4-dimethyl-4,5-dihydro-oxazole, benzoimidazole, pyrimidine, benzothiazole 1,1-dioxide and quinazoline, each of which may be further independently substituted with a halogen atom, CF₃, C₁₋₄-alkyl or C₃₋₆-cycloalkyl.

In yet another embodiment of the invention, V is C(O)-R²⁰, wherein R²⁰ is selected from the group consisting of 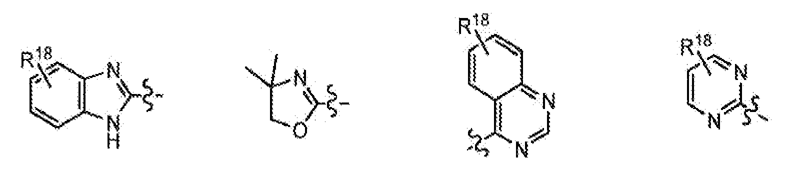 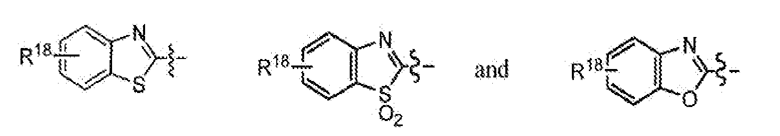 wherein R¹⁸ is selected from the group consisting of hydrogen, a halogen atom, aryl, trihalomethyl, and C₁₋₄-alkyl.

In another embodiment of the invention, V is C(O)-R²⁰, where R²⁰ is selected from the group consisting of 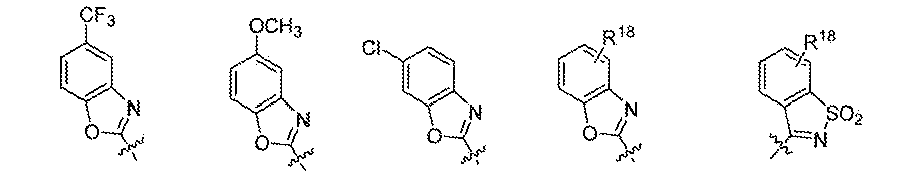 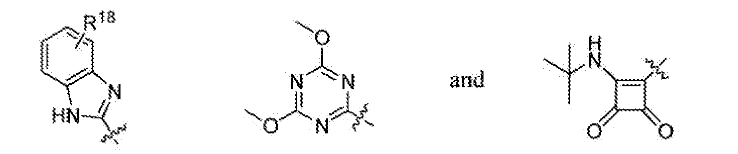 wherein R¹⁸ is selected from the group consisting of hydrogen, a halogen atom, aryl, trihalomethyl, and C₁₋₄alkyl.

In another embodiment of the invention, V is selected from the group consisting of C₃₋₆-cycloalkyl, phenyl, pyrazine, benzooxazole, 4,4-dimethyl-4,5-dihydro-oxazole, benzoimidazole, pyrimidine, benzothiazole 1,1-dioxide and quinazoline, each of which may be further independently substituted with a halogen atom, CF₃, C₁₋₄-alkyl or C₃₋₆-cycloalkyl.

In still another embodiment of the invention, V is selected from the group consisting of 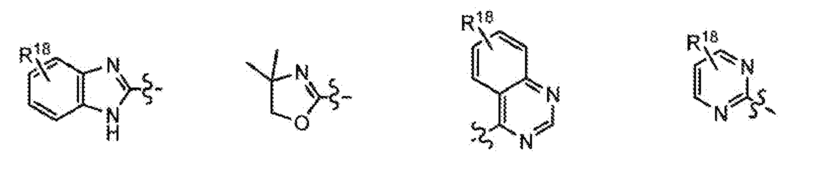 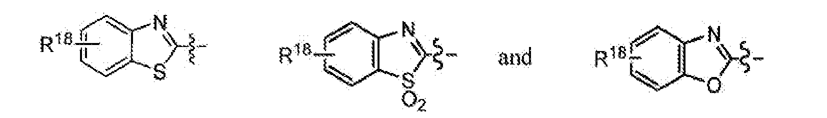 wherein R¹⁸ is selected from the group consisting of hydrogen, a halogen atom, aryl, trihalomethyl, and C₁₋₄-alkyl.

In another embodiment of the invention, V is selected from the group consisting of 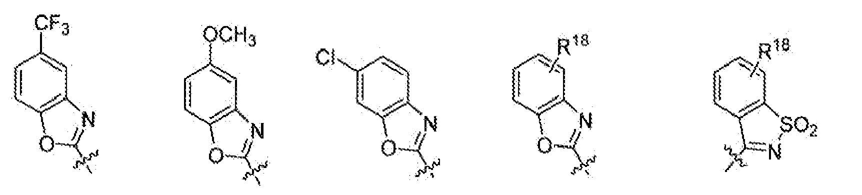 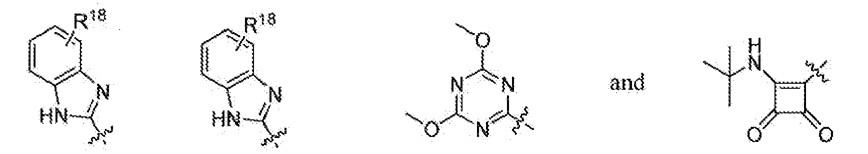 wherein R¹⁸ is selected from the group consisting of hydrogen, a halogen atom, aryl, trihalomethyl, and C₁₋₄-alkyl.

In another embodiment of the invention, R⁵ is selected from the group consisting of 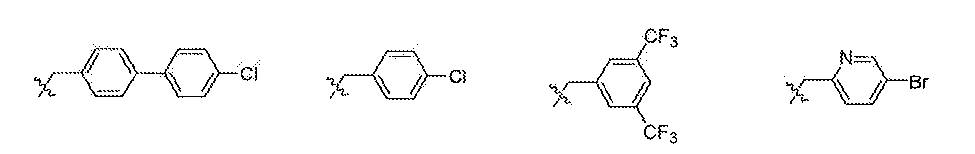 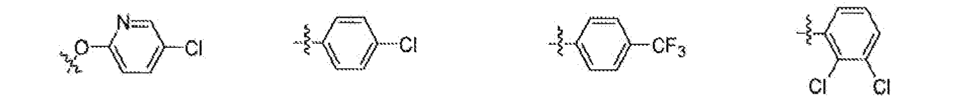 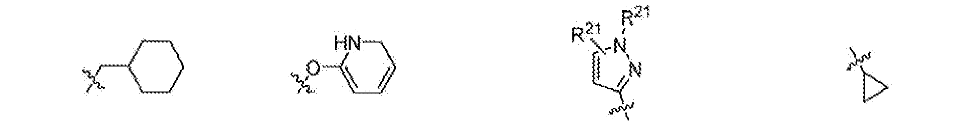 and 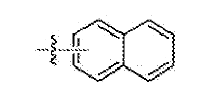 wherein R²¹ is independently selected from the group consisting of C₁₋₄-alkyl and aryl.

In another embodiment of the invention, W is C(O)-C(O)-amino. In another embodiment, R¹⁷ and R¹⁶ together form a ring of the formula III, wherein n and g are each, independently, 0 or 1. In another embodiment of the invention, R¹³ is H and V is selected from the group consisting of C=N(H)NH₂, C=N(CN)NH₂ and C(O)NH₂.

In still another embodiment of the invention, W is C(O)N(H)S(O)₂R²⁴, wherein R²⁴ is selected from hydrogen or from the group consisting of C₁₋₄-alkyl, (CH₂)₀₋₄-C₃₋₆-cycloalkyl, substituted or unsubstituted aryl and substituted or unsubstituted heterocycle, each of which may be independently substituted one or more times with a halogen atom or C₁₋₄-alkyl.

In another embodiment of the invention, W is COOH, R¹ is H, and R² is selected from the group consisting of propyl, 2,2-difluoroethyl and CH₂-cyclobutyl, or R¹ and R² form together a cyclopropyl group that may be further substituted with a vinyl group.

In another embodiment of the invention, R⁵, R⁴ and R^{4a} are each, independently, selected from the group consisting of H, C₁₋₄alkoxy, aryloxy, heterocyclyl-oxy, aralkyloxy, C(O)N(R²⁴)₂, -N(R²⁴)C(O)R²⁴, C₁₋₄alkyl, aryl and aralkyl, wherein R²⁴ is independently selected from hydrogen or halogen or from the group consisting of C₁₋₄-alkyl, amino, mono- and di-C₁₋₄alkylamino, C₁₋₄alkoxy, C₃₋₆-cycloalkylC₀₋₄alkyl, C₃₋₆-eycloalkylC₀₋₄alkoxy, aryl, aralkyl and heterocycleC₀₋₄alkyl, each of which is further substituted with 0 to 5 groups independently selected from halogen, hydroxy, oxo, C₁₋₄-alkyl, amino, mono- and di-C₁₋₄alkylamino, C₁₋₄alkoxy, C₃₋₆cycloalkyl, aryl, and heterocycle.

In another embodiment of the invention, R¹ and R² form a substituent of the following formula:

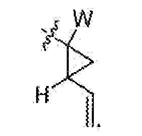

In yet another embodiment of the invention, W, R¹ and R² form a substituent of the following formula:

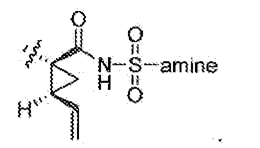

In another embodiment of the invention, W, R¹ and R² form a substituent of the following formula: 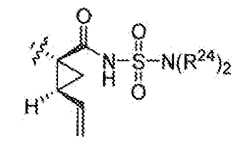 wherein each R²⁴ is independently selected from the group consisting of H, substituted or unsubstituted-C₁₋₄-alkyl, substituted or unsubstituted-(CH₂)₀₋₄-C₃₋₆-cycloalkyl, substituted or unsubstituted aryl and substituted or unsubstituted heterocycle.

In yet another embodiment of the invention, R²⁴ is selected from the group consisting of

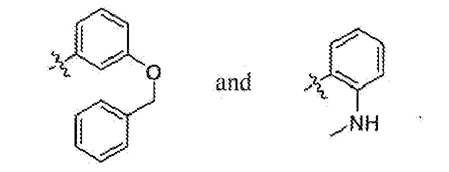

In another embodiment of the invention, W, R¹ and R² form a substituent selected from the group consisting of:

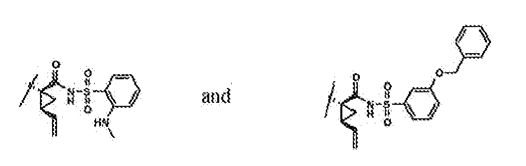

In still another embodiment of the invention, V is selected from the group consisting of acyl, SO₂-R²⁴, C(O)N(R²⁴)₂, C(O)O(R²⁴)₂, and N(H)R²⁴, wherein each R²⁴ is independently selected from hydrogen or from the group consisting of eC₁₋₄-alkyl, C₃₋₆-cycloalkylC₀₋₄alkyl, amino, mono-and diC₁₋₄alkylamin, aryl, aralkyl, aryloxy, and heterocyeleC₀₋₄alkyl, each of which is substituted with 0-5 groups independently selected from halogen, hydroxy, oxo, amino, C₁₋₄-alkyl, mono- and di-C₁₋₄alkylamino, C₁₋₄alkoxy, C₃₋₆cycloalkyl, aryl, and heterocycle.

Preferred embodiments of the compounds of the invention (including pharmaceutically acceptable salts thereof, as well as enantiomers, stereoisomers, rotamers, tautomers, diastereomers, or racemates thereof) are shown below in Table A and Table B, and are also considered to be "compounds of the invention."

**TABLE A**

| **Structure** | **Compound No.** |
|---|---|
| 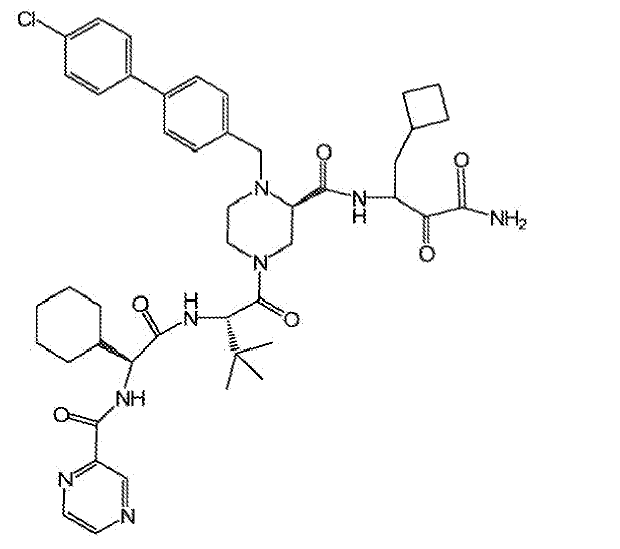 | **A-1** |
| 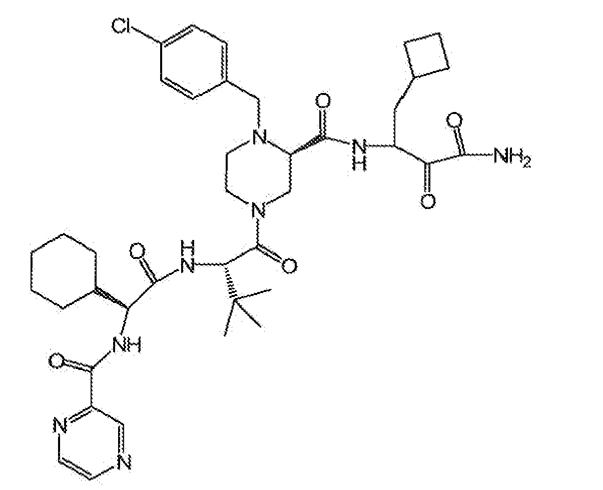 | **A-2** |
| 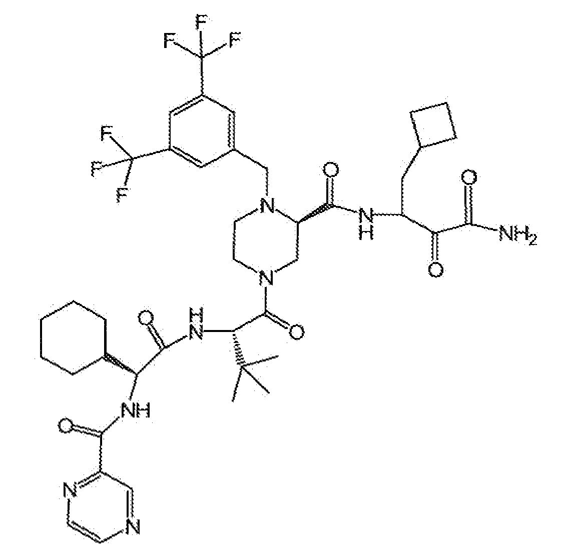 | **A-3** |
| 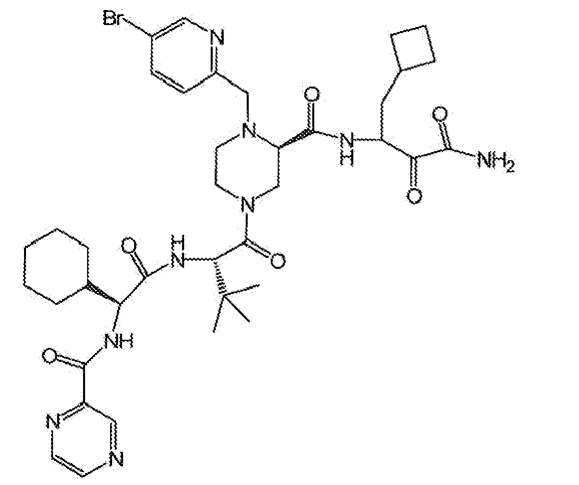 | **A-4** |
| 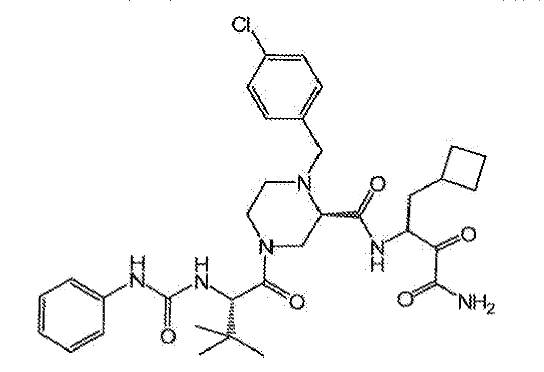 | **A-5** |
| 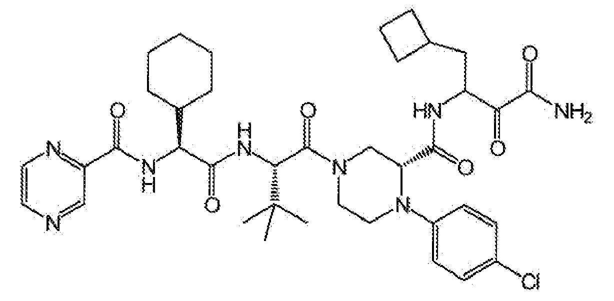 | **A-6** |
| 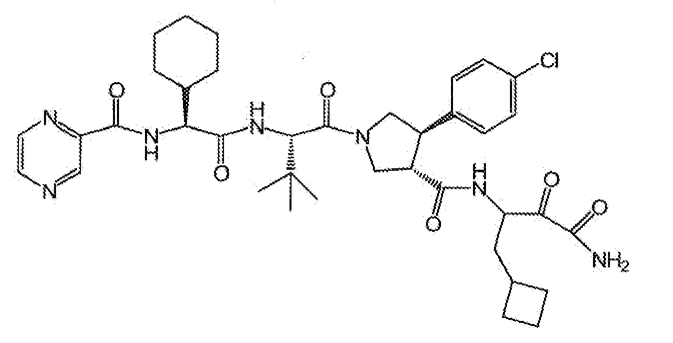 | **A-7** |
| 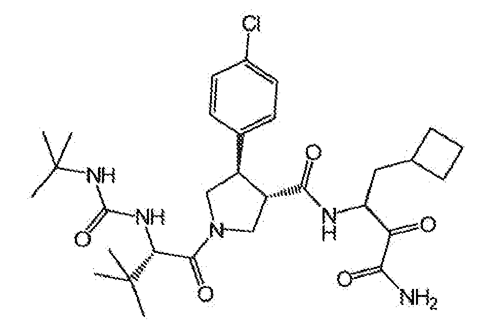 | **A-8** |
| 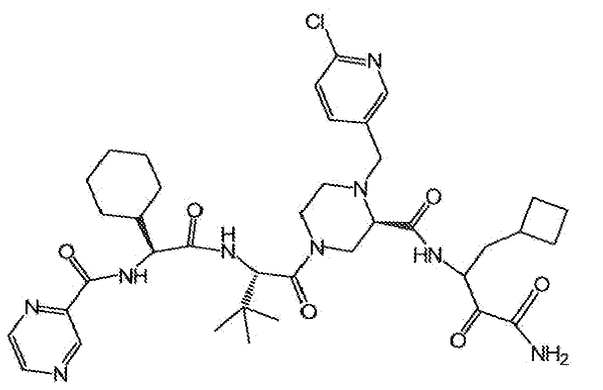 | **A-9** |
| 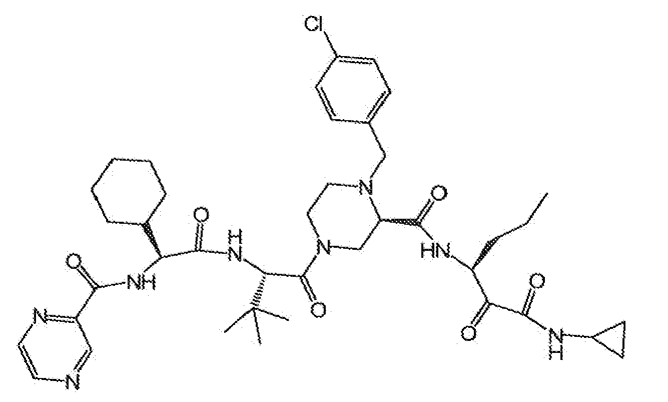 | **A-10** |
| 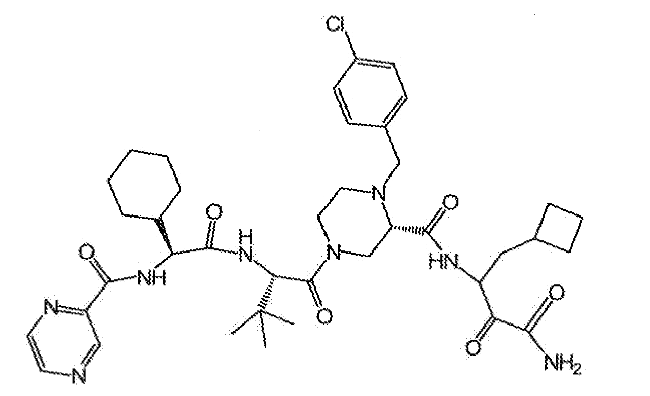 | **A-11** |
| 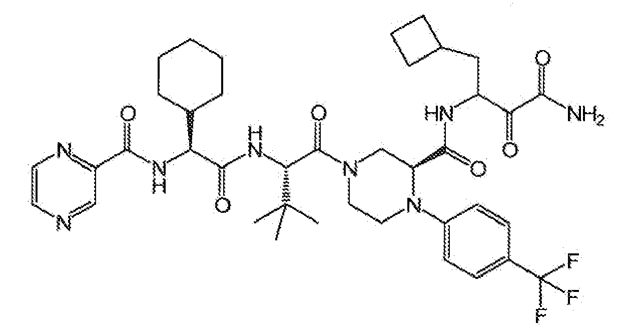 | **A-12** |
| 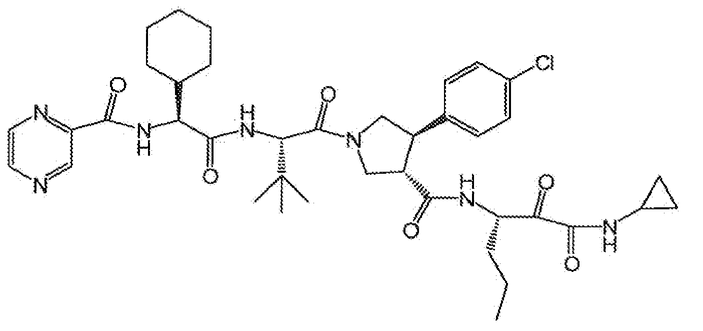 | **A-13** |
| 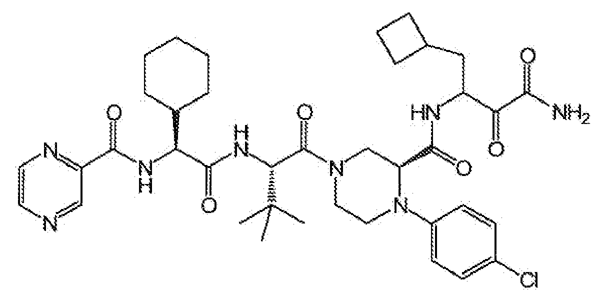 | **A-14** |
| 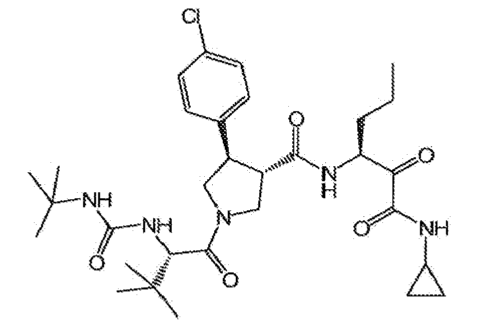 | **A-15** |
| 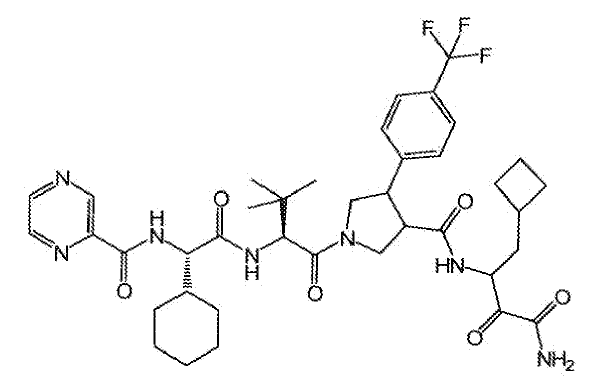 | **A-16** |
| 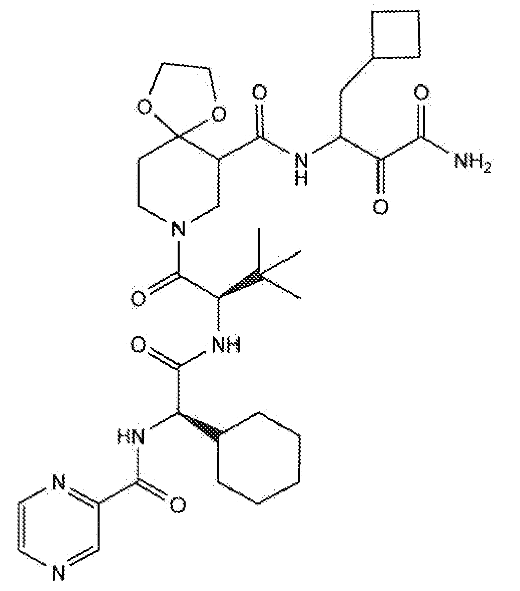 | **A-17** |
| 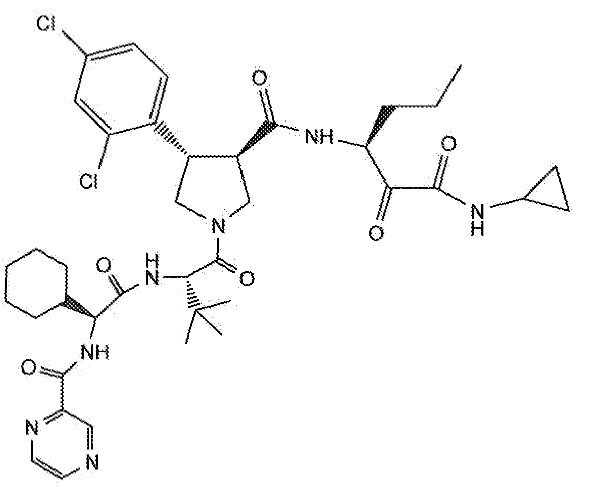 | **A-18** |
| 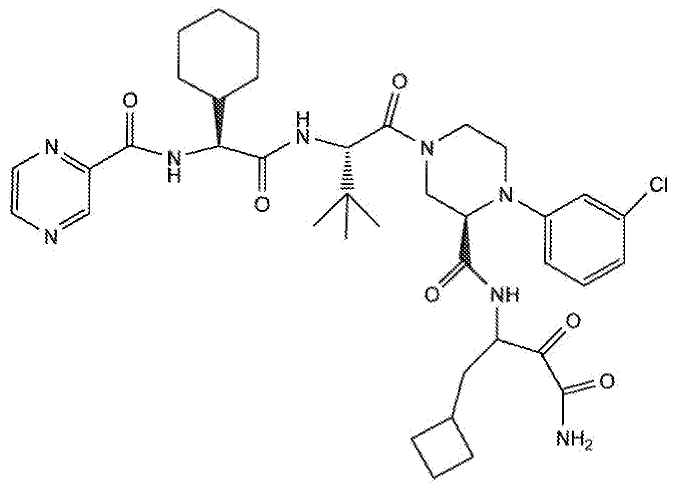 | **A-19** |
| 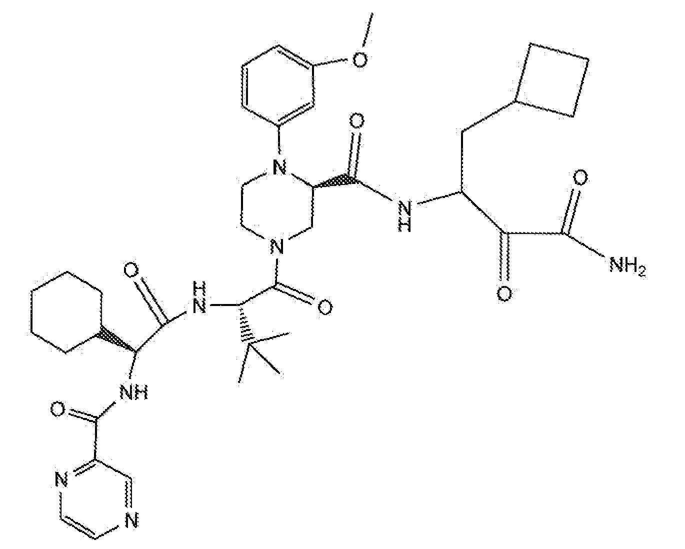 | **A-20** |
| 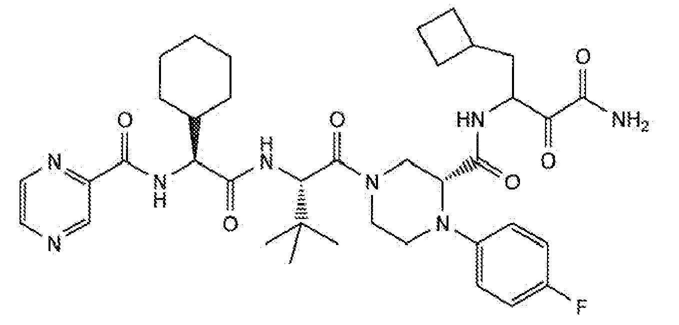 | **A-21** |
| 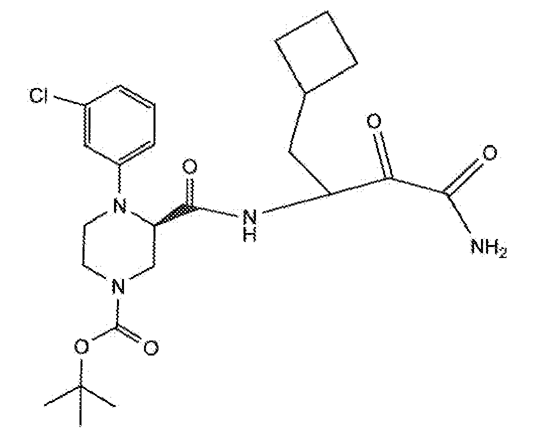 | **A-22** |
| 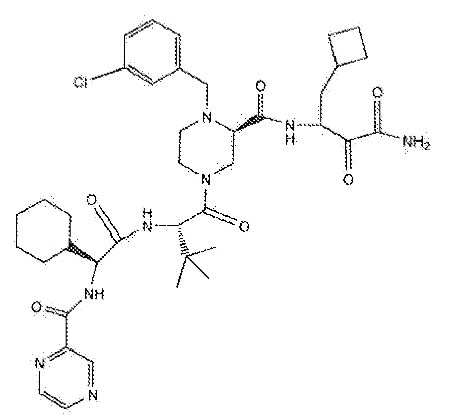 | **A-23** |
| 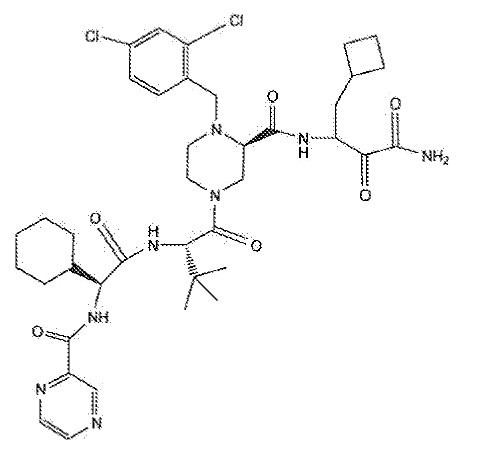 | **A-24** |
| 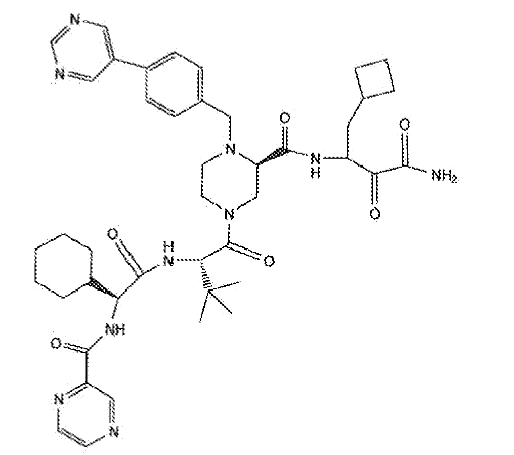 | **A-25** |
| 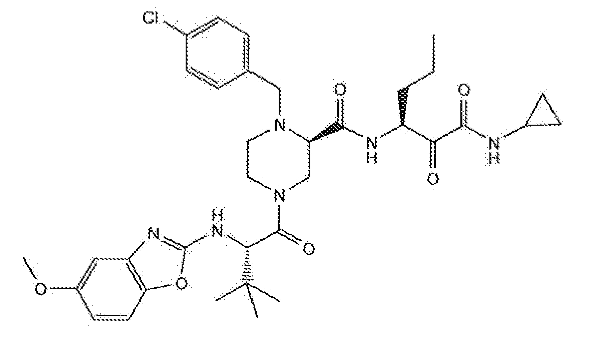 | **A-26** |
| 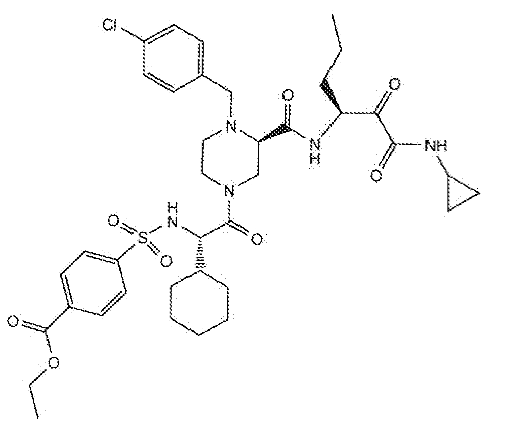 | **A-27** |
| 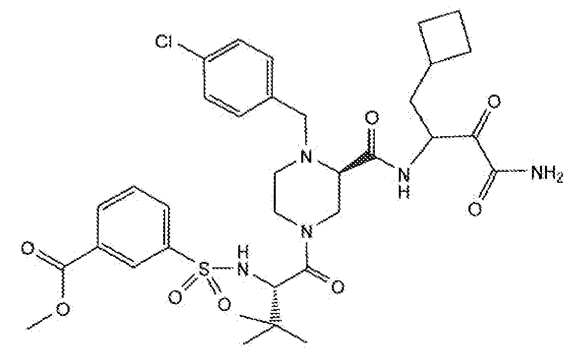 | **A-28** |
| 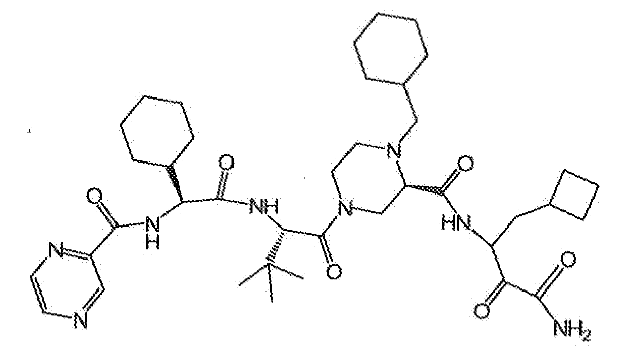 | **A-29** |
| 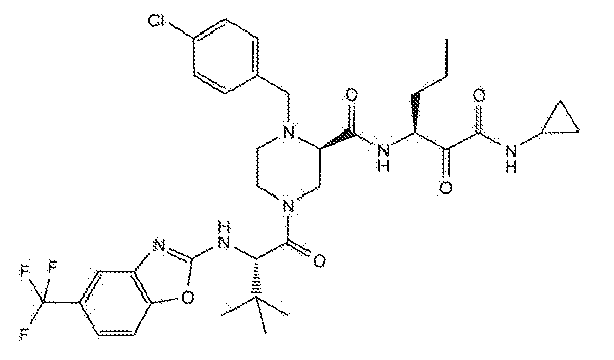 | **A-30** |
| 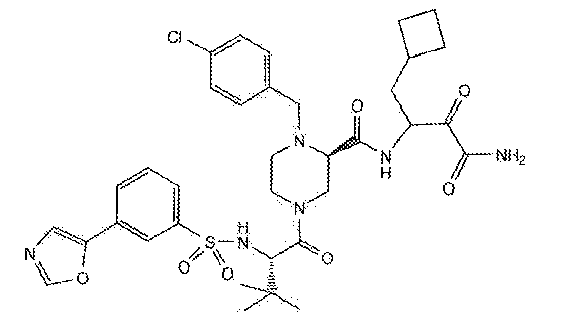 | **A-31** |
| 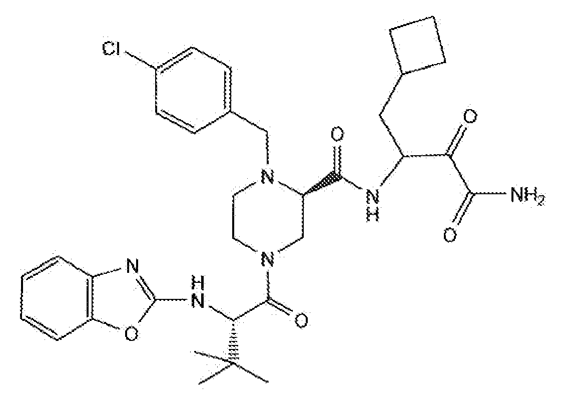 | **A-32** |
| 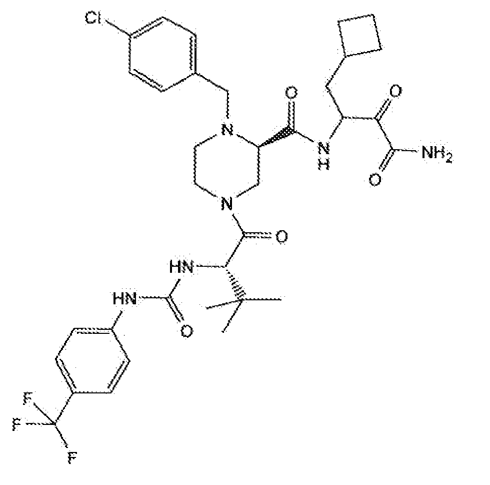 | **A-33** |
| 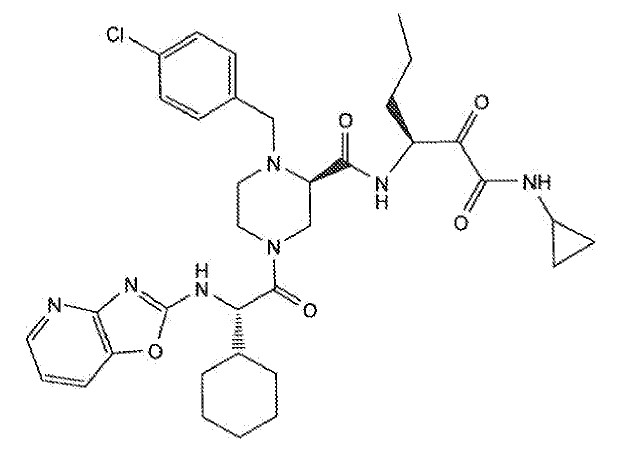 | **A-34** |
| 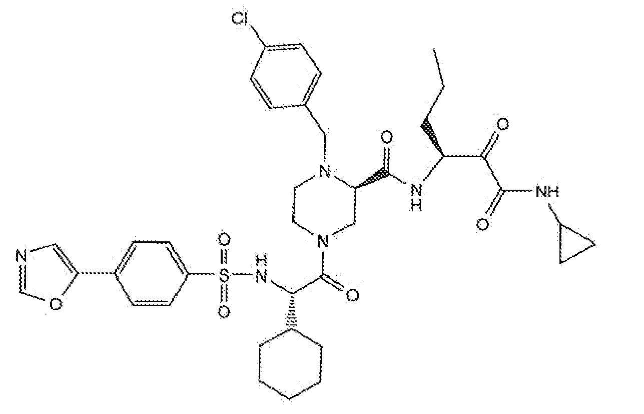 | **A-35** |
| 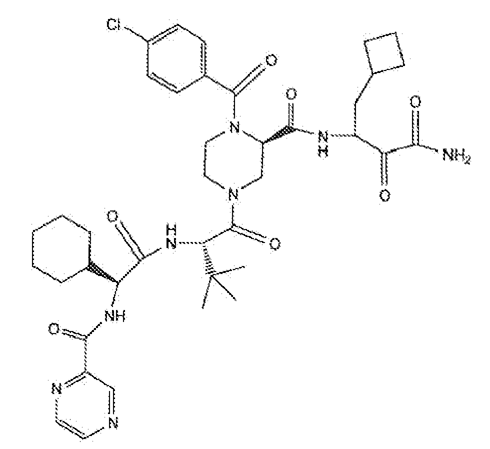 | **A-36** |
| 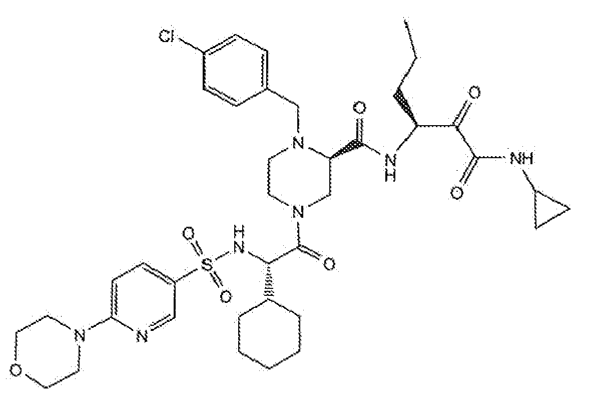 | **A-37** |
| 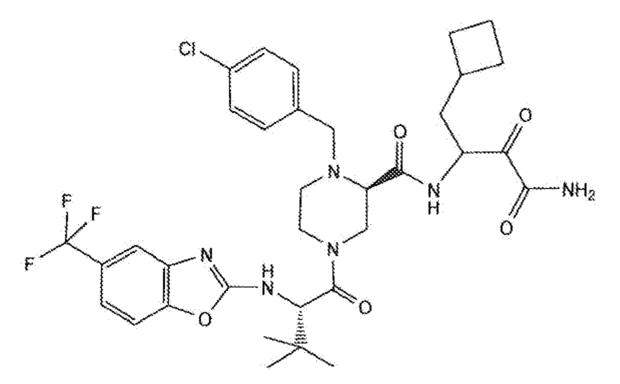 | **A-38** |
| 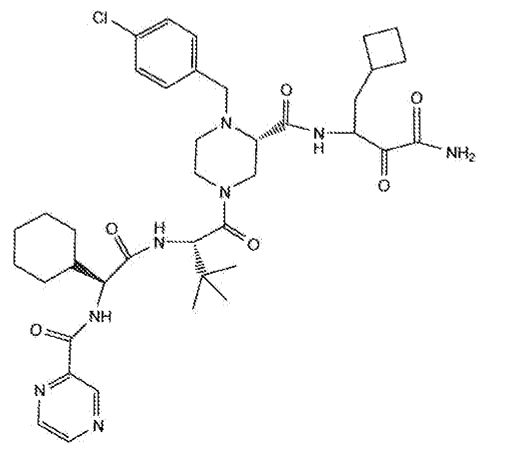 | **A-39** |
| 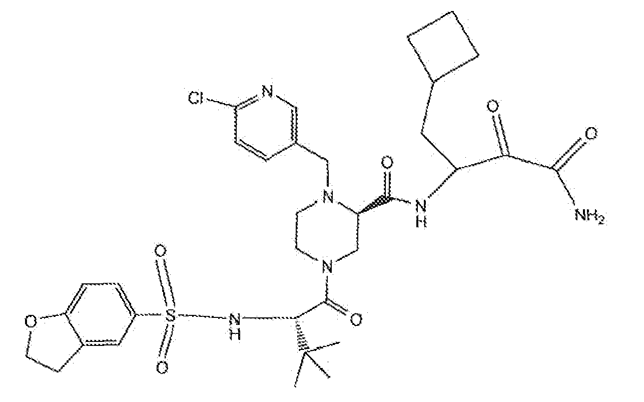 | **A-40** |
| 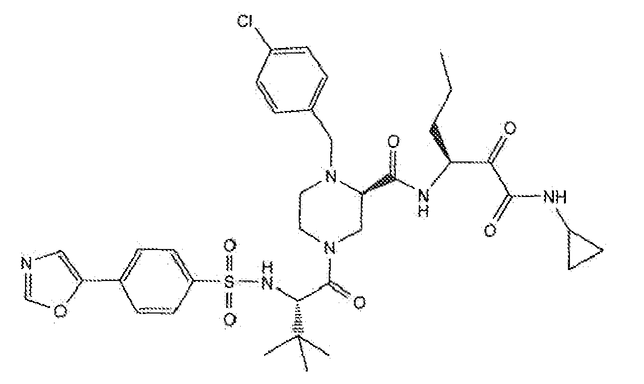 | **A-41** |
| 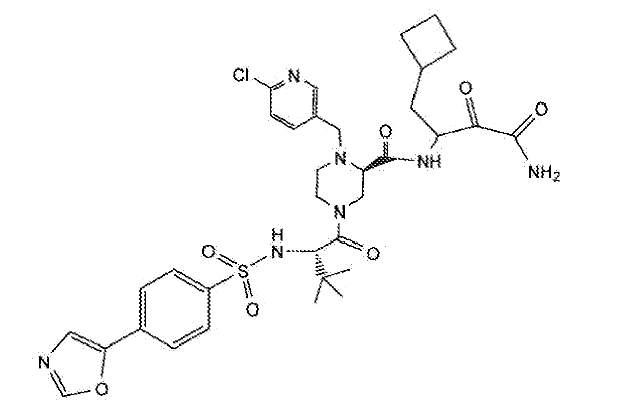 | **A-42** |
| 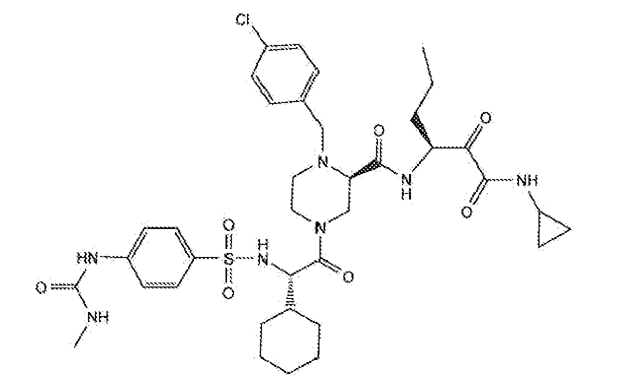 | **A-43** |
| 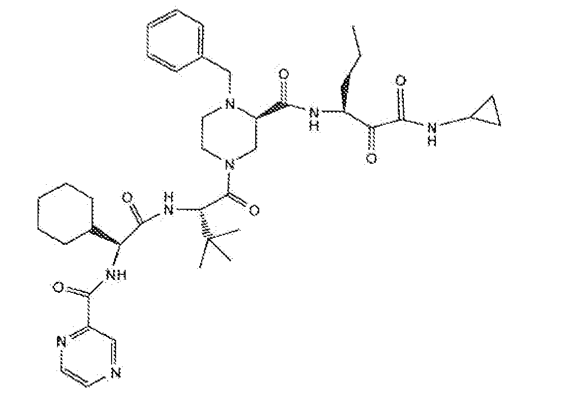 | **A-44** |
| 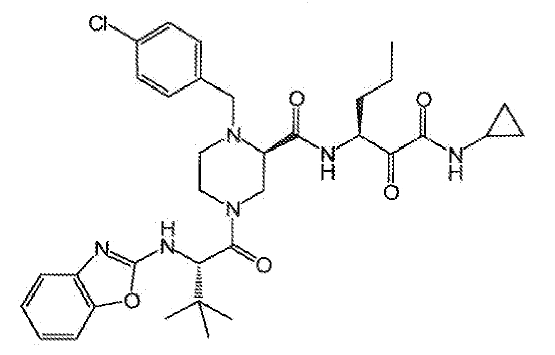 | **A-45** |
| 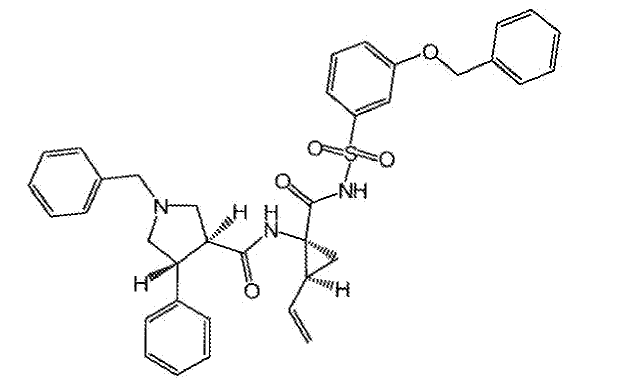 | **A-46** |
| 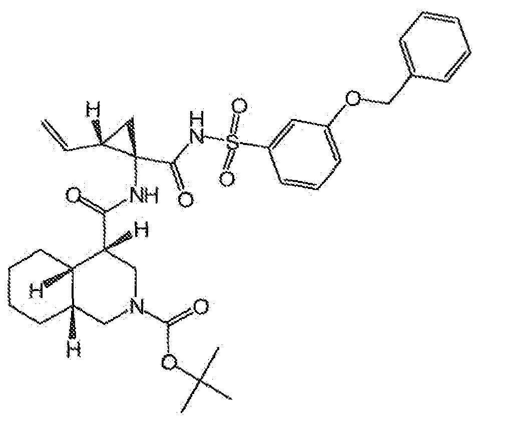 | **A-47** |
| 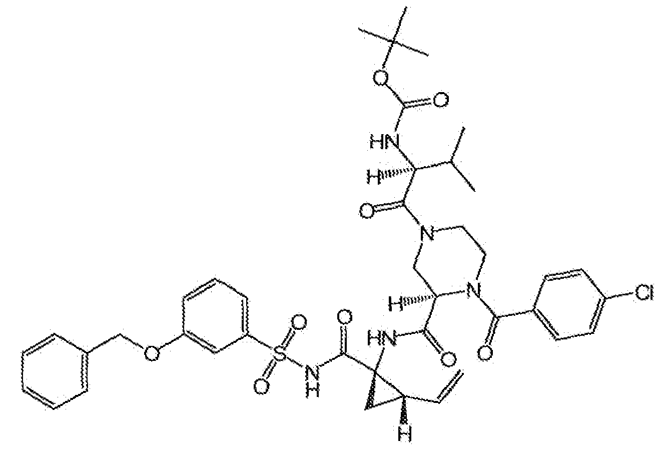 | **A-48** |
| 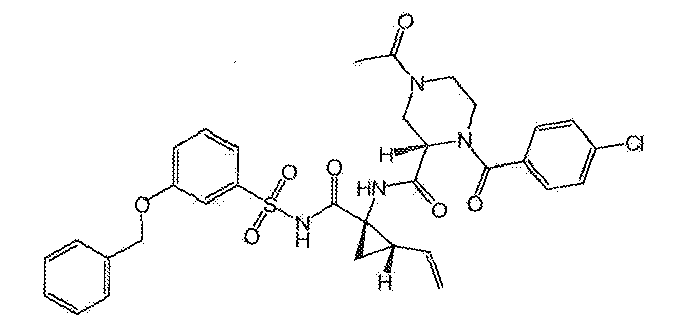 | **A-49** |
| 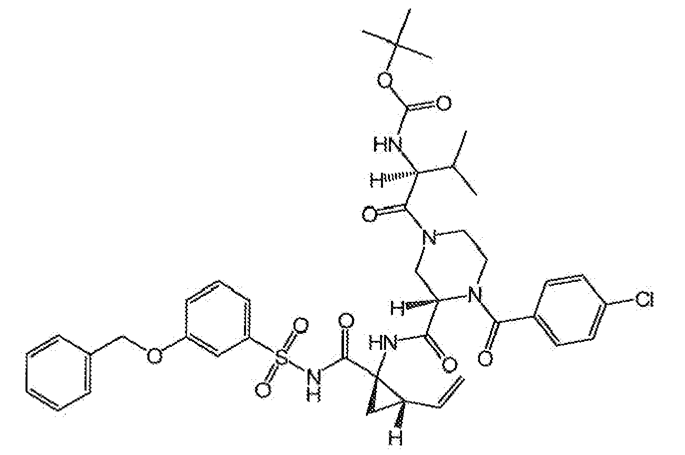 | **A-50** |
| 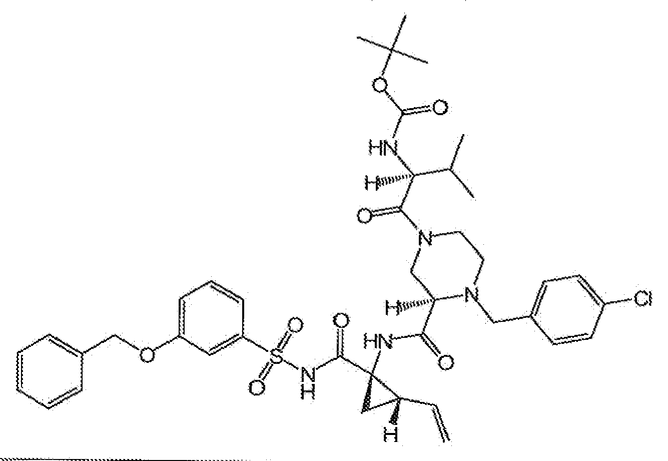 | **A-51** |
| 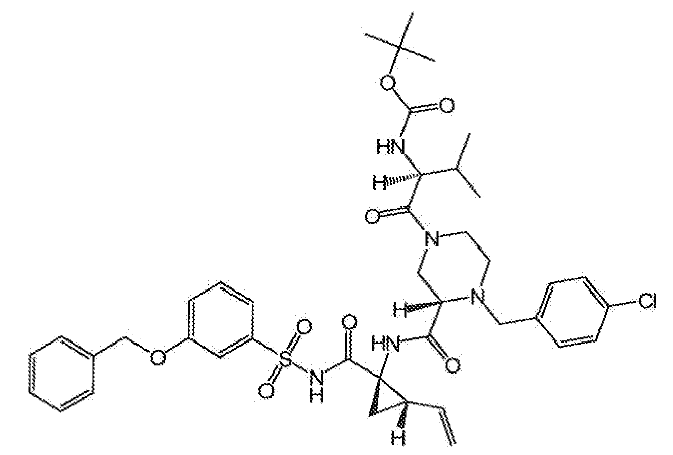 | **A-52** |
| 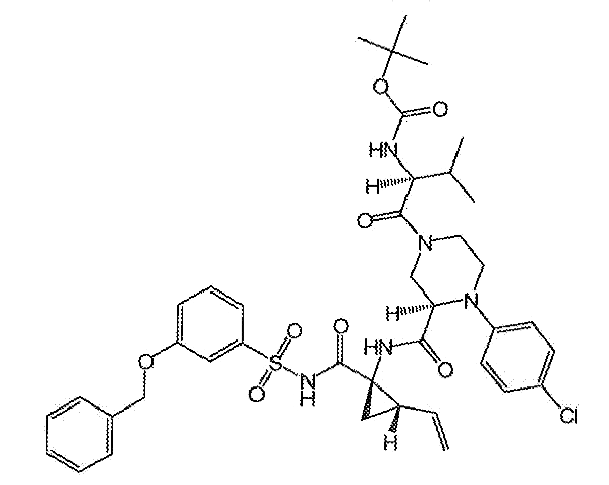 | **A-53** |
| 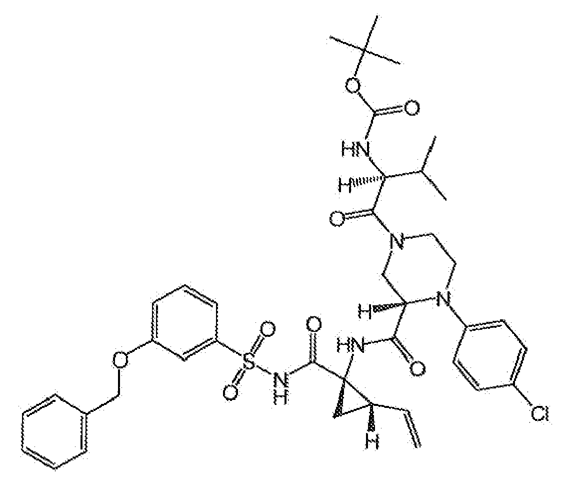 | **A-54** |
| 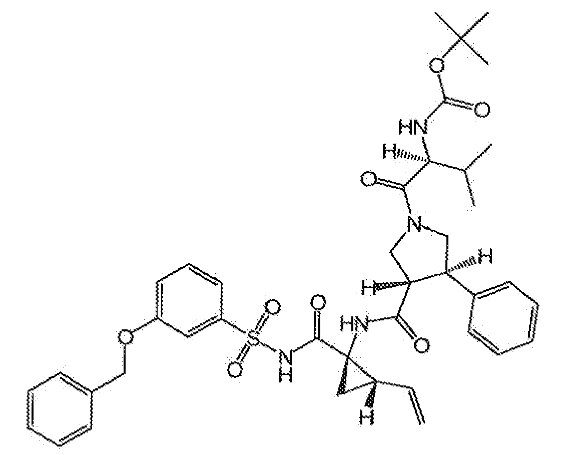 | **A-55** |
| 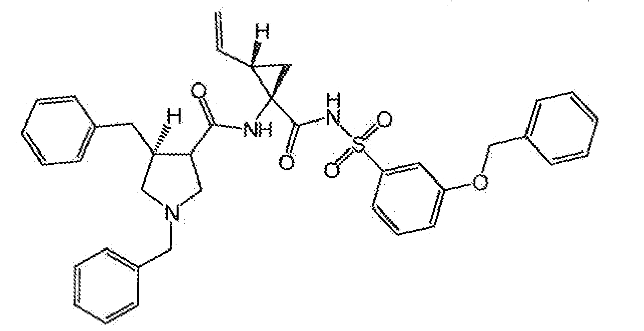 | **A-56** |
| 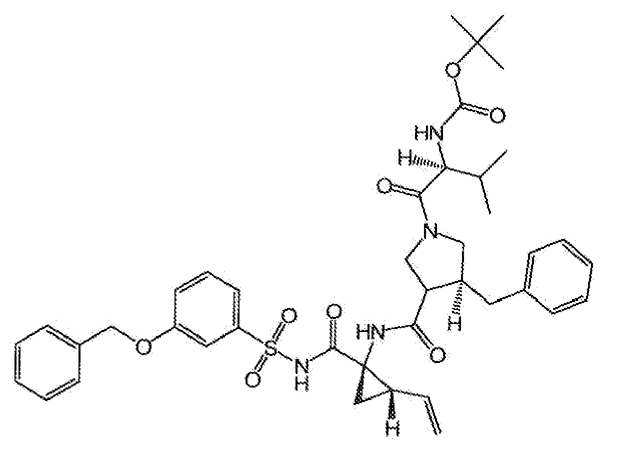 | **A-57** |
| 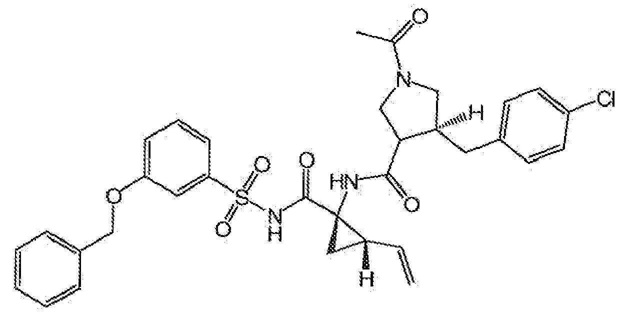 | **A-58** |
| 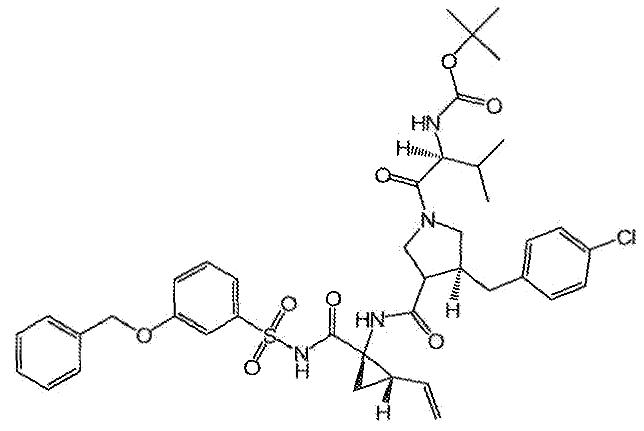 | **A-59** |
| 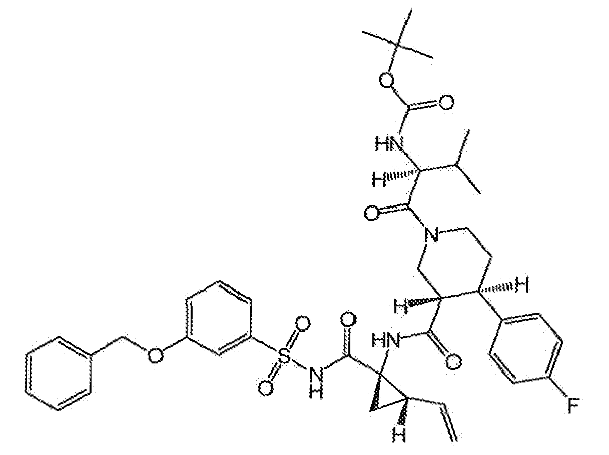 | **A-60** |
| 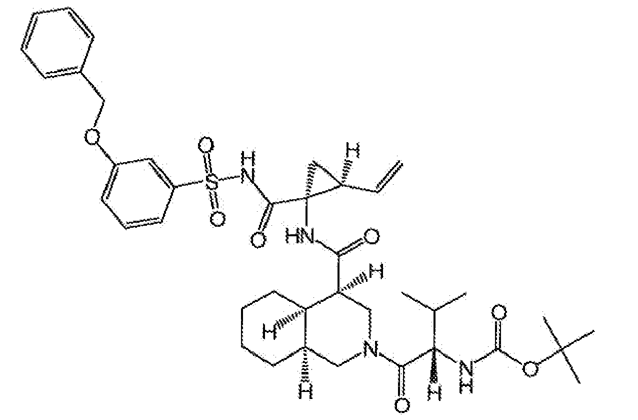 | **A-61** |
| 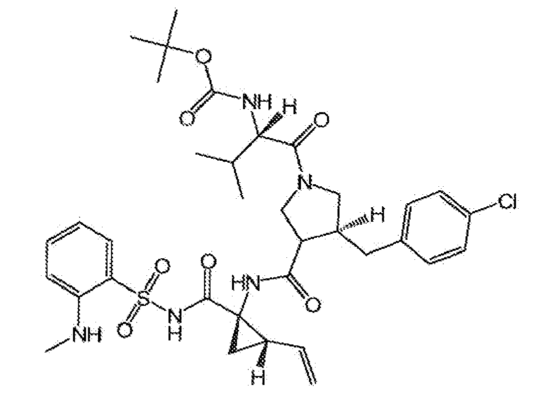 | **A-62** |
| 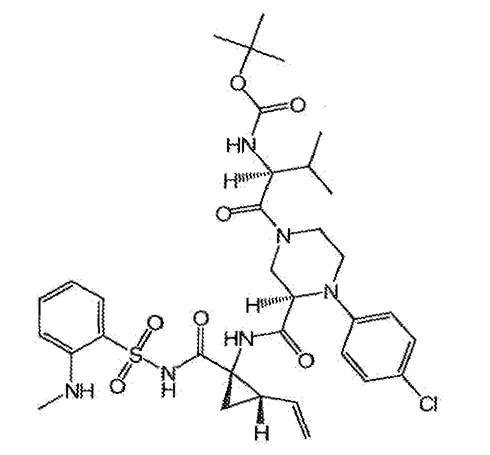 | **A-63** |
| 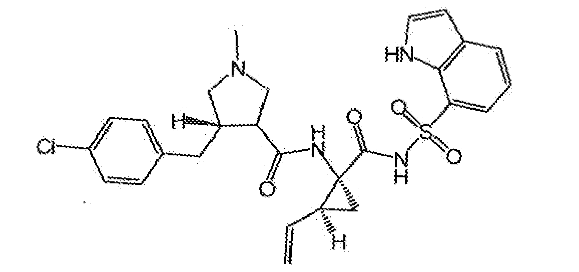 | **A-64** |
| 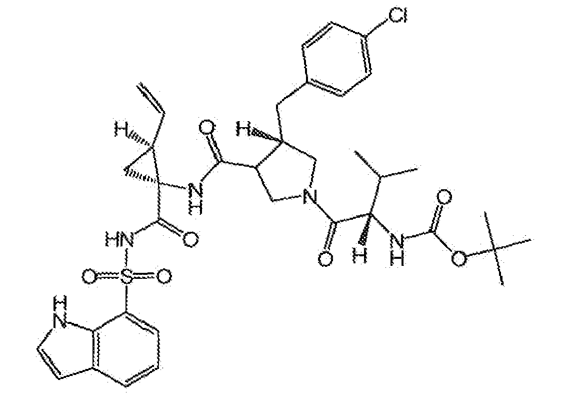 | **A-65** |
| 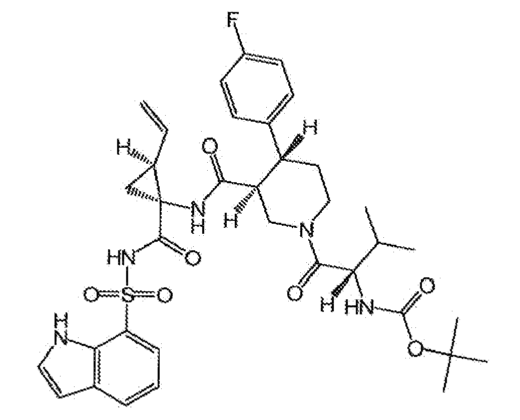 | A**-66** |
| 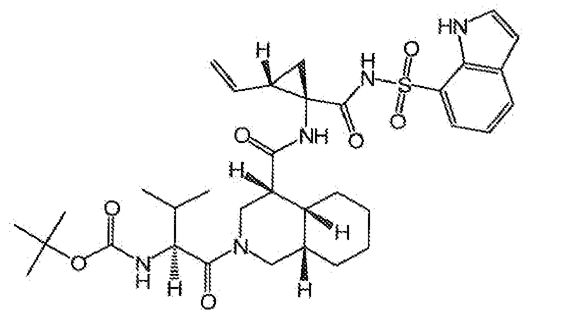 | **A-67** |
| 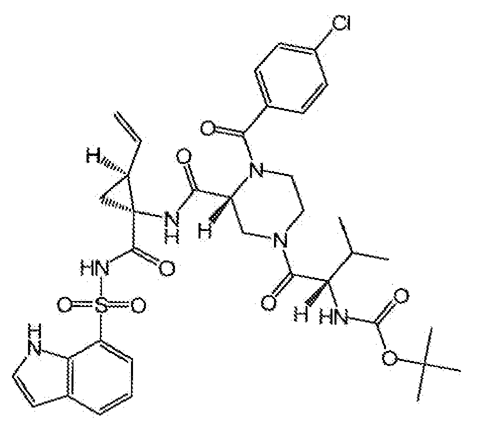 | **A-68** |
| 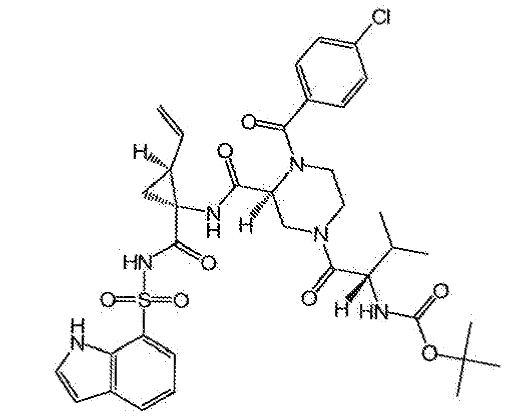 | **A-69** |
| 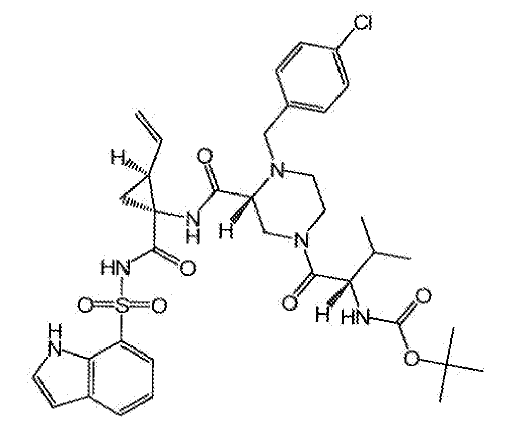 | **A-70** |
| 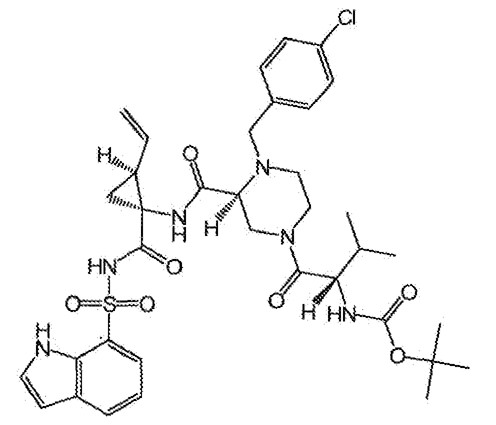 | **A-71** |
| 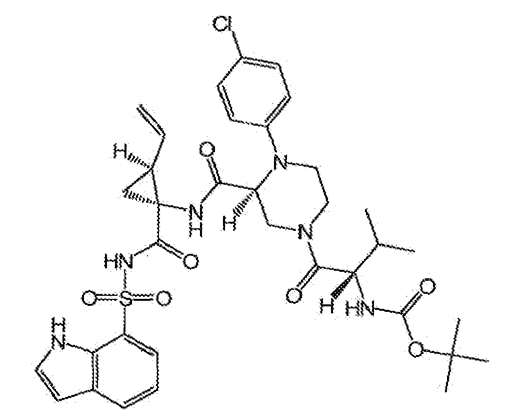 | **A-72** |
| 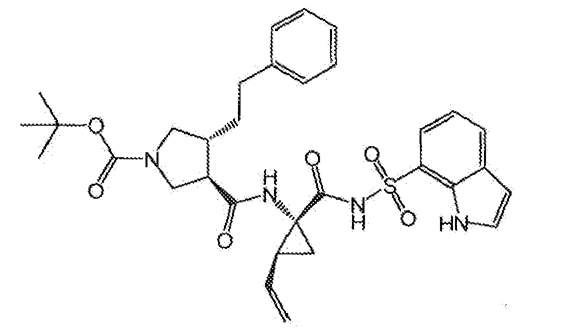 | **A-73** |
| 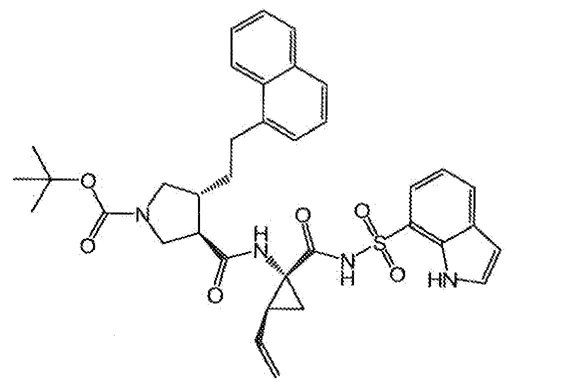 | **A-74** |
| 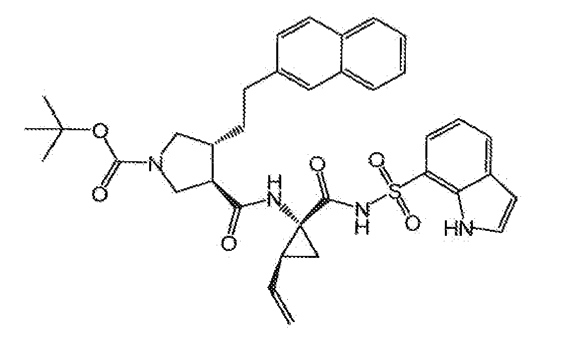 | **A-75** |
| 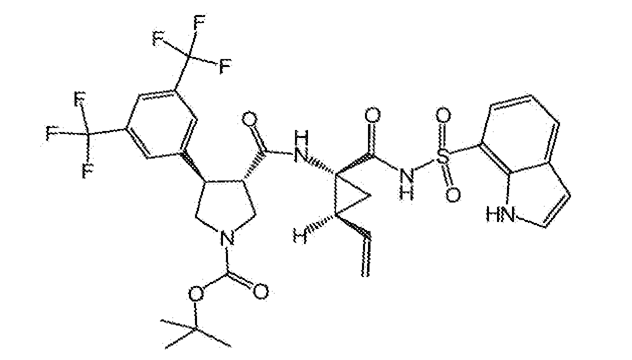 | **A-76** |
| 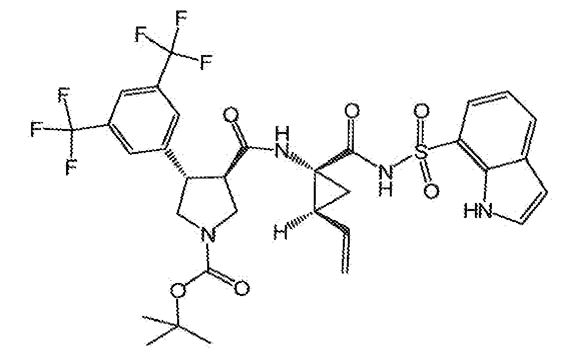 | **A-77** |
| 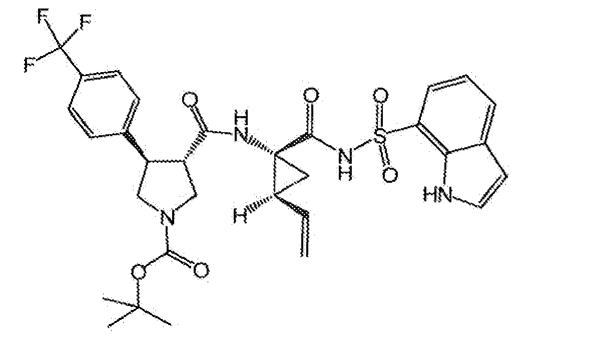 | **A-78** |
| 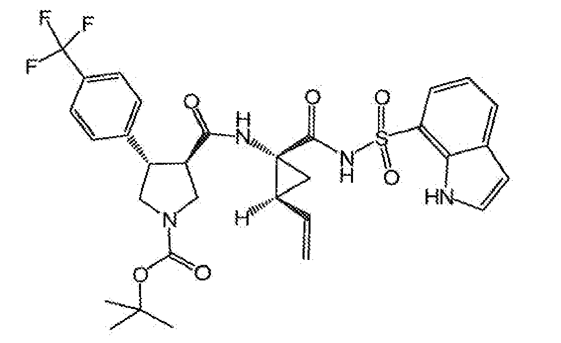 | **A-79** |
| 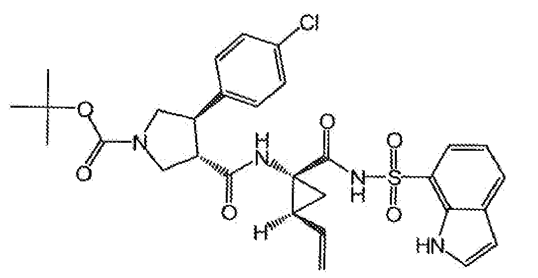 | **A-80** |
| 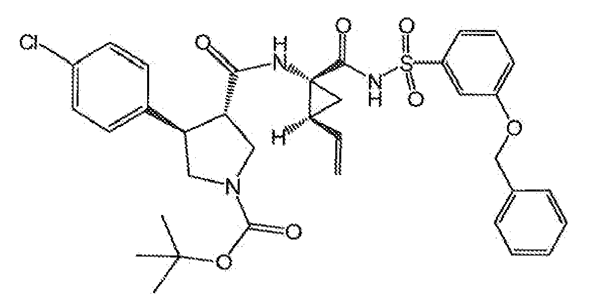 | **A-81** |
| 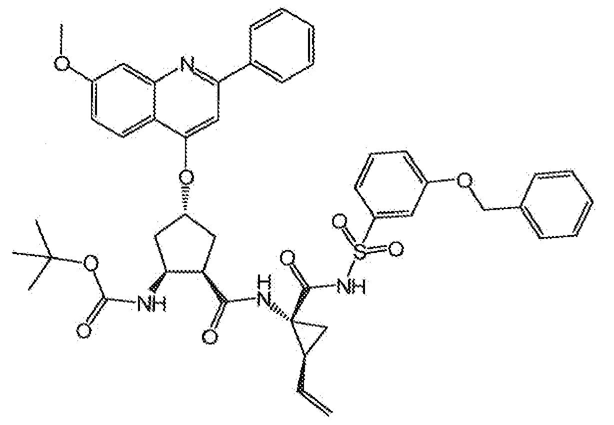 | **A-82** |
| 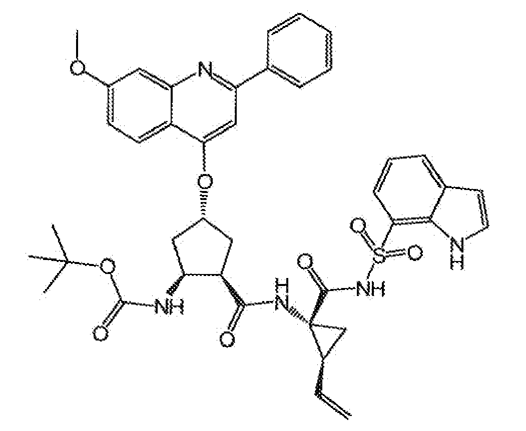 | **A-83** |
| 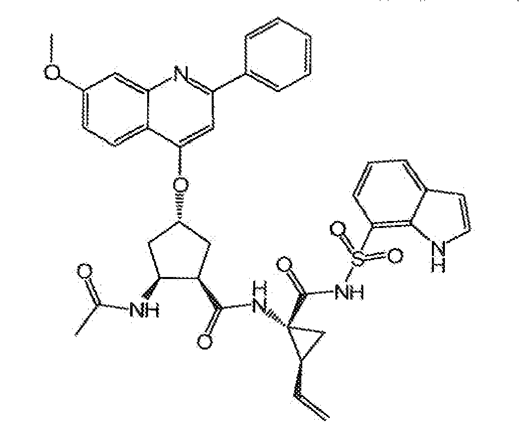 | **A-84** |
| 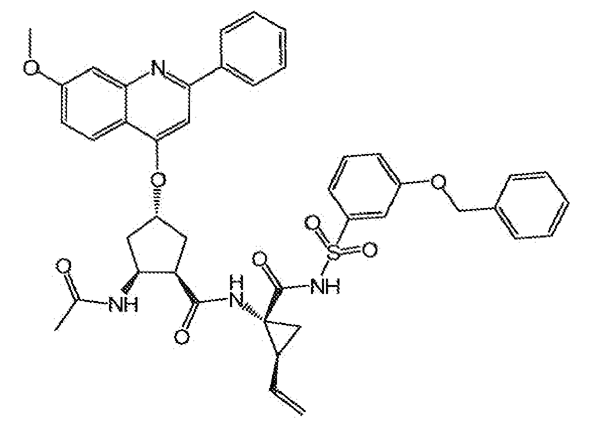 | **A-85** |
| 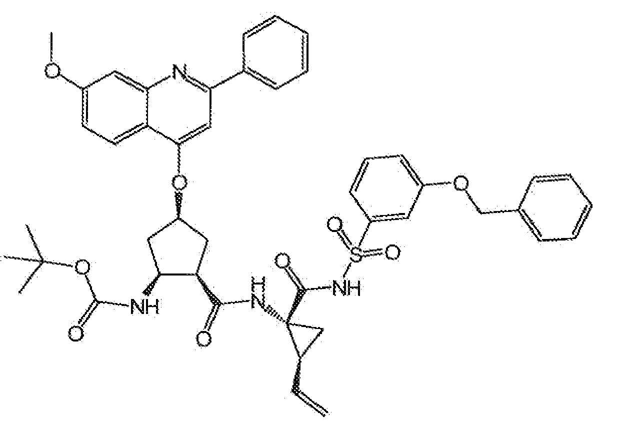 | **A-86** |
| 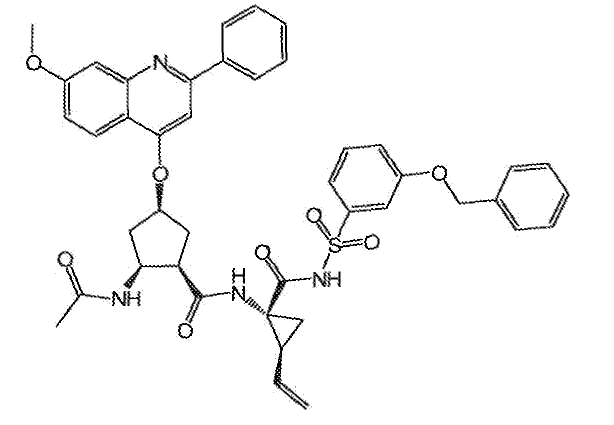 | **A-87** |
| 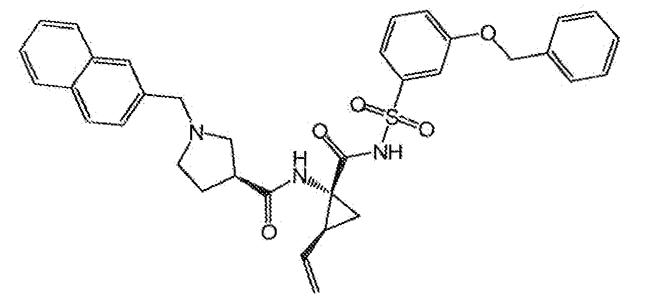 | **A-88** |
| 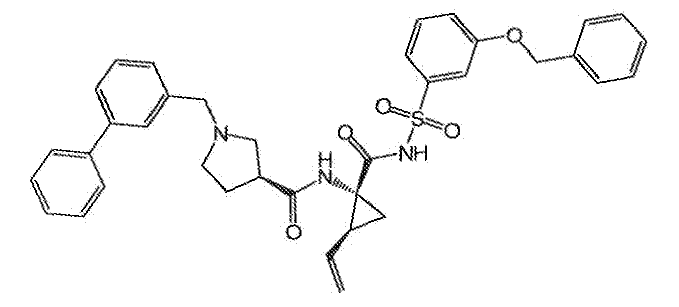 | **A-89** |
| 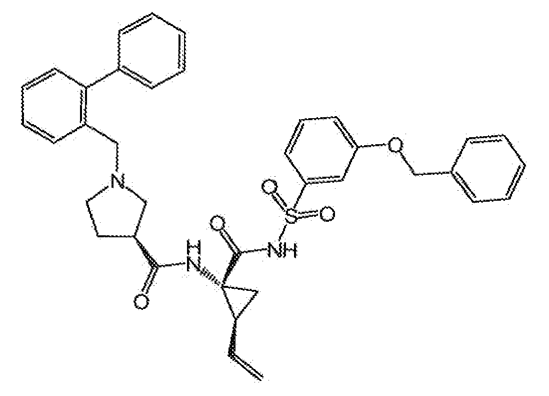 | **A-90** |
| 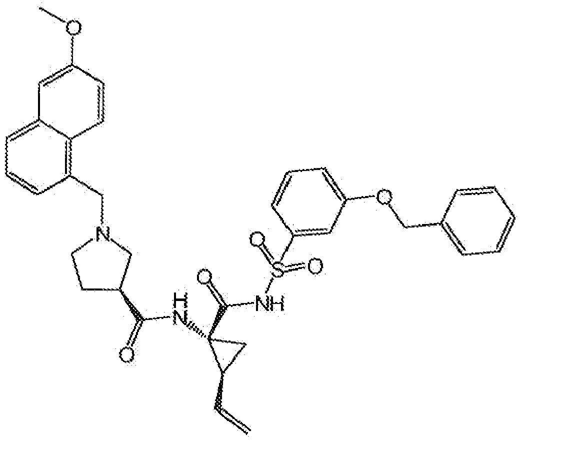 | **A-91** |
| 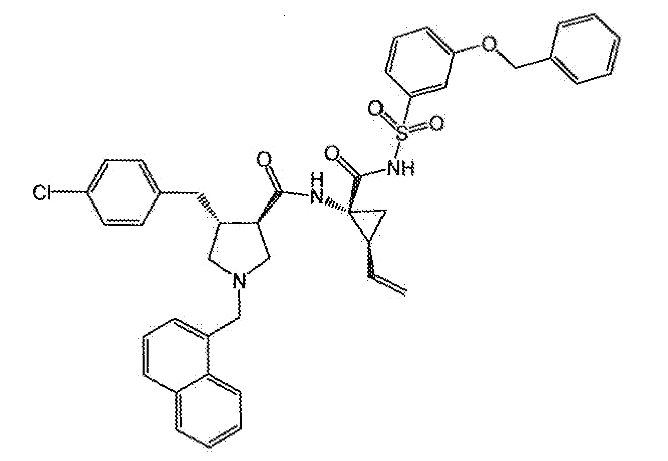 | **A-92** |
| 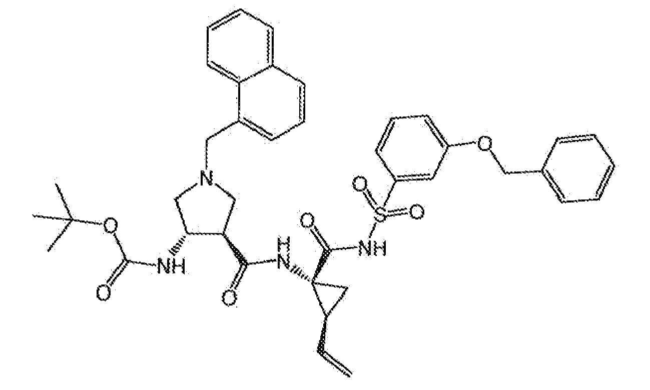 | **A-93** |
| 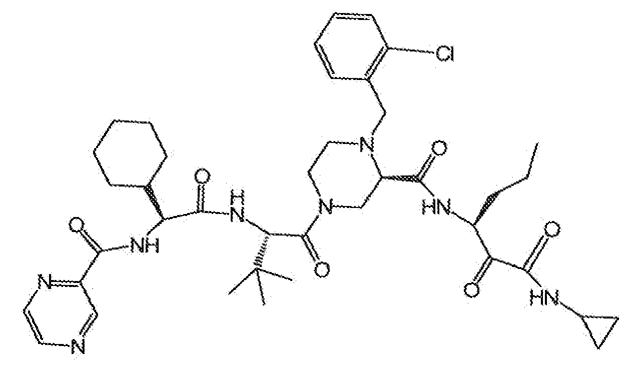 | **A-94** |
| 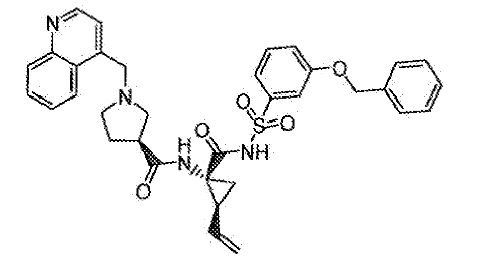 | **A-95** |
| 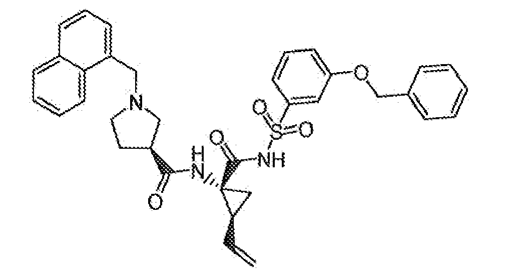 | A-9**6** |
| 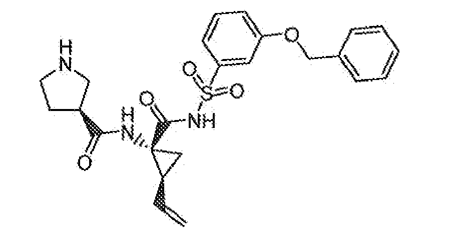 | **A-97** |
| 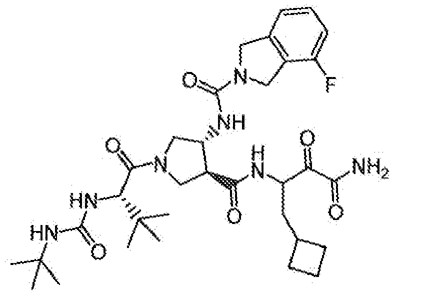 | **A-98** |

**TABLE B**

| **Structure** | **Compound No.** |
|---|---|
| 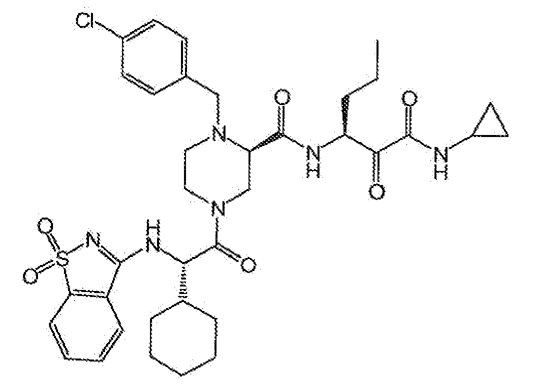 | **B-1** |
| 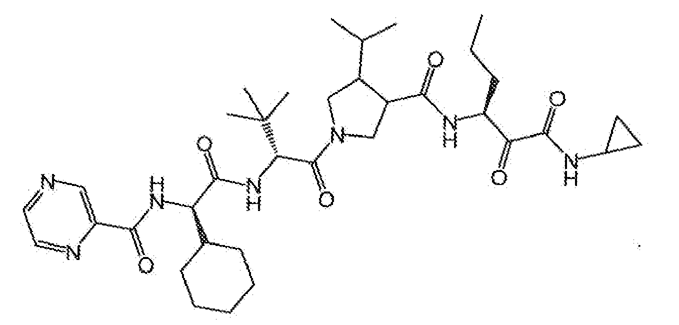 | **B-2** |
| 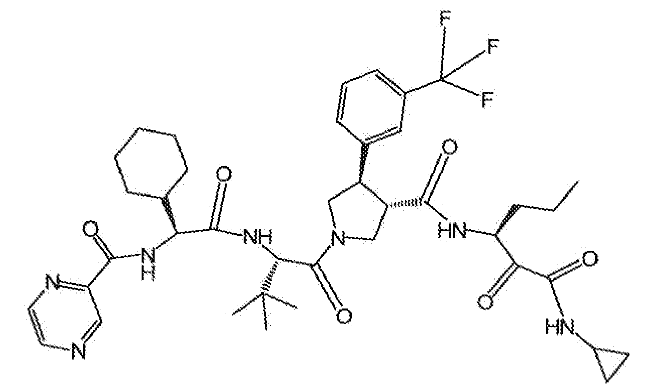 | **B-3** |
| 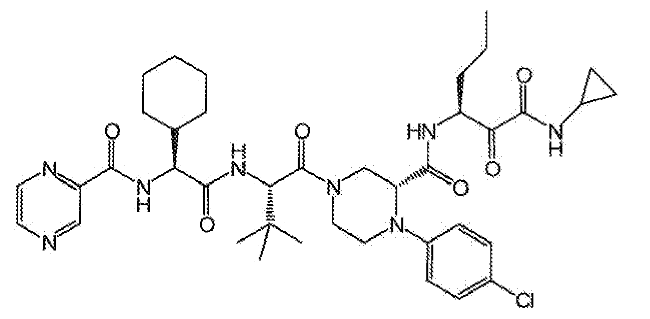 | **B-4** |
| 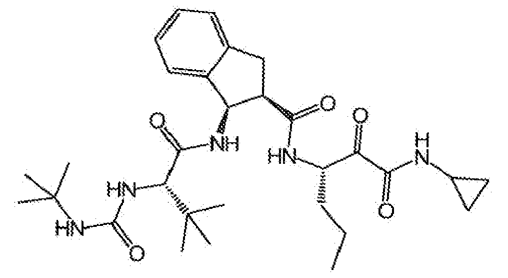 | **B-5** |
| 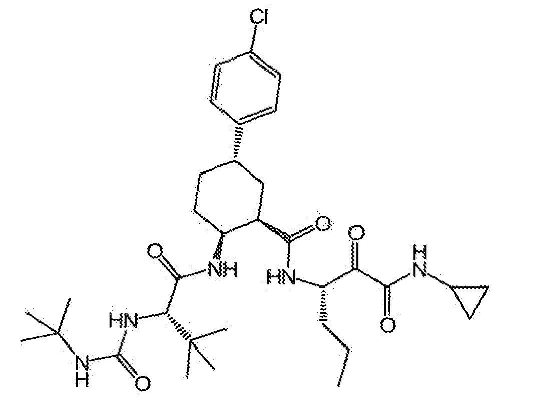 | **B-6** |
| 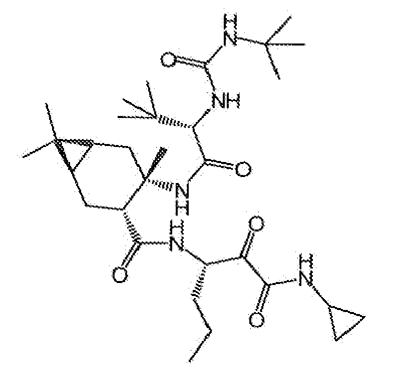 | **B-7** |
| 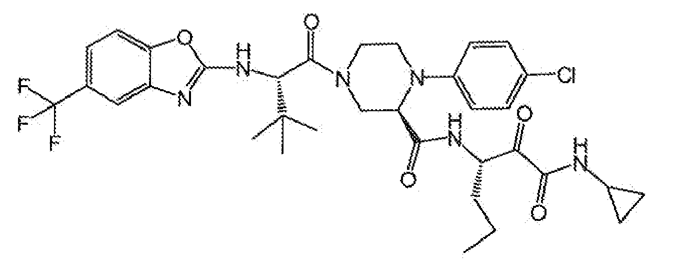 | **B-8** |
| 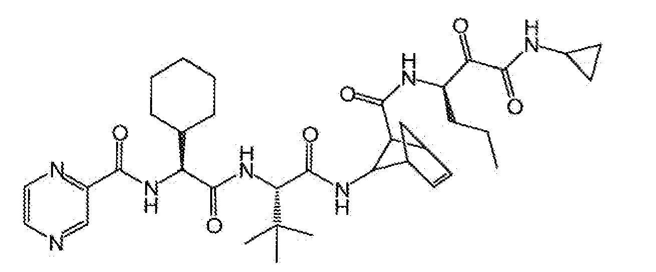 | **B-9** |
| 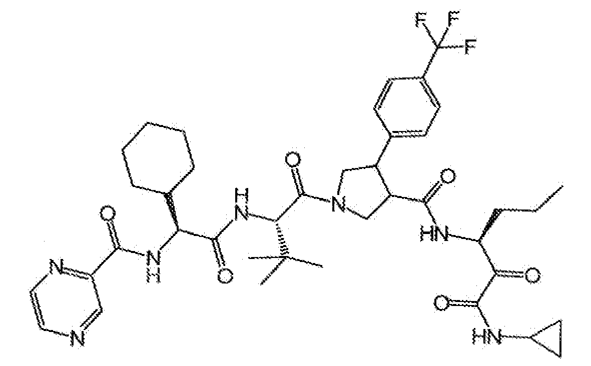 | **B-10** |
| 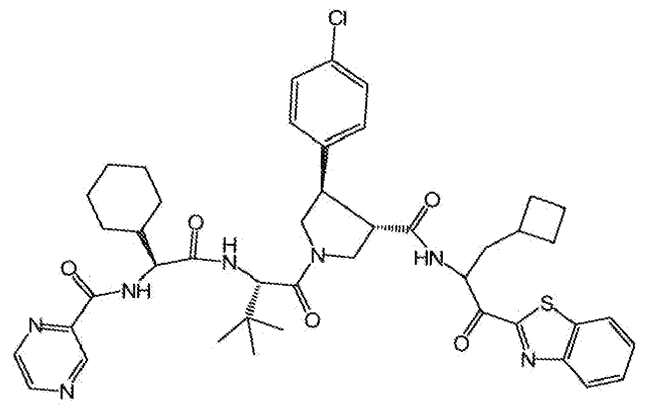 | **B-11** |
| 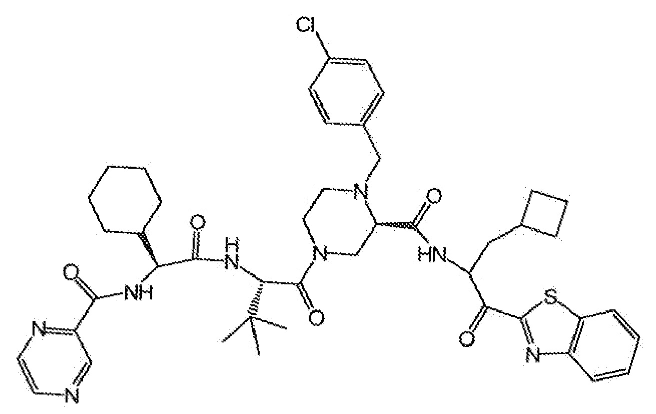 | **B-12** |
| 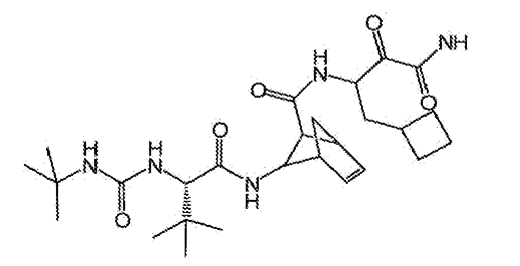 | **B-13** |
| 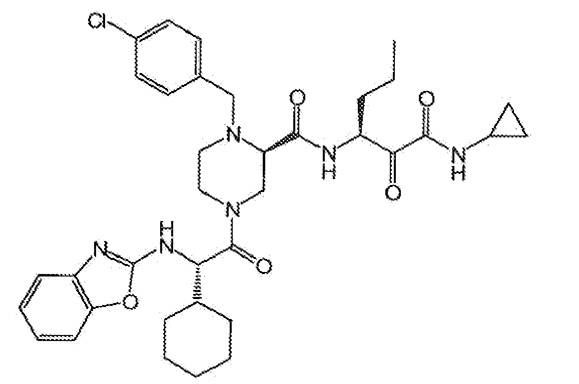 | **B-14** |
| 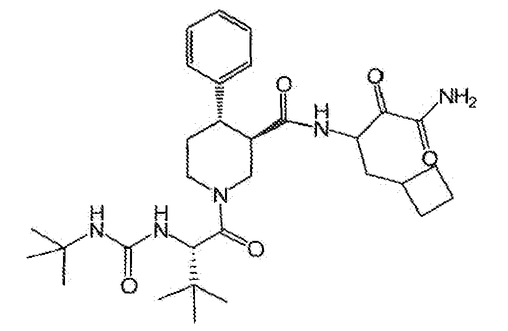 | **B-15** |
| 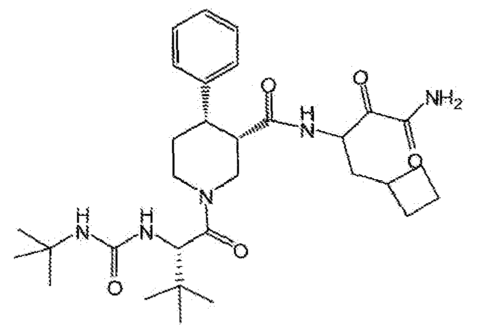 | **B-16** |
| 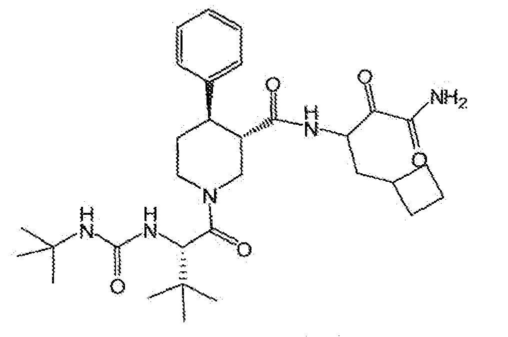 | **B-17** |
| 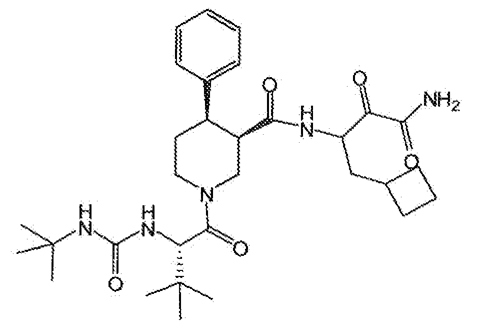 | **B-18** |
| 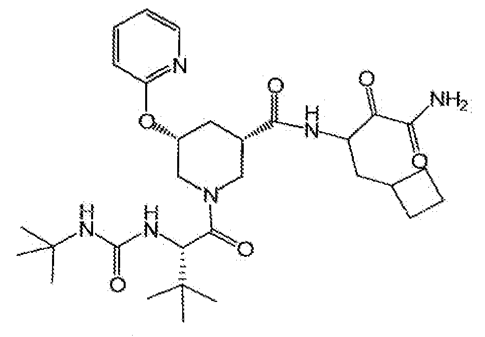 | **B-19** |
| 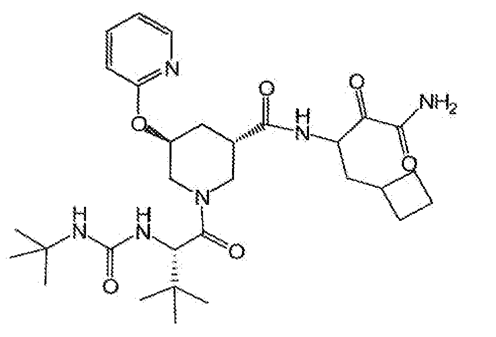 | **B-20** |
| 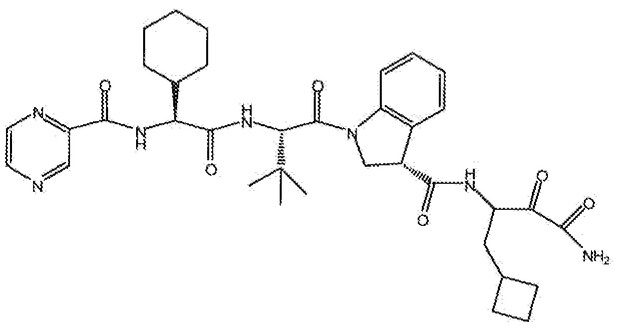 | **B-21** |
| 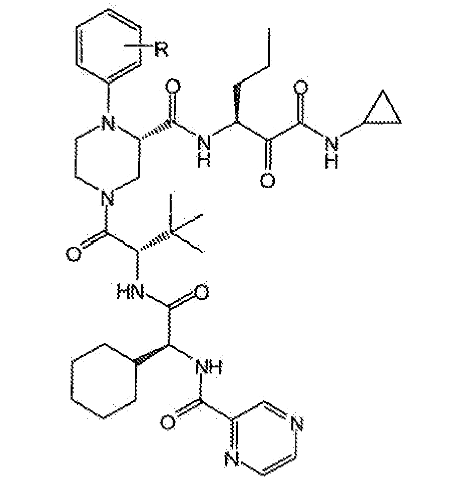 | **B-22** |
| 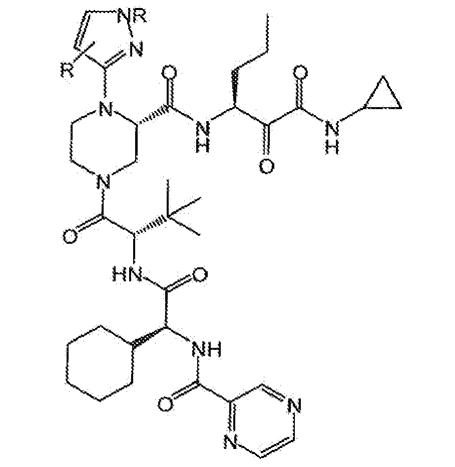 | **B-23** |
| 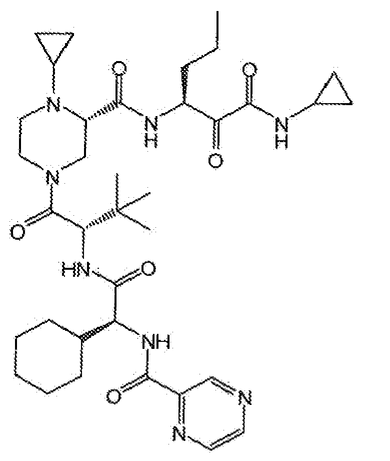 | **B-24** |
| 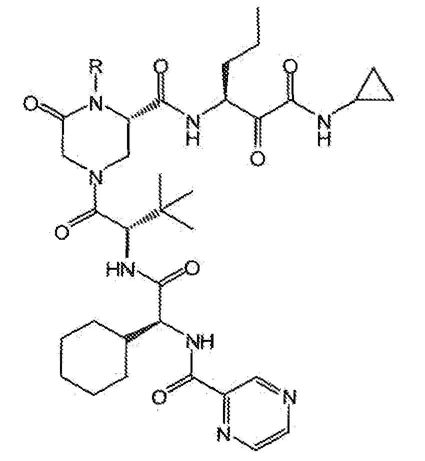 | **B-25** |
| 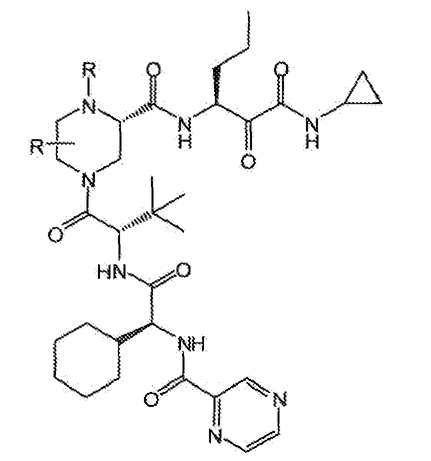 | **B-26** |
| 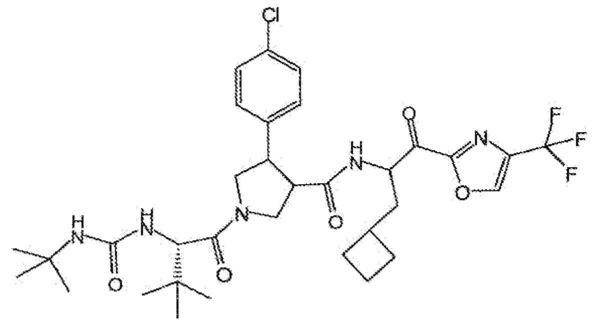 | **B-27** |
| 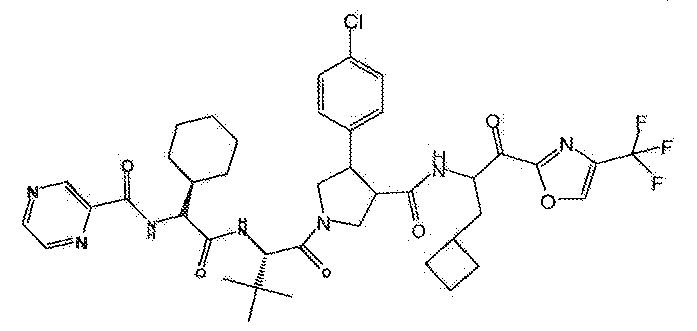 | **B-28** |
| 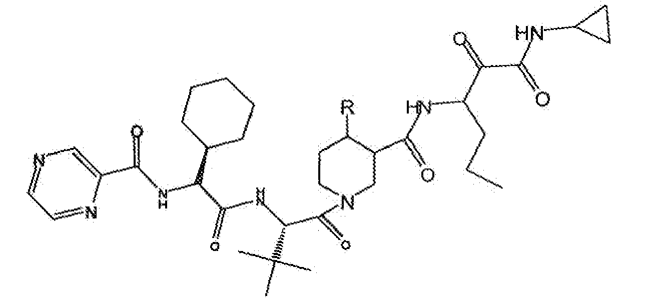 | **B-29** |
| 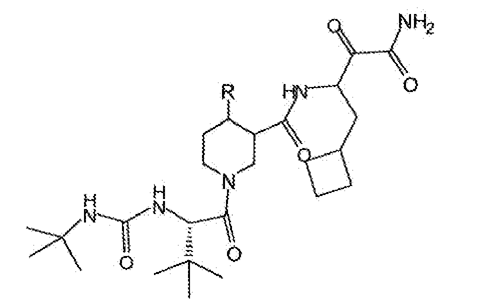 | **B-30** |
| 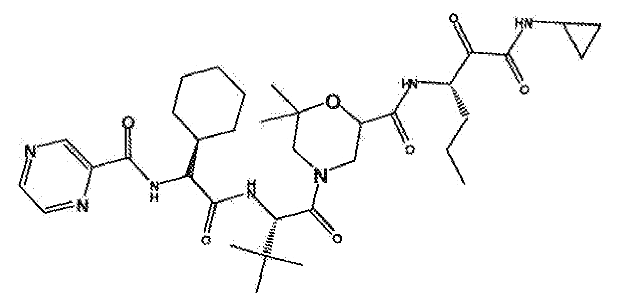 | **B-31** |
| 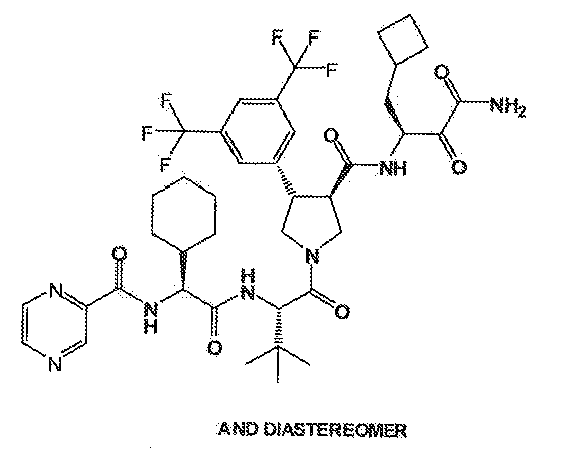 | **B-32** |
| 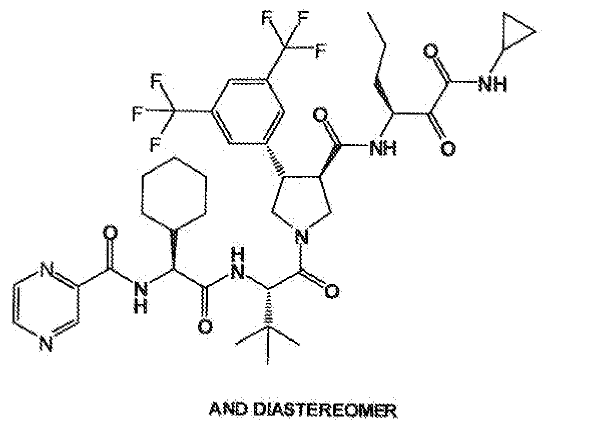 | **B-33** |
| 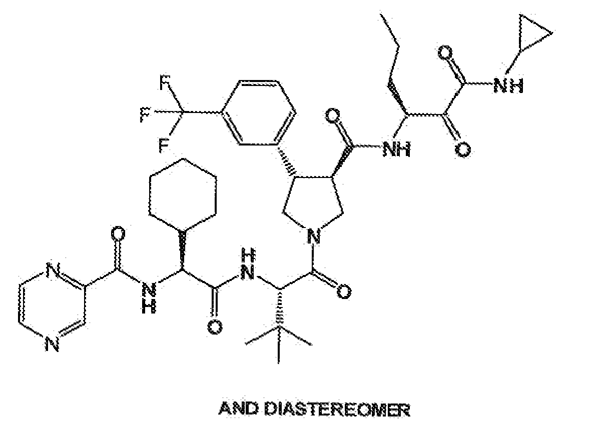 | **B-34** |
| 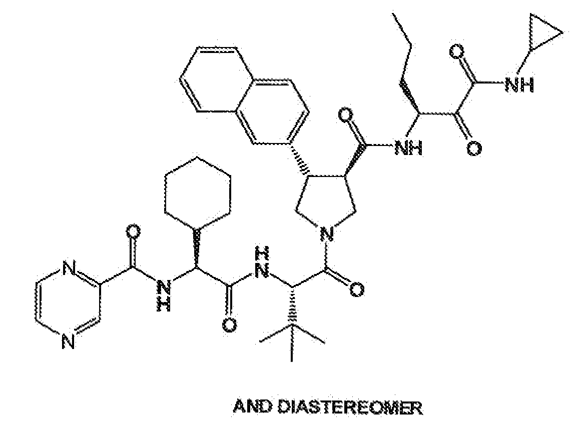 | **B-35** |
| 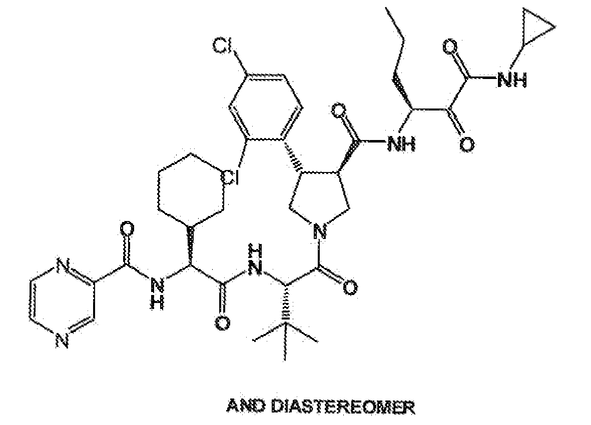 | **B-36** |
| 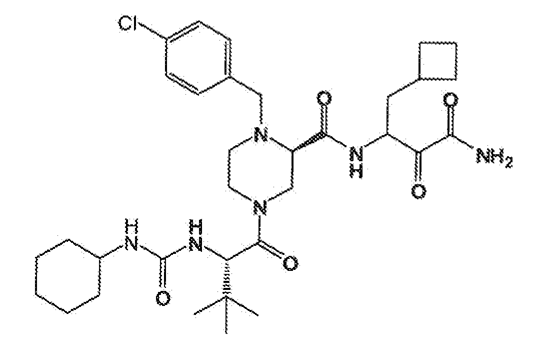 | **B-37** |
| 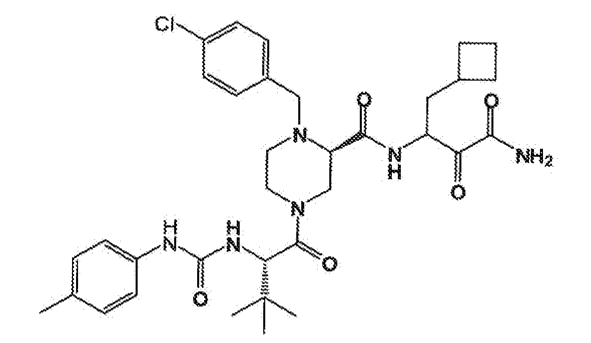 | **B-38** |
| 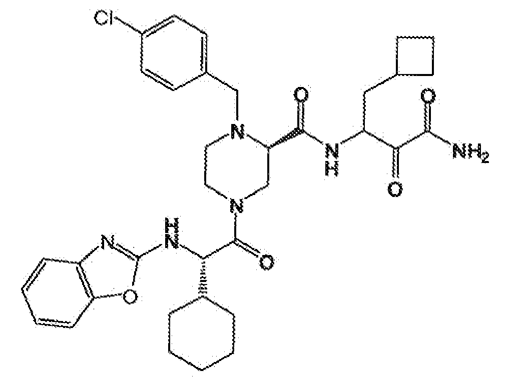 | **B-39** |
| 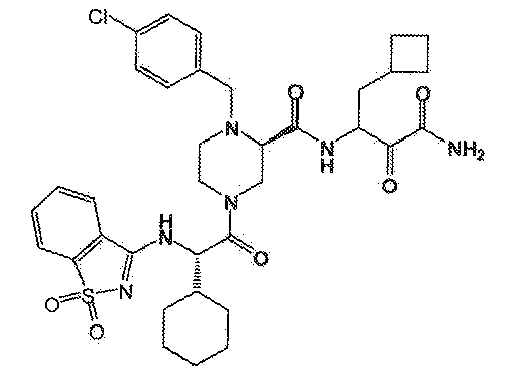 | **B-40** |
| 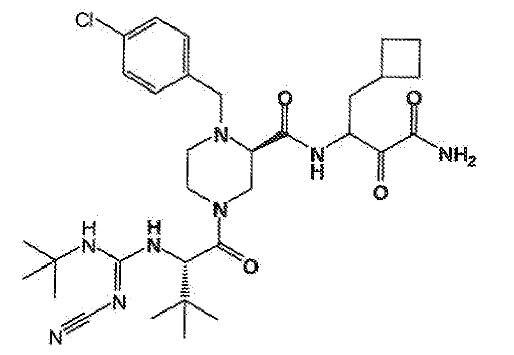 | **B-41** |
| 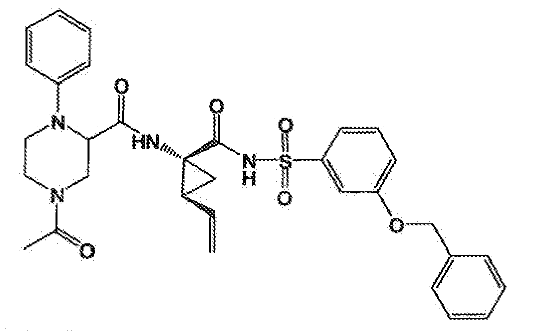 | **B-42** |
| 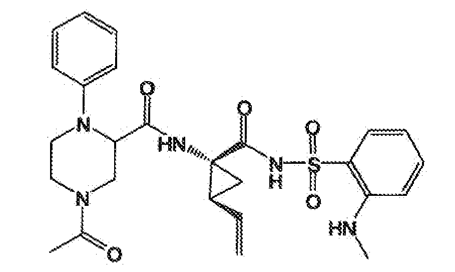 | **B-43** |
| 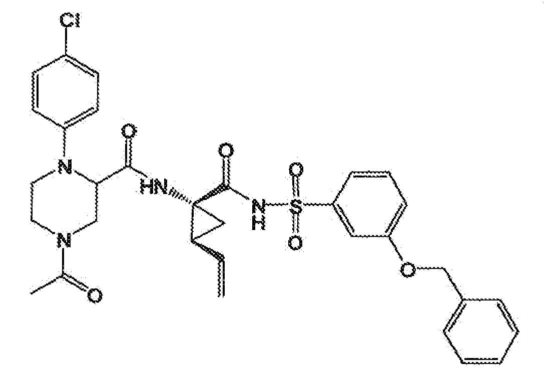 | **B-44** |
| 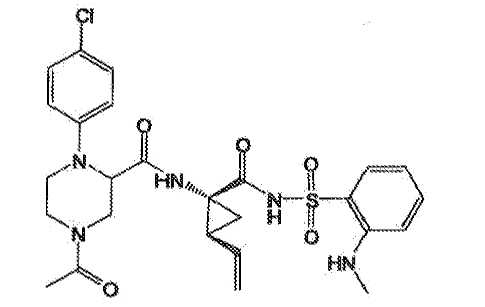 | **B-45** |
| 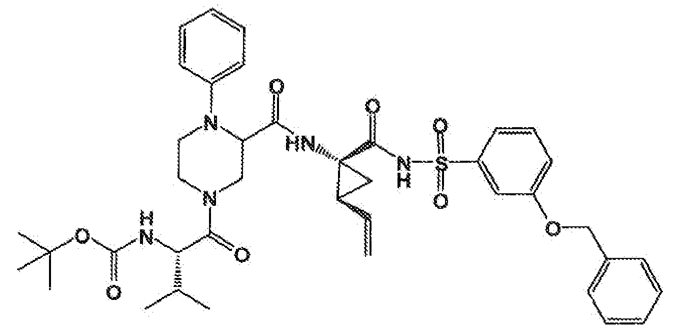 | **B-46** |
| 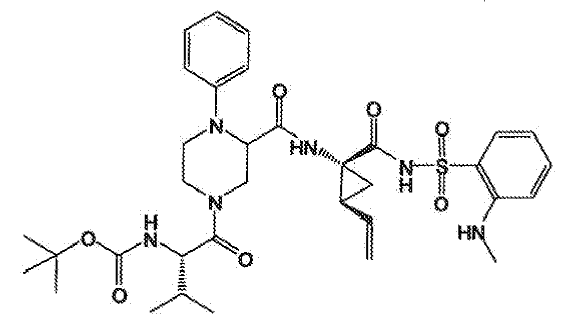 | **B-47** |
| 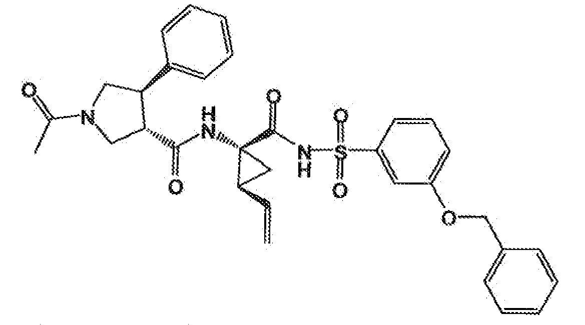 | **B-48** |
| 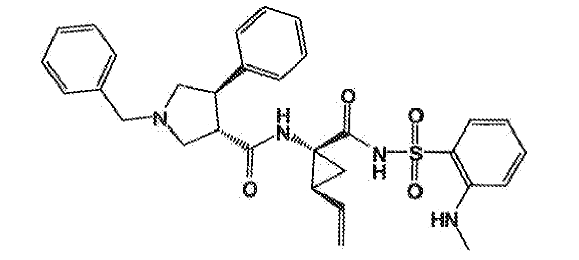 | **B-49** |
| 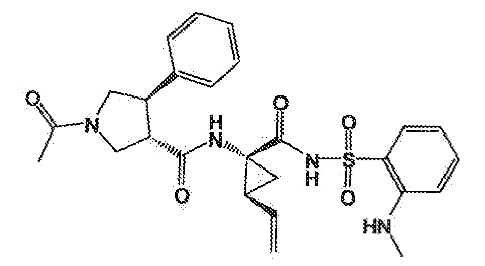 | B-50 |
| 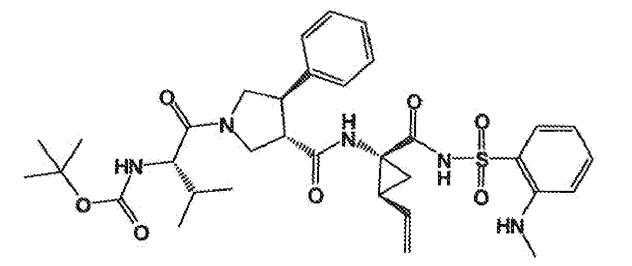 | **B-51** |
| 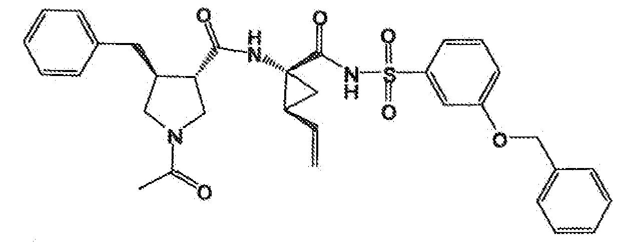 | **B-52** |
| 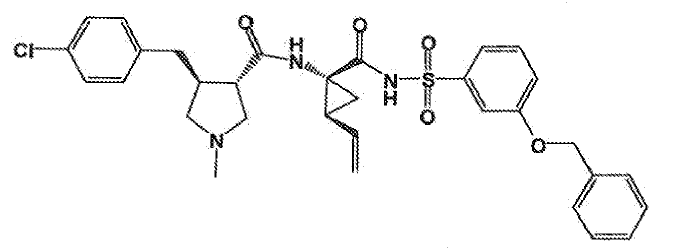 | **B-53** |
| 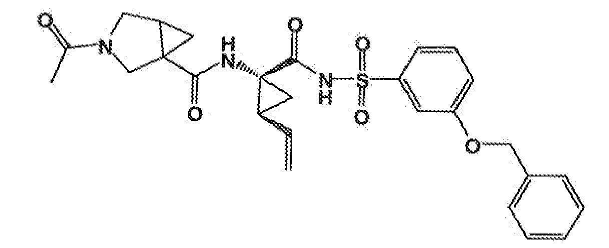 | **B-54** |
| 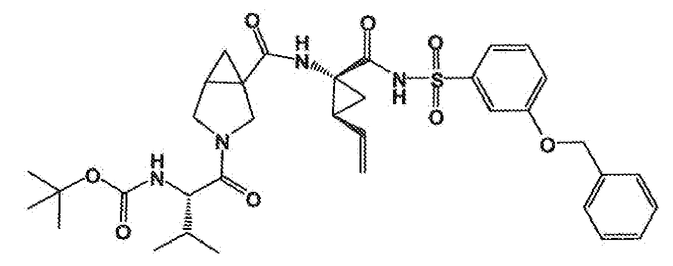 | **B-55** |
| 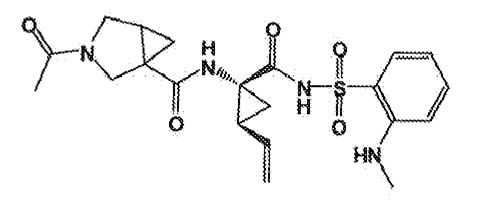 | **B-56** |
| 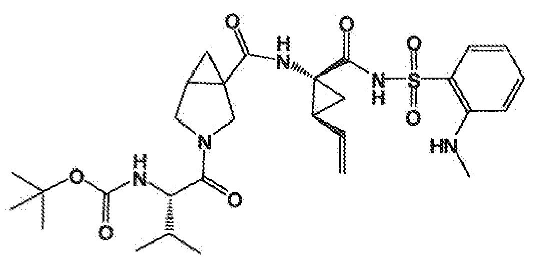 | B**-57** |
| 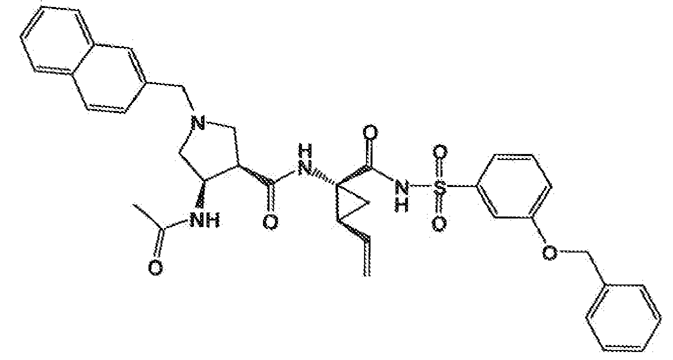 | **B-58** |
| 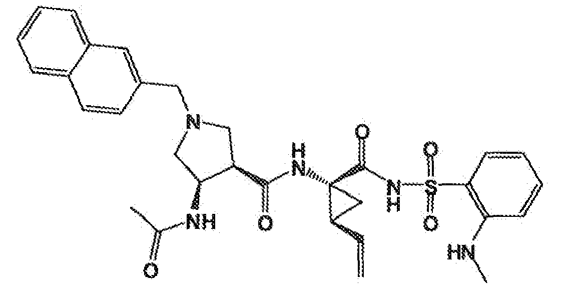 | **B-59** |
| 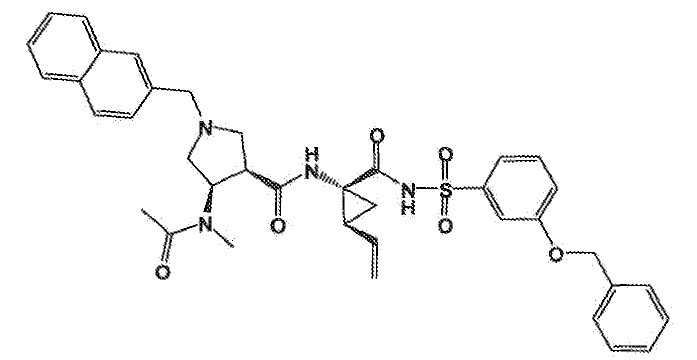 | **B-60** |
| 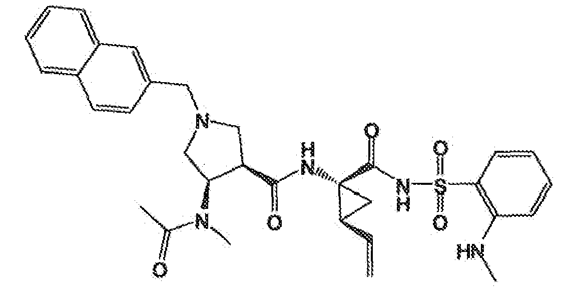 | **B-61** |
| 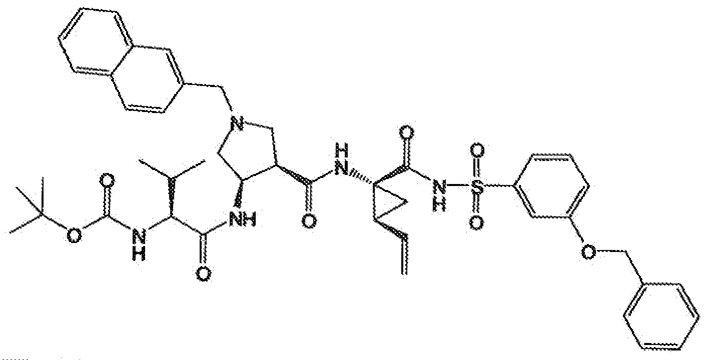 | **B-62** |
| 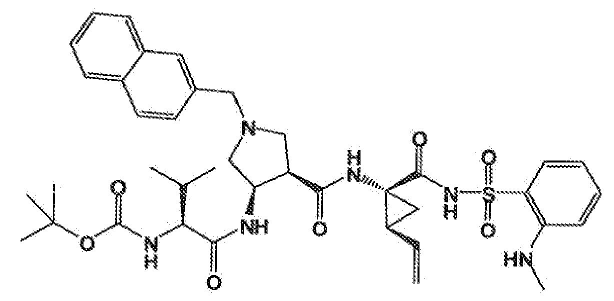 | **B-63** |
| 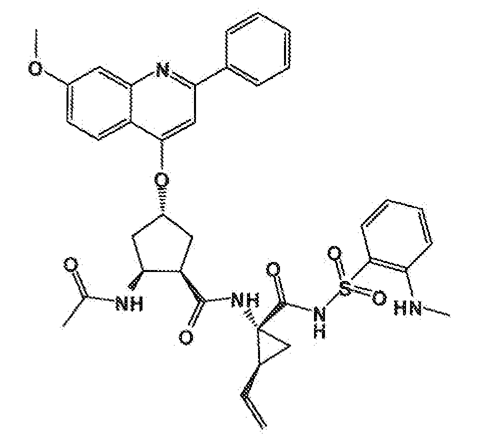 | **B-64** |
| 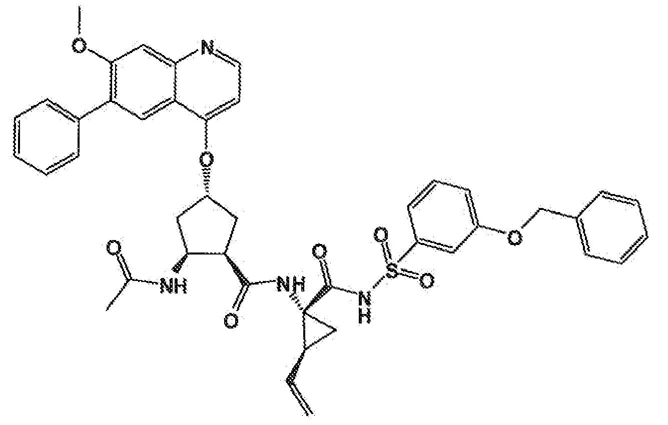 | **B-65** |
| 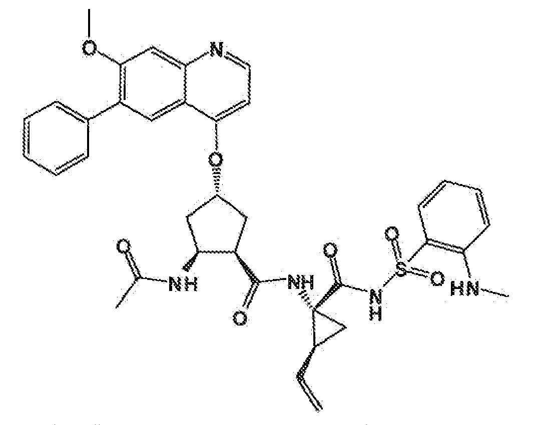 | **B-66** |
| 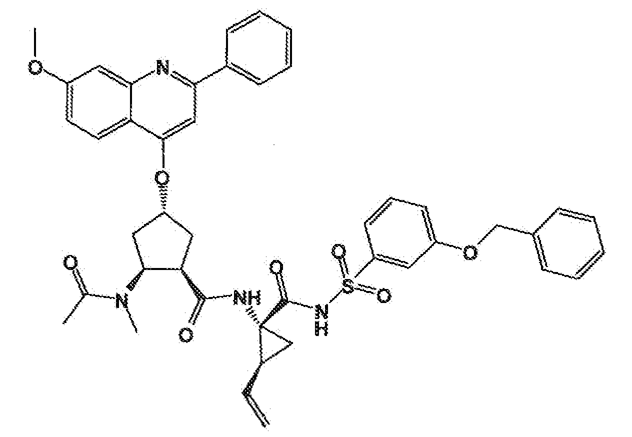 | **B-67** |
| 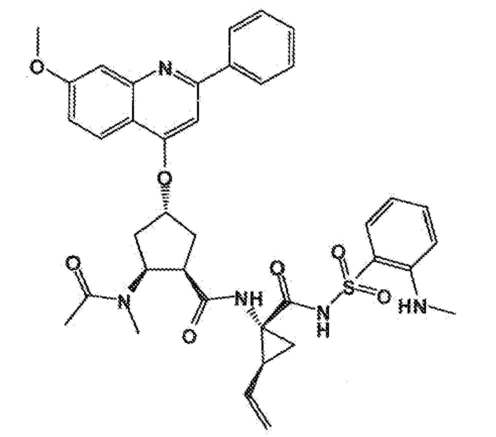 | **B-68** |
| 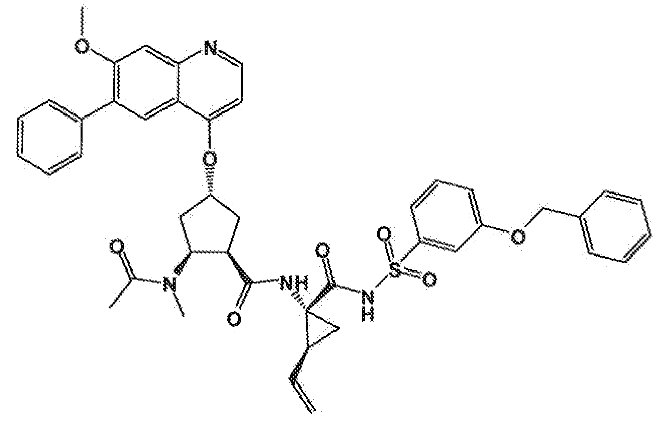 | **B-69** |
| 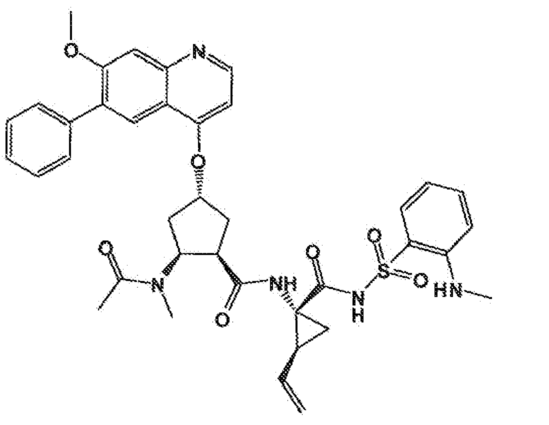 | **B-70** |
| 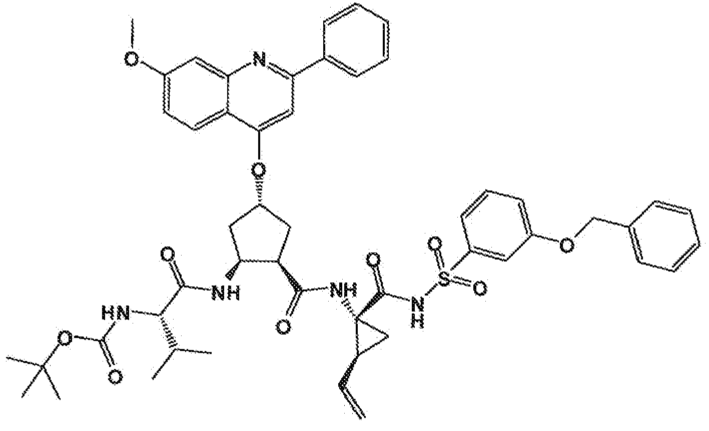 | **B-71** |
| 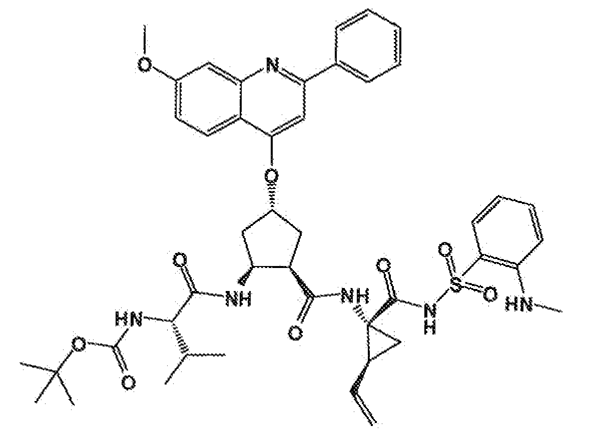 | **B-72** |
| 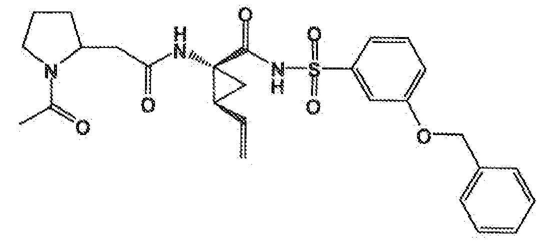 | **B-73** |
| 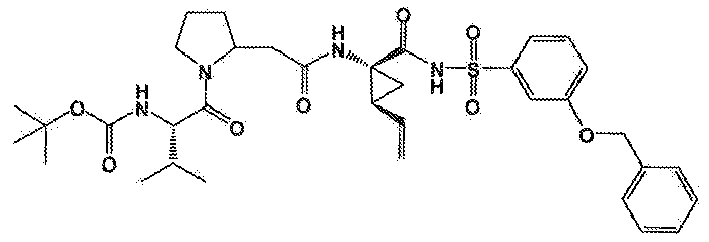 | **B-74** |
| 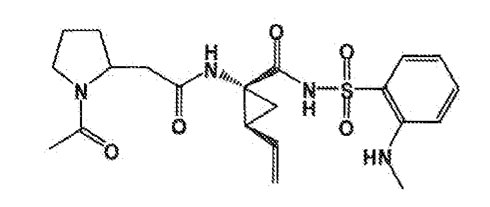 | **B-75** |
| 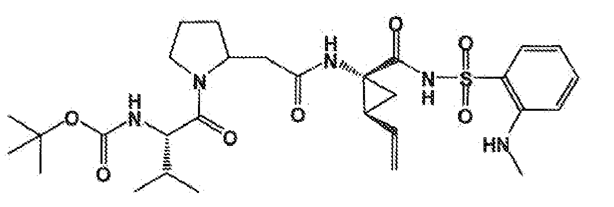 | **B-76** |
| 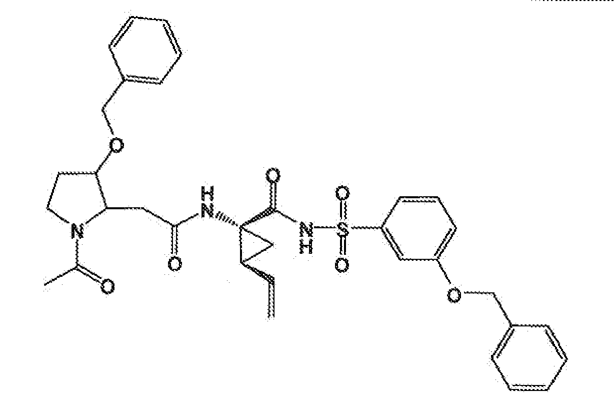 | **B-77** |
| 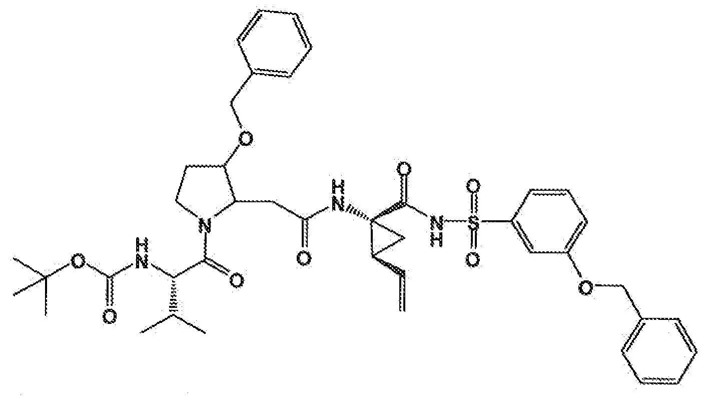 | **B-78** |
| 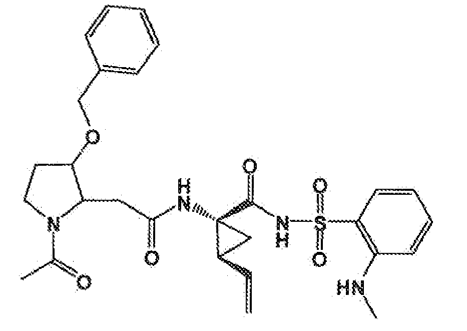 | **B-79** |
| 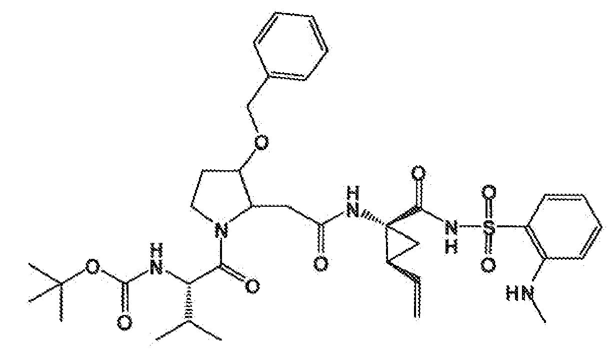 | **B-80** |
| 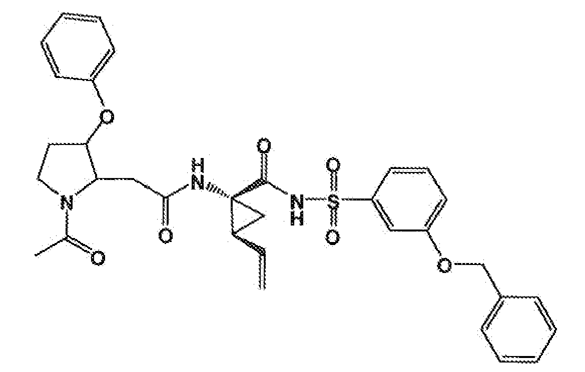 | **B-81** |
| 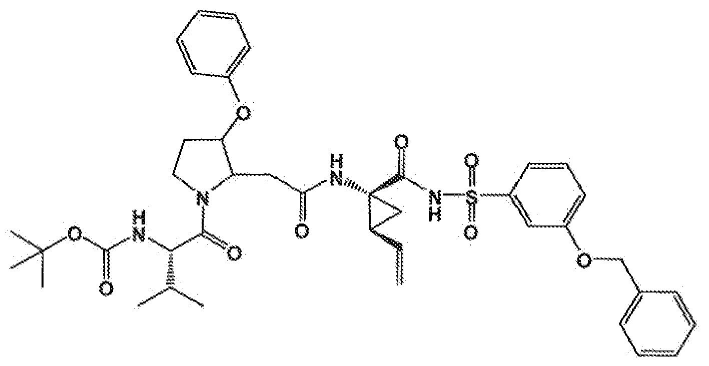 | **B-82** |
| 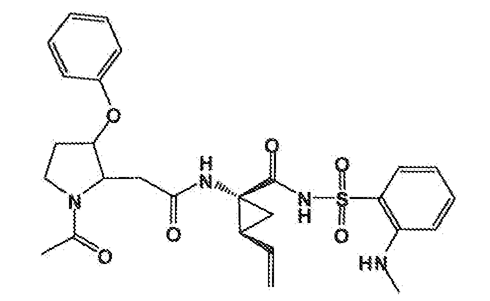 | **B-83** |
| 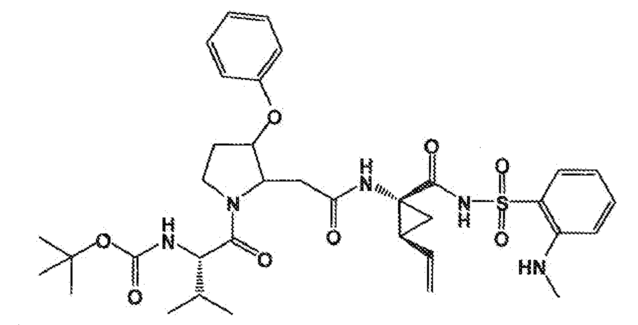 | **B-84** |
| 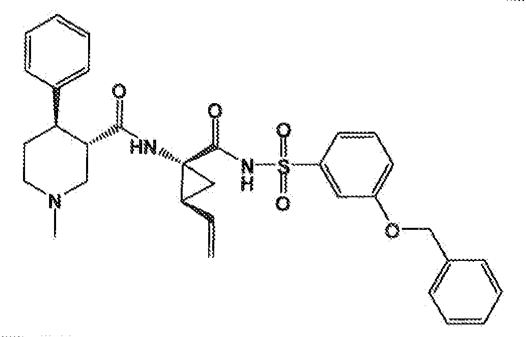 | **B-85** |
| 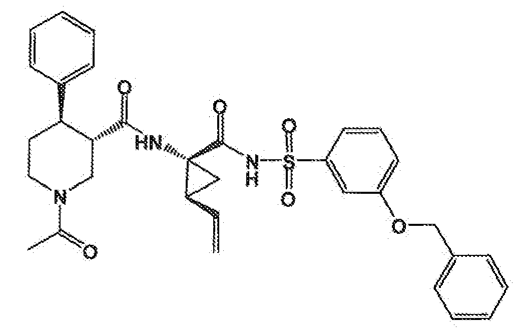 | **B-86** |
| 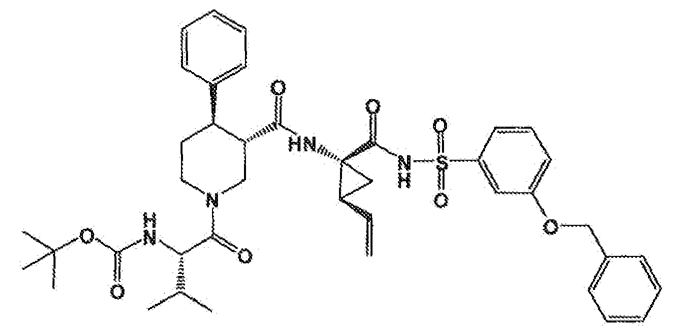 | **B-87** |
| 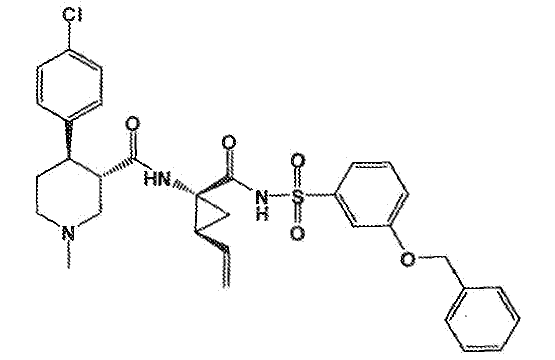 | **B-88** |
| 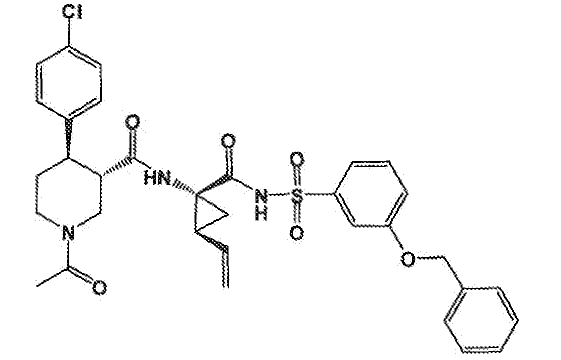 | **B-89** |
| 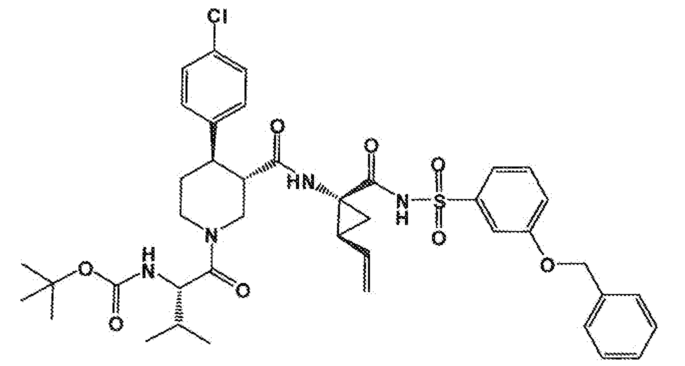 | **B-90** |
| 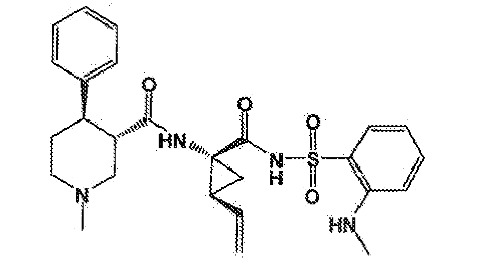 | **B-91** |
| 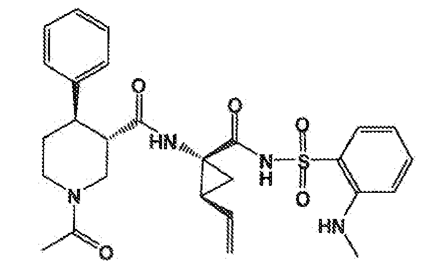 | **B-92** |
| 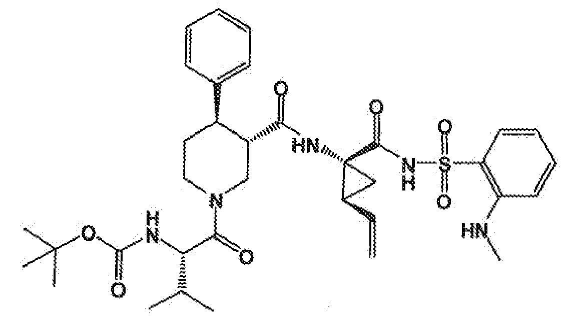 | **B-93** |
| 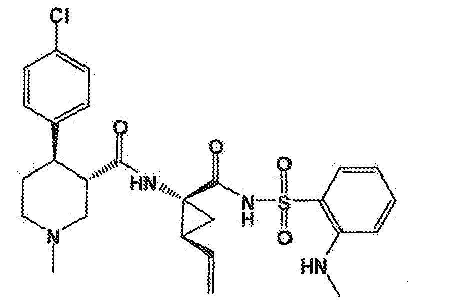 | **B-94** |
| 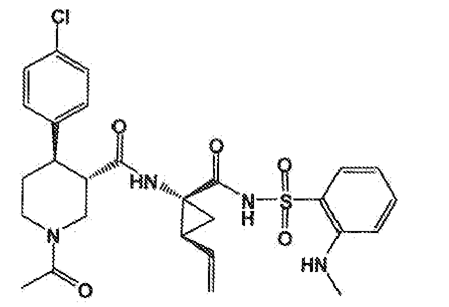 | **B-95** |
| 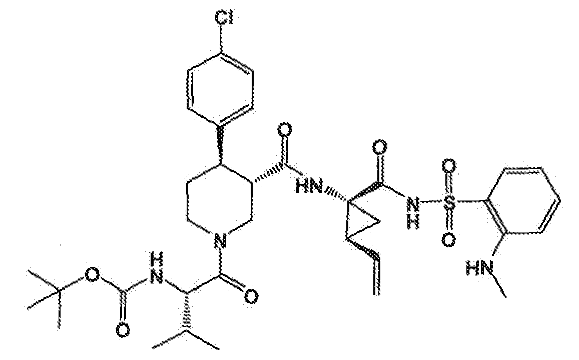 | **B-96** |
| 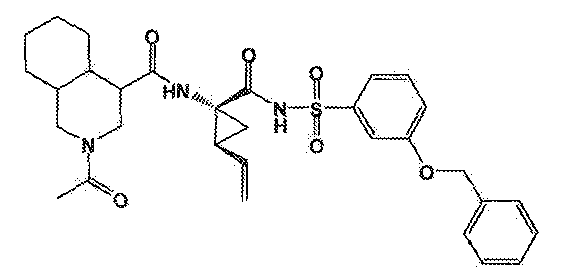 | **B-97** |
| 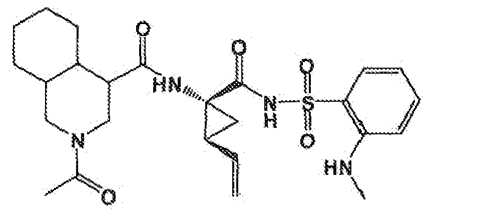 | **B-98** |
| 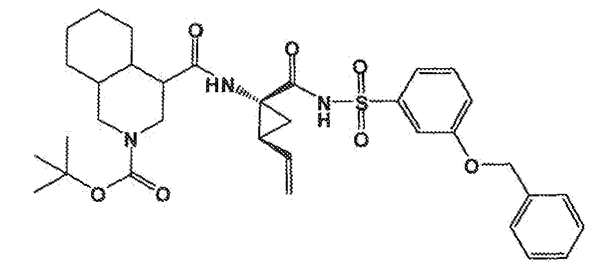 | **B-99** |
| 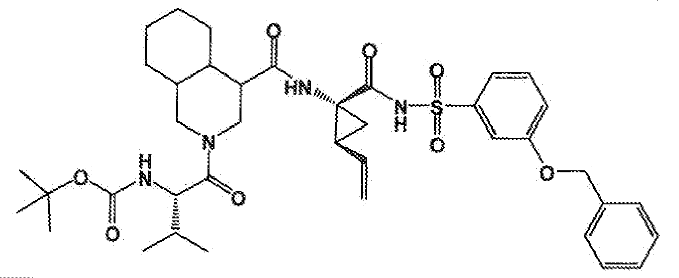 | **B-100** |
| 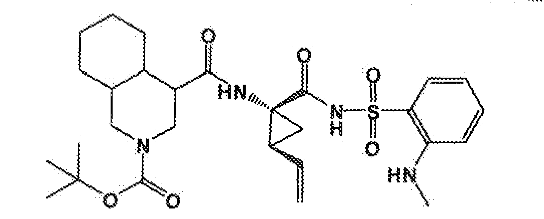 | **B-101** |
| 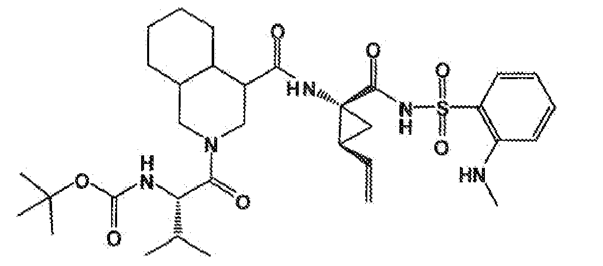 | **B-102** |
| 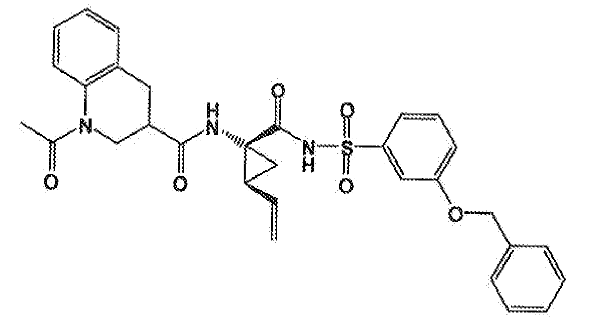 | **B-103** |
| 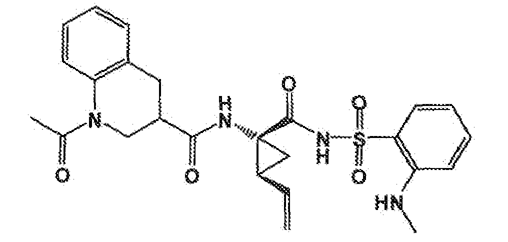 | **B-104** |
| 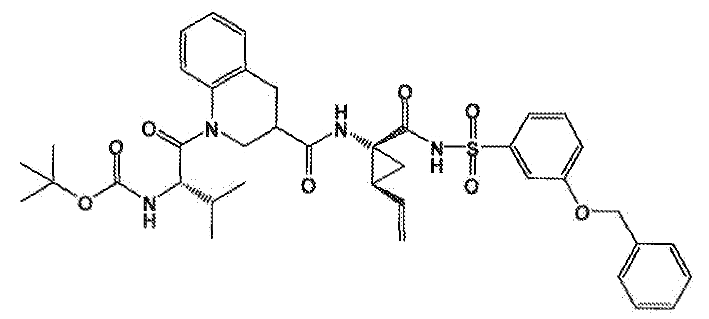 | **B-105** |
| 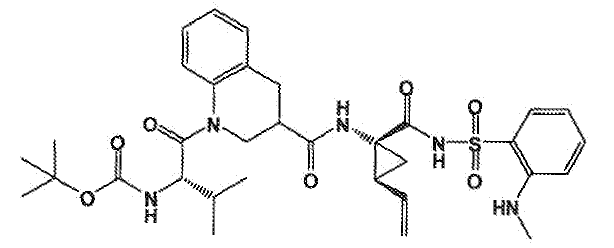 | **B-106** |
| 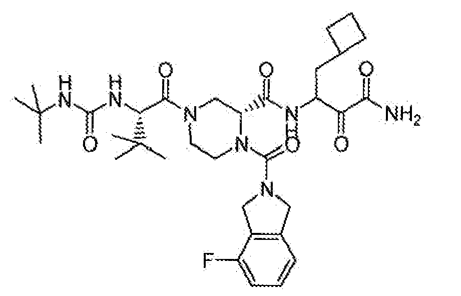 | **B-107** |

Using the HCV NS3-4A protease and Luciferase-HCV replicon assays described in the exemplification section below, the compounds of the invention (including compounds of Tables A depicted above) are found to show IC₅₀ values for HCV inhibition in the range from 10 to more than 100 µM, or 0.5 to 30 µM, including, for example, the range from 0.5 to 10 µM or less.

In certain embodiments, a compound of the present invention is further characterized as a modulator of HCV, including a mammalian HCV, and especially including a human HCV. In a preferred embodiment, the compound of the invention is an HCV inhibitor.

In certain embodiments, the compound of the invention is not VX-950 or Sch 503034 (see, *e.g*., Curr. Med. Chem., 2005, 12, 2317-2342; and Antimicrob Agents Chemother. 2006 Mar;50(3):1013-20, both of which are incorporated herein by reference in their entirety),

In other embodiments, the compounds of the invention are not the species described in International Patent Application Nos. WO 2005/058821, WO/2005/021584, WO/01/18369, WO/03/062265, WO/02/18369, WO/2003/087092 and U.S. Pat. App. No. 2002/0032175.

The terms "HCV-associated state" or "HCV-associated disorder" include disorders and states (*e.g*., a disease state) that are associated with the activity of HCV, *e.g*., infection of HCV in a subject. HCV-associated states include HCV-infection, liver cirrhosis, chronic liver disease, hepatocellular carcinoma, cryoglobulinaemia, non-Hodgkin's lymphoma, and a suppressed innate intracellular immune response.

HCV-associated states are often associated with the NS3 serine protease of HCV, which is responsible tbr several steps in the processing of the HCV polyprotein into smaller functional proteins. NS3 protease forms a heterodimeric complex with the NS4A protein, an essential cofactor that enhances enzymatic activity, and is believed to help anchor HCV to the endoplasmic reticulum. NS3 first autocatalyzes hydrolysis of the NS3-NS4A juncture, and then cleaves the HCV polyprotein intermolecularly at the NS4A-NS4B, NS4B-NS5A and NS5A-NS5B intersections. This process is associated with replication of HCV in a subject. Inhibiting or modulating the activity of one or more of the NS3, NS4A, NS4B, NS5A and NS5B proteins will inhibit or modulate replication of HCV in a subject, thereby preventing or treating the HCV-associated state. In a particular embodiment, the HCV-associated state is associated with the activity of the NS3 protease. In another particular embodiment, the HCV-associated state is associated with the activity of NS3-NS4A heterodimeric complex.

In one embodiment, the compounds of the invention are NS3/NS4A protease inhibitors. In another embodiment, the compounds of the invention are NS2/NS3 protease inhibitors.

Without being bound by theory, it is believed that the disruption of the above protein-protein interactions by the compounds of the invention will interfere with viral polyprotein processing by the NS3 protease and thus viral replication.

HCV-associated disorders also include HCV-dependent diseases. HVC-dependent diseases include, *e.g.*, any disease or disorder that depend on or related to activity or misregulation of at least one strain of HCV.

The present invention includes treatment of HCV-associated disorders as described above, but the invention is not intended to be limited to the manner by which the compound performs its intended function of treatment of a disease. The present invention includes treatment of diseases described herein in any manner that allows treatment to occur, *e.g*., HCV infection.

In a related embodiment, the compounds of the invention can be useful for treating diseases related to HIV, as well is HIV infection and AIDS (Acquired Immune Deficiency Syndrome).

In certain embodiments, the invention provides a pharmaceutical composition of any of the compounds of the present invention. In a related embodiment, the invention provides a pharmaceutical composition of any of the compounds of the present invention and a pharmaceutically acceptable carrier or excipient of any of these compounds. In certain embodiments, the invention includes the compounds as novel chemical entities.

In one embodiment, the invention includes a packaged HCV-associated disorder treatment. The packaged treatment includes a compound of the invention packaged with instructions for using an effective amount of the compound of the invention for an intended use.

The compounds of the present invention are suitable as active agents in pharmaceutical compositions that are efficacious particularly for treating HCV-associated disorders. The pharmaceutical composition in various embodiments has a pharmaceutically effective amount of the present active agent along with other pharmaceutically acceptable excipients, carriers, fillers, diluents and the like. The phrase, "pharmaceutically effective amount" as used herein indicates an amount necessary to administer to a host, or to a cell, issue, or organ of a host, to achieve a therapeutic result, especially an anti-HCV effect, *e.g*., inhibition of proliferation of the HCV virus, or of any other HCV-associated disease.

In one embodiment, the diseases to be treated by compounds of the invention include, for example, HCV infection, liver cirrhosis, chronic liver disease, hepatocellular carcinoma, cryoglobulinaemia, non-Hodgkin's lymphoma, and a suppressed innate intracellular immune response.

In other embodiments, the present invention provides a method for inhibiting the activity of HCV. The method includes contacting a cell with any of the compounds of the present invention. In a related embodiment, the method further provides that the compound is present in an amount effective to selectively inhibit the activity of one or more of the NS3, NS4A, NS4B, NS5A and NS5B proteins. In another related embodiment, the method provides that the compound is present in an amount effective to diminish the HCV RNA load in a subject.

In other embodiments, the present invention provides a use of any of the compounds of the invention for manufacture of a medicament to treat HCV infection in a subject.

In other embodiments, the invention provides a method of manufacture of a medicament, including formulating any of the compounds of the present invention for treatment of a subject.

### Definitions

The term "treat," "treated," "treating" or "treatment" includes the diminishment or alleviation of at least one symptom associated or caused by the state, disorder or disease being treated. In certain embodiments, the treatment comprises the induction of an HCV-inhibited state, followed by the activation of the HCV-modulating compound, which would in turn diminish or alleviate at least one symptom associated or caused by the HCV-associated state, disorder or disease being treated. For example, treatment can be diminishment of one or several symptoms of a disorder or complete eradication of a disorder.

The term "subject" is intended to include organisms, *e.g*., prokaryotes and eukaryotes, which are capable of suffering from or afflicted with an HCV-associated disorder. Examples of subjects include mammals, *e.g*., humans, dogs, cows, horses, pigs, sheep, goats, cats, mice, rabbits, rats, and transgenic non-human animals. In certain embodiments, the subject is a human, *e.g*., a human suffering from, at risk of suffering from, or potentially capable of suffering from an HCV-associated disorder, and for diseases or conditions described herein, *e.g*., HCV infection. In another embodiment, the subject is a cell.

The language "HCV-modulating compound," "modulator of HCV" or "HCV inhibitor" refers to compounds that modulate, *e.g*., inhibit, or otherwise alter, the activity of HCV. Similarly, an "NS3/NS4A protease inhibitor," or an "NS2/NS3 protease inhibitor" refers to a compound that modulates, *e.g*., inhibits, or otherwise alters, the interaction of these proteases with one another. Examples of HCV-modulating compounds include compounds of Formulas I and II, as well as Table A, Table B and Table C (including pharmaceutically acceptable salts thereof, as well as enantiomers, stereoisomers, rotamers, tautomers, diastereomers, or racemates thereof).

Additionally, the method includes administering to a subject an effective amount of an HCV-modulating compound of the invention, *e.g*., HCV-modulating compounds of Formulas I and II, as well as Table A, Table B and Table C (including pharmaceutically acceptable salts thereof, as well as enantiomers, stereoisomers, rotamers, tautomers, diastereomers, or racemates thereof).

The term "alkyl" includes saturated aliphatic groups, including straight-chain alkyl groups (*e.g*., methyl, ethyl, propyl, butyl, pentyl, hexyl, heptyl, octyl, nonyl, decyl, *etc*.)., branched-chain alkyl groups (isopropyl, tert-butyl, isobutyl, *etc*.), cycloalkyl (alicyclic) groups (cyclopropyl, cyclopentyl, cyclohexyl, cycloheptyl, cyclooctyl), alkyl substituted cycloalkyl groups, and cycloalkyl substituted alkyl groups. The term "alkyl" also includes alkenyl groups and alkynyl groups. Furthermore, the expression "Cₓ-C_{y}-alkyl", wherein x is 1-5 and y is 2-10 indicates a particular alkyl group (straight- or branched-chain) of a particular range of carbons. For example, the expression C₁-C₄-alkyl includes, but is not limited to, methyl, ethyl, propyl, butyl, isopropyl, tert-butyl and isobutyl, Moreover, the term C₃₋₆-cycloalkyl includes, but is not limited to, cyclopropyl, cyclopentyl, and cyclohexyl. As discussed below, these alkyl groups, as well as cycloalkyl groups, may be further substituted.

The term alkyl further includes alkyl groups which can further include oxygen, nitrogen, sulfur or phosphorous atoms replacing one or more carbons of the hydrocarbon backbone. In an embodiment, a straight chain or branched chain alkyl has 10 or fewer carbon atoms in its backbone (*e.g*., C₁-C₁₀ for straight chain, C₃-C₁₀ for branched chain), and more preferably 6 or fewer carbons. Likewise, preferred cycloalkyls have from 4-7 carbon atoms in their ring structure, and more preferably have 5 or 6 carbons in the ring structure.

Moreover, alkyl (*e.g*., methyl, ethyl, propyl, butyl, pentyl, hexyl, *etc*.) include both "unsubstituted alkyl" and "substituted alkyl", the latter of which refers to alkyl moieties having substituents replacing a hydrogen on one or more carbons of the hydrocarbon backbone, which allow the molecule to perform its intended function.

The term "substituted" is intended to describe moieties having substituents replacing a hydrogen on one or more atoms, *e.g.* C, O or N, of a molecule. Such substituents can include, for example, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, morpholino, phenol, benzyl, phenyl, piperizine, cyclopentane, cyclohexane, pyridine, 5H-tetrazole, triazole, piperidine, or an aromatic or heteroaromatic moiety.

Further examples of substituents of the invention, which are not intended to be limiting, include moieties selected from straight or branched alkyl (preferably C₁-C₅), cycloalkyl (preferably C₃-C₈), alkoxy (preferably C₁-C₆), thioalkyl (preferably C₁-C₆), alkenyl (preferably C₂-C₆), alkynyl (preferably C₂-C₆), heterocyclic, carbocyclic, aryl (*e*.*g*., phenyl), aryloxy (*e.g*., phenoxy), aralkyl (*e.g.,* benzyl), aryloxyalkyl (*e.g.,* phenyloxyalkyl), arylacetamidoyl, alkylaryl, heteroaralkyl, alkylcarbonyl and arylcarbonyl or other such acyl group, heteroarylcarbonyl, or heteroaryl group, (CR'R")₀₋₃NR'R" (*e.g*., -NH₂), (CR'R")₀₋₃CN (*e.g*., -CN), -NO₂, halogen (*e.g., -*F, -Cl, -Br, or -1), (CR'R")₀₋₃C(halogen)₃ (*e.g., -*CF₃), (CR'R")₀₋₃CH(halogen)₂, (CR'R")₀₋₃CH₂(halogen), (CR'R")₀₋₃CONR'R", (CR'R")₀₋₃(CNH)NR'R", (CR'R")₀₋₃S(O)₁₋₂NR'R", (CR'R")₀₋₃CHO, (CR'R")₀₋₃O(CR'R")₀₋₃H, (CR'R")₀₋₃S(O)₀₋₃R' (*e.g*., -SO₃H, -OSO₃H), (CR'CR")₀₋₃O(CR'R")₀₋₃H (*e.g.,* -CH₂OCH₃ and -OCH₃), (CR'R")₀₋₃S(CR'R")₀₋₃H (*e.g*., -SH and -SCH₃), (CR'R")₀₋₃OH (*e.g*., -OH), (CR'R")₀₋₃COR', (CR'R")₀₋₃(substituted or unsubstituted phenyl), (CR'R")₀₋₃(C₃-C₈ cycloalkyl), (CR'R")₀₋₃CO₂R' (*e.g.,* -CO₂H), or (CR'R")₀₋₃₀R' group, or the side chain of any naturally occurring amino acid; wherein R' and R" are each independently hydrogen, a C₁-C₅ alkyl, C₂-C₅ alkenyl, C₂-C₅ alkynyl, or aryl group. Such substituents can include, for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, oxime, sulfhydryl, alkylthio, arylthio, thiocarboxylate, sulfates, sulfonato, sulfamoyl, sulfonamido, nitro, trifluoromethyl, cyano, azido, heterocyclyl, or an aromatic or heteroaromatic moiety. In certain embodiments, a carbonyl moiety (C=O) may be further derivatized with an oxime moiety, *e.g*., an aldehyde moiety may be derivatized as its oxime (-C=N-OH) analog. It will be understood by those skilled in the art that the moieties substituted on the hydrocarbon chain can themselves be substituted, if appropriate. Cycloalkyls can be further substituted, *e.g*., with the substituents described above. An "aralkyl" moiety is an alkyl substituted with an aryl (*e.g*., phenylmethyl (*i.e*., benzyl)).

The term "alkenyl" includes unsaturated aliphatic groups analogous in length and possible substitution to the alkyls described above, but which contain at least one double bond.

For example, the term "alkenyl" includes straight-chain alkenyl groups (*e.g*., ethenyl, propenyl, butenyl, pentenyl, hexenyl, heptenyl, octenyl, nonenyl, decenyl, etc.), branched-chain alkenyl groups, cycloalkenyl (alicyclic) groups (cyclopropenyl, cyclopentenyl, cyclohexenyl, cycloheptenyl, cyclooctenyl), alkyl or alkenyl substituted cycloalkenyl groups, and cycloalkyl or cycloalkenyl substituted alkenyl groups. The term alkenyl further includes alkenyl groups that include oxygen, nitrogen, sulfur or phosphorous atoms replacing one or more carbons of the hydrocarbon backbone. In certain embodiments, a straight chain or branched chain alkenyl group has 6 or fewer carbon atoms in its backbone (*e.g.,* C₂-C₆ for straight chain, C₃-C₆ for branched chain). Likewise, cycloalkenyl groups may have from 3-8 carbon atoms in their ring structure, and more preferably have 5 or 6 carbons in the ring structure. The term C₂-C₆ includes alkenyl groups containing 2 to 6 carbon atoms.

Moreover, the term alkenyl includes both "unsubstituted alkenyls" and "substituted alkenyls", the latter of which refers to alkenyl moieties having substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamio, and alkylarylamino), acylamino (including alkycarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

The term "alkynyl" includes unsaturated aliphatic groups analogous in length and possible substitution to the alkyls described above, but which contain at one triple bond.

For example, the term "alkynyl" includes straight-chain alkynyl groups (*e*.*g*., ethynyl, propynyl, butynyl, pentynyl, hexynyl, heptynyl, octynyl, nonynyl, decynyl, *etc*.), branched-chain alkynyl groups, and cycloalkyl or cycloalkenyl substituted alkynyl groups. The term alkynyl further includes alkynyl groups that include oxygen, nitrogen, sulfur or phosphorous atoms replacing one or more carbons of the hydrocarbon backbone. In certain embodiments, a straight chain or branched chain alkynyl group has 6 or fewer carbon atoms in its backbone (*e.g*., C₂-C₆ for straight chain. C₃-C₆ for branched chain). The term C₂-C₆ includes alkynyl groups containing 2 to 6 carbon atoms.

Moreover, the term alkynyl includes both "unsubstituted alkynyls" and "substituted alkynyls", the latter of which refers to alkynyl moieties having substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

The term "amine" or "amino" should be understood as being broadly applied to both a molecule, or a moiety or functional group, as generally understood in the art, and may be primary, secondary, or tertiary. The term "amine" or "amino" includes compounds where a nitrogen atom is covalently bonded to at least one carbon, hydrogen or heteroatom. The terms include, for example, but are not limited to, "alkylamino," "arylamino," "diarylamino," "alkylarylamino," alkylaminoaryl." "arylaminoalkyl," "alkaminoalkyl," "amide," "amido," and "aminocarbonyl." The term "alkyl amino" comprises groups and compounds wherein the nitrogen is bound to at least one additional alkyl group, The term "dialkyl amino" includes groups wherein the nitrogen atom is bound to at least two additional alkyl groups. The term "arylamino" and "diarylamino" include groups wherein the nitrogen is bound to at least one or two aryl groups, respectively. The term "alkylarylamino," "alkylaminoaryl" or "arylaminoalkyl" refers to an amino group which is bound to at least one alkyl group and at least one aryl group. The term "alkaminoalkyl" refers to an alkyl, alkenyl, or alkynyl group bound to a nitrogen atom which is also bound to an alkyl group.

The term "amide," "amido" or "aminocarbonyl" includes compounds or moieties which contain a nitrogen atom which is bound to the carbon of a carbonyl or a thiocarbonyl group. The term includes "alkaminocarbonyl" or "alkylaminocarbonyl" groups which include alkyl, alkenyl, aryl or alkynyl groups bound to an amino group bound to a carbonyl group. It includes arylaminocarbonyl and arylcarbonylamino groups which include aryl or heteroaryl moieties bound to an amino group which is bound to the carbon of a carbonyl or thiocarbonyl group. The terms "alkylaminocarbonyl," "alkenylaminocarbonyl," "alkynylaminocarbonyl," "arylaminocarbonyl," "alkylcarbonylamino," "alkenylcarbonylamino," "alkynylcarbonylamino," and "arylcarbonylamino" are included in term "amide." Amides also include urea groups (aminocarbonylamino) and carbamates (oxycarbonylamino).

The term "aryl" includes groups, including 5- and 6-membered single-ring aromatic groups that may include from zero to four heteroatoms, for example, phenyl, pyrrole, furan, thiophene, thiazole, isothiaozole, imidazole, triazole, tetrazole, pyrazole, oxazole, isoxazole, pyridine, pyrazine, pyridazine, and pyrimidine, and the like. Furthermore, the term "aryl" includes multicyclic aryl groups, *e.g*., tricyclic, bicyclic, *e.g*., naphthalene, benzoxazole, benzodioxazole, benzothiazole, benzoimidazole, benzothiophene, methylenedioxyphenyl, quinoline, isoquinoline, anthryl, phenanthryl, napthridine, indole, benzofuran, purine, benzofuran, deazapurine, or indolizine, Those aryl groups having heteroatoms in the ring structure may also be referred to as "aryl heterocycles", "heterocycles," "heteroaryls" or "heteroaromatics." The aromatic ring can be substituted at one or more ring positions with such substituents as described above, as for example, alkyl, halogen, hydroxyl, alkoxy, alkylcarbonyloxy, arylcarbonyloxy, alkoxycarbonyloxy, aryloxycarbonyloxy, carboxylate, alkylcarbonyl, alkylaminoacarbonyl, aralkylaminocarbonyl, alkenylaminocarbonyl, alkylcarbonyl, arylcarbonyl, aralkylcarbonyl,alkenylcarbonyl, alkoxycarbonyl, aminocarbonyl, alkylthiocarbonyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfilnyl, sulfonato, sulfamoyl, sulfonamido, nitro, trifluoromethyl, cyano, azido, heterocyclyl, alkylaryl, or an aromatic or heteroaromatic moiety. Aryl groups can also be fused or bridged with alicyclic or heterocyclic rings which are not aromatic so as to form a polycycle (*e.g*., tetralin).

The term heteroaryl, as used herein, represents a stable monocyclic or bicyclic ring of up to 7 atoms in each ring, wherein at least one ring is aromatic and contains from I to 4 heteroatoms selected from the group consisting of O, N and S. Heteroaryl groups within the scope of this definition include but are not limited to: acridinyl, carbazolyl, cinnolinyl, quinoxalinyl, pyrrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, tetrahydroquinoline. As with the definition of heterocycle below, "heteroaryl" is also understood to include the N-oxide derivative of any nitrogen-containing heteroaryl, In cases where the heteroaryl substituent is bicyclic and one ring is non-aromatic or contains no heteroatoms, it is understood that attachment is via the aromatic ring or via the heteroatom containing ring, respectively.

The term "heterocycle" or "heterocyclyl" as used herein is intended to mean a 5-to 10-membered aromatic or nonaromatic heterocycle containing from 1 to 4 heteroatoms selected from the group consisting of O, N and S, and includes bicyclic groups. "Heterocyclyl" therefore includes the above mentioned heteroaryls, as well as dihydro and tetrathydro analogs thereof. Further examples of "heterocyclyl" include, but are not limited to the following: benzoimidazolyl, benzofuranyl, benzofurazanyl, benzopyrazolyl, benzotriazolyl, benzothiophenyl, benzoxazolyl, carbazotyl, carbolinyl, cinnolinyl, furanyl, imidazolyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, oxazoline, isoxazoline, oxetanyl, pyranyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrahydropyranyl, tetrazolyl, tetrazolopyridyl, thiadiazolyl, thiazolyl, thienyl, triazolyl,azetidinyl, 1,4-dioxanyl, hexahydroazepinyl, piperazinyl, piperidinyl, pyridin-2-onyl, pyrrolidinyl, morpholinyl, thiomorpholinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenzoyl, tetrahydrofuranyl, and tetrahydrothienyl, and N-oxides thereof. Attachment of a heterocyclyl substituent can occur via a carbon atom or via a heteroatom,

The term "acyl" includes compounds and moieties which contain the acyl radical (CH₃CO-) or a carbonyl group. The term "substituted acyl" includes acyl groups where one or more of the hydrogen atoms are replaced by for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

The term "acylamino" includes moieties wherein an acyl moiety is bonded to an amino group. For example, the term includes alkylcarbonylamino, arylcarbonylamino, carbamoyl and ureido groups.

The term "alkoxy" includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups and may include cyclic groups such as cyclopentoxy. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, amino, sulfhydryl, alkylthio, arylthio, thiocarboxylate, sulfates, alkylsulfinyl, sulfonato, sulfamoyl, sulfunamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, *etc*.

The term "carbonyl" or "carboxy" includes compounds and moieties which contain a carbon connected with a double bond to an oxygen atom, and tautomeric forms thereof. Examples of moieties that contain a carbonyl include aldehydes, ketones, carboxylic acids, amides, esters, anhydrides, *etc*. The term "carboxy moiety" or "carbonyl moiety" refers to groups such as "alkylcarbonyl" groups wherein an alkyl group is covalently bound to a carbonyl group, "alkenylcarbonyl" groups wherein an alkenyl group is covalently bound to a carbonyl group, "alkynylcarbonyl" groups wherein an alkynyl group is covalently bound to a carbonyl group, "arylcarbonyl" groups wherein an aryl group is covalently attached to the carbonyl group. Furthermore, the term also refers to groups wherein one or more heteroatoms are covalently bonded to the carbonyl moiety. For example, the term includes moieties such as, for example, aminocarbonyl moieties, (wherein a nitrogen atom is bound to the carbon of the carbonyl group, *e.g*., an amide), aminocarbonyloxy moieties, wherein an oxygen and a nitrogen atom are both bond to the carbon of the carbonyl group (*e.g.*, also referred to as a "carbamate"). Furthermore, aminocarbonylamino groups (*e.g*., ureas) are also include as well as other combinations of carbonyl groups bound to heteroatoms (*e.g*., nitrogen, oxygen, sulfur, *etc,* as well as carbon atoms). Furthermore, the heteroatom can be further substituted with one or more alkyl, alkenyl, alkynyl, aryl, aralkyl, acyl, *etc*. moieties.

The term "thiocarbonyl" or "thiocarboxy" includes compounds and moieties which contain a carbon connected with a double bond to a sulfur atom. The term "thiocarbonyl moiety" includes moieties that are analogous to carbonyl moieties. For example, "thiocarbonyl" moieties include aminothiocarbonyl, wherein an amino group is bound to the carbon atom of the thiocarbonyl group, furthermore other thiocarbonyl moieties include, oxythiocarbonyls (oxygen bound to the carbon atom), aminothiocarbonylamino groups, *etc*.

The term "ether" includes compounds or moieties that contain an oxygen bonded to two different carbon atoms or heteroatoms. For example, the term includes "alkoxyalkyl" which refers to an alkyl, alkenyl, or alkenyl group covalently bonded to an oxygen atom that is covalently bonded to another alkyl group.

The term "ester" includes compounds and moieties that contain a carbon or a heteroatom bound to an oxygen atom that is bonded to the carbon of a carbonyl group. The term "ester" includes alkoxycarboxy groups such as methoxycarbonyl, ethoxycarbonyl, propoxycarbonyl, butoxycarbonyl, pentoxycarbonyl, *etc*. The alkyl, alkenyl, or alkynyl groups are as defined above.

The term "thioether" includes compounds and moieties which contain a sulfur atom bonded to two different carbon or hetero atoms. Examples of thioethers include, but are not limited to alkthioalkyls, alkthioalkenyls, and alkthioalkynyls. The term "alkthioalkyls" include compounds with an alkyl, alkenyl, or alkynyl group bonded to a sulfur atom that is bonded to an alkyl group. Similarly, the term "alkthioalkenyls" and alkthioalkynyls" refer to compounds or moieties wherein an alkyl, alkenyl, or alkynyl group is bonded to a sulfur atom which is covalently bonded to an alkynyl group.

The term "hydroxy" or "hydroxyl" includes groups with an -OH or -O.

The term "halogen" includes fluorine, bromine, chlorine, iodine, *etc*. The term "perhalogenated" generally refers to a moiety wherein all hydrogens are replaced by halogen atoms.

The terms "polycyclyl" or "polycyclic radical" include moieties with two or more rings (*e.g*., cycloalkyls, cycloalkenyls, cycloalkynyls, aryls and/or heterocyclyls) in which two or more carbons are common to two adjoining rings, *e.g.*, the rings are "fused rings". Rings that are joined through non-adjacent atoms are termed "bridged" rings. Each of the rings of the polycycle can be substituted with such substituents as described above, as for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, alkylaminoacarbonyl, aralkylaminocarbonyl, alkenylaminocarbonyl, alkylcarbonyl, arylcarbonyl, aralkylcarbonyl, alkenylcarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkyl, alkylaryl, or an aromatic or heteroaromatic moiety.

The term "heteroatom" includes atoms of any element other than carbon or hydrogen. Preferred heteroatoms are nitrogen, oxygen, sulfur and phosphorus.

Additionally, the phrase "any combination thereof" implies that any number of the listed functional groups and molecules may be combined to create a larger molecular architecture. For example the terms "phenyl," "carbonyl" (or "=O"), "-O-," "-OH," and C₁₋₆ (*i.e.*, -CH₃ and -CH₂CH₂CH₂-) can be combined to form a 3-methoxy-4-propoxybenzoic acid substituent. It is to be understood that when combining functional groups and molecules to create a larger molecular architecture, hydrogens can be removed or added, as required to satisfy the valence of each atom.

It is to be understood that all of the compounds of the invention described above will further include bonds between adjacent atoms and/or hydrogens as required to satisfy the valence of each atom. That is, double bonds and/or hydrogen atoms are added to provide the following number of total bonds to each of the following types of atoms: carbon: four bonds; nitrogen: three bonds; oxygen; two bonds; and sulfur; two bonds.

It is also to be understood that definitions given to the variables of the generic formulae described herein (*e.g*., Formulas I and II) will result in molecular structures that are in agreement with standard organic chemistry definitions and knowledge, *e.g*., valency rules.

It will be noted that the structures of some of the compounds of this invention include asymmetric carbon atoms. It is to be understood accordingly that the isomers arising from such asymmetry (*e.g*., all enantiomers, stereoisomers, rotamers, tautomers, diastereomers, or racemates) are included within the scope of this invention. Such isomers can be obtained in substantially pure form by classical separation techniques and by stereochemically controlled synthesis. Furthermore, the structures and other compounds and moieties discussed in this application also include all tautomers thereof. Compounds described herein may be obtained through art recognized synthesis strategies.

It will be noted that the substituents of some of the compounds of this invention include isomeric cyclic structures. It is to be understood accordingly that constitutional isomers of particular substituents are included within the scope of this invention, unless indicated otherwise. For example, the term "tetrazole" includes tetrazole, 2*H*-tetrazole, 3*H*-tetrazole, 4*H*-tetrazole and 5*H*-tetrazole.

### Use in HCV-associated disorders

The compounds of the present invention have valuable pharmacological properties and are useful in the treatment of diseases. In certain embodiments, compounds of the invention are useful in the treatment of HCV-associated disorders, *e.g*., as drugs to treat HCV infection.

The term "use" includes any one or more of the following embodiments of the invention, respectively; the use in the treatment of HCV-associated disorders; the use for the manufacture of pharmaceutical compositions for use in the treatment of these diseases, *e.g*., in the manufacture of a medicament; methods of use of compounds of the invention in the treatment of these diseases; pharmaceutical preparations having compounds of the invention for the treatment of these diseases; and compounds of the invention for use in the treatment of these diseases; as appropriate and expedient, if not stated otherwise. In particular, diseases to be treated and are thus preferred for use of a compound of the present invention are selected from HCV-associated disorders, including those corresponding to HCV-infection, as well as those diseases that depend on the activity of one or more of the NS3, NS4A, NS4B, NS5A and NS5B proteins, or a NS3-NS4A, NS4A-NS4B, NS4B-NS5A or NS5A-NS5B complex. The term "use" further includes embodiments of compositions herein which bind to an HCV protein sufficiently to serve as tracers or labels, so that when coupled to a fluorine or tag, or made radioactive, can be used as a research reagent or as a diagnostic or an imaging agent.

In certain embodiments, a compound of the present invention is used for treating HCV-associated diseases, and use of the compound of the present invention as an inhibitor of any one or more HCVs. It is envisioned that a use can be a treatment of inhibiting one or more strains of HCV.

### Assays

The inhibition of HCV activity may be measured as using a number of assays available in the art. An example of such an assay can be found in Anal Biochem. 1996 240(1): 60-7; which is incorporated by reference in its entirety. Assays for measurement of HCV activity are also described in the experimental section below.

### Pharmaceutical Compositions

The language "effective amount" of the compound is that amount necessary or sufficient to treat or prevent an HCV-associated disorder, *e.g.* prevent the various morphological and somatic symptoms of an HCV-associated disorder, and/or a disease or condition described herein. In an example, an effective amount of the HCV -modulating compound is the amount sufficient to treat HCV infection in a subject. In another example, an effective amount of the HCV-modulating compound is the amount sufficient to treat HCV infection, liver cirrhosis, chronic liver disease, hepatocellular carcinoma, cryoglobulinaemia, non-Hodgkin's lymphoma, and a suppressed innate intracellular immune response in a subject. The effective amount can vary depending on such factors as the size and weight of the subject, the type of illness, or the particular compound of the invention. For example, the choice of the compound of the invention can affect what constitutes an "effective amount." One of ordinary skill in the art would be able to study the factors contained herein and make the determination regarding the effective amount of the compounds of the invention without undue experimentation.

The regimen of administration can affect what constitutes an effective amount. The compound of the invention can be administered to the subject either prior to or after the onset of an HCV-associated state. Further, several divided dosages, as well as staggered dosages, can be administered daily or sequentially, or the dose can be continuously infused, or can be a bolus injection. Further, the dosages of the compound(s) of the invention can be proportionally increased or decreased as indicated by the exigencies of the therapeutic or prophylactic situation,

Compounds of the invention may be used in the treatment of states, disorders or diseases as described herein, or for the manufacture of pharmaceutical compositions for use in the treatment of these diseases. Methods of use of compounds of the present invention in the treatment of these diseases, or pharmaceutical preparations having compounds of the present invention for the treatment of these diseases.

The language "pharmaceutical composition" includes preparations suitable for administration to mammals, *e.g*., humans. When the compounds of the present invention are administered as pharmaceuticals to mammals, *e.g.,* humans, they can be given per se or as a pharmaceutical composition containing, for example, 0.1 to 99.5% (more preferably, 0.5 to 90%) of active ingredient in combination with a pharmaceutically acceptable carrier.

The phrase "pharmaceutically acceptable carrier" is art recognized and includes a pharmaceutically acceptable material, composition or vehicle, suitable for administering compounds of the present invention to mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include; sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include; water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, α-tocopherol, and the like; and metal chelating agents, such as citric acid, ethylenediamine tetraacetic acid (EDTA), sorbitol, tartaric acid, phosphoric acid, and the like.

Formulations of the present invention include those suitable for oral, nasal, topical, transdermal, buccal, sublingual, rectal, vaginal and/or parenteral administration. The formulations may conveniently be presented in unit dosage form and may be prepared by any methods well known in the art of pharmacy. The amount of active ingredient that can be combined with a carrier material to produce a single dosage form will generally be that amount of the compound that produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a favored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinylpyrrolidone, sucrose and/or acacia; humectants, such as glycerol: disintegrating agents, such as agsr-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmacetical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteris-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluent commonly used in the art, such as, for example, water or other solvents, solubilizing agents and emulsifiers, such as ethyl alcohol, isopropyl, alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, tale, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers call also be used to increase the flux of the compound across the skin. The rate of such flux can be controlled by either providing a rate controlling membrane or dispersing the active compound in a polymer matrix or gel.

Ophthalmic formulations, eye ointments, powders, solutions and the like, are also contemplated as being within the scope of this invention.

Pharmaceutical compositions of this invention suitable for parenteral administration comprise one or more compounds of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption (such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

The preparations of the present invention may be given orally, parenterally, topically, or rectally. They are of course given by forms suitable for each administration route. For example, they are administered in tablets or capsule form, by injection, inhalation, eye lotion, ointment, suppository, *etc*., administration by injection, infusion or inhalation; topical by lotion or ointment: and rectal by suppositories. Oral administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound that is the lowest dose effective to produce a therapeutic effect. Such an effective dose will generally depend upon the factors described above. Generally, intravenous and subcutaneous doses of the compounds of this invention for a patient, when used for the indicated analgesic effects, will range from about 0.0001 to about 100 mg per kilogram of body weight per day, more preferably from about 0.01 to about 50 mg per kg per day, and still more preferably from about 1.0 to about 100 mg per kg per day. An effective amount is that amount treats an HCV-associated disorder.

If desired, the elective daily dose of the active compound may be administered as two, three, four, five, six or more sub-doses administered separately at appropriate intervals throughout the day, optionally, in unit dosage forms.

While it is possible for a compound of the present invention to be administered alone, it is preferable to administer the compound as a pharmaceutical composition.

### Synthetic Procedure

Compounds of the present invention are prepared from commonly available compounds using procedures known to those skilled in the art, including any one of more of the following conditions without limitation:
Within the scope of this text, only a readily removable group that is not a constituent of the particular desired end product of the compounds of the present invention is designated a "protecting group," unless the context indicates otherwise. The protection of functional groups by such protecting groups, the protecting groups themselves, and their cleavage reactions are described for example in standard reference works, such as *e.g*., Science of Synthesis: Houben-Weyl Methods of Molecular Transformation. Georg Thieme Verlag, Stuttgart, Germany, 2005, 41627 pp. (URL: http://www.science-of-synthesis.com (Electronic Version, 48 Volumes)); J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/1, Georg Thieme Verlag, Stuttgart 1974, in H.-D. Jakubke and H. Jeschkeit, "Aminosäuren, Peptide, Proteine" (Amino acids, Peptides, Proteins), Verlag Chemie, Weinheim, Deerfield Beach, and Basel 1982, and in Jochen Lehmann, "Chemie der Kohlenhydrate: Monosaccharide und Derivate" (Chemistry of Carbohydrates: Monosaccharides and Derivatives), Georg Thieme Verlag, Stuttgart 1974. A characteristic of protecting groups is that they can be removed readily (*i.e*., without the occurrence of undesired secondary reactions) for example by solvolysis, reduction, photolysis or alternatively under physiological conditions (*e.g*., by enzymatic cleavage).

Salts of compounds of the present invention having at least one salt-forming group may be prepared in a manner known per se. For example, salts of compounds of the present invention having acid groups may be formed, for example, by treating the compounds with metal compounds, such as alkali metal salts of suitable organic carboxylic acids, *e.g*., the sodium salt of 2-ethylhexanoic acid, with organic alkali metal or alkaline earth metal compounds, such as the corresponding hydroxides, carbonates or hydrogen carbonates, such as sodium or potassium hydroxide, carbonate or hydrogen carbonate, with corresponding calcium compounds or with ammonia or a suitable organic amine, stoichiometric amounts or only a small excess of the salt-forming agent preferably being used. Acid addition salts of compounds of the present invention are obtained in customary manner, *e.g*., by treating the compounds with an acid or a suitable anion exchange reagent. Internal salts of compounds of the present invention containing acid and basic salt-forming groups, *e.g*., a free carboxy group and a free amino group, may be formed, *e.g*., by the neutralisation of salts, such as acid addition salts, to the isoelectric point, *e.g*., with weak bases, or by treatment with ion exchangers.

Salts can be converted in customary manner into the free compounds; metal and ammonium salts can be converted, for example, by treatment with suitable acids, and acid addition salts, for example, by treatment with a suitable basic agent.

Mixtures of isomers obtainable according to the invention can be separated in a manner known per se into the individual isomers; diastereoisomers can be separated, for example, by partitioning between polyphasic solvent mixtures, recrystallisation and/or chromatographic separation, for example over silica gel or by, *e.g*., medium pressure liquid chromatography over a reversed phase column, and racemates can be separated, for example, by the formation of salts with optically pure salt-forming reagents and separation of the mixture of diastereoisomers so obtainable, for example by means of fractional crystallisation, or by chromatography over optically active column materials.

Intermediates and final products can be worked up and/or purified according to standard methods, *e.g*., using chromatographic methods, distribution methods, (re-) crystallization, and the like.

### General process conditions

The following applies in general to all processes mentioned throughout this disclosure.

The process steps to synthesize the compounds of the invention can be carried out under reaction conditions that are known per se, including those mentioned specifically, in the absence or, customarily, in the presence of solvents or diluents, including, for example, solvents or diluents that are inert towards the reagents used and dissolve them, in the absence or presence of catalysts, condensation or neutralizing agents, for example ion exchangers, such as cation exchangers, *e*.*g*., in the H⁺ form, depending on the nature of the reaction and/or of the reactants at reduced, normal or elevated temperature, for example in a temperature range of from about -100 °C to about 190°C, including, for example, from approximately -80°C to approximately 150°C, for example at from -80 to - 60°C, at room temperature, at from -20 to 40°C or at reflux temperature, under atmospheric pressure or in a closed vessel, where appropriate under pressure, and/or in an inert atmosphere, for example under an argon or nitrogen atmosphere.

At all stages of the reactions, mixtures of isomers that are formed can be separated into the individual isomers, for example diastereoisomers or enantiomers, or into any desired mixtures of isomers, for example racemates or mixtures of diastereoisomers, for example analogously to the methods described in Science of Synthesis: Houben-Weyl Methods of Molecular Transformation, Georg Thieme Verlag, Stuttgart, Germany. 2005.

The solvents from which those solvents that are suitable for any particular reaction may be selected include those mentioned specifically or, for example, water, esters, such as lower alkyl-lower alkanoates, for example ethyl acetate, ethers, such as aliphatic ethers, for example diethyl ether, or cyclic ethers, for example tetrahydrofurane or dioxane, liquid aromatic hydrocarbons, such as benzene or toluene, alcohols, such as methanol, ethanol or 1- or 2-propanol, nitriles, such as acetonitrile, halogenated hydrocarbons, such as methylene chloride or chloroform, acid amides, such as dimethylformamide or dimethyl acetamide, bases, such as heterocyclic nitrogen bases, for example pyridine or N-methylpyrrolidin-2-one, carboxylic acid anhydride, such as lower alkanoic acid anhydrides, for example acetic anhydride, cyclic, linear or branched hydrocarbons, such as cyclohexane, hexane or isopentane, or mixtures of those solvents, for example aqueous solutions, unless otherwise indicated in the description of the processes. Such solvent mixtures may also be used in working up, for example by chromatography or partitioning.

The compounds, including their salts, may also be obtained in the form of hydrates, or their crystals may, for example, include the solvent used for crystallization. Different crystalline forms may be present.

The invention relates also to those forms of the process in which a compound obtainable as an intermediate at any stage of the process is used as starting material and the remaining process steps are carried out, or in which a starting material is formed under the reaction conditions or is used in the form of a derivative, for example in a protected form or in the form of a salt, or a compound obtainable by the process according to the invention is produced under the process conditions and processed further in situ,

### Pro-drugs

The present invention also relates to pro-drugs of a compound of the present invention that are converted in vivo to the compounds of the present invention as described herein. Any reference to a compound of the present invention is therefore to be understood as referring also to the corresponding pro-drugs of the compound of the present invention, as appropriate and expedient.

### Combinations

A compound of the present invention may also be used in combination with other agents, *e.g*., an additional HCV-modulating compound that is or is not of the formula 1, for treatment of and HCV-associated disorder in a subject.

By the term "combination", is meant either a fixed combination in one dosage unit form, or a kit of parts for the combined administration where a compound of the present invention and a combination partner may be administered independently at the same time or separately within time intervals that especially allow that the combination partners show a cooperative, *e.g*., synergistic, effect, or any combination thereof.

For example, WO 2005/042020, incorporated herein by reference in its entirety, describes the combination of various HCV inhibitors with a cytochrome P450 ("CYP") inhibitor. Any CYP inhibitor that improves the pharmacokinetics of the relevant NS3/4A protease may be used in combination with the compounds of this invention. These CYP inhibitors include, but are not limited to, ritonavir (WO 94/14436, incorporated herein by reference in its entirety), ketoconazole, troleandomycin, 4-methyl pyrazole, cyclosporin, clomethiazole, cimetidine, itraconazole, fluconazole, miconazole, fluvoxamine, fluoxetine, nefazodone, sertraline, indinavir, nelfinavir, amprenavir, fosamprenavir, saquinavir, lopinavir, delavirdine, erythromycin, VX-944, and VX-497. Preferred CYP inhibitors include ritonavir, ketoconazole, troleandomycin, 4-methyl pyrazole, cyclosporin, and clomethiazole.

Methods for measuring the ability of a compound to inhibit CYP activity are known (see, *e.g*., US 6,037,157 and Yun, et al. Drug Metabolism & Disposition, vol. 21, pp. 403-407 (1993); incorporated herein by reference). For example, a compound to be evaluated may be incubated with 0.1, 0.5, and 1.0 mg protein/ml, or other appropriate concentration of human hepatic microsomes (e. g., commercially available, pooled characterized hepatic microsomes) for 0, 5, 10, 20, and 30 minutes, or other appropriate times, in the presence of an NADPH-generating system. Control incubations may be performed in the absence of hepatic microsomes for 0 and 30 minutes (triplicate). The samples may be analyzed for the presence of the compound. Incubation conditions that produce a linear rate of compound metabolism will be used a guide for further studies. Experiments known in the art can be used to determine the kinetics of the compound metabolism (Kₘ and Vₘₐₓ). The rate of disappearance of compound may be determined and the data analyzed according to Michaelis-Menten kinetics by using Lineweaver-Burk, Eadie-Hofstee, or nonlinear regression analysis.

Inhibition of metabolism experiments may then be performed. For example, a compound (one concentration, < Kₘ) may be incubated with pooled human hepatic microsomes in the absence or presence of a CYP inhibitor (such as ritonavir) under the conditions determined above. As would be recognized, control incubations should contain the same concentration of organic solvent as the incubations with the CYP inhibitor. The concentrations of the compound in the samples may be quantitated, and the rate of disappearance of parent compound may be determined, with rates being expressed as a percentage of control activity.

Methods for evaluating the influence of co-administration of a compound of the invention and a CYP inhibitor in a subject are also known (see, *e.g*., US2004/0028755; incorporated herein by reference). Any such methods could be used in connection with this invention to determine the pharmacokinetic impact of a combination. Subjects that would benefit from treatment according to this invention could then be selected.

Accordingly, one embodiment of this invention provides a method for administering an inhibitor of CYP3A4 and a compound of the invention. Another embodiment of this invention provides a method for administering an inhibitor of isozyme 3A4 ("CYP3A4"), isozyme 2C19 ("CYP2C19"), isozyme 2D6 ("CYP2D6"), isozyme 1A2 ("CYP1A2"), isozyme 2C9 ("CYP2C9"), or isozyme 2E1 ("CYP2E1"). In embodiments where the protease inhibitor is VX-950 (or a sterereoisomer thereof), the CYP inhibitor preferably inhibits CYP3A4.

As would be appreciated, CYP3A4 activity is broadly observed in humans. Accordingly, embodiments of this invention involving inhibition of isozyme 3A4 would be expected to be applicable to a broad range of patients.

Accordingly, this invention provides methods wherein the CYP inhibitor is administered together with the compound of the invention in the same dosage form or in separate dosage forms.

The compounds of the invention (e.g., compound of Formula I or subformulae thereof) may be administered as the sole ingredient or in combination or alteration with other antiviral agents, especially agents active against HCV. In combination therapy, effective dosages of two or more agents are administered together, whereas in alternation or sequentizl-step therapy, an effective dosage of each agent is administered serially or sequentially. In general, combination therapy is typically preferred over alternation therapy because it induces multiple simultaneous stresses on the virus. The dosage given will depend on absorption, inactivation and excretion rate of the drug as well as other factors. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions. The efficacy of a drug against the viral infection can be prolonged, augmented, or restored by administering the compound in combination or alternation with a second, and perhaps third antiviral compound that induces a different gene mutation than that caused by the principle drug in a drug resistant virus. Alternatively, the pharmacokinetic, biodistribution or other parameters of the drug can be altered by such combination or alternation therapy.

Daily dosages required in practicing the method of the present invention will vary depending upon, for example, the compound of the invention employed, the host, the mode of administration, the severity of the condition to be treated. A preferred daily dosage range is about from 1 to 50 mg/kg per day as a single dose or in divided doses. Suitable daily dosages for patients are on the order of from e.g. 1 to 20 mg/kg p.o or i.v. Suitable unit dosage forms for oral administration comprise from ca. 0.25 to 10 mg/kg active ingredient, e.g. compound of Formula I or any subformulae thereof, together with one or more pharmaceutically acceptable diluents or carriers therefor. The amount of co-agent in the dosage form can vary greatly, e.g., 0.00001 to 1000mg/kg active ingredient.

Daily dosages with respect to the co-agent used will vary depending upon, for example, the compound employed, the host, the mode of administration and the severity of the condition to be treated. For example, lamivudine may be administered at a daily dosage of 100mg. The pegylated interferon may be administered parenterally one to three times per week, preferably once a week, at a total weekly dose ranging from 2 to 10 million IU, more preferable 5 to 10 million IU, most preferable 8 to 10 million IU. Because of the diverse types of co-agent that may be used, the amounts can vary greatly, e.g., .0001 to 5,000 mg/kg per day.

The current standard of care for treating hepatitis C is the combination of pegyfated interferon alpha with ribavirin, of which the recommended doses are1.5 µg/kg/wk peginterferon alfa-2b or 180 µg/wk peginterferon alfa-2a, plus 1,000 to 1,200 mg daily of ribavirin for 48 weeks for genotype 1 patients, or 800 mg daily of ribavirin for 24 weeks for genotype 2/3 patients.

The compound of the invention (e.g., compound of Formula I or subformulae thereof) and co-agents of the invention may be administered by any conventional route, in particular enterally, e.g. orally, for example in the form of solutions for drinking, tablets or capsules or parenterally, for example in the form of injectable solutions or suspensions. Certain preferred pharmaceutical compositions may be e.g. those based on microemulsions as described in UK 2,222,770 A.

The compound of the invention (e.g., compound of Formula I or subformulae thereof) are administered together with other drugs (co-agents) e.g. a drug which has anti-viral activity, especially anti-Flaviviridae activity, most especially anti-HCV activity, e.g. an interferon, e.g. interferon-α-2a or interferon-α-2b, e.g. Intron^{R} A, Roferin^{R}, Avonex^{R}, Rebif^{R} or Bctaferon^{R}, or an interferon conjugated to a water soluble polymer or to human albumin, e.g, albuferon, an anti-viral agent, e.g. ribavirin, lamivudine, the compounds disclosed in US patent no. 6,812,219 and WO 2004/002422 A2 (the disclosures of which are incorporated herein by reference in their entireties), an inhibitor of the HCV or other Flaviviridae virus encoded factors like the NS3/4A protease, helicase or RNA polymerase or a prodrug of such an inhibitor, an anti-fibrotic agent, e.g. a N-phenyl-2-pyrimidine-amine derivative, e.g. imatinib, an immune modulating agent, e.g. mycophenolic acid, a salt or a prodrug thereof, e.g. sodium mycophenolate or mycophenolate mofetil, or a SIP receptor agonist, e.g. FTY720 or an analogue thereof optionally phosphorylated, e.g. as disclosed in EP627406A1 EP778263A1, EP1002792A1, WO02/18395, WO02/76995, WO 02/06268, JP2002316985, WO03/29184, WO03/29205, WO03/62252 and WO03/62248, the disclosures of which are incorporated herein by reference in their entireties.

Conjugates of interferon to a water-soluble polymer are meant to include especially conjugates to polyalkylene oxide homopolymers such as polyethylene glycol (PEG) or polypropylene glycols, polyoxyethylenated polyols, copolymers thereof and block copolymers thereof. As an alternative to polyalkylene oxide-based polymers, effectively non-antigenic material such as dextran, polyvinyl pyrrolidones, polyacrylamides, polyvinyl alcohols, carbohydrate-based polymers and the like can be used. Such interferon-polymer conjugates are described in U.S. Pat. Nos. 4,766,106, 4,917,888, European Patent Application No. 0 236 987, European Patent Application No. 0 510 356 and International Application Publication No. WO 95/13090, the disclosures of which are incorporated herein by reference in their entireties. Since the polymeric modification sufficiently reduces antigenic responses, the foreign interferon need not be completely autologous. Interferon used to prepare polymer conjugates may be prepared from a mammalian extract, such as human, ruminant or bovine interferon, or recombinantly produced. Preferred are conjugates of interferon to polyethylene glycol, also known as pegylated interferons.

Especially preferred conjugates of interferon are pegylated alfa-interferons, for example pegylated interferon-α-2a, pegylated interferon-α-2b; pegylated consensus interferon or pegylated purified interferon- α product. Pegylated interferon- α -2a is described e.g. in European Patent 593,868 (incorporated herein by reference in its entirety) and commercially available e. g. under the tradename PEGASYS^{®} (Hoffmann-La Roche). Pegylated interferon- α -2b is described, e.g. in European Patent 975,369 (incorporated herein by reference in its entirety) and commercially available e.g. under the tradename PEC-INTRON A^{®} (Schering Plough). Pegylated consensus interferon is described in WO 96/11953 (incorporated herein by reference in its entirety). The preferred pegylated α-interferons are pegylated interferon-α-2a and pegylated interferon-α-2b. Also preferred is pegylated consensus interferon.

Other preferred co-agents are fusion proteins of an interferon, for example fusion proteins of interferon- α -2a, interferon- a -2b; consensus interferon or purified interferon-α product, each of which is fused with another protein. Certain preferred fusion proteins comprise an interferon (e.g., interferon- α -2b) and an albumin as described in U.S. Patent 6,973,322 and international publications WO03/60071, WO05/003296 and WO05/077042 (Human Genome Sciences). A preferred interferon conjugated to a human albumin is Albuferon (Human Genome Sciences).

Cyclosporins which bind strongly to cyclophilin but are not immunosuppressive include those cyclosporins recited in U.S. Patents 5,767,069 and 5,981,479 and are incorporated herein by reference. Melle⁴-Cyclosporin is a preferred non-immunosuppressive cyclosporin. Certain other cyclosporin derivatives are described in WO2006039668 (Scynexis) and WO2006038088 (Debiopharm SA) and are incorporated herein by reference. A cyclosporin is considered to be non-immunosuppressive when it has an activity in the Mixed Lymphocyte Reaction (MLR) of no more than 5%, preferably no more than 2%, that of cyclosporin A. The Mixed Lymphocyte Reaction is described by T. Meo in "Immunological Methods", L. Lefkovits and B. Peris, Eds., Academic Press, N.Y. pp. 227 - 239 (1979). Spleen cells (0.5 x 10⁶) from Balb/c mice (female, 8 - 10 weeks) are co-incubated for 5 days with 0.5 x 10⁶ irradiated (2000 rads) or mitomycin C treated spleen cells from CBA mice (female, 8 - 10 weeks). The irradiated allogeneic cells induce a proliferative response in the Balb c spleen cells which can be measured by labeled precursor incorporation into the DNA. Since the stimulator cells are irradiated (or mitomycin C treated) they do not respond to the Balb/c cells with proliferation but do retain their antigenicity. The IC₅₀ found for the test compound in the MLR is compared with that found for cyclosporin A in a parallel experiment. In addition, non-immunosuppressive cyclosporins lack the capacity of inhibiting CN and the downstream NF-AT pathway. [Melle]⁴-ciclosporin is a preferred non-immunosuppressive cyclophilin-binding cyclosporin for use according to the invention.

Ribavirin (1-β-D-ribofuranosyl-1-1,2,4-triazole-3-caroxamide) is a synthetic, non-interferon-inducing, broad spectrum antiviral nucleoside analog sold under the trade name, Virazole (The Merk Index, 11th edition, Editor: Budavar, S, Merck & Co., Inc., Rahway, NJ, p1304,1989). United States Patent No. 3,798,209 and RE29,835 (incorporated herein by reference in their entireties) disclose and claim ribavirin. Ribavirin is structurally similar to guanosine, and has *in vitro* activity against several DNA and RNA viruses including *Flaviviridae* (Gary L. Davis, Gastroenterology 118:S104-S114, 2000).

Ribavirin reduces serum amino transferase levels to normal in 40% of patients, but it does not lower serum levels of HCV-RNA (Gary L. Davis, Gastroenterology 118:S104-S114, 2000). Thus, ribavirin alone is not effective in reducing viral RNA levels. Additionally, ribavirin has significant toxicity and is known to induce anemia. Ribavirin is not approved for monotherapy against HCV; it is approved in combination with interferon alpha-2a or interferon alpha-2b for the treatment of HCV.

A further preferred combination is a combination of a compound of the invention (e.g., a compound of Formula I or any subformulae thereof) with a non-immunosuppressive cyclophilin-binding cyclosporine, with mycophenolic acid, a salt or a prodrug thereof, and/or with a S1P receptor agonist, e.g. FTY720.

Additional examples of compounds that can be used in combination or alternation treatments include:
(1) Interferons, including interferon alpha 2a or 2b and pegylated (PEG) interferon alpha 2a or 2b, for example:
   (a) Intron-A®, interferon alfa-2b (Schering Corporation, Kenilworth, NJ);
   (b) PEG-Intron®, peginteferon alfa-2b (Schering Corporation, Kenilworth, NJ);
   (c) Roferon®, recombinant interferon alfa-2a (Hoffmann-La Roche, Nutley, NJ);
   (d) Pegasys®, peginterferon alfa-2a (Hoffmann-La Roche, Nutley, NJ);
   (e) Berefor®, interferon alfa 2 available (Boehringer Ingelheim Pharmaceutical, Inc., Ridgefield, CT);
   (f) Sumiferon®, a purified blend of natural alpha interferons (Sumitomo, Japan)
   (g) Wellferon®, lymphoblastoid interferon alpha n1 (GlaxoSmithKline);
   (h) Infergen®, consensus alpha interferon (InterMune Pharmaceuticals, Inc., Brisbane, CA);
   (i) Alferon®, a mixture of natural alpha interferons (Interferon Sciences, and Purdue Frederick Co., CT);
   (j) Viraferon®;
   (k) Consensus alpha interferon from Amgen, Inc., Newbury, Park, CA, Other forms of interferon include: interferon beta, gamma, tau and omega, such as Rebif ( Interferon beta 1a) by Serono, Omniferon (natural interferon) by Viragen, REBIF (interferon beta-1a) by Ares-Serono, Omega Interferon by BioMedicines; oral interferon Alpha by Amarillo Biosciences, an interferon conjugated to a water soluble polymer or to a human albumin, e.g., Albuferon (Human Genome Sciences), an antiviral agent, a consensus interferon, ovine or bovine interferon-tau
   Conjugates of interferon to a water-soluble polymer are meant to include especially conjugates to polyalkylene oxide homopolymers such as polyethylene glocol (PEG) or polypropylene glycols, polyoxyethylenated polyols, copolymers thereof and block copolymers thereof. As an alternative to polyalkylene oxid-based polymers, effectively non-antigenic materials such as dextran, polyvinyl pyrrolidones, polyacrylamides, polyvinyl alcohols, carbohydrate-based polymers and the like can be used. Since the polymeric modification sufficiently reduces antigenic response, the foreign interferon need not be completely autologous. Interferon used to prepare polymer conjugates may be prepared from a mammalian extract, such as human, ruminant or bovine interferon, or recombinantly produced. Preferred are conjugates of interferon to polyethylene glycol, also known as pegylated interferons.
(2) Ribavirin, such as ribavirin (1-beta-D-ribofuranosyl-1H-1,2,4-triazole-3-carboxamide) from Valeant Pharmaceuticals, Inc., Costa Mesa, CA); Rebetol® from Schering Corporation, Kenilworth, NJ, and Copegus® from Hoffmann-La Roche, Nutley, NJ; and new ribavirin analogues in development such as Levovirin and Viramidine by Valeant.
(3) Thiaxolidine derivatives which show relevant inhibition in a reverse-phase HPLC assay with an NS3/4A fusion protein and NS5A/5B substrate (Sudo K. et al., Antiviral Research, 1996, 32, 9-18), especially compound RD-1-6250, possessing a fused cinnamoyl moiety substituted with a long alkyl chain, RD4 6205 and RD4 6193;
(4) Thiazolidines and benzanilides identified in Kakiuchi N. et al, J. FEBS Letters 421, 217-220; Takeshita N. et al. Analytical Biochemistry, 1997, 247, 242-246*;*
(5) A phenan-threnequinone possessing activity against protease in a SDS-PACE and autoradiography assay isolated from the fermentation culture broth of Streptomyces sp., Sch 68631 (Chu M. et al., Tetrahedron Letters, 1996, 37, 7229-7232), and Sch 351633, isolated from the fungus *Penicillium griseofulvum,* which demonstrates activity in a scintillation proximity assay (Chu M. et al, Bioorganic and Medicinal Chemistry Letters 9, 1949-1952);
(6) Protease inhibitors.
   Examples include substrate-based NS3 protease inhibitors (Attwood et al., *Antiviral peptide derivatives*, PCT WO 98/22496, 1998; Attwood et al., Antiviral Chemistry and Chemotherapy 1999, 10, 259-273; Attwood et al, *Preparation and use of amino acid derivatives as anti-viral agents*, German Patent Pub. DE 19914474; Tung et al. *Inhibitors of serine proteases, particularly hepatitis C virus NS3 protease*; PCT WO 98/17679), including alphaketoamides and hydrazinoureas, and inhibitors that terminate in an electrophile such as a boronic acid or phosphonate (Llinas-Brunet et al. *Hepatitis C inhibitor peptide analogues*, PCT WO 99/07734) are being investigated.
   Non-substrate-based NS3 protease inhibitors such as 2,4,6-trihydroxy-3-nitrobenzamide derivatives (Sudo K. et al., Biochemiscal and Biophysical Research Communication, 1997, 238 643-647; Sudo K. et al. Antiviral Chemistry and Chemotherapy, 1998, 9, 186), including RD3-4082 and RD3-4078, the former substituted on the amide with a 14 carbon chain and the latter processing a *para*-phenoxyphenyl group are also being investigated.
   Sch 68631, a phenanthrenequinone, is an HCV protease inhibitor (Chu M et al., Tetrahedron Letters 37:7229-7232, 1996). In another example by the same authors, Sch 351633, isolated from the fungus *Penicillium grieofulvum*, was identified as a protease inhibitor (Chu M. et al., Bioorganic and Medicinal Chemistry Letters 9:1949-1952). Nanomolar potency against the HCV NS3 protease enzyme has been achieved by the design of selective inhibitors based on the macromolecule eglin c. Eglin c, isolated from leech, is a potent inhibitor of several serine proteases such as S. griseus proteases A and B, ∀-chymotrypsin, chymase and subtilisin. Qasim M.A. et al., Biochemistry 36:1598-1607, 1997.
   U.S. patents disclosing protease inhibitors for the treatment of HCV include, for example, U.S. Patent No. 6,004,933 to Spruce et al (incorporated herein by reference in its entirety) which discloses a class of cysteine protease inhibitors for inhibiting HCV endopeptidase 2; U.S. Patent No. 5,990,276 to Zhang et al.(incorporated herein by reference in its entirety) which diseases synthetic inhibitors of hepatitis C virus NS3 protease; U.S. Patent No. 5,538,865 to Reyes et al.(incorporated herein by reference in its entirety). Peptides as NS3 serine protease inhibitors of HCV are disclosed in WO 02/008251 to Corvas International, Inc., and WO 02/08187 and WO 02/008256 to Schering Corporation (incorporated herein by reference in their entireties). HCV inhibitor tripeptides are disclosed in U.S. Patent Nos. 6,534,523, 6,410,331 and 6,420,380 to Boehringer Ingelheim and WO 02/060926 to Bristol Myers Squibb (incorporated herein by reference in their entireties). Diaryl peptides as NS3 serine protease inhibitors of HCV are disclosed in WO 02/48172 to Sobering Corporation (incorporated herein by reference). Imidazoleidinones as NS3 serine protease inhibitors of HCV are disclosed in WO 02/18198 to Schering Corporation and WO 02/48157 to Bristol Myers Squibb (incorporated herein by reference in their entireties). WO 98/17679 to Vertex Pharmaceuticals and WO 02/48116 to Bristol Myers Squibb also disclose HCV protease inhibitors (incorporated herein by reference in their entireties).
   HCV NS3-4A serine protease inhibitors including BILN 2061 by Boehringer Ingelheim, VX-950 by Vertex, SCH 6/7 by Schering-Plough, and other compounds currently in preclinical development;
   Substrate-based NS3 protease inhibitors, including alphaketoamides and hydrazinoureas, and inhibitors that terminate in an elecrophile such as a boronic acid or phosphonate; Non-substrate-based NS3 protease inhibitors such as 2.,4,6-trihydroxy-3-nitro-benzamide derivatives including RD3-4082 and RD3-4078, the former substituted on the amide with a 14 carbon chain and the latter processing a para-phenoxyphenyl group; and Sch68631, a phenanthrenequinone, an HCV protease inhibitor.
   Sch 351633, isolated from the fungus *Penicillium griseofulvum* was identified as a protease inhibitor. Eglin c, isolated from leech is a potent inhibitor of several serine proteases such as S. griseus proteases A and B, a-chymotrypsin, chymase and subtilisin.
   US patent no. 6004933 (incorporated herein by reference in its entirety) discloses a class of cysteine protease inhibitors from inhibiting HCV endopeptidase 2; synthetic inhibitors of HCV NS3 protease (pat), HCV inhibitor tripeptides (pat), diaryl peptides such as NS3 serine protease inhibitors of HCV (pat), Imidazolidindiones as NS3 serine protease inhibitors of HCV (pat).
   Thiazolidines and benzanilides (ref). Thiazolidine derivatives which show relevant inhibition in a reverse-phase HPLC assay with an NS3/4A fusion protein and NS5A/5B substrate especially compound RD-16250 possessing a fused cinnamoyl moiety substituted with a long alkyl chain, RD4 6205 and RD4 6193
   Phenan-threnequinone possessing activity against protease in a SDS-PAGE and autoradiography assay isolated from the fermentation culture broth of *Streptomyces* sp, Sch68631 and Sch351633, isolated from the fungus *Penicillium griseofulvum,* which demonstrates activity in a scintillation proximity assay.
(7) Nucleoside or non-nucleoside inhibitors of HCV NS5B RNA-dependent RNA polymerase, such as 2'-C-methyl-3'-O-L-valine ester ribofuranosyl cytidine (Idenix) as disclosed in WO 2004/002422 A2 (incorporated herein by reference in its entirety), R803 (Rigel), JTK-003 (Japan Tabacco), HCV-086 (ViroPharma/Wyeth) and other compounds currently in preclinical development;
   gliotoxin (ref) and the natural product cerulenin;
   2*-fluoronucleosides;
   other nucleoside analogues as disclosed in WO 02/057287 A2, WO 02/057425 A2, WO 01/90121, WO 01/92282, and US patent no. 6,812,219, the disclosures of which are incorporated herein by reference in their entirety.
   Idenix Pharmaceuticals discloses the use of branched nucleosides in the treatment of flaviviruses (including HCV) and pestiviruses in International Publication Nos. WO 01/90121 and WO 01/92282 (incorporated herein by reference in their entireties). Specifically, a method for the treatment of hepatitis C infection (and flaviviruses and pestiviruses) in humans and other host animals is disclosed in the Idenix publications that includes administering an effective amount of a biologically active 1', 2', 3' our 4'-branched B-D or B-L nucleosides or a pharmaceutically acceptable salt or prodrug thereof, administered either alone or in combination with another antiviral agent, optionally in a pharmaceutically acceptable carrier. Certain preferred biologically active 1', 2', 3', or 4' branched B-D or B-L nucleosides, including Telbivudine, are describedi n U.S. Patents 6,395,716 and 6,875,751, each of which are incorporated herein by reference.
   Other patent applications disclosing the use of certain nucleoside analogs to treat hepatitis C virus include: PCTCA00/01316 (WO 01/32153; filed November 3,2000) and PCT/CA01/00197(WO 01/60315; filed February 19, 2001) filed by BioChem Parma, Inc., (now Shire Biochem, Inc.); PCT/US02/01531 (WO 02/057425; filed January 18, 2002) and PCT/US02/03086 (WO 02/057287; filed January 18, 2002) filed by Merck & Co., Inc., PCT/EP01/09633 (WO 02/18404; published August 21, 2001) filed by Roche, and PCT Publication Nos. WO 01/79246 (filed April 13, 2001), WO 02/32920 (filed October 18, 2001) and WO 02/48165 by Pharmasset, Ltd. (the disclosures of which are incorporated herein by reference in their entireties)
   PCT Publication No. WO 99/43691 to Emory University (incorporated herein by reference in its entirety), entitled "2-Fluoronucleosides" discloses the use of certain 2'-fluoronucleosides to treat HCV.
   Eldrup et al. (Oral Session V, Hepatitis C Virus, Flaviviridae; 16th International Conference on Antiviral Research (April 27, 2003, Savannah, GA)) described the structure activity relationship of 2'-modified nucleosides for inhibition of HCV.
   Bhat et al. (Oral Session V, Hepatitis C Virus, Flaviviridae, 2003 (Oral Session V, Hepatitis C Virus, Flaviviridae; 16th International conference on Antiviral Research (April 27, 2003, Savannah, Ga); p A75) describes the synthesis and pharmacokinetic properties of nucleoside analogues as possible inhibitors of HCV RNA replication. The authors report that 2'-modified nucleosides demonstrate potent inhibitory activity in cell-based replicon assays.
   Olsen et al. (Oral Session V, Hepatitis C Virus, Flaviviridae; 16th International Conference on Antiviral Research (April 27, 2003, Savannah, Ga)p A76) also described the effects of the 2'-modified nucleosides on HCV RNA replication.
(8) Nucleotide polymerase inhibitors and gliotoxin (Ferrari R. et al. Journal of Virology, 1999, 73, 1649-1654), and the natural product cerulenin (Lohmann V, et al. Virology, 1998, 249, 108-118);
(9) HCV NS3 helicase inhibitors, such as VP_50406 by ViroPhama and compounds from Vertex. Other helicase inhibitors (Diana G,D et al., *Compounds, compositions and methods for treatment of hepatitis C*, U.S. Patent No. 5,633,358 (incorporated herein by reference in its entirety); Diana G.D. et at. *Piperidine derivatives, pharmaceutical compositions thereof and their use in the treatment of hepatitis C*, PCT WO 97/36554);
(10) Antisense phosphorothioate oligodeoxynucleotides (S-ODN) complementary to sequence stretches in the 5' non-coding region (NCR) of the virus (AIt M. et al., Hepatology, 1995, 22, 707-717), or nucleotides 326-348 comprising the 3' end of the NCR and nucleotides 371-388 located in the core coding region of one HCV RNA (Alt M. et al., Archives of Virology, 1997, 142, 589-599; Galderisi U. et al., Journal of Cellular Physiology, 199, 181, 251-257); such as ISIS 14803 by Isis Pharm/Elan, antisense by Hybridon, antisense by AVI bioPharma,
(11) Inhibitors of IRES-dependent translation (Ikeda N et al., *Agent for the prevention and treatment of hepatitis C*, Japanese Patent Pub. JP-08268890; Kai Y et al, *Prevention and treatment of viral diseases,* Japanese Patent Pub. JP-10101591); such as ISIS 14803 by Isis Pharm/Elan, IRES inhibitor by Anadys, IRES inhibitors by Immusol, targeted RNA chemistry by PTC Therapeutics
(12) Ribozymes, such as nuclease-resistant ribozymes (Maccjak, D.J. et al., Hepatology 1999, 30, abstract 995) and those directed in U.S. Patent No. 6,043,077 to Barber et al., and U.S. Patent Nos. 5,869,253 and 5,610,054 to Draper et al.(incorporated herein by reference in their entireties) for example, HEPTAZYME by RPI
(13) siRNA directed against HCV genome
(14) HCV replication inhibitor of any other mechanisms such as by VP50406ViroPharama/Wyeth, inhibitors from Achillion, Arrow
(15) An inhibitor of other targets in the HCV life cycle including viral entry, assembly and maturation
(16) An immune modulating agent such as an IMPDH inhihitor, mycophenolic acid, a salt or a prodrug thereof sodium mycophenolate or mycophenolate mofetil, or Merimebodib (VX-497); thymosin alpha-1 (Zadaxin, by SciClone); or a SIP receptor agonist, e.g. FTY720 or analogue thereof optionally phosphorylated.
(17) An anti-fibrotic agent, such as a N-phenyl-2-pyrimidine-amine derivative, imatinib (Gleevac), IP-501 by Indevus, and Interferon gamma 1b from InterMune
(18) Therapeutic vaccine by Intercell, Epimmune/Genecor, Merix, Tripep (Chron-VacC), immunotherapy (Therapore) by Avant, T cell therapy by CellExSys, monoclonal antibody XTL-002 by STL, ANA 246 and ANA 246 BY Anadys,
(19) Other miscellaneous compounds including 1-amino-alkylcyclohexanes (U.S. Patent No. 6,034,134 to Gold et al.), alkyl lipids (U.S. Pat. No. 5,922,757 to Chojkier et al.), vitamin E and other antitoxidants (U.S. Patent. No. 5,922,757 to Chojkier et al.), amantadine, bile acids (U.S. Pat. No. 5,846,99964 to Ozeki et al.), N-(phosphonoacetl)-L-aspartic acid, )U.S. Pat. No. 5,830,905 to Diana et al.), benzenedicarboxamides (U.S. Pat. No. 5,633,388 to Diane et al.), polyadenylic acid derivatives (U.s. Pat. No. 5,496,546 to Wang et al.), 2'3'-dideoxyinosine (U.S. Pat. No. 5,026,687 to Yarchoan et al.), benzimidazoles (U.S. Pat. No. 5,891,874 to Colacino et al.), plant extracts (U.S. Pat. No. 5,837,297 to Tsai et al., U.S. Pat. No. 5,725,859 to Omer et al., and U.S. Pat. No. 6,056,961) and piperidines (U.S. Pat. No. 5,830,905 to Diana et al.); the disclosures of which are incorporated herein by reference in their entireties. Also,squalene, telbivudine, N-(phosphonoacetyl)-L-aspartic acid, benzenedicarboxamides, polyadenylic acid derivatives, glycosylation inhibitors, and nonspecific cytoprotective agents that block cell injury caused by the virus infection.
(20) Any other compound currently in preclinical or clinical development for the treatment of HCV, including Interleukin-10 (Schering-Plough), AMANTADINE (Symmetrel) by Endo Labs Solvay, caspase inhibitor IDN-6556 by Idun Pharma, HCV/MF59 by Chiron, CIVACIR (Hepatitis C Immune Globulin) by NABI, CEPLENE (histamine dichloride) by Maxim, IDN-6556 by Idun PHARM, T67, a beta-tubulin inhibitor, by Tularik, a therapeutic vaccine directed to E2 by Innogenetics, FK788 by Fujisawa Helathcare, 1dB1016(Siliphos,oral silybin-phospliatidyl choline phytosome), fusion inhibitor by Trimeris, Dication by Immtech, hemopurifier by Aethlon Medical, UT 231B by United Therapeutics.
(21) Purine nucleoside analog antagonists of TIR7 (toll-like preceptors) developed by Anadys, e.g., Isotorabine (ANA245) and its prodrug (ANA975), which are described in European applications EP348446 and EP636372, International Publications WO03/045968, WO05/121162 and WO05/25583, and U.S. Patent 6/973322, each of which is incorporated by reference.
(21) Non-nucteoside inhibitors developed by Genelabs and described in International Publications WO2004/108687, WO2005/12288, and WO2006/076529, each of which is incorporated by reference.
(22) Other co-agents (*e.g*., non-immunomodulatory or immunomodulatory compounds) that may be used in combination with a compound of this invention include, but are not limited to, those specified in WO 02/18369, which is incorporated herein by reference.

Methods of this invention may also involve administration of another component comprising an additional agent selected from an immunomodulatory agent; an antiviral agent; an inhibitor of HCV protease; an inhibitor of another target in the HCV life cycle; a CYP inhibitor; or combinations thereof.

Accordingly, in another embodiment, this invention provides a method comprising administering a compound of the invention and another anti-virat agent, preferably an anti-HCV agent. Such anti-viral agents include, but are not limited to, immunomodulatory agents, such as α, β, and δ interferons, pegylated derivatized interferon-a compounds, and thymosin; othe anti-viral agents, such as ribavirin, amantadine, and telbivudine; other inhibitors of hepatitis C proteases (NS2-NS3 inhibitors and NS3-NS4A inhibitors); inhibitors of other targets in the HCV life cycle, including helicase, polymerase, and metalloprotease inhibitors; inhibitors of internal ribosome entry; broad-spectrum viral inhibitors, such as IMPDH inhibitors (*e.g*., compounds of United States Patent 5,807, 876,6, 498, 178 6,344, 465,6, 054,472,WO 97/40028, WO 98/40381, WO 00/56331, and mycophenolic acid and derivatives thereof, and including, but bot limited to VX-497, VX-148, and/or VX-944); or combinations of any of the above.

In accordance with the forgoing the present invention provides in a yet further aspect,
- A pharmaceutical combination comprising a) a first agent which is a compound of the invention, e,g, a compound of formula I or any subformulae thereof, and b) a co-agent, e.g. a second drug agent as defined above.
- A method as defined above comprising co-administration, e.g. concomitantly or in sequence, of a therapeutically effective amount of a compound of the invention, e.g. a compound of formula 1 or any subformulae thereof, and a co-agent, e.g. a second drug agent as defined above.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time. Fixed combinations are also within the scope of the present invention. The administration of a pharmaceutical combination of the invention result in a beneficial effect, e.g. a synergistic therapeutic effect, compared to a monotherapy applying only one of its pharmaceutically active ingredients.

Each component of a combination according to this invention may be administered separately, together, or in any combination thereof. As recognized by skilled practitioners, dosages of interferon are typically measured in IU (*e.g*., about 4 minion IU to about 12 million IU).

If an additional agent is selected from another CYP inhibitor, the method would, therefore, employ two or more CYP inhibitors. Each component may be administered in one or more dosage forms. Each dosage form may be administered to the patient in any order.

The compound of the invention and any additional agent may be formulated in separate dosage forms. Alternatively, to decrease the number of dosage forms administered to a patient, the compound of the invention and any additional agent may be formulated together in any combination. For example, the compound of the invention inhibitor may be formulated in one dosage form and the additional agent may be formulated together in another dosage form. Any separate dosage forms may be administered at the same time or different times.

Alternatively, a composition of this invention comprises an additional agent as described herein. Each component may be present in individual compositions, combination compositions, or in a single composition.

### Exemplification of the Invention

The invention is further illustrated by the following examples, which should not be construed as further limiting. The assays used throughout the Examples are accepted. Demonstration of efficacy in these assays is predictive of efficacy in subjects.

### GENERAL SYNTHESIS METHODS

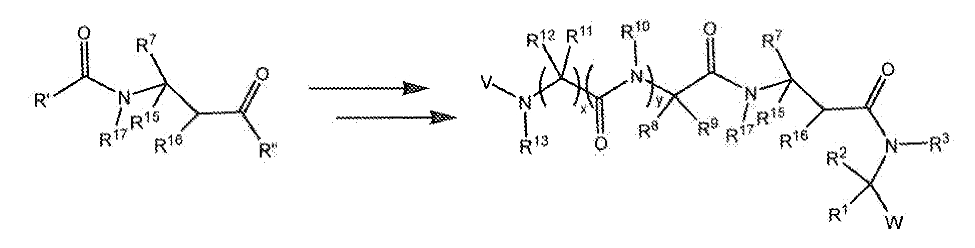

All starting materials, building blocks, reagents, acids, bases, dehydrating agents, solvents, and catalysts utilized to synthesis the compounds of the present invention are either commercially available of can be produced by organic synthesis methods known to one of ordinary skill in the art (Houben-Weyl 4th Ed. 1952, Methods of Organic Synthesis, Thieme, Volume 21). Further, the compounds of the present invention can be produced by organic synthesis methods known to one of ordinary skill in the art as shown in the following examples.

### LIST OF ABBREVIATIONS

- Ac: acetyl
- ACN: Acetonitrile
- AcOEt / EtOAc: Ethyl acetate
- AcOH: acetic acid
- aq: aqueous
- Ar: aryl
- Bn: benzyl
- Bu: butyl (nBu = n-butyl, tBu = tert-butyl)
- CDI: Carbonyldiimidazole
- CH₃CN: Acetonitrile
- DBU: 1,8-Diazabicyclo[5.4.0]-undec-7-ene
- DCE: 1,2-Dichloroethane
- DCM: Dichloromethane
- DIPEA: N-Ethyldiisopropylamine
- DMAP: Dimethylaminopyridine
- DMF: N,N'-Dimethylformamide
- DMSO: Dimethylsulfoxide
- El: Electronspray ionisation
- Et₂O: Diethylether
- Et₃N: Triethylamine
- Ether: Diethylether
- EtOH: Ethanol
- FC: Flash Chromatography
- h: hour(s)
- HATU: O-(7-Azabenzotriazole-1-yl)-N,N,N'N'- tetramethyluronium hexafluorophosphate
- HBTU: O-(Benzotriazol-1-yl)-N,N,N',N'- tetramethyluronium hexafluorophosphate
- HCl: Hydrochloric acid
- HOBt: 1-Hydroxybenzotriazole
- HPLC: High Performance Liquid Chromatography
- H₂O: Water
- L: liter(s)
- LC-MS: Liquid Chromatography Mass Spectrometry
- Me: methyl
- MeI: lodomethane
- MeOH: Methanol
- mg: milligram
- min: mtnule(s)
- mL: milliliter
- MS: Mass Spectrometry
- Pd/C: palladium on charcoal
- PG: protecting group
- Ph: phenyl
- Prep: Preparative
- Rf: ratio of fronts
- RP: reverse phase
- Rt: Retention time
- rt: Room temperature
- SiO₂: Silica gel
- TBAF: Tetrabutylammonium fluoride
- TEA: Triethylamine
- TEA: Trifluoroacetic acid
- THF: Tetrahydrofurane
- TLC: Thin Layer Chromatography

### HPLC methods:

### Method A

Agilent 1100 LC chromatographic system with Micromass ZMD MS detection. A binary gradient composed of A (water containing 5 % acetonitrile and 0.05% trifluoroacetic acid) and B (acetonitrile containing 0.045% trifluoroacetic acid) is used as a mobile phase on a Waters X Terra^{™} C-18 column (30 x 3 mm, 2.5 µm particle size) as a stationary phase.

The following elution profile is applied: a linear gradient of 3.5 minutes at a flow rate of 0.6 ml/min from 5% of B to 95% of B, followed by an isocratic elution of 0.5 minutes at a flow rate of 0.7 ml/min of 95% of B, followed by an isocratic elution of 0.5 minutes at a flow rate of 0.8 ml/min of 95% of B, followed by a linear gradient of 0.2 minutes at a flow rate of 0.8 ml/min from 95% of B to 5% of B, followed by a isocratic elution of 0.2 minutes at a flow rate of 0.7 ml/mm of 5% of B.

### Method B:

Agilent 1100 LC chromatographic system with Micromass ZMD MS detection. A binary gradient composed of A (water containing 5 % acetonitrile and 0.05% trifluoroacetic acid) and B (acetonitrile containing 0.045% trifluoroacetic acid) is used as a mobile phase on a Waters X Terra^{™} C-18 column (30 x 3 mm, 2.5µm particle size) as a stationary phase.

The following elution profile is applied: a linear gradient of 1.5 minutes at a flow rate of 0.6 ml/min from 10% of B to 95% of B, followed by an isocratic elution of 0.5 minutes at a flow rate of 0.7 ml/min of 95% of B, followed by an isocratic elution of 0.5 minutes at a flow rate of 0.8 ml/min of 95% of B, followed by a linear gradient of 0.2 minutes at a flow rate of 0.8 ml/min from 95% ofB to 10% ofB, followed by an isocratic elution of 0.2 minutes at a flow rate of 0.7 ml/min of 10% of B.

### Method C:

### LC-MS

| | |
|---|---|
| Instrument: | Agilent system |
| Column: | Waters symmetry, 3.5 µm, 50 x 2.1 mm, 5 min, 20% to 95% CH₃CN |
| solvent: | CH₃CN (0.1% HCO₂H); H₂O (0.1% HCO₂H) |
| gradient: | 0-3.5 min : 20-95% CH₃CN, 3.3-5 min : 95% CH₃CN, 5.5-5.33 min 95 % to |
| 20% CH₃CN | |

### Method D:

### HPLC

| | |
|---|---|
| instrument: | Kontron, Kroma-System |
| Column: | Macherey-Nagel, Lichrosphere 100-5 RP 18 |
| Solvent: | CH₃CN (0.1% CF₃CO₂H); H₂O (0.1% CF₃CO₂M) |
| Gradient: | 0-5 min: 10-100% CH₃CN; 5-7.5 min: 100% CH₃CN (Flow 1.5mL/min) |

### Method E:

### HPLC

| | |
|---|---|
| lnstrument: | Agilent system |
| column: | waters symmetry C18, 3.3 µm, 2.1 x 50mm, flow 0.6 ml/min |
| solvent: | CH₃CN (0.1% CF₃CO₂H); H₂O (0.1% CF₃CO₂H) |
| gradient: | 0-3.5 min : 20-95% CH₃CN, 3.5-5 min: 95% CH₃CN, 5.5-3.55 min 95 % to |
| 20% CH₃CN | |

### Method F:

### MS

| | |
|---|---|
| Instrument: | Agilent 1100 Series |
| Detection: | API-ES, positive/negative |

### Method G:

### HPLC

| | |
|---|---|
| Instrument: | Agilent system |
| column: | Macherey-Nagel Nucleosil 100-3 C18 HD, particle size 3.5 µm, pore size |
| 100Å, length 70 mm, internal diameter 4 mm, flow 1.0 ml/min | |
| solvent: | CH₃CN (0.1% CF₃CO₂H); H₂O (0.1% CF₃CO₂H) |
| gradient: | 0-6 min: 20-100% CH₃CN, 1.5 min : 100% CH₃CN, 0.5 min 100-20% |
| CH₃CN | |

### Methods H:

### Preparative HPLC

| | |
|---|---|
| Instrument: | Gilson |
| Column: | Sun-Fire prep C18 OBD 5 µm, Column 19 x 50 mm (flow 20mL/min) or |
| Column | 30 x 100 mm (flow 4CmL/min) |
| Solvent: | CH₃CN (0.1% CF₃CO₂H) and H₂O (0.1% CF₃CO₂H) |
| Gradient: | 0-20 min: 5-100% CH₃CN |

### Examples 1-16

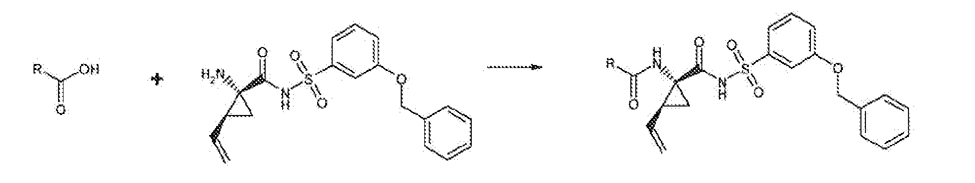

To an array of glass tubes is added one of 16 carboxylic acids (0.121 mmol) (for preparation of the corresponding acids (RCO₂H) see below) and DMF (0.25 ml) in each tube. O-(7-Azabenzotriazol-1-yl)-N,N,N'N'-tetramethyluronium-hexafluorophosphste (0.133 mmol) and N-ethyldiisopropylamine (0.182 mmol) is added to each tube. The resulting reaction mixtures are stirred at 25°C for 45 minutes and a solution (0.165 ml) of N-((1R,2S)-)-amino-2-vinyl-cyclopropanecarbonyl)-3-benzyloxy-benzenesulfonamide (BB29) (1.936 mmol) in DMF (2.63 ml) is added in each tube followed by the addition of N-ethyldiisopropylamine (0.182 mmol). The resulting reaction mixtures are stirred at 50°C for 17 hours. Methanol (1.0 ml) is added to each tube and each reaction mixture is filtered over a 0.45 µm PTFA membrane. The filtrates are then individually purified by a preparative LC-MS procedure.

This generic procedure is used to prepare the following compounds:

| Example | R | Rt [min] | Detected mass (MH⁺) | HPLC | RCO₂H |
|---|---|---|---|---|---|
| | | | | method | BB# |
| 1 | 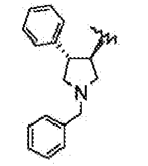 | 2.82 | 636 | A | see ref. in BB 10 |
| 2 | 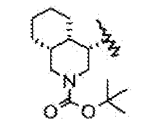 | 1.94 | 638 | B | 9 |
| 3 | 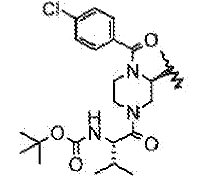 | 3.61 | 823 | A | 1 |
| 4 | 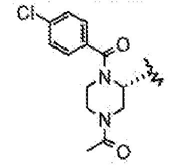 | 3.20 | 666 | A | 4 |
| 5 | 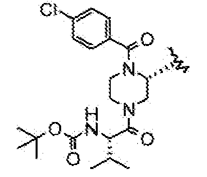 | 3.61 | 823 | A | 5 |
| 6 | 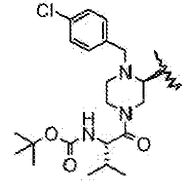 | 2.02 | 809 | B | 2 |
| 7 | 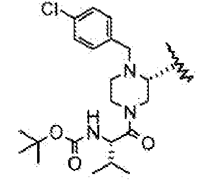 | 1.93 | 809 | B | 6 |
| 8 | 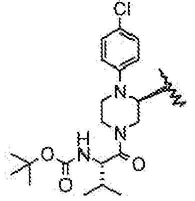 | 2.01 | 795 | B | 3 |
| 9 | 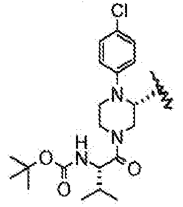 | 2.02 | 795 | B | 7 |
| 10 | 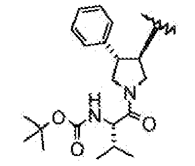 | 3.50 | 745 | A | 10 |
| 11 | 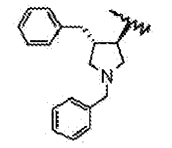 | 2.88 | 650 | A | see ref. in BB 11 |
| 12 | 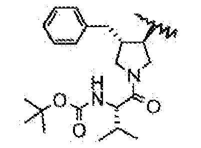 | 3.53 | 759 | A | see ref. in BB 11 |
| 13 | 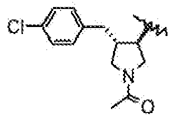 | 3.30 | 637 | A | 14 |
| 14 | 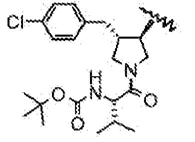 | 3.65 | 794 | A | 12 |
| 15 | 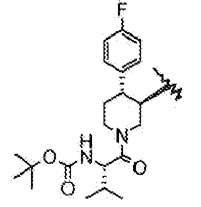 | 1.99 | 777 | B | 15 |
| 16 | 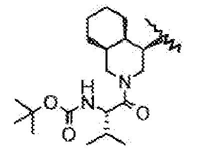 | 3.57 | 737 | A | 8 |

### Examples 17-18

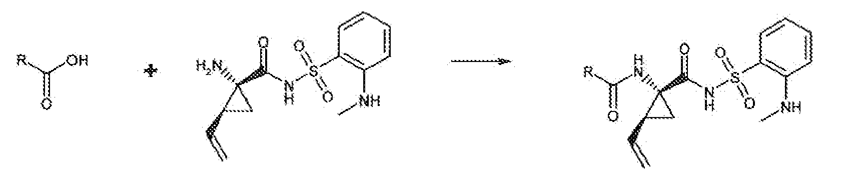

To an array of glass tubes is added one of 2 carboxylic acids (0.121 mmol) (for preparation of the corresponding acids see below) and DMF (0.25 ml) in each tube. O-(7-Azabenzotriazol-1-yl)-N,N,N'N'-tetramethyluronium-hexafluorophosphate (0.133 mmol) and N-ethyldiisopropylamine (0.182 mmol) is added to each tube. The resulting reaction mixtures are stirred at 25°C for 45 minutes and a solution (0.165 ml) of N-((1R,2S)-1-Amino-2-vinyl-cyclopropanecarbonyl)-2-methylamino-benzenesulfonamide (BB28) (0.242 mmol) in DMF (0.33 ml) is added in each tube followed by the addition of N-ethyldiisopropylamine (0.182 mmol). The resulting reaction mixtures are stirred at 50°C for 17 hours. Methanol (1.0 ml) is added to each tube and each reaction mixture is filtered over a 0.45 µm PTFA membrane. The filtrates are then individually purified by a preparative LC-MS procedure.

This generic procedure is used to prepare the following compounds:

| Example | R | Rt [min] | Detected mass(MH⁺) | HPLC method | RCO₂H BB# |
|---|---|---|---|---|---|
| 17 | 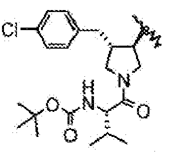 | 3.48 | 717 | A | 12 |
| 18 | 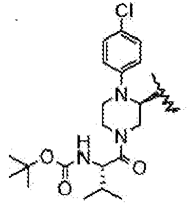 | 1.93 | 718 | B | 3 |

### Examples 19-27

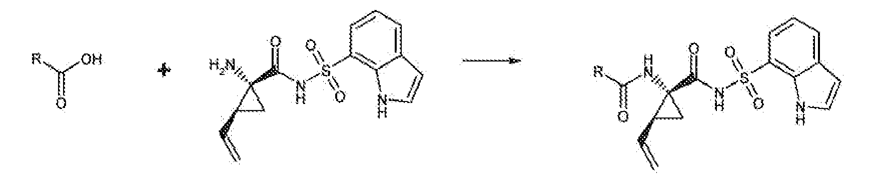

To an array of glass tubes is added one of 9 carboxylic acids (0.130 mmol) and DMF (0.25 ml) in each tube, O-(7-Azabenzotriazol-1-yl)-N,N,N'N'-tetramethyluronium-hexafluorophosphate (0.143 mmol) and N-ethyldiisopropylamine (0.1.95 mmol) is added to each tube. The resulting reaction mixtures are stirred at 25°C for 45 minutes and a solution (0.145 ml) of 1H-Indole-7-sulfonic acid ((1R,2S)-1-amino-2-vinyl-cyclopropanecarbonyl)-amide (BB27) (1.170 mmol) in DMF (1.31 ml) is added in each tube followed by the addition of N-ethyldiisopropylamine (0.195 mmol). The resulting reaction mixtures are stirred at 50°C for 17 hours. Methanol (1.0 ml) is added to each tube and each reaction mixture is filtered over a 0.45 µm PTFA membrane. The filtrates are then individually purified by a preparative LC-MS procedure.

This generic procedure is used to prepare the following compounds:

| Position | R | Rt [min] | Detected | HPLC method | RCO₂H BB# |
|---|---|---|---|---|---|
| | | | mass | | |
| | | | (MH ⁺) | | |
| 19 | 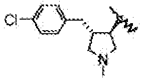 | 1.49 | 542 | B | see step 4, BB 12 |
| 20 | 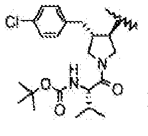 | 3.48 | 727 | A | 12 |
| 21 | 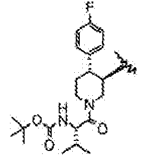 | 3.44 | 710 | A | 15 |
| 22 | 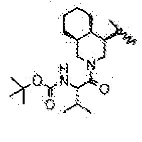 | 3.38 | 670 | A | 8 |
| 23 | 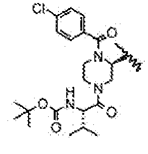 | 3.45 | 756 | A | 1 |
| 24 | 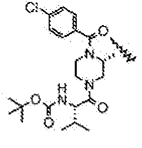 | 3.45 | 756 | A | 5 |
| 25 | 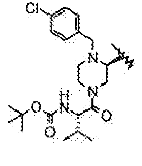 | 3.52 | 742 | A | 2 |
| 26 | 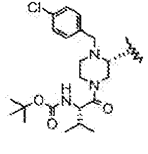 | 3.50 | 742 | A | 6 |
| 27 | 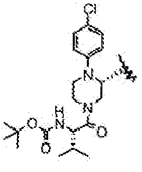 | 3.56 | 728 | A | 7 |

### Example 28

### (3R,4R)-3-[(1R,2S)-1-(1H-Indole-7-sulfonylaminocarbonyl)-2-vinyl-cyclopropylcarbamoyl]-4-phenethyl-pyrrolidine-1-carboxylic acid tert-butyl ester

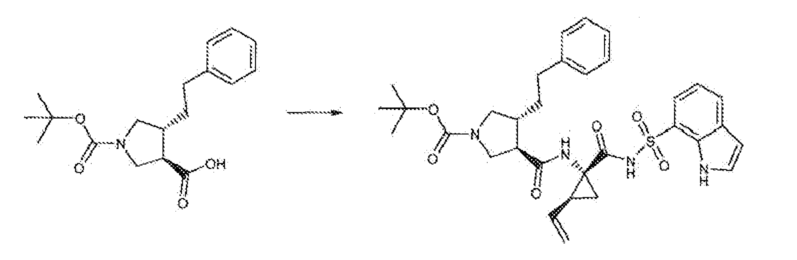

At 0°C, 394 mg (1.04 mmol) HBTU are added to a solution of 89 mg (0.28 mmol) (3R,4R)-4-phenethyl-pyrrolidine-1,3-dicarboxylic acid 1-tert-butyl ester (BB16), 114 mg (0.33 mmo;) 1,1-indole-7-sulfonic acid (1-amino-cyclopropanecarbonyl)-amide hydrochloride (BB27) and 146 µl (0.84 mmol) DIPEA and the reaction is stirred at room temperature for 72 hours. The reaction is evaporated to dryness and taken up in EtOAc and 1N HCl. The phases are separated and the aqueous phase is extracted with EtOAc. The combined organic phases are washed with sat. aq. NaHCO₃ and brine, dried with Na₂SO₄ and evaporated to dryness. The residue is chromatographed by preparative reverse phase HPLC (CH₃CN, H₂O, HCO₂H) to give (3R,4R)-3-[(1R,2S)-1-(1H-indole-7-sulfonylaminocarbonyl)-vinyl-cyclopropylcarbamoyl]-4-phenethyl-pyrrolidine-1-carboxylic acid tert-butyl ester as an off-white solid.
HPLC (method C): Rt = 4.025 min, MS (method F): M+Na = 629.2, M-H = 605.2.

### Example 29

### (3R,4R)-3[(1r,2S)-1-(1H-Indole-7-sulfonylaminocarbonyl)-2-vinyl-cyclopropylcarbamoyl]-4-(2-naphthalen-1-yl-ethyl)-pyrrolidine-1-carboxylic acid tert-butyl ester

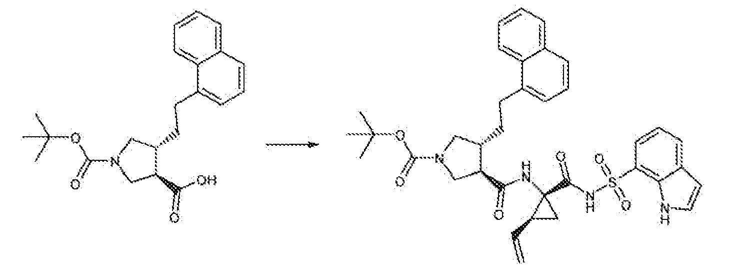

(3R,4R)-3-[(1R,2S)-1-(1H-Indole-7-sulfonylaminocarbonyl)-2-vinyl-cyclopropylcarbamoyl]-4-(2-naphthalen-1-yl-ethyl)-pyrrolidine-1-carboxylic acid tert-butyl ester is prepared in an analogous fashion as (3R,4R)-3-[(1R,2S)-1-(1H-indole-7-sulfonylaminocarbonyl)-2-vinyl-cyclopropylcarbamoyl]-4-phenethyl-pyrrolidine-1-carboxylic acid tert-butyl ester starting from 200 mg (0.54 mmol) (3R,4R)-4-(2-naphthalen-1-yl-ethyl)-pyrrolidine-1,3-dicarboxylic acid 1-tert-butyl ester (BB17) and 200 mg (0.59 mmol) 1H-indole-7-sulfonic acid (1-amino-cyclopropanecarbonyl)-amide hydrochloride (BB27). HPLC (method D): Rt = 4.228 min, MS (method F): M+H-Boc = 557.2, M-H = 655.2.

### Example 30

### (3R,4R)-3-[(1R,2S)-1-(1H-Indole-7-sulfonylaminocarbonyl)-2-vinyl-cyclopropylcarbamoyl]-4-(2-naphthalen-2-yl-ethyl)-pyrrolidine-1-carboxylic acid tert-butyl ester

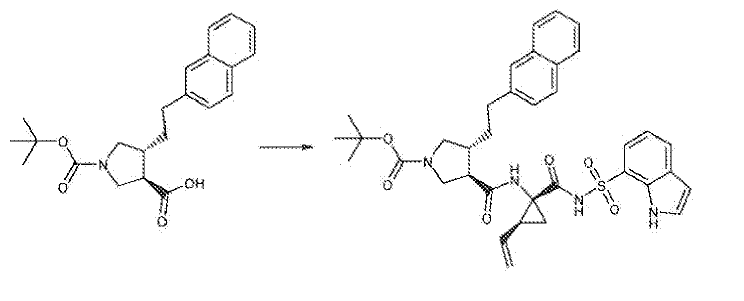

(3R,4R)-3-[(1R,2S)-1-(1H-Indole-7-sulfonylaminocarbonyl)-2-vinyl-cyclopropylcarbamoyl]-4-(2-naphthalen-2-yl-ethyl)-pyrrolidine-1-carboxylic acid tert-butyl ester is prepared in an analogous fashion as (3R,4R)-3-[(1R,2S)-1-(1H-indole-7-sulfonylaminocarbonyl)-2-vinyl-cyclopropylcarbamoyl]-4-phenethyl-pyrrolidine-1-carboxylic acid tert-butyl ester starting from 320 mg (0.87 mmol) (3R,4R)-4-(2-naphthalen-2-yl-ethyl)-pyrrolidine-1,3-dicarboxylic acid 1-tert-but-yl ester (BB18) and 333 mg (1.04 mmol)1H-indole-7-sulfonic acid (1-amino-cyclopropanecarbonyl)-amide hydrochloride (BB27). HPLC (method D): Rt = 4.279 min, MS (method F): M+Na = 679.3, M+H-Boc = 557.2, M-H = 655.3.

### Examples 31 and 32

The following two compounds were prepared from racemic, diasteromerically pure (3r*,4S*)-4-(3,5-Bis-trifluoromethyl-phenyl)-pyrrolidine-1,3-dicarboxylic acid 1-tert-butyl ester (BB 19)in an analogous fashion as described for example 30. The resulting two diastereomers were separated by preparative HPLC and the absolute stereochemistry on the proline was not assigned.

### trans-3-(3,5-Bis-trifluoromethyl-phenyl)-4-[(1R,2S)-1-(1H-indole-7-sulfonylaminocarbonyl)-2-vinyl-cyclopropylcarbamoyl]-pyrrolidine-1-carboxylic acid tert-butyl ester, Isomer 1

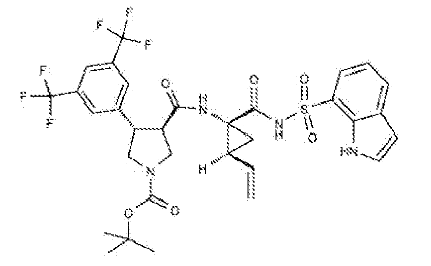

HPLC (method D): Rt = 6.08 min; MS (method F): M-H = 713.

### trans-3-(3,5-Bis-trifluoromethyl-phenyl)-4-[(1R,2S)-1-(1H-indole-7-sulfonylaminocarbonyl)-2-vinyl-cyclopropylcarbarnoyl]-pyrrolidine-1-carboxylic acid tert-butyl ester, Isomer 2

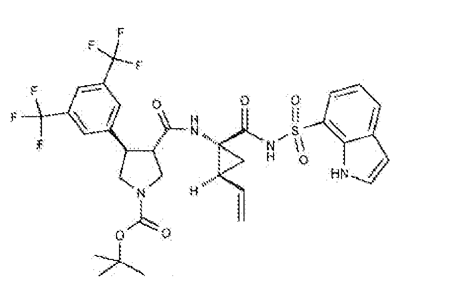

HPLC (method D): Rt = 6.02 mm; MS (method F): M-H = 713.

### Examples 33 and 34

The following two compounds were prepared from racemic, diasteromerically pure (3R*,4S*)-4-(4-Trifluoromethyl-phenyl)-pyrrolidine-1,3-dicarboxylic acid 1-tert-butyl ester in an analogous fashion as described for example 30. The resulting two diastereomers were separated by preparative HPLC and the absolute stereochemistry on the proline was not assigned.

### trans-3-[(1R,2S)-1--(1H-Indole-7-sulfonylaminocarbonyl)-2-vinyl-cyclopropylcarbamoyl]-4-(4-trifluoromethyl-phenyl)-pyrrolidine-1-carboxylic acid tert-butyl ester, Isomer 1

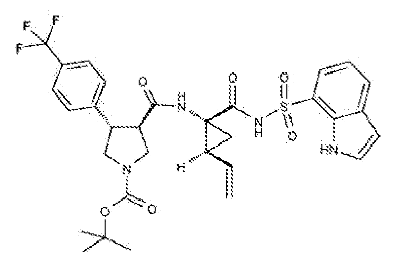

HPLC (method D): Rt = 5.82 mm; MS (method F): M-H = 645.

### trans-3-[(1R,2S)-1-(1H-indole-7-sulfonylaminocarbonyl)-2-vinyl-cyclopropykarbamoyl]-4-(4-trifluoromethyl-phenyl)-pyrrolidine-1-carboxylic acid tert-butyl ester, Isomer 2

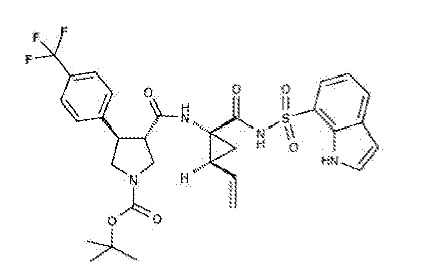

HPLC (method D): Rt = 5.82 min; MS (method F): M-H = 645.

### Example 35 and 36

The following two compounds were prepared from (3R,4S)-4-(4-Chloro-phenyl)-pyrrolidine-1,3-dicarboxylic acid 1-tert-butyl ester (for preparation see US 2005/0176772 and WO 2005/040109) in an analogous fashion as described for example 30.

### (3S,4R)-3-(4-Chloro-phenyl)-4-[(1R,2S)-1-(1H-indole-7-sulfoylaminocarbonyl)-2-vinyl-cyclopropylcarbamoyl]-pyrrolidine-1-carboxylic acid tert-butyl ester

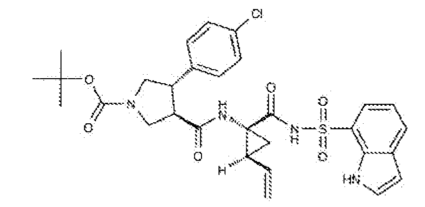

HPLC (method D); Rt = 5.81 min; MS (method F): M-H = 612.

### (3R,4S)-3-[(1R,2S)-1-(3-Benzyloxy-benzenesulfonylaminocarbonyl)-2-vinyl-cyclopropylcarbamoyl]-4-(4-chloro-phenyl)-pyrrolidine-1-carboxylic acid tert-butyl ester

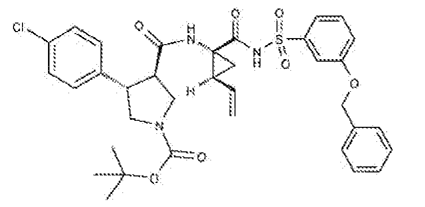

HPLC (method D): Rt = 6.15 min; MS (method F): M-1 = 679.

### Example 37

### [(1S,2R,4R)-2-[(1R,2S)-1-(3-Benzyloxy-benzenesulfonylaminocarbonyl)-2-vinyl-cyclopopylcarbamoyl]-4-(7-methoxy-2-phenyl-quinolin-4-yloxy)-cyclopentyl]-carbamic acid tert-butyl ester

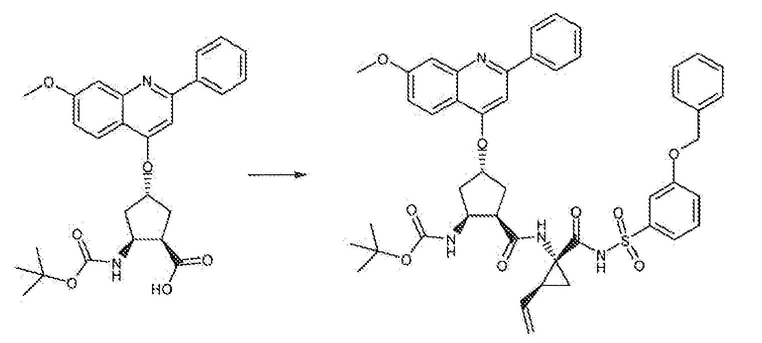

At 0°C, 171 mg (0.45 mmol) HBTU are added to a solution of 180 mg (0.376 mmol) (1R,2S,4R)-2-tert-butoxycarbonylamino-4-(7-methoxy-2-phenyl-quinolin-4-yloxy)-cyclopentanecarboxylic acid (BB20), 195 mg (0.414 mmol) *N*-((1*R*, 2*S*)-1-amino-2-vinyl-cyclopropanecarbonyl)-3-benzyloxy-benzenesulfonamide trifluoroacetate (BB29) and 197 µl (0.451 mmol) DIPEA and the reaction is stirred at room temperature overnight. The reaction is evaporated to dryness and taken up in EtOAc and 1N HCl. The phases are separated and the aqueous phase is extracted twice with EtOAc. The combined organic phases are washed with sat. aq. NaHCO₃ and brine, dried with Na₂SO₄ and evaporated to dryness. The residue is chromatographed by preparative reverse phase HPLC (CH₃CN, H₂O, HCO₂H) to give [(1S,2R,4R)-2-[(1R,2S)-1-(3-benzyloxy-benzenesulfonylaminocarbonyl)-2-vinyl-cyclopropylcarbamoyl]-4-(7-methoxy-2-phenyl-quinolin-4-yloxy)-cyclopentyl]-carbamic acid tert-butyl ester as a white solid. HPLC (method D): Rt = 3.645 min, MS (method F): M+H = 831.0.

### Example 38

### [(1S,2R,4R)-2-[(1R,2S)-1-(1H-Indole-7-sulfonylaminocarbonyl)-2-vinyl-cyclopropylcarbamoyl]-4-(7-methoxy-2-phenyl-quinolin-4-yloxy)-cyclopentyl]-carbamic acid tert-butyl ester

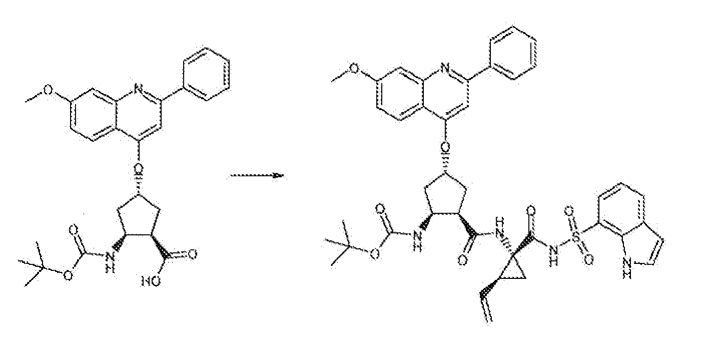

To a solution of 75 mg (0.16 mmol) (1R,2S,4R)-2-tert-Butoxycarbonylamino-4-(7-methoxy-2-phenyl-quinolin-4-yloxy)-cyclopentanecarboxylic acid (BB20) 75 mg (0.22 mmol) 1H-Indole-7-sulfonic acid ((1R,2S)-1-amino-2-vinyl-cyclopropanecarbonyl)-amide (BB27) and 82 □1 (0.47 mmol) DIPEA in DMF (2 mL) is added 71 mg (0.22 mmol) TBTU and the reaction is stirred overnight at RT. the reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC to give the title compound. HPLC (method D) Rt = 5.85 min; MS (method F): 766 [M+H].

### Example 39

### (1R,3S,4R)-2-Acetylamino-4-(7-methoxy-2-phenyl-quinolin-4-yloxy)-cyclopentanecarboxylic acid [(1R,2S)-1-(1H-indole-7-sulfonylaminocarbonyl)-2-vinyl-cyclopropyl]-amide

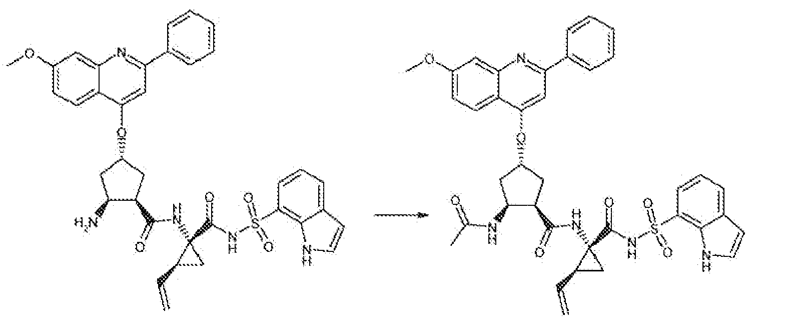

To a solution of 5 mg (0.073 mmol) (1R,2S,4R)-2-Amino-4-(7-methoxy-2-phenyl-quinolin-4-yloxy)-cyclopentanecarboxylic acid [(1R,2S)-1-(1H-indole-7-sulfonylaminocarbonyl)-2-vinyl-cyclopropyl]-amide (BB21), 13 mg (0.22 mmol) AcOH and 64 µl (0.36 mmol) DIPEA in DMF (2 mL) is added 70 mg (0.22 mmol) TBTU and the reaction is stirred overnight at RT. The reaction is diluted with EtOAc, washed with aq. saturated bicarbonate and brine, dried with Na₂SO₄, filtered and the solvent is removed in vacuo. The residue is purified by preparative reverse phase HPLC to give the title compound. HPLC (method D) Rt = 5.37 min; MS (method F): 708 [M+H].

### Example 40

### (1R,2S,4R)-2-Acetylamino-4-(7-Methoxy-2-phenyl-quinolin-4-yloxy)-cyclopentanecarboxylic acid [(1R,2S)-1-(3-benxyloxy-benzenesulfonylaminocarbonyl)-2-vinyl-cyclopropyl]-amide

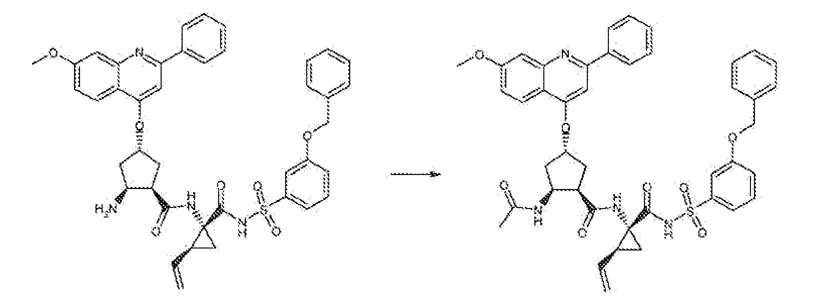

To a solution of 70 mg (0.09 mmol) (1R,2S,4R)-2-Amino-4-(7-methoxy-2-p henyl-quinolin-4-yloxy)-cyclopentancearboxylic acid [(1R,2S)-1-(3-benzyloxy-benzenesulfonylaminocarbonyl)-2-vinyl-cyclopropyl]-amide (BB22), 16 □L (0.27 mmol) AcOH and 80 µl (0.46 mmol) DIPEA in DMF (1 mL) is added 88 mg (0.27 mmol) TBTU and the reaction is stirred overnight at RT. The reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC to give the title compound. HPLC (method D) Rt = 5.65 min; MS (method F): 775 [M+H].

### Example 41

### [(1S,2R,4S)-2-[(1R,2S)-1-(3-Benzyloxy-benzenesulfonylaminocarbonyl)-2-vinyl-cyclopropylcarbamoyl]4-(7-methoxy-2-phenyl-quinolin-4-yloxy)-cyclopentyl]-carbamic acid tert-butyl ester

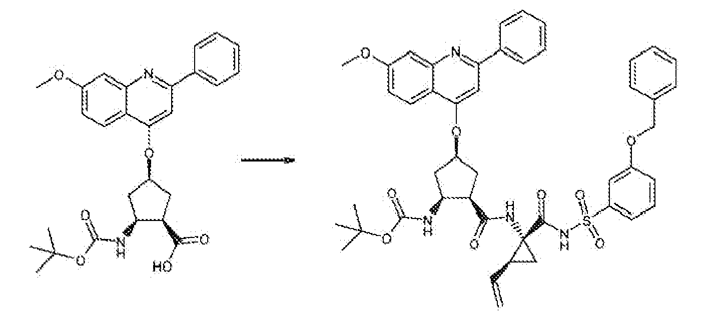

To a solution of 25 mg (0.05 mmol) ((1R,2S,4S)-2-tert-Butoxycarbonylamino-4-(7-methxy-2-phenyl-quinolin-4-yloxy)-cyclopentanecarboxylic acid (BB23), 27 mg (0.065 mmol) *N*-((1*R*, 2*S*)-1-amino-2-vinyl-cyclopropanecarbonyl)-3-benxyloxy-benzenesulfonamide (TFA-salt, BB29) and 27 µl (0.16 mmol) DIPEA in DMF (0.5 mL) is added 21 mg (0.065mmol) TBTU and the reaction is stirred overnight at RT. The reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC to give the title compound. HPLC (method D) Rt = 6.15 min; MS (method F): 833 [M+H].

### Example 42

### (1R,2S,4S)-2-Acetylamino-4-(7-Methoxy-2-phenyl-quinolin-4-yloxy)-cyclopentanecarboxylic acid [(1R,2S)-1-(3-benzyloxy-benzenesulfonylaminocarbonyl)-2-vinyl-cyclopropyl]-amide

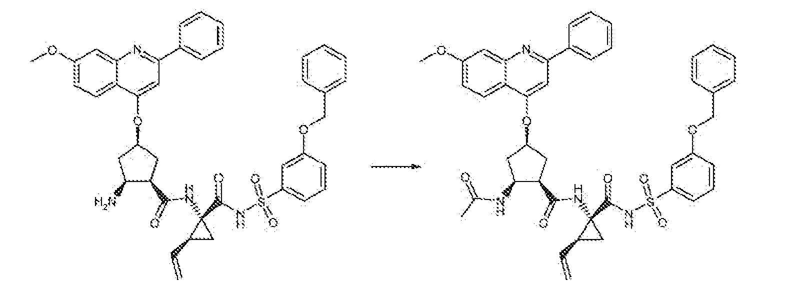

To a solution of 17 mg (0.022 mmol) (1R,2S,4S)-3-Amino-4-(7-methoxy-2-phenyl-quinolin-4-yloxy)-cyclopentanecarboxylic acid [(1R,2S)-1-(3-benzyloxybenzenesulfonyl-aminocarbonyl)-2-vinyl-cyclopropyl]-amide (BB24) 1.9 □L (0.033 mmol) AcOH and 12 µl (0.066 mmol) DIPEA in DMF (0.5 mL) is added 11 mg (0.033mmol) TBTU and the reaction is stirred overnight at RT. The reaction is diluted with EtOAc and 0.1N aq. HCl, extracted with EtOAc, washed with brine, dried with Na₂SO₄, filtered and the solvent ss removed in vacuo. The residue is purified by preparative reverse phase HPLC to give the title compound. HPLC (method D) Rt = 5.71 min; MS (method F): 775 [M+H].

### Example 43

### (S)--1-Napthalen-2-ylmethyl-pyrrolidine-3--carboxylic acid [(1R,2S)-1-(3-benxyloxy-benzenesulfonylaminocarbonyl)-2-vinyl-cyclopropyl]-amide

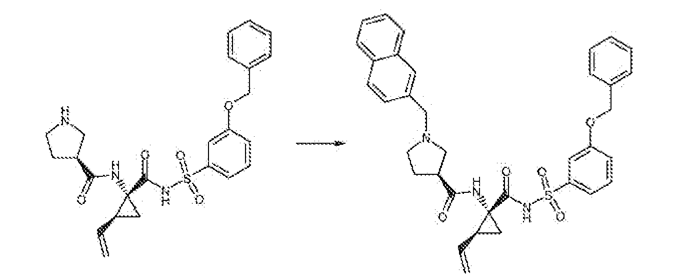

To solution of 100 mg (0.20 mmol) (S)-Pyrrolidine-3-carboxylic acid [(1R,2S)-1-(3₋benzyloxy-bonzenesulfonylamminocarbonyl)--2-vinyl-cyclopropyl]-amide (BB25), 46 mg (0.20 mmol) 2-Bromomethyl-naphthalene in DMF (1 mL) is added 83 mg (0.60 mmol) K₂CO₃ and the mixture is stirred overnight at RT. The reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC (method H) to give the title compound. HPLC (method G) Rt = 5.05 min; MS (method F): 610 [ M+H

### Example 44

### (S)-1-Biphenyl-3-ylmethyl-pyrrolidine-3-carboxylic acid [(1R,2S)-1-(3-benzyloxy-benzenesulfonylaminocarbonyl)-2-vinyl-cyclopropyl]-amide

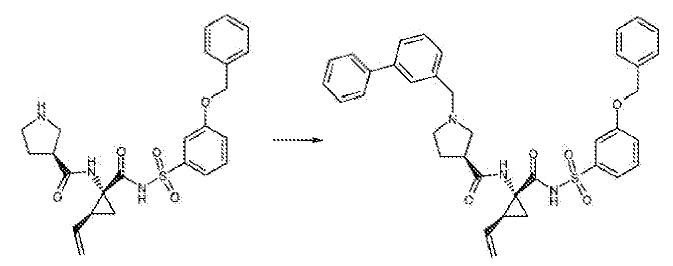

To a solution of 95 mg (0.19 mmol) (S)-Pyrrolidine-3-carboxylic acid [(1R,2S)-1-(3-benzyloxy-benzenesulfonylaminocarbonyl)-2-vinyl-cyclopropyl]-amide (BB25), 48 mg (0.19 mmol) 3-Phenylbenzyl bromide in DMF (1 mL) is added 79 mg (0.56 mmol) K₂CO₃ and the mixture is stirred 6 h at RT. The reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC (method H) to give the title compound. HPLC (method D) Rt = 5.85 min; MS (method F): 636 [M+H],

### Example 45

### (S)-1-Biphenyl-2-ylmethyl-pyrrolidine-3-carboxylic acid [(1R,2S)-1-(3-benzyloxy-benzenesulfonylaminocarbonyl)-2-vinyl-cyclopropyl]-amide

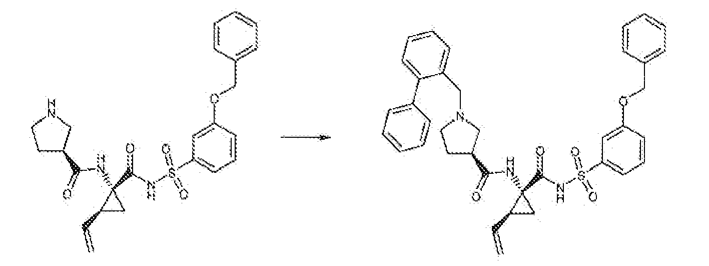

To a solution of 100 mg (0.20 mmol) (S)-Pyrrolidine-3-carboxylic acid [(1R,2S)-1-(3-benzyloxy-benzenesulfonylaminocarbonyl)-2-vinyl-cyclopropyl]-amide (BB25), 43 □L (0.24 mmol) 2-Phenylbenzyl bromide in DMF (1 mL) is added 83 mg (0.59 mmol) K₂CO₃ and the mixture is stirred overnight at RT. The reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC (method H) to give the title compound. HPLC (method D) Rt = 5.92 min; MS (method F): 636 [M+H].

### Example 46

### (S)-1-(6-Methoxy-naphthalen-1-ylmethyl)-pyrrolidine-3-carboxylic acid [(1R,2S)-1-(3-benzyloxy-benzenesulfonylaminocarbonyl)-2vinyl-cyclopropropyl]-amide

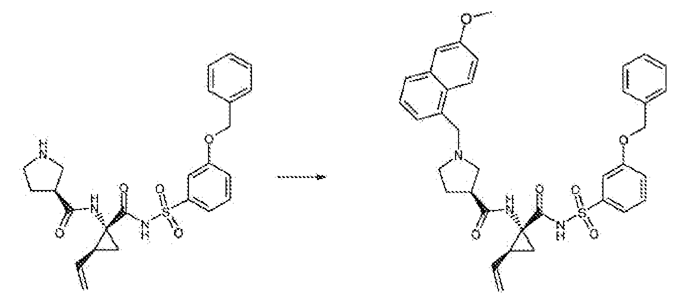

To a solution of 100 mg (0.20 mmol) (S)-Pyrrolidine-3-carboxylic acid [(1R,2S)-1-(3-benzyloxy-benzenesulfonylaminocarbonyl)-2-vinyl-cyclopropyl]-amine (BB25), 58 mg (028 mmol) 1-Chloromethyl-6-methoxy-naphthalene in DMF (1.5 mL) is added 83 mg (0.59 mmol) K₂CO₃ and the mixture is stirred overnight at RT. The reaction is diluted with EtOAc and 0.1N aq. HCl, extracted with BtOAc, washed with brine, dried with Na₂SO₄, filtered and the solvent is removed in vacuo. The residue is purified by preparative reverse phase HPLC (method H) to give the title compound. HPLC (method D) Rt = 5.87 min; MS (method F): 640 [M+H].

### Example 47

### (3R*,4R*)-4-(4-Chloro-benzyl)-1-naphalen-1-ylmethyl-pyrrolidine-3-carboxylic acid [(1R,2S)-1-(3-benzyloxy-benzenesulfonylaminocarbonyl)-2-vinyl-cyclopropyl]-amide

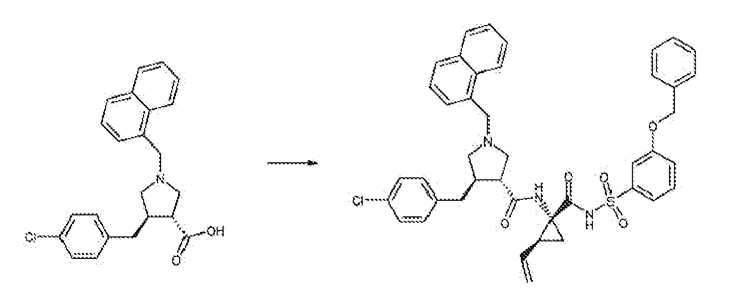

To a solution of 125 mg (0.33 mmol) (3R*,4R*)-4-(4-Chloro-benzyl)-1-naphthalen-1-ylmethyl-pyrrolidine-3-carboxylic acid (BB13), 175 mg (0.43 mmol) N-((1R,2S)-1-Amino-2-vinyl-cyclopropanecarbonyl)-3-benzyloxy-benzenesulfonamide (BB29) and 144 µl (0.82 mmol) DIPEA in DMF (2 mL) is added 148 mg (0.46 mmol) TBTU and the reaction is stirred overnight at RT. The reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC (method H) to give the title compound. HPLC (method D) Rt = 6.87 min; MS (method F): 734 [M+H].

### Example 48

### {(3S*,4R*)-4-[(1R,2S)-1-(3-Benzyloxy-benzenesulfonylaminocarbonyl)-2-vinyl-cyclopropylcarbamoyl]-1-naphthalen-1-ylmethyl-pyrrolidin-3-yl}-carbamic acid tert-butyl ester

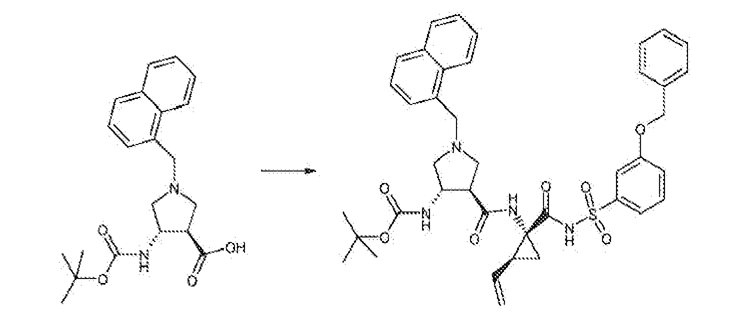

To a solution of 400 mg (1.1 mmol) (3R*,4R*)-4-tert-Butoxycarbonylamino-1-naphthalen-1-ylmethyl-pyrrolidine-3-carboxylic acid (BB26), 569 mg (1.4 mmol) N-((1R,2S)-1-Amino-2-vinyl-cyclopropanccarbonyl)-3-bezyloxy-benzenesulfonamide (BB29) and 566 µl (3.2 mmol) DIPEA in DMF (8 mL) is added 438 mg (1.4 mmol) TBTU and the reaction is stirred overnight at RT. The reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC (method H) to give the title compound. HPLC (method D) Rt = 6.30 min; MS (method F): 725 [M+H].

### Synthesis of building blocks used in Examples 1-48:

### (R)-4-((S)-2-tert-Butoxycarbonylamino-3-methyl-butryl)-1-(4-chloro-benzoyl)-piperazine-2-carboxylic acid (BB 1)

### Step 1

### (R)-4-(4-Chloro-benzoyl)-piperazine-1,3-dicarboxylic acid 1-tert-butyl ester 3-methyl ester

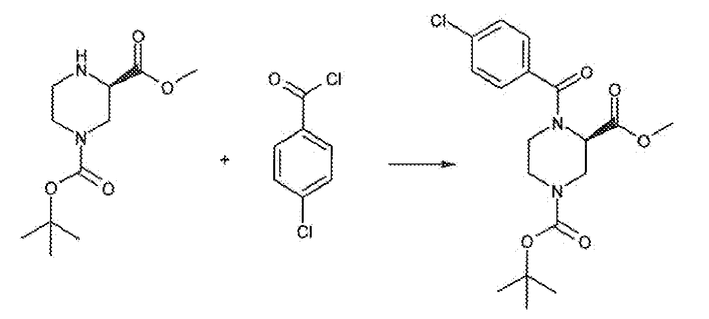

At 0°C, 0.47 ml (3.68 mmol) 4-chlorobenzoyl chloride are added to a solution of 750 mg (3.07 mmol) (R)-piperazine-1,3-dicarboxylic acid 1-tert-butyl ester 3-methyl ester, 0.64 ml (4.60 mmol) triethylamine and 19 mg (0.15 mmol) DMAP in 15 ml CH₂Cl₂ and the resulting mixture is stirred at room temperature overnight. 15 ml 1N HCl are added and the phases are separated. The organic phase is washed with brine, dried with Na₂SO₄ and concentrated under reduced pressure. The residue is chromatographed on SiO₂ (eluent hexanes/EtOAc) to give (R)-4-(4-chloro-benzoyl)-piperazine-1,3-dicarboxylic acid 1-tert-butyl ester 3-methyl ester LC-MS (method C) Rt = 4.040 min, M+Na = 405.0.

### Step 2

### (R)-4-(4-Chloro-benzoyl)-piperazine-1,3-dicarboxylic acid 1-tert-butyl ester 3-methyl ester trifluoroacetate

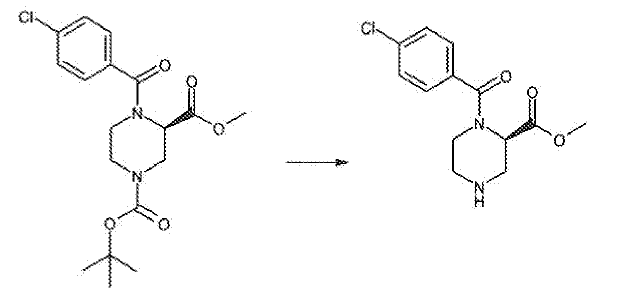

A solution of 1.02 g (2.66 mmol) (R)-4-(4-chloro-benzoyl)-piperazine-1,3-dicarboxylic acid 1-tert-butyl ester 3-methyl ester, 6.2 ml trifluoroacetic acid and 15 ml CH₂Cl₂ is stirred at room temperature for 4 hours. The reaction is concentrated in vacuo to afford (R)-4-(4-chloro-benzoyl)-piperazine-1,3-dicarboxylic acid 1-tert-butyl ester 3-methyl ester trifluoroacetate. LC-MS (method C) Rt = 0.485 min, M+1 = 283.1.

### Step 3

### (R)4-((S)-2-tert-Butoxycarbonylamino-3-methyl-butyryl)-1-(4-chloro-benzoyl)-piperazine-2-carboxylic acid methyl ester

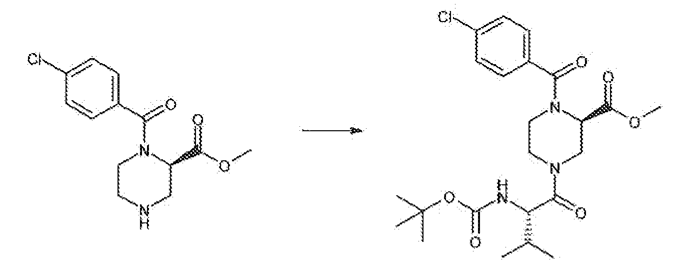

To a solution of 353 mg (0.89 mmol) (R)-4-(4-chloro-benzoyl)-piperazine-1,3-dicarboxylic acid 1-tert-butyl ester 3-methyl ester trifluoroacetate, 154 mg (1.07 mmol) (S)-2-tert-butoxycarbonylamino-3-methyl-butyric acid and 0.62 ml (3.56 mmol) DIPEA in 4.0 ml DMF are added 404 mg (1.07 mmol) HBTU at 0°C. The reaction mixture is allowed to warm to room temperature and is stirred for 12 hours. 10 ml EtOAc are added and the organic phase is washed once with 1N HCl and twice with sat. NaHCO₃ (aq.). The organic layer is dried with MgSO₄ and concentrated in vacuo. The residue is chromatographed on SiO₂ (eluent hexanes/EtOAc) to give (R)-4-((S)-2-tert-butoxycarbonylamino-3-methyl-butyryl)-1-(4-chloro-benzoyl)-pipepazine-2-carboxylic acid methyl ester. LC-MS (method C): Rt = 4.091 min, M+Na = 504.0, M+H = 482.1.

### Step 4

### (R)-4-((S)-2-tert-Butoxycarbonylamino-3-methyl-butyryl)-1-(4-chloro-benzoyl)-piperazine-2-carboxylic acid

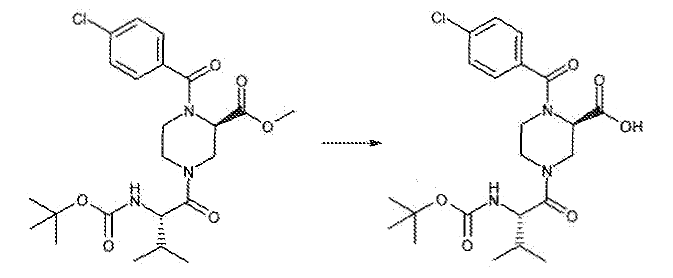

315 mg (0.65 mmol) (R)-4-((S)-2-tert-Butoxycarbonylamino-3-methyl-butyryl)-1-(4-chloro-benzoyl)-piperazine-2-carboxylic acid methyl ester are added to a solution of 36 mg (0.85 mmol) LiOH* H₂O in 3 ml of a THF/MeOH/H2O-mixture (2:1:1) and the reaction is stirred at room temperature overnight. The reaction is neutralized with 1N HCl, concentrated in vacuo and taken up in 10 ml EtOAc and 10 ml H₂O. The phases are separated and the aqueous phase is extracted twice with EtOAc. The combined organic phases are dried with MgSO₄ and concentrated in vacuo to afford (R)-4-((S)-2-tert-butoxycarbonylamino-3-methyl-butyryl)-1-(4-chloro-benzoyl)-piperazine-2-carboxylic acid as a white solid. LC-MS (method C) Rt = 3.760 min, M+Na = 490.1, M+H=468.0, M-H=466.1.

### (R)-4-((S)-2-tert-Butoxycarbonylamino-3-methyl-butyryl)-1-(4-chloro-benzyl)-piperazine-2-carboxylic acid (BB 2)

### Step 1

### (R)-1-(4-Chloro-benzyl)-piperazine-2-carboxylic acid methyl ester trifluoroacetate

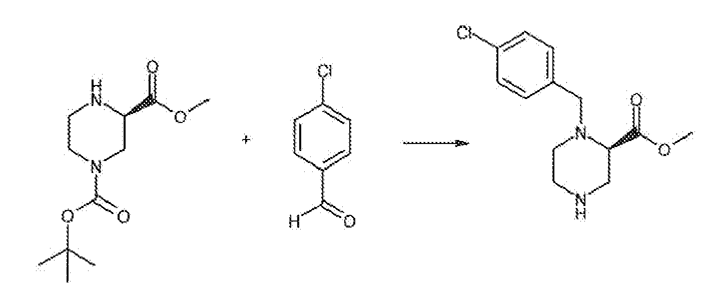

679 mg (2.78 mmol) (R)-Piperazine-1,3-dicarboxylic acid 1-tert-butyl ester 3-methyl ester and 391 mg (2.78 mmol) 4-chlorobenzaldehyde are dissolved in 10 ml CH₂Cl₂ and the mixture is stirred for 30 min. Sodium triacetoxyborohydride (843 mg, 3.78 mmol) is added and the reaction is stirred at room temperature overnight H₂O is added and the phases are separated. The aqueous phase is extracted with 10 ml CH₂Cl₂ and the combined organic layers are dried with Na₂SO₄. The CH₂Cl₂-solution is treated with 30 ml trifluoroacetic acid. After 4 hours the reaction mixture is concentrated in vacuo to yield (R)-1-(4-chloro-benzyl)-piperazine-2-carboxylic acid methyl ester trifluoroacetate as a colorless oil. LC-MS (method C) Rt = 0.909 min, M+H = 269.0.

### Step 2

### (R)-4-((S)-2-tert-Butoxycarbonylamino-3-methyl-butyryl)-1-(4-chloro-benzyl)-pipcrazine-2-carboxylic acid methyl ester

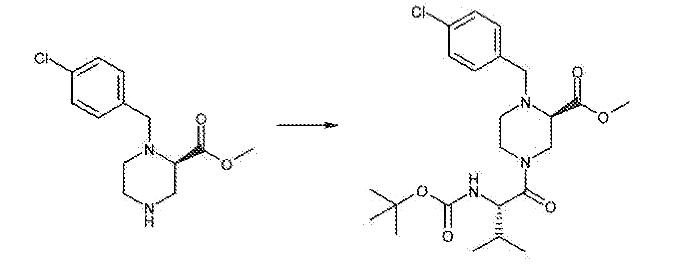

To a solution of 395 mg (1.03 mmol) (R)-1-(4-chloro-benzyl)-piperazine-2-carboxylic acid methyl ester trifluoroacetate, 179 mg (1.24 mmol) (S)-2-tert-butoxycarbonylamino-3-methyl-butyric acid and 0.72 ml (4.13 mmol) DIPEA in 5 ml DMF are added 470 mg (1.24 mmol) HBTU at 0°C. The reaction mixture is allowed to warm to room temperature and is stirred for 12 hours. 10 ml EtOAc are added and the organic phase is washed once with H₂O and twice with sat. NaHCO₃ (aq.). The organic phase is dried with MgSO₄ and concentrated in vacuo. The residue is chromatographed on SiO₂ (eluent hexanes/EtOAc) to give (R)-4-((S)-2-tert-butoxycarbonylamino-3-methyl-butyryl)-1-(4-chloro-benzyl)-piperazine-2-carboxylic acid methyl ester. LC-MS (method C) Rt = 4.601 min, M+H = 468.0.

### Step 3

### (R)-4-((S)-2-tert-Butoxycarbonylamino-3-methyl-butyryl)-1-(4-chloro-benzyl)-piperazine-2-carboxylic acid

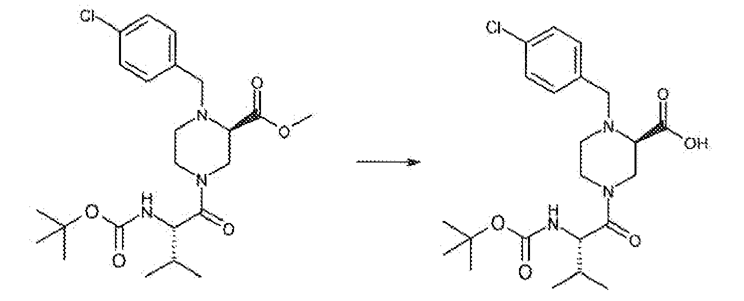

240 mg (0.51 mmol) (R)-4-((S)-2-tert-Butoxycarbonylamino-3-methyl-butyryl)-1-(4-chloro-benzyl)-piperazine-2-carboxylic acid methyl ester are added to a solution of 26 mg (0.62 mmol) LiOH* H₂O in 2 ml of a THF/MeOH/ H₂O-mixture (2:1:1) and the reaction is stirred at room temperature overnight. The reaction is neutralized with 1N HCl, concentrated in vacuo and taken up in 10 ml EtOAc and 10 ml H₂O. The phases are separated and the aqueous phase is extracted twice with EtOAc. The combined organic phases are dried with MgSO₄ and concentrated in vacuo to afford (R)-4-((S)-2-tert-butoxycarbonylamino-3-methyl-butyryl)-1-(4-chloro-benzyl)-piperazine-2-carboxylic acid as a white solid. LC-MS (method C) Rt = 3.267 min, M+H = 454.1, M-H = 452.2.

### (R)-4-((S)-2-tert-Butoxycarbonylamino-3-methyl-butyryl)-1-(4-chloro-phenyl)-piperazine-2-carboxylic acid (BB 3)

### Step 1

### (R)-4-(4-Chloro-phenyl)-piperazine-1,3-dicarboxylic acid 1-tert-butyl ester 3-methyl ester

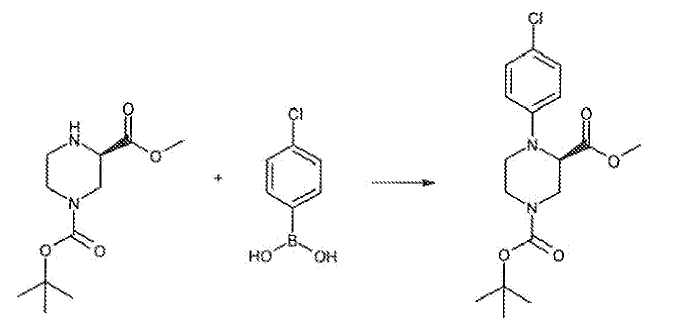

1.33 g (5.5 mmol) (R)-Piperazine-1,3-dicarboxylic acid 1-tert-butyl ester and 1.71 g (10.9 mmol) 4-chlorophenylboronic acid are mixed in 17 ml CH₂Cl₂ followed by the addition of 0.99 g (5.5 mmol) cupric acetate, 0.4 g 4Å molecular sieves and 0.88 ml (10.9 mmol) pyridine. The mixture is stirred at room temperature for 50 hours, concentrated in vacuo and taken up in EtOAc. After filtration through Celite, the reaction mixture is concentrated in vacuo. The residue is chromatographed on SiO₂ (eluent hexanes/EtOAc) to give (R)-4-(4-chloro-phenyl)-piperazine-1,3-dicarboxylic acid 1-tert-butyl ester 3-methyl ester. LC-MS (method C) Rt = 4.486 min, M+M = 355.1.

### Step 2

### (R)-1-(4-Chloro-phenyl)-piperazine-2-carboxylic acid methyl ester trifluoroacetate

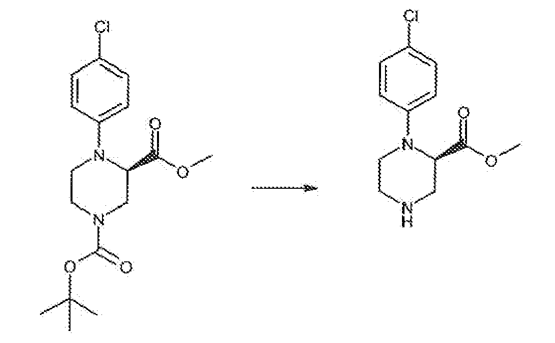

A solution of 1.02 g (2.66 mmol) (R)-4-(4-chloro-phenyl)-piperazine-1,3-dicarboxylic acid 1-tert-butyl ester 3-methyl ester, 13 ml trifluoroacetic acid and 30 ml CH₂Cl₂ is stirred at room temperature for 4 hours. The reaction is concentrated in vacuo to afford (R)-1-(4-chloro-phenyl)-piperazine-2-carboxylic acid methyl ester trifluoroacetate. LC-MS (method C) Rt = 0.748 min, M+1 = 255.1.

### Step 3

### (R)-4-((S)-2-tert-Butoxycarbonylamino-3-methyl-butyryl)-1-(4-chloro-phenyl)-piperazine-2-carboxylic acid methyl ester

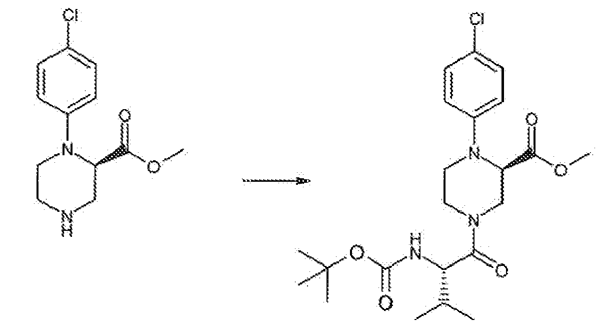

To a solution of 468 mg (1.27 mmol) (R)-1-(4-chloro-phenyl)-piperazine-2-carboxylic acid methyl ester trifluoroacetate, 220 mg (1.52 mmol) (S)-2-tert-butoxycarbonylamino-3-mothyl-butyric acid and 0.89 ml (5.08 mmol) DIPEA in 7 ml DMF are added 578 mg (1.52 mmol) HBTU at 0°C. The reaction mixture is allowed to warm to room temperature and is stirred for 12 hours. 10 ml EtOAc are added and the organic phase is washed once with H₂O and twice with sat. NaHCO₃ (aq.). The organic layer is dried with MgSO₄ and concentrated in vacuo. The residue is chromatographed on SiO₂ (eluent hexanes/EtOAc) to give (R)-4-((S)-2-tert-butoxycarbonylamino-3-methyl-butyryl)-1-(4-chloro-phenyl)-piperazine-2-carboxylic acid methyl ester. LC-MS (method C) Rt = 4.482 min, M+Na = 476.0, M+H=454.1.

### Step 4

### (R)-4-((S)-2-tert-Butoxycarbonlylamino-3-methyl-butyryl)-1-(4-chloro-phenyl)-piperazine-2-carboxylic acid

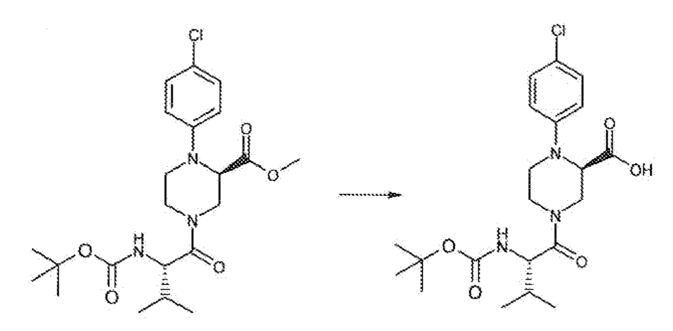

468 mg (1.03 mmol) (R)-4-((S)-2-tert-Butoxycarbonylamino-3-methyl-butyryl)-1-(4-chloro-phenyl)-piperazine-2-carboxylic acid methyl ester are added to a solution of 57 mg (1.34 mmol) LiOH* H₂O in 4 ml of a THF/MeOH/H₂O-mixture (2:1:1) and the reaction is stirred at room temperature overnight. The reaction is neutralized with 1N HCl, concentrated in vacuo and taken up in 10 ml EtOAc and 10 ml H₂O. The phases are separated and the aqueous phase is extracted twice with EtOAc. The combined organic phases are dried with MgSO₄ and concentrated in vacuo to afford (R)-4-((S)-2-tert-butoxycarbonylamino-3-methyl-butyryl)-1-(4-chloro-phenyl)-piperazine-2-carboxylic acid as a white solid. LC-MS (method C) Rt = 4.022 min, M+H = 440.1, M-H = 438.1.

### (S)-4-Acetyl-1-(4-chloro-benzoyl)-piperazine-2-carboxylic acid (BB 4)

### step 1

### (S)-4-Acetyl-1-(4-chloro-benzoyl)-piperazine-2-carboxylic acid methyl ester

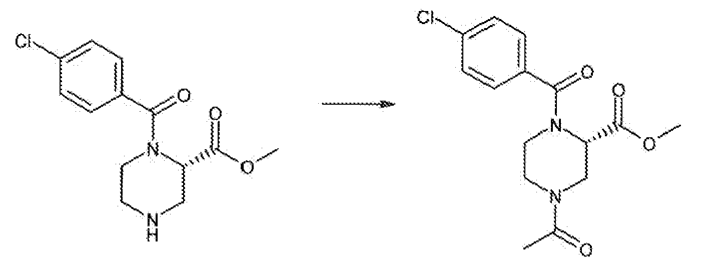

At 0°C, 61 µl (0.86 mmol) acetyl chloride are added to a solution of 202 mg (0.71 mmol) (S)-4-(4-chloro-benzoyl)-piperazine-1,3-dicarboxylic acid 1-tert-butyl ester 3-methyl ester trifluoroacetate (prepared in an analogous fashion as (R)-4-(4-chloro-benzoyl)-piperazine-1,3-dicarboxylic acid 1-tert-butyl ester 3-methyl ester trifluoroacetate starting from (S)-piperazine-1,3-dicarboxylic acid 1-tert-butyl ester 3-methyl ester) and 0.22 ml (1.57 mmol) triethylamine in 10 ml CH₂Cl₂ and the resulting mixture is stirred at room temperature overnight. 10 ml CH₂Cl₂ and 10 ml 1N HCl are added and the phases are separated. The organic phase is dried with Na₂SO₄ and concentrated in vacuo. The residue is chromatographed on SiO₂ (eluent hexanes/EtOAc) to give (S)-4-acetyl-1-(4-chloro-benzoyl)-piperazine-2-carboxylic acid methyl ester. LC-MS (method C) Rt = 2.735 min, M+Na = 347.0, M+H = 325.1.

### Step 2

### (S)-4-Acetyl-1-(4-chloro-benzoyl)-piperazine-2-carboxylic acid

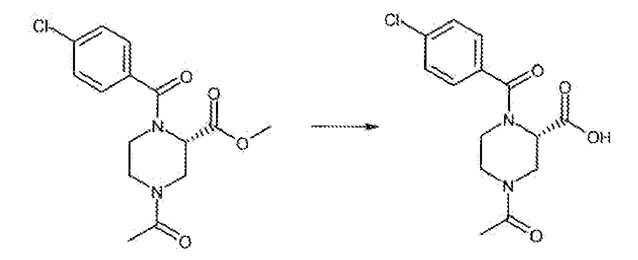

220 mg (0.68 mmol) (S)-4-Acetyl-1-(4-chloro-benzoyl)-piperazine-2-carboxylic acid methyl ester are added to a solution of 37 mg (0.88 mmol) LiOH* H₂O in 3 ml of a THF/MeOH/H₂O-mixture (2:1:1) and the reaction is stirred at room temperature overnight. The reaction is neutralized with 1N HCl, concentrated in vacuo and taken up in 10 ml THF and 10 ml brine. The phases are separated and the aqueous phase is extracted twice with THF. The combined organic phases are dried with Na₂SO₄ and concentrated in vacuo to afford (S)-4-acetyl-1-(4-chloro-benzoyl)-piperazine-2-carboxylic acid as a white solid. LC-MS (method C) Rt = 1.956 min, M+H = 311.0, M-H = 309.1.

The following compounds are prepared in a fashion similar to the analogous compounds described above starting from (S)-piperazine-1,3-dicarboxylic acid 1-tert-butyl ester 3-methyl ester instead of its enantiomer (R)-piperazine-1,3-dicarboxylic acid 1-tert-butyl ester 3-methyl ester:

### (S)-4-((S)-2-tert-Butoxycarbonylamino-3-methyl-butyryl)-1-(4-chloro-benzoyl)-pipeyazine-2-carboxylic acid (BB 5)

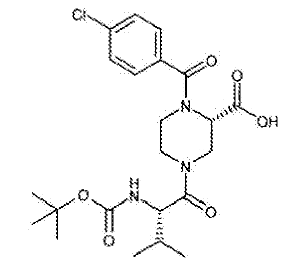

LC-MS (method C) Rt = 3.710 min, M+H-Boc = 368.0, M-H = 466.1.

### (S)-4-((S)-2-tert-Butoxycarbonylamino-3-methyl-butyryl)-1-(4-chloro-benzyl)-pipcrazine-2-carboxylic acid (BB 6)

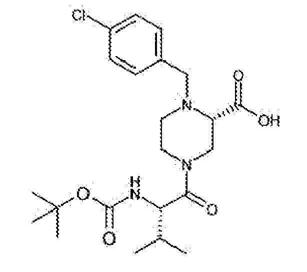

LC-MS (method C) Rt = 3.167 min, M+H = 454.1, M-H = 452.2.

### (S)-4-((S)-2-tert-Butoxycarbonylamino-3-methyl-butyryl)-1-(4-chloro-phenyl)-piperazine-2-carboxylic acid (BB 7)

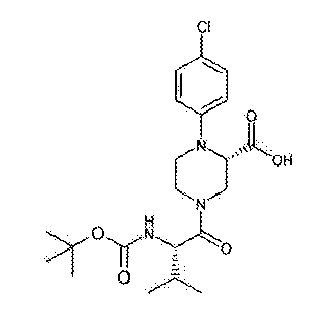

LC-MS (method C) Rt = 4.019 min, M+H = 440.1, M-H = 438.3.

### (4R,4aR,8aS)-2-((S)-2-tert-Butoxycarbonylamino-3-methyl-butyryl)-decahydro-isoquinoline-4-carboxylic acid (BB 8)

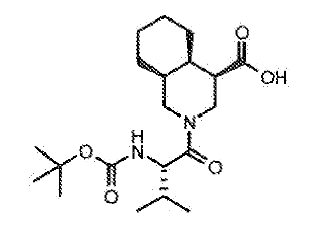

For preparation see M. Bänziger et al., Tetrahedron Asym. 2003, 14, 3469.

### (4S,4aS,8aR)-Octahydro-isoquinoline-2,4-dicarboxylic acid 2-tert-butyl ester (BB 9)

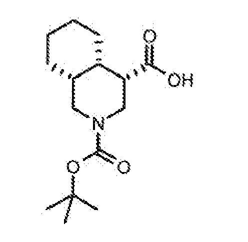

For preparation see M. Bänziger et al., Tetrahedron Asym. 2003, 14, 3469.

### (3R*,4S*)-1-((S)-2-tert-Butoxycarbonylamino-3-methyl-butyryl)-4-phenylpyrrolidine-3-carboxylic acid (BB 10)

### Step 1

### (3R*,4S*)-1-Benzyl-4-phenyl-pyrrolidine-3-carboxylic acid methyl ester

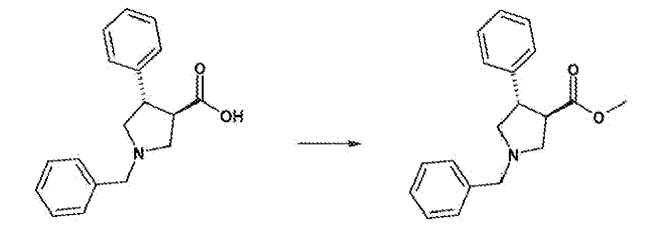

A solution of 3 g (10.66 mmol) of (3R*,4S*)-1-Benzyl-4-phenyl-pyrrolidine-3-carboxylic acid (for preparation see R. Achini, Hel. Chim. Acta 1981, 64, 2203.) in 50 mL methanol is treated with 0.5 mL of concentrated sulfuric acid and healed up to reflux overnight. The reaction mixture is concentrated, taken up in EtOAc, washed with aqueous NaHCO₃ and brine, and concentrated in vacuo to give (3R*,4S*)-1-Benzyl-4-phenyl-pyrrolidine-3-carboxylic acid methyl ester. TLC (95:5 CH₂Cl₂/EtOH) Rf = 0.50 ; MS (method F): M+H = 296.

### Step 2

### (3R*,4S*)-4-Pheny)-pyrrolidine-3-carboxylic acid methyl ester

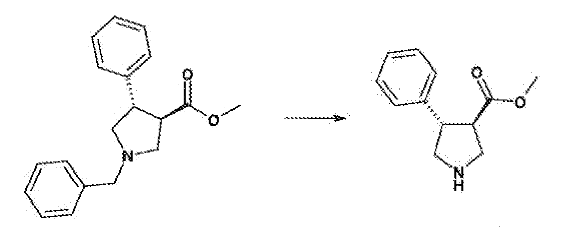

A solution of 2.84 g (9.615 mmol) of (3R*,4S*)-1-Benzyl-4-phenyl-pyrrolidine-3-carboxylic acid methyl ester in 50 mL methanol is shaken with 0.6 g of Pd (10% on carbon) under an H₂ atmosphere (1 atm) at RT until completion of the reaction. The catalyst is removed by filtration over Celite, washed with methanol. The filtrate is concentrated and the crude material purified by preparative reverse phase HPLC to give (3R*,4S*)-4-Phenyl-pyrrolidine-3-carboxylic acid methyl ester as its TFA salt. HPLC (method D): Rt = 3.78 min; MS (method F): M+H = 206.

### Step 3

### (3R*,4S*)-1-((S)-2-tert-Butoxycarbonylamino-3-methyl-butyryl)-4-pheny-pyri-oliditie-3-carboxylic acid methyl ester

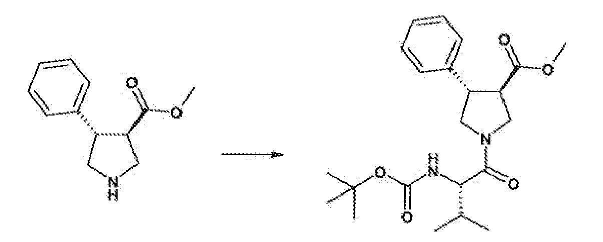

A mixture of 0.34 g (1.566 mmol) of BOC-L-valine in 15 mL CH₂Cl₂ is treated with 0.553 g(1.723 mmol) TBTU, followed by 0.3 mL of DIPEA. After 15 min the resulting solution is treated with a solution of 0.5 g (1.566 mmol) of (3R*,45*)-4-Phenylpyrrolidine-3-carboxylic acid methyl ester in 10 mL CH₂Cl₂ and 0.6 mL of DIPEA, and stirred overnight at RT. The reaction mixture is washed sequentially with 0.05N HCl, water, and saturated aqueous NaHCO₃. dried over Na₂SO₄ and concentrated in vacuo to (3R*,4S*)-1-((S)-2-tert-Butoxycarbonylamino-3-methyl-butyryl)-4-phenyl-pyrrolidine-3-carboxylic acid methyl ester as an oil. HPLC (method D): R1 = 5.50 min; MS (method F): M+H = 405.

### Step 4

### (3R*,4S*)-1-((S)-2-tert-Butoxycarbonylamino-3-methyl-butyryl)-4-phenylpyrrolidine-3-carboxylic acid

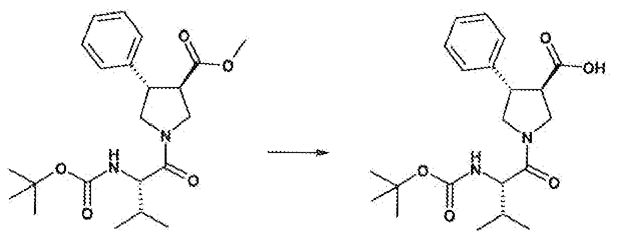

A solution of 0.6 g (1.483 mmol) of (3R*,4S*)-1-((S)-2-tert-Butoxycarrbonylamino-3-methyl-butyryl)-4-phenyl-pyrrlidine-3-carboxylic acid methyl ester in 10 mL THF is treated with methanol/water (5 mL + 5 mL) and 0.249 g (5.932 mmol) LiOH and allowed to stir at RT for 16 hours. The reaction mixture is concentrated, taken up in EtOAc, washed with 0.1N HCl and brine, dried over Na₂SO₄ and concentrated in vacuo to (3R*,4S*)-1-((S)-2-tert-Butoxycarbonylamino-3-methyl-butyryl)-4-phenyl-pyrrolidine-3-carboxylic acid as a white foam. HPLC (method D): Rt = 5.05 min; MS (method F): M+H=391.

### (3R*,4R*)-4-Benzyl-1-((S)-2-tert-butoxycarbonylamino-3-methyl-butyryl)-pyrrolidine-3-carboxylic acid (BB 11)

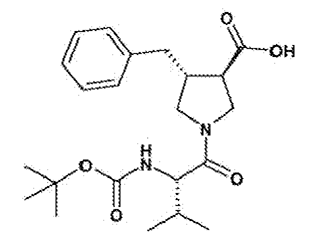

(3R*,4R*)-4-Benzyl-1-((S)-2-tert-butoxycarbonylamino-3-methyl-butyryl)-pyrrolidine-3-carboxylic acid is obtained according to the methods described for (3R*,4S*)-1-((S)-2-tert-Butoxycarbonylamino-3-methyl-butyryl)-4-phenyl-pyrrolidine-3-carboxylic acid starting from (3R*,4R*)-1,4-Dibenzyl-pyrrolidine-3-carboxylic acid (for preparation see WO2006/066896).
HPLC (method D): Rt = 5.13 min; MS (method F): M+1 = 405.

### (3R*,4R*)-1-((S)-2-tert-Butoxycarbonylamino-3-methyl-butyryl)-4-(4-chlorobenzyl)-pyrrolidine-3-carboxylic acid (BB 12)

### Step 1

### 3-{[(E)-3-(4-Chloro-pbenyl)-allyl]-methyl-amino}-propionitrile

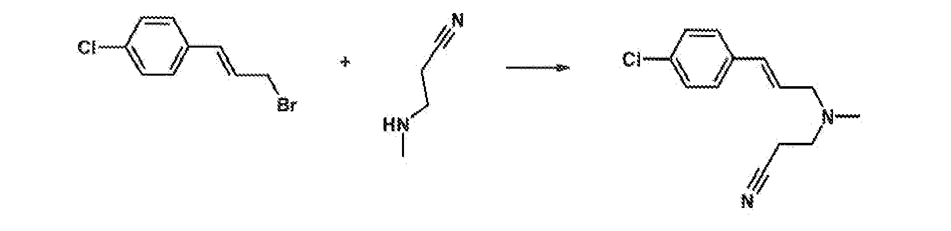

To a mixture of 0.84 kg (10 mol) 3-Methylamino-propionitrile, 0.18 kg (0.5 mol) benzyl-tri-(n-butyl) ammonium bromide and 10 L 2 N aq. sodium hydroxide in 20 L DCM is added a solution of 2.32 kg (10 mol) 1-((E)-3-Bromo-propenyl)-4-chloro-benzen (for preparation see for example: M. Mori, S. Watanuki, J. Chem. Soc. Chem. Commun. 1992, 15, 1082-1084) in 10 L of DCM and the resulting mixture is stirred at rt overnight The organic layer is separated, washed twice with 10 L water, dried over MgSO₄ and concentrated in vacuo to give the title compound which is used in the next step without further purification.
TLC (toluene/ethanol/ammonia 84:15:1) Rf = 0.5.

### Step 2

### (3R*,4R*)-4-(4-Chloro-benzyl)-1-methyl-pyrrolidine-3-carbonitrile

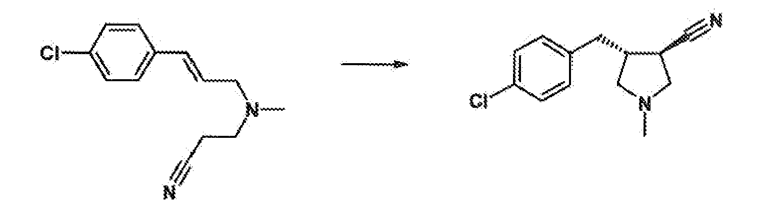

To 0.21 kg (7 mol) NaH (80% in mineral oil) is added under N₂ atmosphere 6 L HMPA and the mixture is cooled to 0°C. A mixture of 1.5 kg (6.4 mol) 3-{[(E)-3-(4-Chlorophenyl)-allyl]-methyl-amino}-propionitrile in 6 L HMPA is added and the mixture is allowed to warm to rt overnight, before 0.51 kg AcOH is added (exothermicl). 1 0 L water and 15 L toluene are added and the aq. layer is extracted twice with 7 L toluene. The combined organic layers are washed twice with 5 L water, dried over MgSO₄ and concentrated in vacuo to give the title compound. This residue is taken up in 3 L MeOH and a solution of 0.77 kg (6 mol) oxalic acid dihydrate in 1.5 L MeOH is added at 50°C, and the resulting mixture is cooled to 35°C. 6 L Et₂O are added and the mixture is cooled to -5°C overnight, centrifuged, filtered and dried to give the title compound as oxalate salt. This salt is dissolved in a mixture of water and toluene, the pH is adjusted to 10 using MH₄OH (25%) and the layers are separated. The aqueous layer is extracted twice with toluene and the combined organic layers are dried over MgSO₄ and concentrated to give the title compound.
TLC (toluene/ethanol/ammonia 84:15:1) Rf = 0.3

### Step 3

### (3R*,4R*)-4-(4-Chloro-beuzyl)-1-methyl-pyrrolidine-3-carboxylic acid

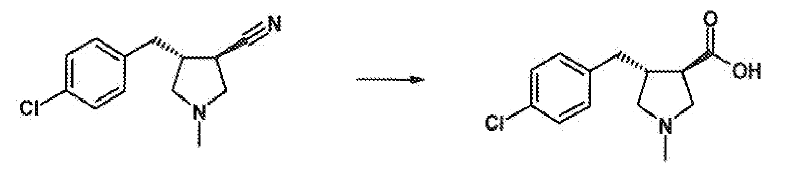

A mixture of 0.93 kg (4 mole) of (3R*,4R*)-4-(4-Chloro-benzyl)-1-methyl-pyrrolidine-3-carbonitrile, 1.2 L cone. HCl, 1.2 L water and 3.6 L acetic acid is refluxed for 20 h. To the solution, charcoal is added at a temperature of 70°C and the resulting mixture is further stirred at 70°C before filtration over a pad of Celite. The filtrate is concentrated under reduced pressure, and the residue is dissolved in 8 L of water at 50°C, cooled to rt and adjusted to pH 9. The mixture is extracted three times with 1.5 L DCM and the aqueous layer is now adjusted to pH 6 before it is concentrated under reduced pressure. The residue is triturated twice with 1 L of toluene/EtOH 1/1 and concentrated again. To this residue is added twice 1 L of toluene/EtOH 1/1 and the resulting mixture is concentrated again. The residue is triturated with 8 L EtOH at 50°C, filtered and washed with an additional 1.5 L EtOH. The filtrate is concentrated to a volume of 2 L, filtered again and finally concentrated under reduced pressure to give an oil which is dissolved in 1 L EtOH. To this mixture 1.5 L Et₂O are added drop wise, the resulting mixture is refluxed for 15 min to allow the title compound to precipitate. The mixture is cooled to 0°C, filtered and the solid product is dried to give the title compound.
TLC (DCM/EtOH/ammonia 50:45:5) Rf = 0.35

### Step 4

### (3R*,4R*)-4-(4-Chloro-benzyl)-1-methyl-pyrrolidine-3-carboxylic acid methyl ester

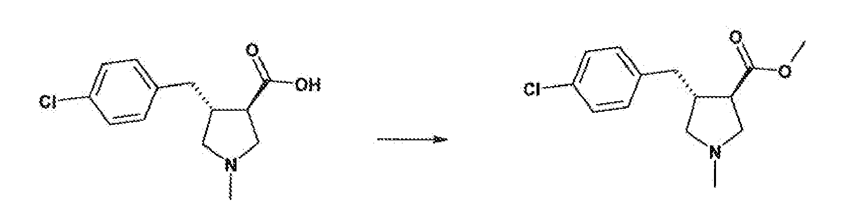

This step is performed as described in the synthesis of (3R*,4S*)-1-((S)-2-tert-Butoxycarbonylamino-3-methyl-butyryl)-4-phenyt-pyrmtid!ne-3-cat-boxyhcacid.TLC (CH₂Cl₂/EtOH 95:5) Rf = 0.17; MS (method F): M+H = 268.

### Step 5

### (3R*,4R*)-4-(4-Chloro-benzyl)-pyrrolidine-3-carboxylic acid methyl ester

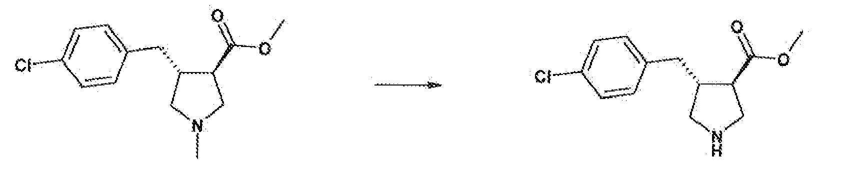

A solution of 2.67 g (9.972 mmol) of (3R*,4R*)-4-(4-Chloro-benzyl)-1-methyl-pyrrolidine-3-carboxylic acid methyl ester in 50 mL 1,2-dicholoroethane is treated with 1.069 g (4.986 mmol) Proton Sponge, cooled to 0 °C, treated with 2.851 g (19.944 mmol) of 1-chloroethyl-chloroformate, and warmed up to RT. The reaction mixture is heated up to reflux for 30 min, concentrated to 1/3 volume, treated with 50 mL methanol, heated up to reflux for 15 min and concentrated in vacuo. The residue is chromatographed on SiO₂ (eluent EtOAc/MeOH 95:5 with 0.5% NH₄ᵢOH) to give (3R*,4R*)-4-(4-Chloro-benzyl)-pyrrolidine-3-carboxylic acid methyl ester as an oil, TLC (EtOAc/McOH 95:5 with 0.5% NH₄OH) Rf = 0.10; MS (method F): M+H = 254.

### Step 6

### (3R*,4R*)-1-((S)-2-tert-Butoxycarbonylamino-3-methyl-butyryl)-4-(4-chlorobenzyl)-pyrrolidine-3-carboxylic acid methyl ester

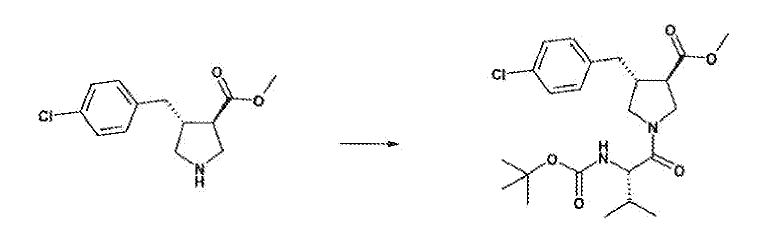

This step is performed as described in the synthesis of (3R*,4S*)-1-((S)-2-tert-Butoxycarbonylamino-3-methyl-butyryl)-4-phenyl-pyrrolidin-3-carboxylic acid. HPLC (method D): Rt = 5.78 min; MS (method F): M+H = 453.

### Step 7

### (3R*,4R*)-1-((S)-2-tert-Butoxycarbonylamino-3-methyl-butyryl)-4-(chlorobenzyl)-pyrrolidine-3-carboxylic acid

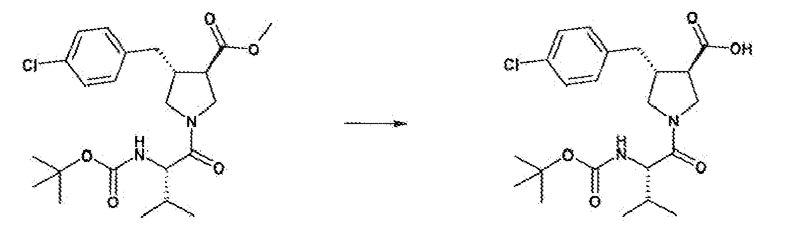

This step is performed as described in the synthesis of (3R*,4S*)-1-((S)-2-tert-Butoxycarbonylamino-3-methyl-butyryl)-4-phenyl-pyrrolidine-3-carboxylic acid. HPLC (method D): Rt = 5.33 min; MS (method F): M+H = 439.

### (3R*,4R*)-4-(4-Chloro-benzyl)-1-naphthalen-1-ylmethyl-pyrrolidine-3-carboxylic acid (BB 13)

### Step 1

### (3R*,4R*)-4-(4-Chloro-benzyl)-1-naphthalen-1-ylmethyl-pyrrolidine-3-carboxylic acid methyl ester

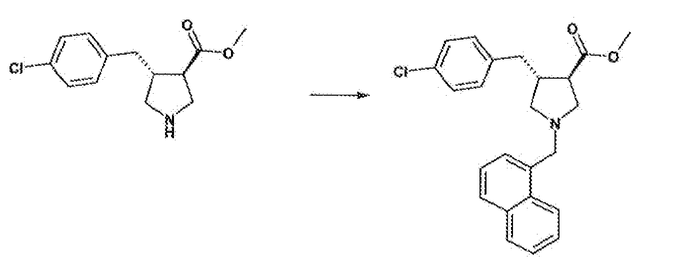

To a solution of 280 mg (1.10 mmol) (3R*,4R*)-4-(4-Chloro-benzyl)-pyrrolidine-3-carboxylic acid methyl ester and 292 mg (1.66 mmol) 1-Chloromethyl-naphthalene in DMF (2 mL) is added 462 mg (3.3 mmol) K₂CO₃ and the mixture is stirred 3h at RT. The reaction is diluted with EtOAc and 0.1N aq. HCl, extracted with EtOAc, washed with brine, dried with Na₂SO₄, filtered and the solvent is removed in vacuo. The residue is purified by FC (silica gel, eluent: hexane/EtOAc 4:1 -> hexane /MeQH 9:1) to give the title compound. HPLC (method D) Rt = 5.89 min; MS (method F): 394 [M+H].

### Step 2

### (3R*,4R*)-4-(4-Chloro-benzyl)-1-naphthalen-1-ylmethyl-pyrrolidine-3-carboxylic acid

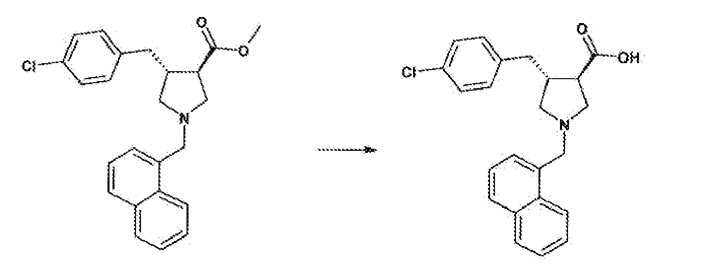

To a solution of 250 mg (0.64 mmol) (3R*,4R*)-4-(4-Chloro-benzyl)-1-naphthalen-1-ylmethyl-pyrrolidine-3-carboxylic acid methyl ester in THF/MeOH/H₂O (10 mL, 2:1:1) is added 108mg (2.54 mmol) LiOH at RT and the reaction is stirred overnight. The reaction is diluted with EtOAc and 0.1N aq. HCl, extracted with EtOAc. washed with brine, dried with Na₂SO₄, filtered and the solvent is removed in vacuo. The residue is used without further purification. HPLC (method D) Rt = 5.51 min; MS (method F): 380 [M+H].

### (3R*,4R*)-1-Acetyl-4-(4-chloro-benzyl)-pyrrolidine-3-carboxylic acid (BB 14)

### Step 1

### (3R*,4R*)-1-Acetyl-4-(4-chloro-benzyl)-pyolidine-3-carboxylic acid methyl ester

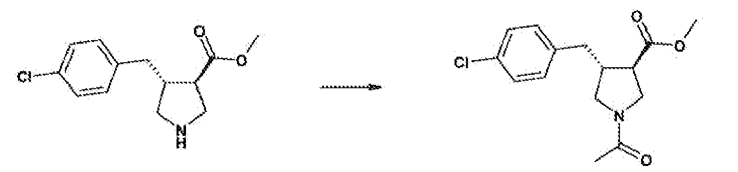

A solution of 0.6 g (2.365 mmol) (3R*,4R*)-4-(4-Chloro-benzyl)-pyrrolidine-3-carboxylic acid methyl ester in 15 mL CH₂Cl₂ is treated with 1.215 mL (7.095 mmol) DIPEA, cooled to 0°C, treated with a solution of 0.252 mL (3.547 mmol) of acetyl chloride in 5 mL CH₂Cl₂. The reaction mixture is stirred at 0 °C for 5 min, at RT for 1h, and then washed with saturated aqueous NaHCO₃ and brine, The organic phase is dried over Na₂SO₄ and concentrated in vacuo to give (3R*,4R*)-1-Acetyl-4-(4-chloro-benzyl)-pyrrolidine-3-carboxylic acid methyl ester as an oil. HPLC (method D): Rt = 2.64 min; LC-MS (method F): M+H = 296.

### Step 2

### (3R*,4R*)-1-Acetyl-4-(4-chloro-benzyl)-pyrrolidine-3-carboxylie acid

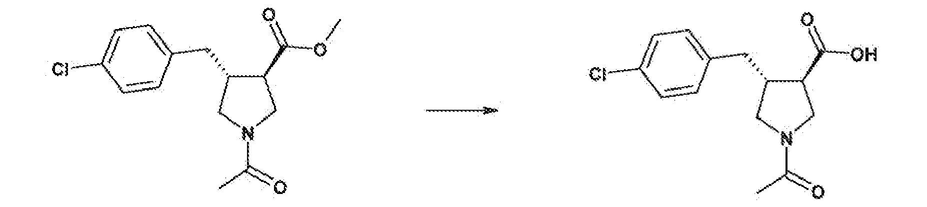

This step is performed according as described above. HPLC (method D): Rt = 4.62 min; MS (method F); M+H = 282.

### (3R*,4R*)-4-Benzyl-1-((S)-2-tert-butoxyearbonylamino-3-methyl-butyryl)-pyrrolidine-3-carboxylic acid (BB 15)

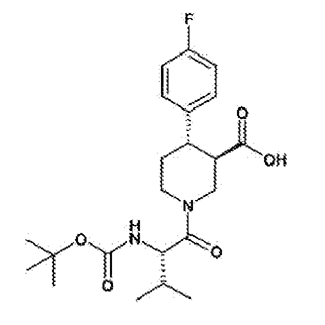

(3R*,4R*)-4-Benzyl-1-((S)-2-tert-butoxycarbonylamino-3-methyl-butyryl)-pyrrolidine-3-carboxylic acid is obtained from (3R*,4S*)-4-(4-Fluoro-phenyl-1-methy)-piperdine-3-carboxylic acid methyl ester (for preparation see WO2001/029032) according to the methods described above. HPLC (method D): Rt = 5.23 min; MS (method F): M+H = 423.

### (3R,4R)-4-Phenethyl-pyrrolidine-1,3-dicarboxylic acid 1-tert-butyl ester (BB 16)

### Step 1

### (3R,4R)-4-(tert-Butyl-dimethyl-silanyloxymethyl)-pyrrolidine-1,3-dicarboxylic acid 1-tert-butyl ester

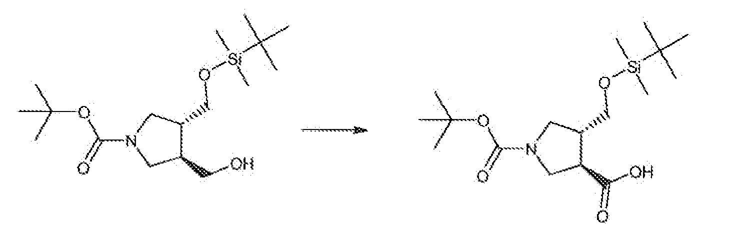

274 mg (1.76 mmol) TEMPO are added to a mixture of 7.6 g (22.0 mmol) (3R,4R)-3-(tert-butyl-dimethyl-silanyloxymethyl)-4-hydroxymethyl-pyrrolidine-1-carboxylic acid tert-butyl ester (synthesized in an analogous fashion as described in WO 2006/1 00036 starting from (3R,4R)-3,4-Bis-hydroxymethyl-pyrrolidine-1-carboxylic acid tert-butyl ester) in 80 ml MeCN and 60 ml phosphate buffer (pH 6.7, 0.67 M). The mixture is heated to 35 °C and simultaneously, a solution of 5.22 g (46.2 mmol) NaClO₂ in 23 ml H₂O and a solution of 427 µl (0.66 mmol) NaOCl (11.5 % in H₂O) in 14 ml H₂O are added over 30 minutes. The reaction is stirred at 35°C for 3 hours. After cooling to room temperature, the reaction is acidified to pH3 with IN HCl and extracted three times with EtOAc. The combined organic phases are washed with brine, dried with MgSO₄ and evaporated to dryness. The residue is chromatographed on SiO₂ (eluent cycbhexane/EtOAc) to give (3R,4R)4-(tert-butyl-dimethyl-silanyloxymethyl)-pyrrolidine-1,3-dicarboxylic acid 1-tert-butyl ester as a colorless oil. LC-MS (method C) Rt = 4.574 min; M+Na = 382.1, M-H = 358.1.

### Step 2

### (3R,4R)-4-(tert-Butyl-dimethyl-silanyloxymenthyl)-pyrrolidine-1,3-dicarboxylic acid 1-tert-butyl ester 3-methyl ester

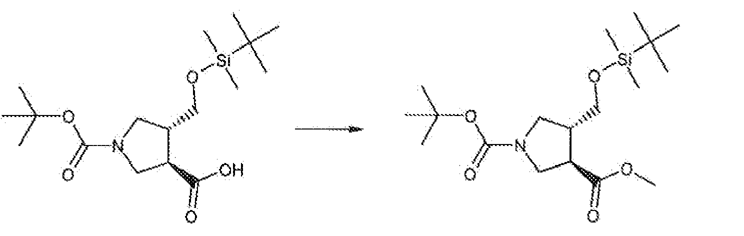

13 ml (Trimethylstilyl)diazomethane (2M in hexane) are added drop wise to a solution of 4.8 g (13.4 mmol) (3R,4R)-4-(tert-butyl-dimethyl-silanyloxymethyl)-pyrrolidine-1,3-dicarboxylic acid 1-tert-butyl ester in 26 ml MeOH and 26 ml benzene. The reaction is stirred for 1 hour at room temperature and then evaporated to dryness to afford (3R,4R)-4-(tert-butyl-dimethyl-silanyloxymethyl)-pyrrolidine-1,3-dicarboxylic acid 1-tert-butyl ester 3-methyl ester, which is used without further purification. LC-MS (method C) Rt = 5.075 min; M+Na = 396.2.

### Step 3

### (3R,4R)-4-Hydroxymethyl-pyrrolidine-1,3-dicarboxylic acid 1-tert-butyl easter 3-methyl ester

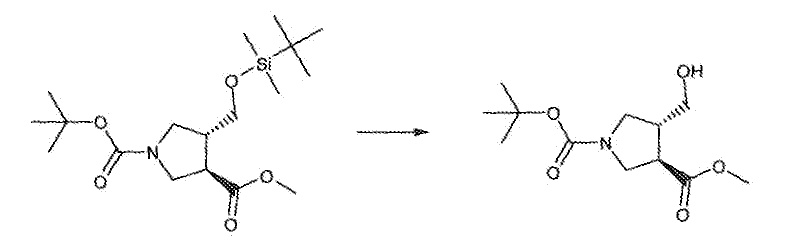

To a solution of 4.32 g (11.6 mmol) (3R,4R)-4-(tert-butyl-dimethyl-silanyloxymethyl)-pyrrolidine-1,3-dicarboxylic acid 1-tert-butyl ester 3-methyl ester in 50 ml pyridine is added 7.0 ml HF in pyridine (70% HF, 30% pyridine) and the mixture is stirred overnight. H₂O is added and the reaction is extracted twice into EtOAc. The combined organic phases are washed with H₂O, dried with Na₂SO₄ and evaporated to dryness. The residue is chromatographed on SiO₂ (eluent cyclohexane/EtOAc) to give (3R,4R)-4-hydroxymethyl-pyrrolidine-1,3-dicarboxylic acid 1-tert-butyl ester 3-methyl ester as a yellowish oil. LC-MS (method C) Rt = 2.822 min; M+Na = 282.2.

### Step 4

### (3R,4R)-4-Formyl-pyrrolidine-1,3-dicarboxylic acid 1-tert-butyl ester 3-methyl ester

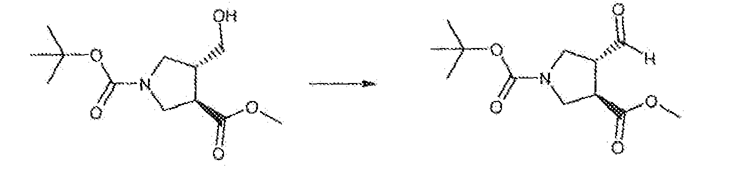

To a well-stirred mixture of 2.98 g (11.5 mmol) (3R,4R)-4-hydroxymethyl-pyrrolidine-1,3-dicarboxylic acid 1-tert-butyl ester 3-methyl ester in 30 ml CH₂Cl₂ and 0.23 ml H₂O are added 5.53 g (12.6 mmol) Dess Martin-periodinane. The mixture is stirred vigorously for 2 hours and then diluted with Et₂O. It is then concentrated and taken up in Et₂O, washed with 10% aq. Na₂S₂O₃ / sat. aqueous NaHCO₃ (1:1 v/v), H₂O and brine. The ethereal phase is dried with Na₂SO₄ and evaporated to dryness to afford (3R,4R)-4-formyl-pyrrolidine-1,3-dicarboxylic acid 1-tert-butyl ester 3-methyl ester as a yellowish oil. LC-MS (method C): Rt = 2.640 min; M+H-Boc = 158.0.

### Step 5

### (3R,4R)-4-((E)-Styryl)-pyrrolidine-1,3-dicarboxylic acid 1-tert-butyl ester 3-methyl ester and (3R,4R)-4-((Z)-Styryl)-pyrrolidine-1,3-dicarboxylic acid 1-tert-butyl ester 3-methyl ester

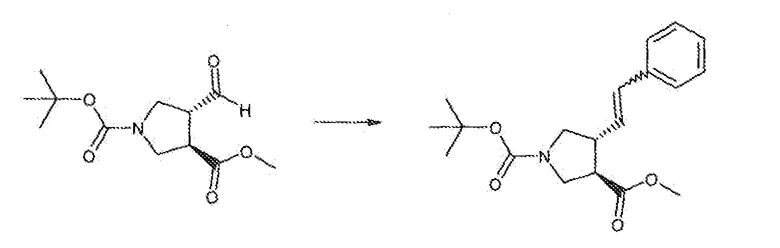

To 0.88 g (1.94 mmol) benzyltriphenylphosphonium bromide in 13 ml anhydrous THF are added drop wise 1.28 ml (2.0 mmol) *n*-BuLi (1.6 M in hexanes) at 0°C and the mixture is stirred at that temperature for 30 minutes. 0.5 g (1.94 mmol) (3R,4R)-4-formyl-pyrrolidine-1,3-dicarboxylic acid 1-tert-butyl ester 3-methyl ester in 3 ml THF are added drop wise and the solution is stirred for 30 minutes at 0°C and for 3 hours at room temperature. Sat. aq. NH₄Cl and then EtOAc are added and the phases are separated. The organic phase is washed with H₂O, dried with Na₂SO₄ and evaporated to dryness. The residue is chromatographed on SiO₂ (eluent cyclohexane/EtOAc 100/0 to 50/50) to give (3R,4R)-4-(styryl)-pyrrolidine-1,3-dicarboxylic acid 1-tert-butyl ester 3-methyl ester as a mixture of E/Z-isomers as a yellow oil, HPLC (method D): Rt = 3.927 min; MS (method F): M+Na = 354.0).

### Step 6

### (3R,4R)-4-Phenethyl-pyrrolidine-1,3-dicarboxylic acid 1-tert-butyl ester 3-methyl ester

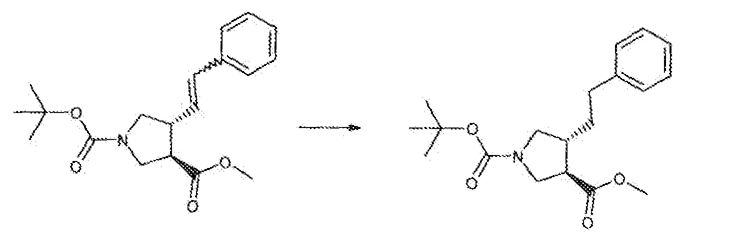

A mixture of 130 mg (0.39 mmol) (3R,4R)-4-(styryl)-pyrrolidine-1,3-dicarboxylic acid 1-tert-butyl ester 3-methyl ester (mixture of H/Z-isomers), 2.1 mg Pd/C (10%, Engelhardt) in 5 ml EtOH is stirred in a H₂-atmosphere overnight. After purging with N₂, the reaction is filtered through Celite and evaporated to dryness to afford (3R,4R)-4-phenethyl-pyrrolidine-1,3-dicarboxylic acid 1-tert-butyl ester 3-methyl ester as a yellow oil, which is used without further purification. HPLC (method D): Rt = 3.945 min; MS (method F): M+Na = 356.1.

### Step 7

### (3R,4R)-4-Phenethyl-pyrrolidine-1,3-dicarboxylic acid 1-tert-butyl ester

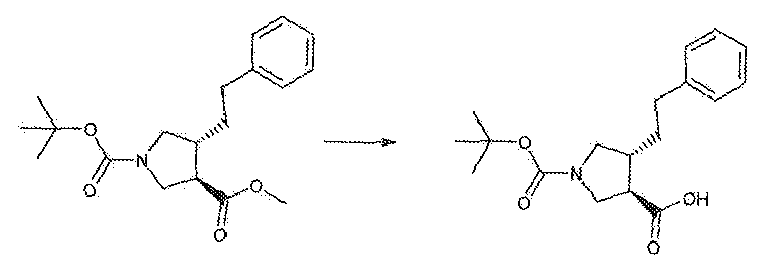

A mixture of 110 mg (0.33 mmol) (3R,4R)-4-phenethyl-pyrrolidine-1,3-dicarboxylic acid 1-tert-butyl ester 3-methyl ester in 1.8 ml 1N KOH and 3.6 ml EtOH is stirred at 50°C for I hour. The reaction is brought to pH 3 with I N HCl and extracted twice into CH₂Cl₂. The combined organic layers are washed with H₂O, dried with Na₂SO₄ and evaporated to dryness to afford (3R,4R)-4-phenethyl-pyrrolidine-1,3-dicarboxylic acid 1-tert-butyl ester, which is used without further purification. LC-MS (method C): Rt = 4.020 min; M-H = 318.1.

### (3R,4R)-4-(2-Naphthalen-1-yl-ethyl)-pyrrolidine-1,3-dicarboxylic acid 1-tert-butyl ester (BB 17)

### Step 1

### (3R,4R)-4-((E)-2-Naphthalen-1-yl-vinyl)-pyrrolidine-1,3-dicarboxylic acid 1-tert-butyl ester 3-methyl ester and (3R,4R)-4-((Z)-2-Naphthalen-1-yl-vinyl)-pyrrolidine-1,3-dicarboxylic acid 1-tert-butyl ester 3-methyl ester

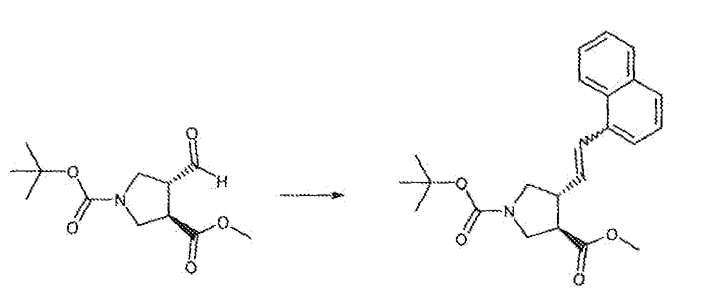

(3R,4R)-4-(2-Naphthalen-1-yl-vinyl)-pyrrolidine-1,3-dicarboxylic acid 1-tert-butyl ester 3-methyl ester (mixture of E/Z-isomers) is prepared in an analogous fashion as (3R,4R)-4-(styryl)-pyrrolidine-1,3-dicarboxylic acid 1-tert-butyl ester 3-methyl ester (mixture of E/Z-isomers) starting from 800 mg (3.11 mmol) (3R,4R)-4-formyl-pyrrolidine-1,3-dicarboxylic acid 1-tert-butyl ester 3-methyl ester and 1.42 g (3.11 mmol) naphthalen-1-ylmethyl-triphenyl-phosphonium chloride. HPLC (method D): Rt = 4.662 min, MS (method F): M+H-Boc = 282.0)

### Step 2

### (3R,4R)-4-(2-Naphthalen-1-yl-ethyl)-pyrrolidine-1,3-dicarboxylic acid -tert-butyl ester 3-methyl ester

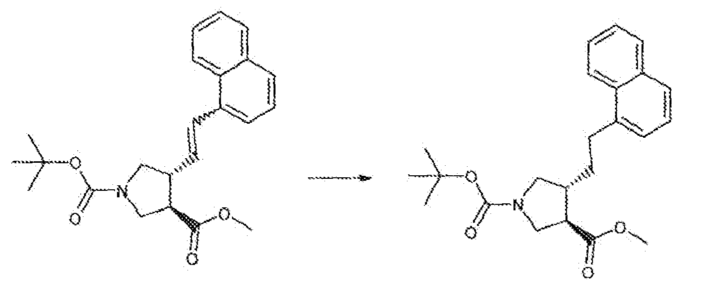

A mixture of 760 mg (1.99 mmol)(3R,4R)-4-(2-naphthalen-1-yl-vinyl)-pyrrolidine-1,3-dicarboxylic acid 1-tert-butyl ester 3-methyl ster (mixture of E/Z-isomers), 4.64 g (23.9 mmol) potassium diazodicarboxylate in 30 ml CH₂Cl₂ is heated to reflux and 20 ml AcOH (10 mmol, 0.5 M in CH₂Cl₂) is added. The reaction is refluxed to 72 hours and extracted with 1N HCl. The aq. phase is extracted with CH₂Cl₂ and the combined organic phases are washed with sat aqueous NaHCO₃, dried with Na₂SO₄ and evaporated to dryness. The residue is chromatographed by preparative reverse phase HPLC (CH₃CN, H₂O, HCO₂H) to give (3R,4R)-4-(2-naphthalen-1-yl-ethyl)-pyrrolidine-1,3-dicarboxylic acid 1-tert-butyl ester 3-methyl ester as a colorless oil. HPLC (method D): Rt = 4.281 min, MS (method F): M+H-Boc = 282.0.

### Step 3

### (3R,4R)-4-(2-Naphthalen-1-yl-ethyl)-pyrrolidine-1,3-dicarboxylic acid -tert-butyl ester

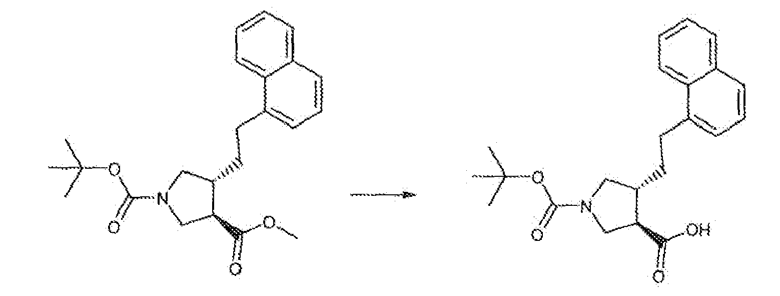

(3R,4R)-4-(2-Naphthalen-1-yl-ethyl)-pyrrolidine-1,3-dicarboxylic acid 1-tert-butyl ester is prepared in an analogous fashion as (3R,4R)-4-phenethyl-pyrrolidine-1,3-dicarboxylic acid 1-tert-butyl ester starting from 370 mg (0.97 mmol) (3R,4R)-4-(2-naphthalen-1-yl-ethyl)-pyrrolidine-1,3-dicarboxylic acid 1-tert-butyl ester 3-methyl ester. HPLC (method D): Rt = 3.833 min; MS (method F): M-H = 368.2.

### (3R,4R)-4-(2-Naphthalen-2-yl-ethyl)-pyrrolidine-1,3-dicarboxylic acid 1-tert-butyl ester (BB 18)

### Step 1

### (3R,4R)-4-((E)-2-Naphthalen-2-yl-vinyl)-pyrrolidine-1,3-dicarboxylic acid 1-tert-butyl ester 3-methyl ester and (3R,4R)-4-((Z)-2-Naphthalen-2-yl-vinyl)-pyrrolidine-1,3-dicarboxylic acid 1-tert-butyl ester 3-methyl ester

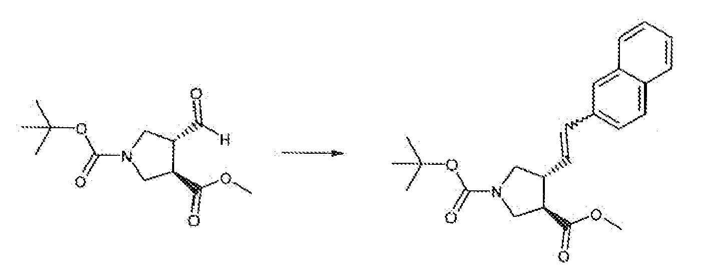

(3R,4R)-4-(2-Naphthalen-2-yl-vinyl)-pyrrolidine-1,3-dicarboxylic acid 1-tert-butyl ester 3-methyl ester (mixture of E/Z-isomers) is prepared in an analogous fashion as (3R,4R)-4-(styryl)-pyrrolidine-1,3-dicarboxylic acid 1-tert-butyl ester 3-methyl ester (mixture of E/Z-isomers) starting from 500 mg (1.93 mmol) (3R,4R)-4-formyl-pyrrolidine-1,3-dicarboxylic acid 1-tert-butyl ester 3-methyl ester and 0.97 g (1.94 mmol) naphthalen-2-ylmethyl-triphenyl-phosphonium chloride. HPLC (method D): Rt = 4.307 min, MS (method F): M+Na = 404.1.

### Step 2

### (3R,4R)-4-(2-Naphthalen-2-yl-ethyl)-pyrrolidine-1,3-dicarboxylic acid -tert-butyl ester 3-methyl ester

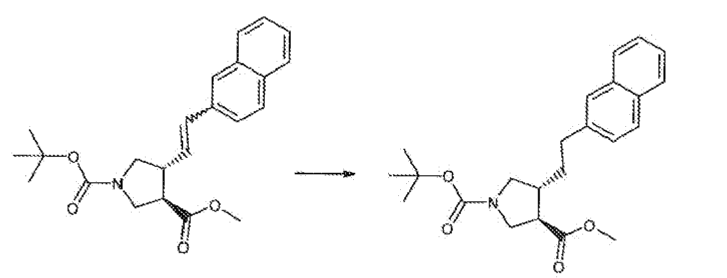

(3R,4R)-4-(2-Naphthalen-2-yl-ethyl)-pyrrolidine-1,3-dicarboxylic acid 1-tert-butyl ester 3-methyl ester is prepared in an analogous fashion as (3R,4R)-4-(2-naphthalen-1-yl-ethyl)-pyrrolidine-1,3-dicarboxylic acid 1-tert-butyl ester 3-methyl ester starting from 380 mg (1.00 mmol) (3R,4R)-4-(2-naphthalen-2-yl-vinyl)-pyrrolidine-1,3-dicarboxylic acid 1-tert-butyl ester 3-methyl ester (mixture of E/Z-isomers) and 4.64 g (23.9 mmol) potassium diazodicarboxylate. HPLC (method D): Rt = 4.297 min, MS (method F): M+Na = 406.1, M+H-Boc = 284.1.

### Step 3

### (3R,4R)-4-(2-Naphthalen-2-yl-ethyl)-pyrrolidine-1,3-dicarboxylic acid 1-tert-butyl ester

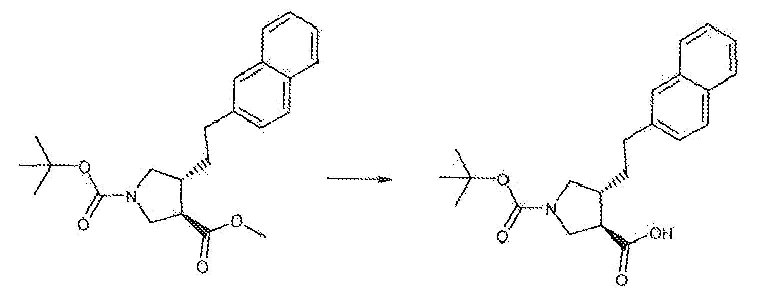

(3R,4R)-4-(2-Naphthalen-2-yl-ethyl)-pyrrolidine-1,3-dicarboxylic acid 1-tert-butyl ester is prepared in an analogous fashion as (3R,4R)-4-phenethyl-pyrrolidine-1,3-dicarboxylic acid 1-tert-butyl ester starting from 335 mg (0.87 mmol) (3R,4R)-4-(2-naphthalen-2-yl-ethyl)-pyrrolidine-1,3-dicarboxylic acid 1-tert-butyl ester 3-methyl ester. HPLC (method D): Rt = 3.844 min, MS (method F): M+H-Boc = 270.0, M-H = 368.2.

### (3R*,4S*)-4-(3,5-Bis-trifluoromethyl-phenyl)-pyrrolidine-1,3-dicarboxylic acid 1-tert-butyl ester (BB 19)

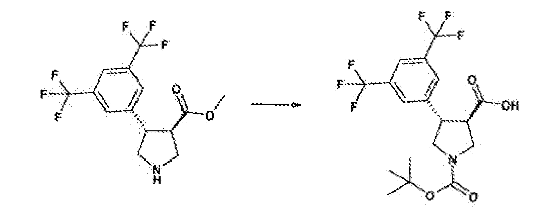

To a solution of 0.333 g (0.976 mmol) of (3R*,4S*)-4-(3,5-Bis-trifluoromethyl-phenyl)-pyrrolidine-3-carboxylic acid methyl ester in 3 mL THF is added 1.5 mL water and 0.4 mL of 10% NaOH, followed by 0.224 g (1.025 mmol) of BOC₂O. After 3 h at RT the reaction mixture is taken up in water, extracted with EtOAc, dried over Na₂SO₄, and concentrated. The resulting residue is purified on preparative reverse phase HPLC to give (3R*,4S*)-4-(3,5-Bis-trifluoromethyl-phenyl)-pyrrolidine-1,3-dicarboxylic acid 1-tert-butyl ester. HPLC (method D): Rt = 5.62 min; MS (method F): M-H = 426.

### (1R,2S,4R)-2-tert-Butoxycarbonylamino-4-(7-methoxy-2-phenyl-quinolin-4-yloxy)-cyclopentanecarboxylic acid (BB 20)

### Step 1

### (1R,2S)-2-tert-Butoxycarbonylamino-4-methylene-cyclopentanecarboxylic acid

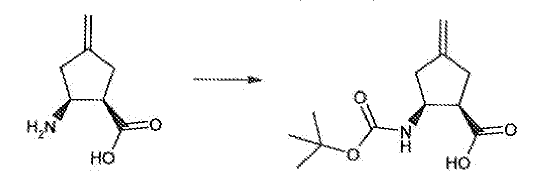

To a solution of 0.79 g (5.60 mmol)(1R,2S)-2-amino-4-methylene-cyclopentanecarboxylic acid (for preparation see J. Mittendorf et al., Synthesis 2003, 136) and 1.95 ml (14.0 mmol) triethylamine in 20 ml dioxane/H₂O (3:1 v/v) is added 1.22 g (5.60 mmol) Boc₂O. The reaction mixture is stirred overnight and evaporated to dryness to afford crude (1R,2S)-2-tert-butoxycarbonylamino-4-methylene-cyclopentanecarboxylic acid as a pale yellow oil, which is used without further purification, LC-MS (method C): Rt = 2.88 min, M+Na = 264.2, M-H = 240.1.

### Step 2

### (1R,2S)-2-tert-Butoxycarbonylamino-4-oxo-cyclopentanecarboxylic acid

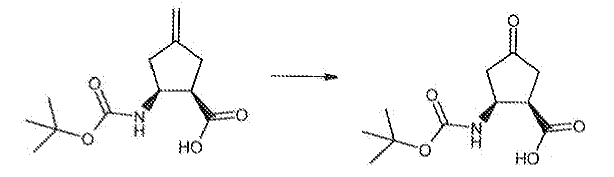

Ozone is bubbled through a solution of 1.32 g (5.5 mmol) (1R,2S)-2-tert-butoxycarbonylamino-4-methylene-cyclopentanecarboxylic acid in 50 ml MeOH at - 78°C until the blue color persists. Argon is then bubbled through the solution until it becomes colorless. 2.01 ml (27.4 mmol) dimethyl sulfide are slowly added at -78°C and the reaction is stirred at that temperature for 1 hour and then allowed to warm to room temperature overnight. The reaction is evaporated to dryness and the residue is taken up with EtOAc and 1N NaOH. The phases are separated and the aqueous phase is acidified to pH3 with 1N HCl. It is extracted twice with EtOAc, and the combined organic phases are dried with Na₂SO₄ and evaporated to dryness to afford (1R,2S)-2-tert-butoxycarbonylamino-4-oxo-cyclopentanecarboxylic acid as a white foam, which is used without further purification. LC-MS (method C): Rt = 0.992 min, M+Na = 266.1, M-H = 242.2.

### Step 3

### (1R,2S,4S)-2-tert-Butoxycarbonylamino-4-hydroxy-cyclopentanecarboxylic acid

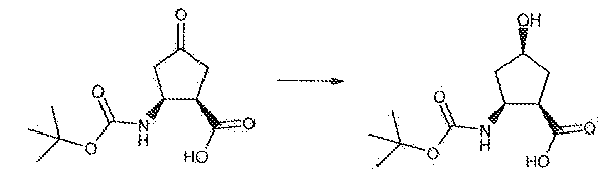

To a solution of 1.34 g (5.51 mmol)(1R,2S)-2-tert-butoxycarbonylamino-4-oxo-cyclopentanecarboxylic acid in 30 ml MeOH is slowly added 326 mg (8.26 mmol) sodium borohydride in portions and the reaction mixture is stirred overnight. EtOAc and 1N HCl are added and the phases are separated. The organic phase is washed with brine, dried with Na₂SO₄ and evaporated to dryness to afford (1R,2S,4S)-2-tert-butoxycarbonylamino-4-hydroxy-cyclopentanecarboxylic acid as a white foam, which is used without further purification. LC-MS (method C): Rt = 1.092 min, M+Na = 268.1, M-H = 244.1.

### Step 4

### (1R,2S,4S)-2-tert-Butoxycarbonylamino-4-hydroxy-cyclopentanecarboxylic acid methyl ester

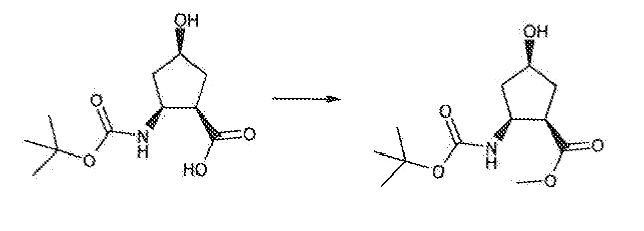

To a solution of 200 mg (0.815 mmol) (1R,2S,4S)-2-tert-butoxycarbonylamino-4-hydroxy-cyclopentanecarboxylic acid in 1.6 ml MeOH and 1.6 ml benzene are added drop wise 0.82 ml (1.6 mmol) (trimethylsilyl)diazomethane (2M in hexane) and the reaction is stirred for 1 hour at room temperature. The reaction mixture is evaporated to dryness to afford (1R,2S,4S)-2-tert-butoxycarbonylamino-4-hydroxy-cyclopentanecarboxylic acid methyl ester as a white solid, which is used without further purification. LC-MS (method C): Rt = 2.468 min, M+Na = 282.0.

### Step 5

### (1R,2S,4R)-2-tert-Butoxycarbonylamino-4-(7-methoxy-2-phenyl-quinolin-4-yloxy)-cyclopentanecarboxylic acid methyl ester

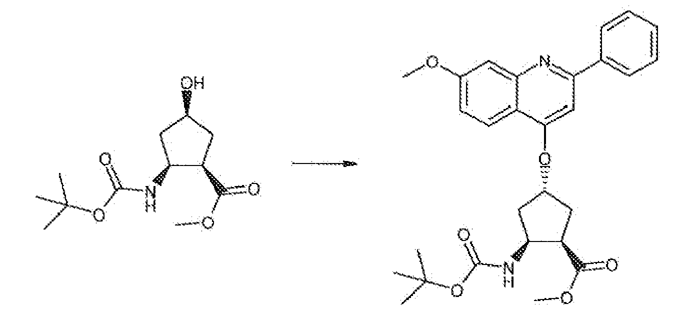

To a solution of 816 mg (3.15 mmol) (1R,2S,4S)-2-tert-butoxycarbonylamino-4-hydroxy-cyclopentanecarboxylic acid methyl ester, 1.19 g (4.72 mmol) 7-methoxy-2-phenyl-quinolin-4-ol (for preparation see: N. Goudreau et al., J. Org. Chem. 2004, 69, 6185) and 2.12 g (7.87 mmol) triphenylphosphine in 60 ml anhydrous THF are added drop wise 1.62 ml (7.87 mmol) diisopropyl azodicarboxylate at 0°C. The reaction mixture is slowly allowed to come to room temperature and is then stirred for 72 hours. EtOAc and sat. aq. NaHCO₃ are added and the phases are separated. The organic layer is washed with H₂O, dried with Na₂SO₄ and evaporated to dryness. The residue is chromatographed by preparative reverse phase HPLC (CH₃CN, H₂O, HCO₂H) to give (1R,2S,4R)-2-tert-butoxycarbonylamino-4-(7-methoxy-2-phenyl-quinolin-4-yloxy)-cyclopentanecarboxylic acid methyl ster as a white solid. HPLC (method D): Rt = 3.023 min, MS (method F): M+H = 493.1, M-H = 491.1.

### Step 6

### (1R,2S,4R)-2-tert-Butoxycarbonylamino-4-(7-methoxy-2-phenyl-quinolin-4-yloxy)-cyclopentanecarboxylic acid

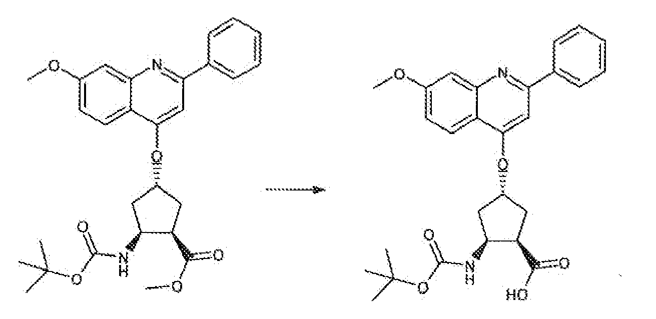

41 mg (0.974 mmol) LiOH*H₂O are added to a solution of 400 mg (0.812 mmol) (1R,2S,4R)-2-tert-butoxycarbonylamino-4-(7-methoxy-2-phenyl-quinolin-4-yloxy)-cyclopentanecarboxylic acid ethyl ester in 30 ml THF/MeOH/H₂O 3:1:1 v/v/v) at 0°C. The reaction mixture is slowly allowed to reach room temperature and stirring is continued overnight. 1N HCl and EtOAc are added and the phases are separated. The organic layer is washed with H₂O, dried with Na₂SO₄ and evaporated to dryness. The residue is chromatographed by preparative reverse phase HPLC (CH₃CN, H₂O, HCO₂H) to give (1R,2S,4R)-2-tert-butoxycarbonylamino-4-(7-methoxy-2-phenyl-quinolin-4-yloxy)-cyclopentanecarboxylic acid as a white solid. HPLC (method D): Rt = 1.245 min, MS (method F): M+H = 479.2, M-H = 477.0.

### (1R,2S,4R)-2-Amino-4-(7-methoxy-2-phenyl-quinolin-4-yloxy)-cyclopentanecarboxylic acid [(1R,2S)-1-(1H-indole-7-sulfonylaminocarbonyl)-2-vinyl-cyclopropyl]-amide (BB 21)

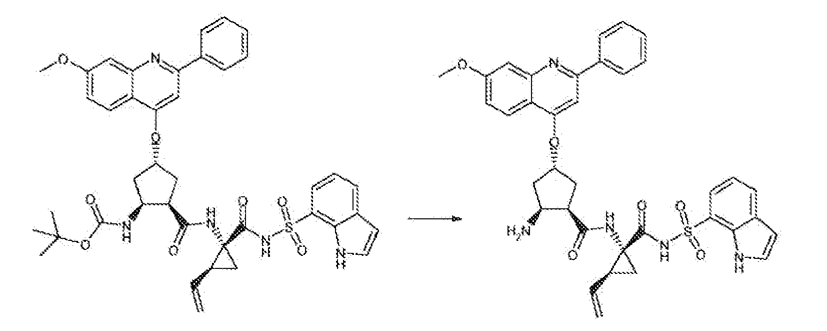

To a solution of 56 mg (0.073 mmol) [(1S,2R,4R)-2-[(1R,2S)-1-(1H-Indole-7-sulfonylaminocarbonyl)-2-vinyl-cyclopropylcarbamoyl]-4-(7-methoxy-2-phenyl-quinolin-4-yloxy)-cyclopentyl]-carbamic acid tert-butyl ester (see Example 38) in 0.5 mL dioxane is added at RT 3 mL HCl (4N in dioxane). After 2 h at RT the solvent is removed in vacuo and the residue is used without further purification. HPLC (method D) Rt = 5.23 min; MS (method F): 666 [M+H].

### (1R,2S,4R)-2-Amino-4-(7-methoxy-2-phenyl-quinolin-4-yloxy)-cyclopentanecarboxylic acid [(1R,2S)-1-(3-benzyloxy-benzenesulfonylaminocarbonyl)-2-vinyl-cyclopropyl]-amide hydrochloride (BB 22)

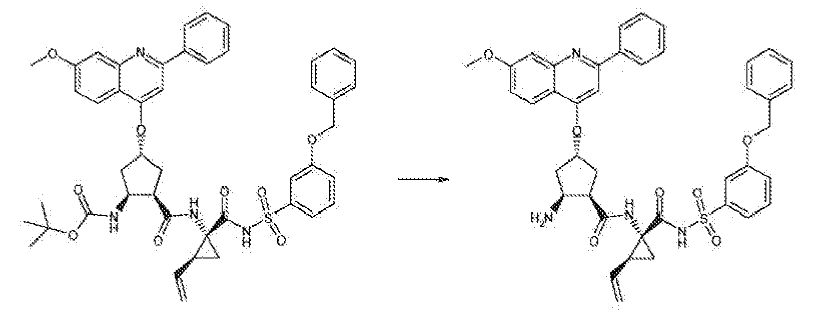

A mixture of 42 mg (0.050 mmol) [(1S,2R,4R)-2-[(1R,2S)-1-(3-benzyloxy-benzenesulfonylaminocarbonyl)-2-vinyl-cyclopropylcarbamoyl]-4-(7-methoxy-2-phenyl-quinolin-4-yloxy)-cyclopentyl]-carbamic acid tert-butyl ester (see Example 37) in 2 ml HCl (4M in dioxane) and 4 ml dioxane is stirred for 2 hours at room temperature. Evaporation to dryness affords (1R,2S,4R)-2-amino-4-(7-methoxy-2-phenyl-quinolin-4-yloxy)-cyclopentanecarboxylic acid [(1R,2S)-1-(3-benzyloxy-benzenesulfonylaminocarbonyl)-2-vinyl-cyclopropyl]-amide hydrochloride as an off-white solid. LC-MS (method C): Rt = 2.734 min, M-H = 730.9.

### (1R,2S,4S)-2-tert-Butoxycarbonylamino-4-(7-methoxy-2-phenyl-quinolin-4-yloxy)-cyclopentanecarboxylic acid (BB 23)

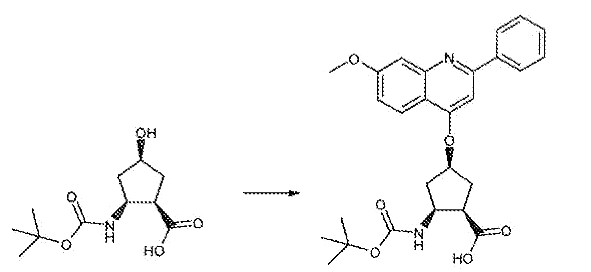

To a solution of 182 mg (0.74 mmol) (1R,2S,4S)-2-tert-Butoxycarbonylamino-4-hydroxy-cyclopentanecarboxylic acid in 4 mL DMSO is added 229 mg (2.0 mmol) potassium tert-butylate and the mixture is stirred for 90 min at RT. 200 mg (0.74 mmol) 4-Chloro-7-methoxy-2-phenyl-quinoline (for preparation see WO2003/99316) is added in three portions over 45 min and stirring is continued at RT overnight. The reaction is diluted with water, extracted with EtOAc, dried with Na₂SO₄, filtered and the solvent is removed in vacuo. The residue is purified by FC on SiO₂ (eluent DCM -> DCM/MeOH 19:1 -> 1:1) to give the title compound. HPLC (method D) Rt = 5.19 min; MS (method F): 479 [M+H].

### (1R,2S,4S)-2-Amino-4-(7-methoxy-2-phenyl-quinolin-4-yloxy)-cyclopentanecarboxylic acid [(1R,2S)-1-(3-benzyloxy-benzenesulfonyl-aminocarbonyl)-2-vinyl-cyclopropyl]-amide (BB 24)

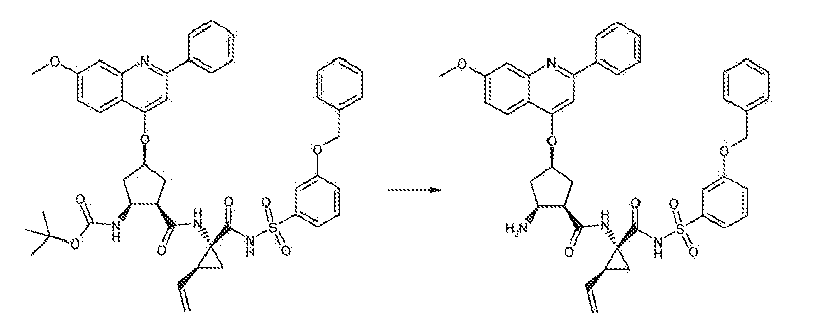

To a solution of 19 mg (0.023 mmol) [(1S,2R,4S)-2-[(1R,2S)-1-(3-Benzyloxy-benzenesulfonylaminocarbonyl)-2-vinyl-cyclopropylcarbamoyl]-4-(7-methoxy-2-phenyl-quinolin-4-yloxy)-cyclopentyl]-carbamic acid tert-butyl ester in 0.5 mL dioxane is added at RT 3 mL HCl (4N in dioxane). After 2 h at RT the solvent is removed in vacuo and the residue is used without further purification, HPLC (method D) Rt = 5.45 min; MS (method F): 733 [M+H].

### (S)-Pyrrolidine-3-carboxylic acid [(1R,2S)-1-(3-benzyloxy-benzenesulfonylamino-carbonyl)-2-vinyl-cyclopropyl]-amide (BB 25)

### Step 1

### (S)-3-[(1R,2S)-1-(3-Benzyloxy-benzenesulfonylaminocarbonyl)-2-vinyl-cyclopropyl-carbamoyl]-pyrrolidine-1-carboxylic acid tert-butyl ester

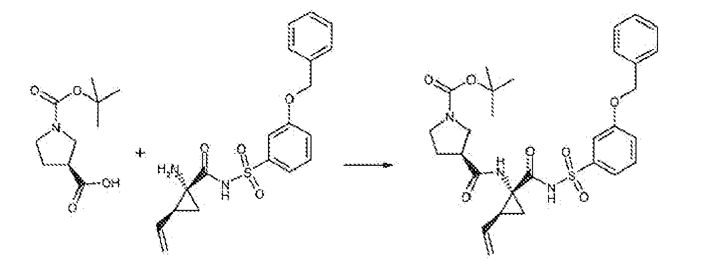

To a solution of 350 mg (1.63 mmol) (S)-Pyrrolidine-1,3-dicarboxylic acid 1-tert-butyl ester, 665 mg (1.63 mmol) N-((1R,2S)-1-Amino-2-vinyl-cyclopropanecarbonyl)-3-benzyloxy-benzenesulfonamide and 0.85 mL (4.9 mmol) DIPEA in DMF (40 mL) is added 627 mg (1.95 mmol) TBTU and the reaction is stirred overnight at RT. The reaction is diluted with EtOAc and 0.1N aq. HCl, extracted with EtOAc, washed with brine, dried with Na₂SO₄, filtered and the solvent is removed in vacuo. The residue is purified by preparative reverse phase HPLC (method H) to give the title compound. HPLC (method D) Rt = 5.79 min; MS (method F): 568 [M-H].

### Step 2

### (S)-Pyrrolidine-3-carboxylic acid [(1R,2S)-1-(3-benzyloxy-benzenesulfonylamino-carbonyl)-2-vinyl-cyclopropyl]-amide

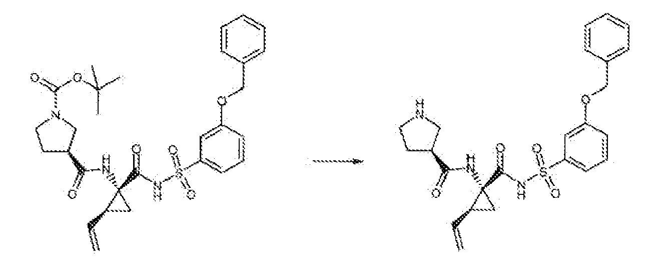

To a solution of 669 mg (1.2 mmol) (S)-3-[(1R,2S)-1-(3-Benzyloxy-benzenesulfonylaminocarbonyl)-2-vinyl-cyclopropyl-carbamoyl]-pyrrolidine-1-carboxylic acid tert-butyl ester in 20 mL dioxane is added at RT 5 mL HCl (4N in dioxane). After 3 h at RT the solvent is removed in vacuo and the residue is used without further purification. HPLC (method D) Rt = 5.14 min; MS (method F): 470 [M+H].

### (3R*,4S*)-4-tert-Butoxycarbonylamino-1-naphthalen-1-ylmethyl-pyrrolidine-3-carboxylic acid (BB 26)

### Step 1

### (3R*,4R*)-1-Benzyl-pyrrolidine-3,4-dicarboxylic acid monomethyl ester

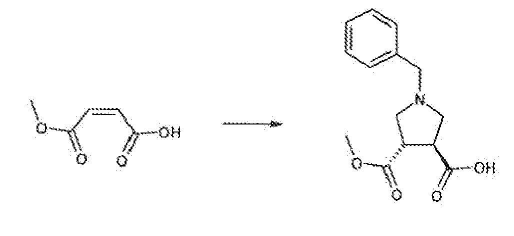

To a suspension of 12.0 g (90 mmol) mono-Methyl fumarate and 26.6 g (107 mmol) N-(Methoxymethyl)-N-(trimethylsilyl)-benzylamine in DCM (150 mL) is added TFA (150 □L) and the reaction is stirred at RT overnight. The solvent is removed in vacuo and the residue is used without further putification. MS (method F): 264 [M+H].

### Step 2

### (3R*,4S*)-1-Benzyl-4-tert-butoxycarbonylamino-pyrrolidine-3-carboxylic acid methyl ester

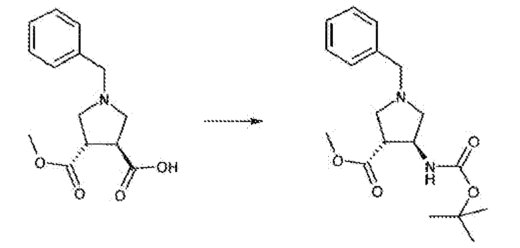

To a solution of 27 g (89 mmol) (3R*,4R*)-1-Benzyl-pyrrolidine-3,4-dicarboxylic acid monomethyl ester and 12.4 mL (89 mmol) NEt₃ in toluene (150 mL) is added 19.3 mL (89 mmol) DPPA within 10 min. The reaction is heated slowly to 90°C, after gas evolution has stopped (∼15 min) 30 mL (390 mmol) tert-butanol is added and the reaction is stirred at 90°C for 6 h. After cooling to RT the solvent is removed in vacuo and the residue is purified by FC (silica gel, cluent: hexane/EtOAc 4:1) to give the title compound. MS (method F): 335 [M+H].

### Step 3

### (3R*,4S*)-4-tert-Butoxycarbonylamino-pyrrolidine-3-carboxylic acid methyl ester

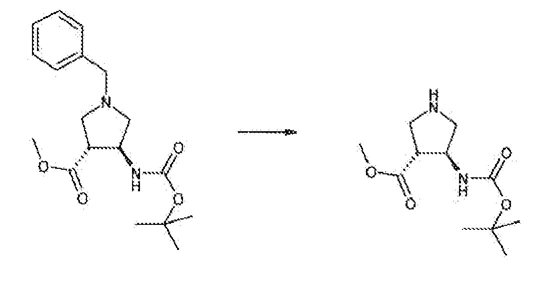

A suspension of 2 g (5.6 mmol) (3R*,4S*)-1-Benzyl-4-tert-butoxycarbonylamino-pyrrolidine-3-carboxylic acid methyl ester and 10% Pd on charcoal (250 mg) in MeOH (50 mL) is stirred under H₂-atmosphere for 2 h at RT. The reaction is filtered, washed with MeOH and the filtrate is concentrated and dried in vacuo to give the title compound, which is used without further purification. MS (method F): 245 [M+H].

### Step 4

### (3R*,4S*)-4-tert-Butoxycarbonylamino-1-naphthalen-1-ylmethyl-pyrrolidine-3-carboxylic acid methyl ester

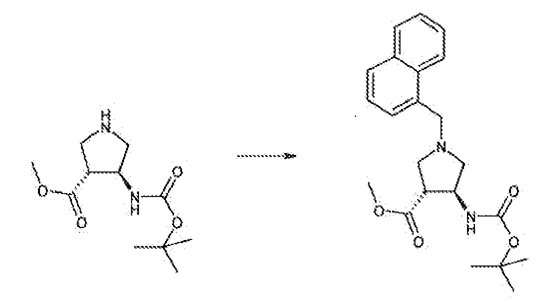

To a solution of 1.37 g (5.6 mmol) (3R*,4S*)-4-tert-Butoxycarbonylamino-pyrrolidine-3-carboxylic acid methyl ester and 2.1 mL (14 mmol) 1-Chloromethyl-naphthalene in DCM (25 mL) is added 1.2 mL (16.8 mmol) Pyridine and the mixture is stirred at RT overnight. The solvent is removed in vacuo and the residue is purified by FC (silica gel, eluent: hexane/EtOAc 9:1) to give the title compound. HPLC (method G) Rt = 3.94 min; MS (Method F): 385 [M+H].

### Step 5

### (3R*,4S*)-4-Amino-1-naphthalen-1-ylmethyl-pyrrolidine-3-carboxylic acid

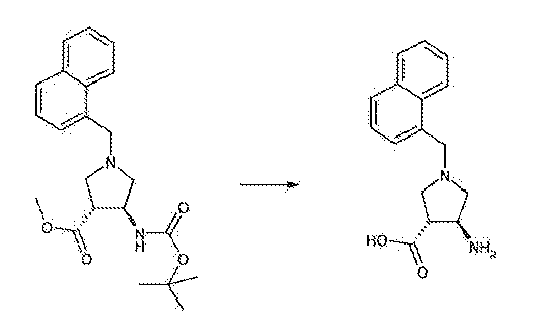

A solution of 950 mg (2.5 mmol) (3R*,4S*)-4-tert-Butoxycarbonylamino-1-naphthalen-1-ylmethyl-pyrrolidine-3-carboxylic acid methyl ester in 37% aq HCl (20 mL) is stirred 1h at RT and 2h at 80°C. After cooling to RT the solvent is removed in vacuo and the residue is used without further purification. MS (method F): 271 [M+H].

### Step 6

### (3R*,4S*)-4-tert-Butoxycarbonylamino-1-naphthalen-1-ylmethyl-pyrrolidine-3-carboxylic acid

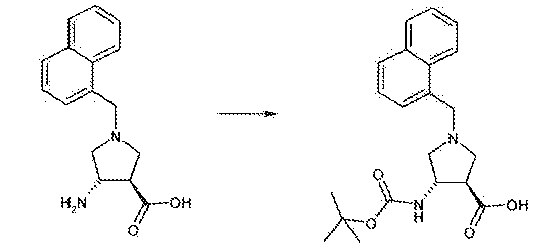

A solution of 990 mg (2.5 mmol) (3R*,4S*)-4-Amino-1-naphthalen-1-ylmethyl-pyrrolidine-3-carboxylic acid, 596 mg (2.7 mmol) (BOC)₂O and 1.04 mL (7.4 mmol) NEt₃ in dioxane/H₂O (5 mL, 3: 1) is stirred overnight at RT. The reaction is acidified with 0.1 N HCl, extracted with EtOAc dried with Na₂SO₄, filtered and the solvent is removed in vacuo. The residue is used without further purification. MS (method F): 371 [M+H].

### [(1R,2S)-1-(1H-Indole-7-sulfonylaminocarbonyl)-2-vinyl-cyclopropyl]-carbamic acid tert-butyl ester (Hydrochloride) (BB 27)

### Step 1

### [(1R,2S)-1-(1H-Indole-7-sulfonylaminocarbonyl)-2-vinyl-cyclopropyl]-carbamic acid tert-butyl ester

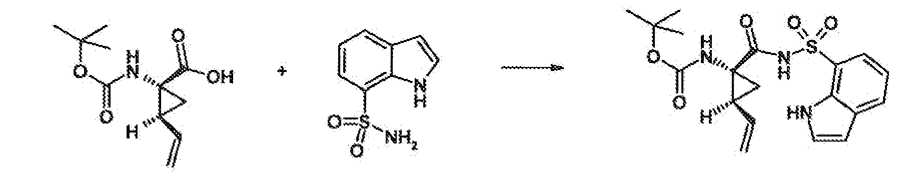

A mixture of 8.3 g (37 mmol) (1R,2S)-1-tert-Butoxycarbonylamino-2-vinyl-cyclopropane-carboxylic acid and 9.0 g (55 mmol) CD1 in 200 mL THF is refluxed for 1h, cooled to RT and 8.6 g ( 44 mmol) 1H-Indole-7-sulfonic acid amide (prepared as described in US 468300, July 1987) and 8.3 mL (55 mmol) DBU are added. The mixture is stirred at RT overnight, diluted with EtOAc and washed three times with aq. NaHCO₃-solution. The combined aq. layers are extracted with EtOAc and the combined organic layers are dried over Na₂SO₄ and concentrated under reduced pressure. The residue is purified by FC (silica gel, eluent: DCM/MeOH 19:1) to give the title compound. TLC (hexane/EtOAc 1:1): Rf = 0.52; LC-MS (method C): Rt = 3.803, M+H = 404.2.

### Step 2

### [(1R,2S)-1-(1H-Indole-7-sulfonylaminocarbonyl)-2-vinyl-cyclopropyl]-carbamic acid tert-butyl ester (Hydrochloride)

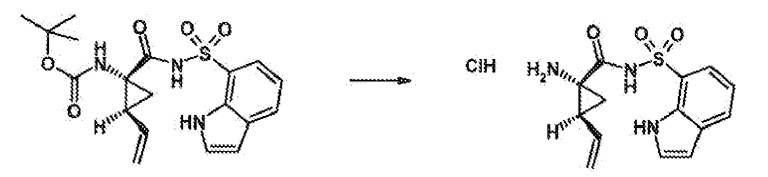

A mixture of 8.2 g (20 mmol) [(1R,2S)-1-(1H-Indole-7-sulfonylaminocarbonyl)-2-vinyl-cyclopropyl]-carbamic acid tert-butyl ester and 38 mL HCl (4 M in dioxane) in 38 mL dioxane is stirred at RT for 1.5 h. The mixture is concentrated under reduced pressure and co-evaporated with DCM to give the title compound. LC-MS (method C): Rt = 1.025, M+H = 304.1.

### N-((1R,2S)-1-Amino-2-vinyl-cyclopropanecarbonyl)-2-methylamino-benzene sulfonamide hydrochloride (BB 28)

### Step 1

### (2-Sulfamoyl-phenyl)-carbamic acid 2-trimethylsilanyl-ethyl ester

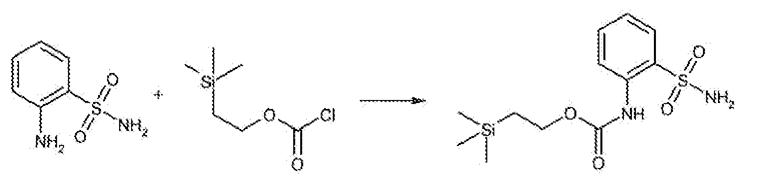

To a solution of 27.0 g (0.157 mol) of 2-Aminobenzenesulfonamide and 17.0 g (0.160 mol) Na₂CO₃ in a mixture of 150 mL dioxane and 150 mL H₂O is added a solution of 28.9 g (0.160 mol) Teoc-Cl in 50 mL dioxane at 0°C and the resulting mixture is stirred for 18 hours at RT. 200 mL of 1N HCl and 300 mL ether are added. The organic phase is separated and the aqueous phase is extracted twice with 300 mL Et₂O each. The combined organic phases are dried with MgSO₄ and concentrated in vacuo. The residue is chromatographed on SiO₂ (eluent hexanes/EtOAc 6:1 to hexanes/EtOAc 2:1) to give (2-Sulfamoyl-phenyl)-carbamic acid 2-trimethylsilanyl-ethyl ester as a white solid. LC-MS (method C): Rt = 4.13 min; M+Na = 339.0, M-1 = 315.1.

### Step 2

### [2-[((1R,2S)-1-tert-Butoxycarbonylamino-2-vinyl-cyclopropanecarbonyl)-sulfamoyl]-phenyl]-carbamic acid 2-trimethylsilanyl-ethyl ester

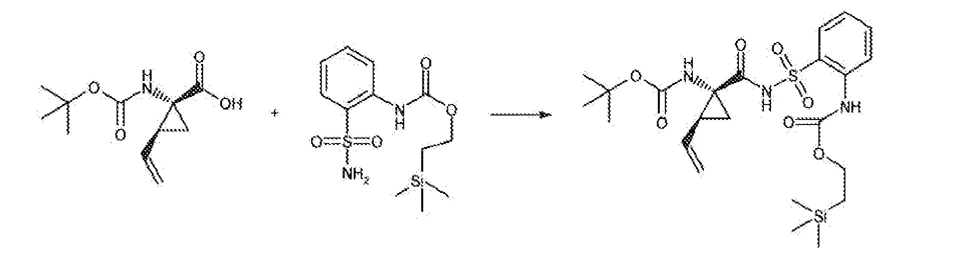

To a solution of 8.6 g (37.8 mmol) (1R,2S)-4-tert-Butoxycarbonylamino-2-vinyl-cyclopropanecarboxylic acid in 120 mL THF is added 9.69 g (56.8 mmol) CDI and the mixture is stirred at 70 °C for 2 hours. The mixture is allowed to cool to RT and 12.8 g (40.5 mmol) (2-Sulfamoyl-phenyl)-carbamic acid 2-trimethylsilanyl-ethyl ester and 8.6 mL (56.8 mmol) DBU are added. The reaction mixture is stirred at RT for 12 hours. 400 mL EtOAc are added and the mixture is washed twice with 150 mL 0.5 N HCl each. The organic layer is dried with MgSO₄ and concentrated in vacuo. The residue is chromatographed on SiO₂ (hexanes/EtOAc 6:1 to EtOAc) to give [2-[((1R,2S)-1-tert-Butoxycarbonylamino-2-vinyl-cyclopropanecarbonyl)-sulfamoyl]-phenyl]-carbamic acid 2-trimethylsilanyl-ethyl ester as a colorless oil. LC-MS (method C): Rt = 4.97 min; M+Na = 548.2, M-1 = 524.2.

### Step 3

### [(1R,2S)-1-(2-Amino-benzenesulfonylaminocarbonyl)-2-vinyl-cyclopropyl]-carbamic acid tert-butyl ester

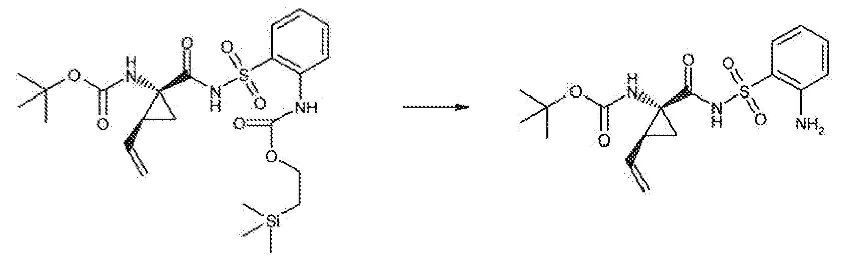

A mixture of 10 g (19.0 mmol) [2-[((1R,2S)-1-tert-Butoxycarbonylamino-2-vinyl-cyclopropanecarbonyl)-sulfamoyl]-phenyl]-carbamic acid 2-trimethylsilanyl-ethyl ester and 8.5 g (57.1 mmol) tetraethyl ammonium fluoride in 150 mL acetonitrile is stirred at 90°C for 1.5 hours. The reaction mixture is concentrated in vacuo and the residue is chromatographed on SiO₂ (CH₂Cl₂/MeOH 98:2 to 9:1) to give [(1R,2S)-1-(2-Amino-benzenesulfonylaminocarbonyl)-2-vinyl-cyclopropyl]-carbamic acid tert-butyl ester as a white solid. LC-MS (method C) Rt = 3.75 min; M+Na = 404.0, M-1 = 380.0.

### Step 4

### [(1R,2S)-1-(2-Methylamino-benzenesulfonylaminocarbonyl)-2-vinyl-cyclopropyl]-carbamic acid tert-butyl ester

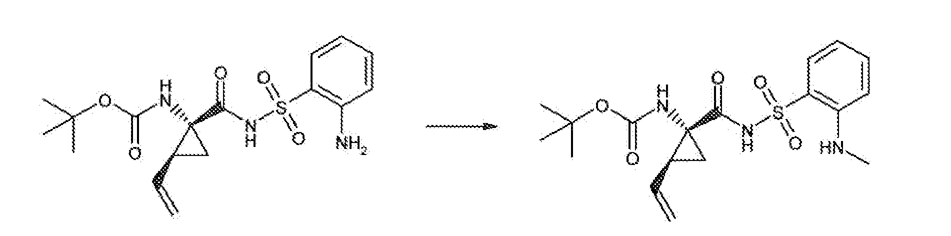

178 µl (2.83 mmol) Methyl iodide are added to a mixture of 1.08 g (2.83 mmol) [(1R,2S)-1-(2-Amino-benzenesulfonylaminocarbonyl)-2-vinyl-cyclopropyl]-carbamic acid tert-butyl ester and 435 mg K₂CO₃ (3.11 mmol) in 30 ml DMF. After stirring for 1 hour, the reaction mixture is concentrated in vacuo and the residue is chromatographed by preparative reverse phase HPLC (CH₃CN, H₂O, HCO₂H) to give [(1R,2S)-1-(2-Methylamino-benzenesulfonylaminocarbonyl)-2-vinyl-cyclopropyl]-carbamic acid tert-butyl ester as a white solid. LC-MS (method C) Rt = 4.025; M+H = 396.0.

### Step 5

### N-((1R,2S)-1-Amino-2-vinyl-cyclopropanecarbonyl)-2-methylamino-benzene sulfonamide hydrochloride

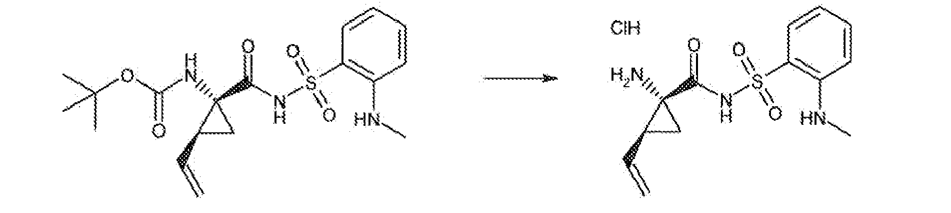

A mixture of 558 mg (1.41 mmol) [(1R,2S)-1-(2-Methylamino-benzenesulfonylaminocarbonyl)-2-vinyl-cyclopropyl]-carbamic acid tert-butyl ester in 3.5 ml HCl (4M in dioxane) and 3.5 ml dioxane is stirred at room temperature for 2 hours. Evaporation of the solvent affords N-((1R,2S)-1-Amino-2-vinyl-cyclopropanecarbonyl)-2-methylamino-benzene sulfonamide hydrochloride as a yellowish solid. HPLC (method E) Rt = 0.952 min; LC-MS (method C) Rt = 0.870; M+H = 296.0.

### N-((1R,2S)-1-Amino-2-vinyl-cyclopropanecarbonyl)-3-benzyloxy-benzenesulfonamide (BB 29)

### Step 1

### 1-Benzyloxy-3-bromo-benzene

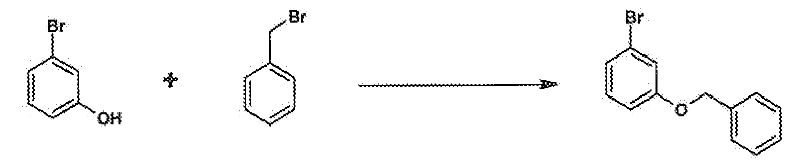

3-Bromophenol (19 g) and benzyl bromide (15.7 ml) in acetone (200ml) are treated with potassium carbonate (60.1 g) and the reaction mixture is stirred at RT for 72 hours. The reaction is filtered and the filter cake is washed with acetone. The filtrate is concentrated and purified *via* chromatography on SiO₂ gel (eluent hexanes/ethyl acetate 96:4) to give 1-benzyloxy-3-bromo benzene as a white solid.

### Step 2

### 3-Benzyloxy-benzenesulfonamide

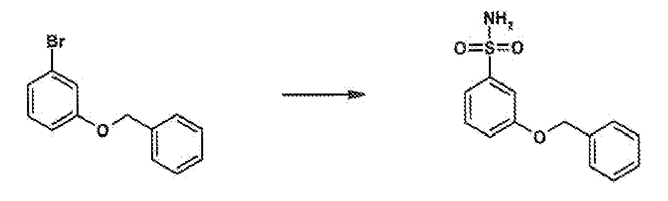

A solution of 1-benzyloxy-3-bromobenzene (28.3 g) in Et₂O (375 ml) was cooled to -70 °C and treated with TMEDA (19.2 ml) and *n*-BuLi in hexane (1.6 M, 79 ml). The solution is stirred at -70 °C for 1 h and transferred into a cooled solution (-70 °C) of SO₂ (54.4 g) in Et₂O (375 ml). The mixture is kept at -70°C for 15 min, then allowed to warm to room temperature over 1 h. The solvent is evaporated and the residue is suspended aq. sodium phosphate (1M, 750 ml, pH 6). EtOAc (500 ml) is added and the solution is cooled to 0°C. *N*-Chlorosuccinimide (43.5 g) is slowly added and the pH is readjusted to pH 6 by addition of Na₃PO₄. The reaction mixture is stirred vigorously for 1 h. The phases are separated and the aq. phase is extracted twice with EtOAc. The combined organic phases are washed with H₂O and brine, dried and concentrated to give a yellowish oil. The residue is dissolved in dioxane (400 ml) and NH₃ in H₂O (28%, 200 ml) is added. The reaction mixture is stirred for 12 h and then concentrated to dryness. Residue chromatographed on SiO₂ gel (eluent hexanes/EtOAc 4:1 to 3:7) to give 19.5 g of 3-benzyloxy-benzenesulfonamide as a white powder. MS (method F): M-H = 262.

### Step 3

### [(1R,2S)-1-(3-Benzyloxy-benzenesulfonylaminocarbonyl)-2-vinyl-cyclopropyl]-carbamic acid tert-butyl ester

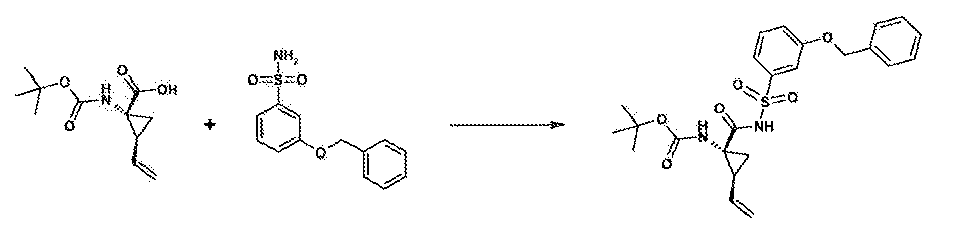

A solution of (1*R*,2*S*)-1-*tert*-Butoxycarbonylamino-2-vinyl-cyclopropanecarboxylic acid (700 mg) in THF (10 ml) is treated with carbonyl diimidazole (789 mg) and the reaction mixture is stirred at 65 °C for 30 min. The mixture is allowed to cool to RT and - benzyloxy-benzenesulfonamide (1.05 g) and DBU (0.697 ml) are added. The solution is stirred at RT for 12 h. The reaction mixture is taken up in EtOAc, washed with 0.1 M HCl (aq.), aq. NaHCO3 and brine, dried with Na2SO4 and concentrated. The residue is chromatographed on SiO₂ gel (eluent hexanes/EtOAc 7:3 to EtOAc, then EtOAc/MeOH 9:1) to give [(1*R*,2*S*)-1-(3-Benzyloxy-benzenesulfonylaminocarbonyl)-2-vinyl-cyclopropyl]-carbamic acid *tert*-butyl ester. MS (method F): M+H = 473.

### Step 4

### N-((1R,2S)-1-Amino-2-vinyl-cyclopropanecarbonyl)-3-benzyloxy-benzenesulfonamide

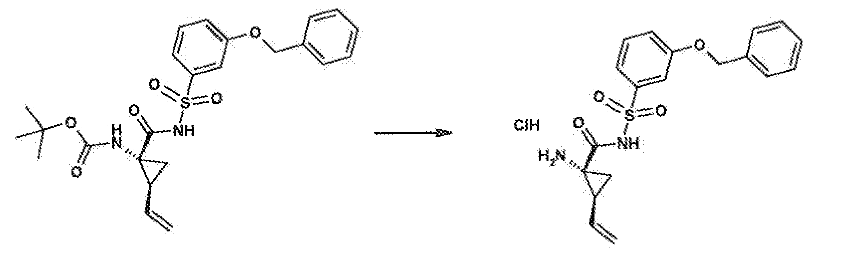

A solution of [(1*R*,2*S*)-1-(3-Benzyloxy-benzenesulofonylaminocarbonyl)-2-vinyl cyclopropyl]-carbamic acid *tert*-butyl ester (850 mg) in dioxane (5 ml) is treated with HCl in dioxane (4M, 10 ml) and is stirred at RT for 4 h. The reaction mixture is evaporated to give *N*-((1*R*, 2*S*)-1-amino-2-vinyl-cyclopropanecarbonl)-3-benzyloxy-benzenesulfonamide hydrochloride. MS (method F): M+H = 373.

### Example 49:

### Pyrazine-2-carboxylic acid ((S)-{(S)-1-[(R)-3-(2-carbamoyl-1-cyclobutylmethyl-2-oxo-ethylcarbamoyl)-4-(4-chloro-benzyl)-piperazine-1-carbonyl]-2,2-dimethyl-propylcarbamoyl}-cyclohexyl-methyl)-amide

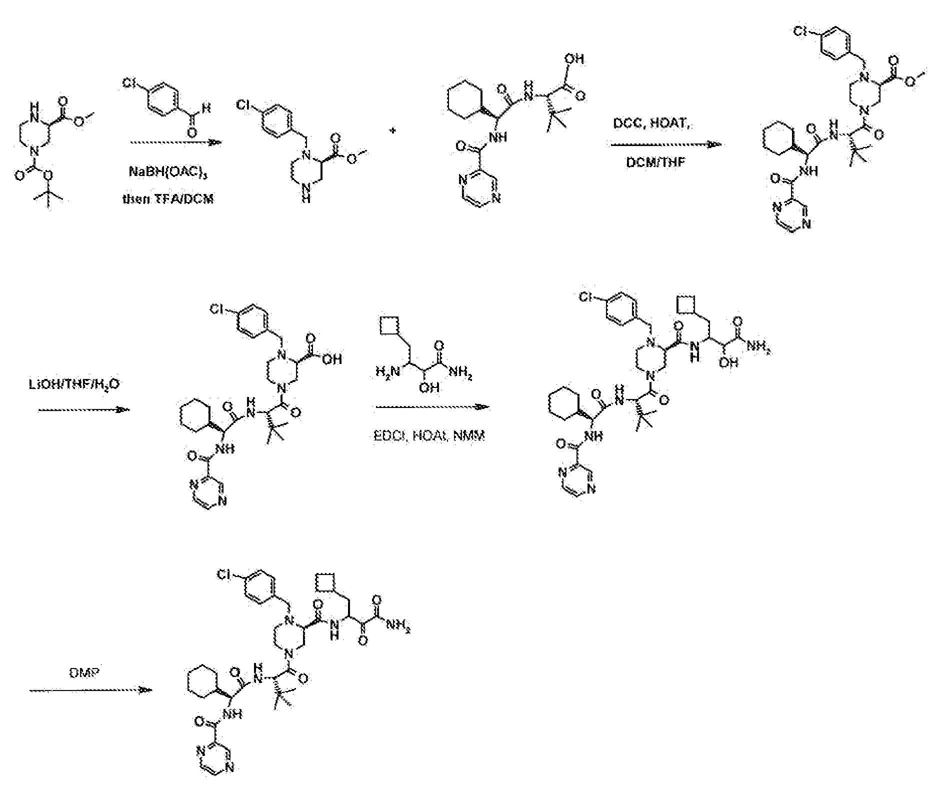

### Step A: (R)-1-(4-Chloro-benzyl)-piperazine-2-carboxylic acid methyl ester

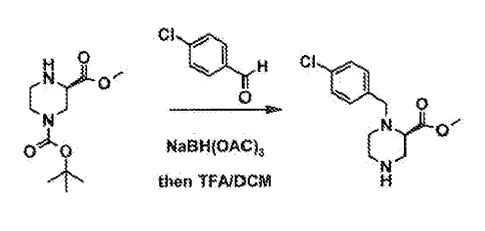

R-4N-Boc-piperazine 2-carboxylic acid methyl ester (679*A*mg, 2.78mmol) and 4-chlorobenzaldehyde (390.94mg, 2.78mmol) are mixed in dichloromethane(10ml) for 30 minutes. Sodium triacetoxyborohydride (800mg, 3.77mmol) is added. The mixture is stirred at room temperature for 16 hr. Water is added. The aqueous layer is extracted with dichloromethane twice (30mL x2). The dichloromethane solution is treated with trifluoracetic acid (30ml). After 4 hrs the solvent is evaporated and re-dissolved in water. The water solution is basified by adding K₂CO₃ (solid). The water solution is extracted with EtOAc three times. The organic layer is dried over NaSO₄. The product is colorless oil (748mg, 100%) after removing solvent to dryness. Found m/z ES+=269

### Step B: (R)-(4-Chloro-benzyl)-4((S)-2-{(S)-2-cyclohexyl-2-[(pyrazine-2-carbonyl)-amino]-acetylamino}-3,3-dimethyl-butyryl)piperazine-2-carboxylic acid methyl ester

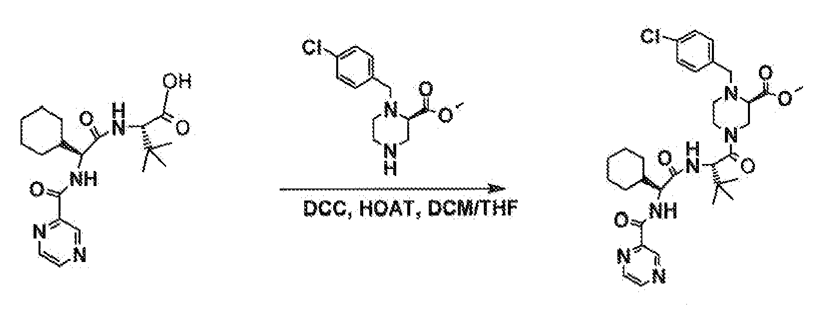

A dichloromethane (5ml) solution of (*S*)-2-{(*S*)-2-Cyclohexyl-2-[(pyrazine-2-carbonyl)-amino]-acetylamino}-3,3-dimethyl-butyric acid (268mg, 0.71mmol) is treated with 1,3-dicyclohexyl carbodiimide (160mg, 0.77mmol), and 7-aza-1-hydroxy (96.9mg, 0.71mmol). After stirring for 30 min., the reaction mixture is treated with a THF solution (5ml) of (*R*)-1-(4-Chloro-benzyl)-piperazine-2-carboxylic acid methyl ester (174mg, 0.65mmol). The reaction mixture is stirred at room temperature for 16hrs. The white solid is removed by filtration. The filtrates are concentrated in vacuo to give a residue that is purified by flash column chromatography 2%-100% EtOAc/Hexane. The product is obtained as a colorless oil (369mg). Found m/z ES+=627

### Step C: (R)-1-(4-Chloro-benzyl)-4-((S)-2-{(S)-2-cyclohexyl-2-[(pyrazine-2-carbonyl)-amino]-acetylamino}-3,3-dimethyl-butyryl)-piperazine-2-carboxylic acid

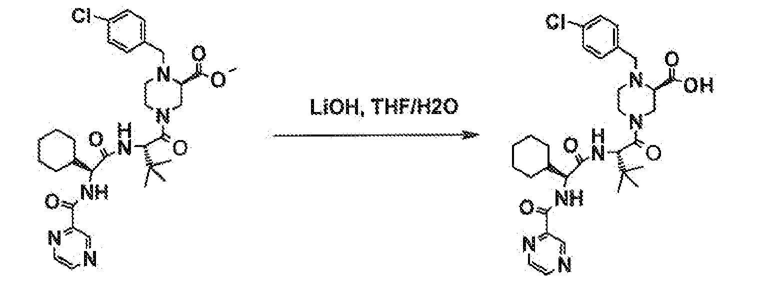

To the solution of compound (*R*)-1-(4-Chloro-benzyl)-4-(*(S*)-2-{(*S*)-2-cyclohexyl-2-[(pyrazine-2-carbonyl)-amino]-acetylamino}-3,3-dimethyl-butyryl)-pipcrazine-2-carboxylic acid methyl ester(369mg, 0.589mmol) in THF/H₂O (10ml/4ml) is added Lithium hydroxy (53mg, 1.26mmol). The mixture is stirred at room temperature for 16 hours. The solution is acidified by 1N HCl. The aqueous layer is extracted by EtOAc. Dried over NaSO₄. The product is obtained as a white solid (390mg) after removing the solvent to dryness. Found m/z ES+=613

### Step D: Pyrazine-2-carboxylic acid ((S)-{(S)-1-(R)-3-(2-carbamoyl-1-cyclobutylmethyl-2-hydroxy-ethylcarbamoy l)-4-(4-chloro-benzyl)-piperazine-1-carbonyl]-2,2-dimethyl-propylcarbamoyl}-cyclohexyl-methyl)-amide

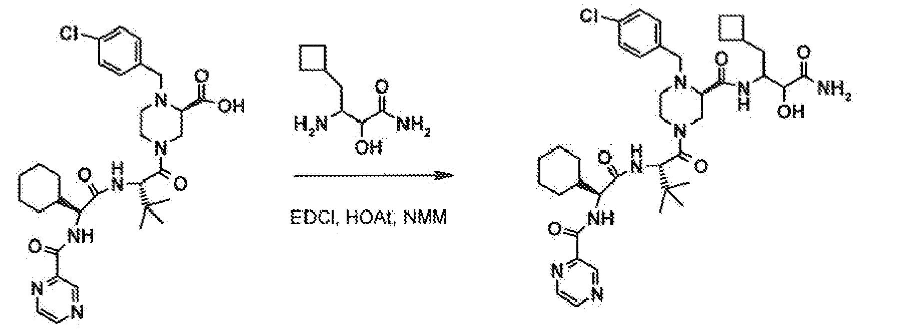

(*R*)-1-(4-Chloro-benzyl)-4-((*S*)-2-{(S)-2-cyclohexyl-2-[(pyrazine-2-carbonyl)-amino]-acetylamino}-3,3-dimethyl-butyryl)-piperazine-2-carboxylic acid (62.72mg; 0.1mmol), 1-ethyl-3-(3'-(dimethylamino)propyl)carbodiimide hydrochloride (28.65, 0.15mmol), 1-hydroxybenzotriazole (20.26mg, 0.15mmol) are mixed in CH₂Cl₂/DMF(3mL/3mL). N-methyl morpohline (0.04ml, 0.36mmol) is added. The mixture is stirred for 16 hrs. Purified by Biotage 2%-100% EtOAc/Hexane, then 2%-10% MeOH/Hexane. The product is obtained as colorless oil 50mg. Found m/z ES+=766

### Step E: Pyrazine-2-carboxylic acid ((S)-{(S)-1-[(R)-3-(2-carbamoyl-1-cyclobutylmethyl-2-oxo-ethylcarbamoyl)-4-(4-chloro-benzyl)-piperazine-1-carbonyl]-2,2-dimethyl-propylcarbamoyl}-cyclohexyl-methyl)-amide

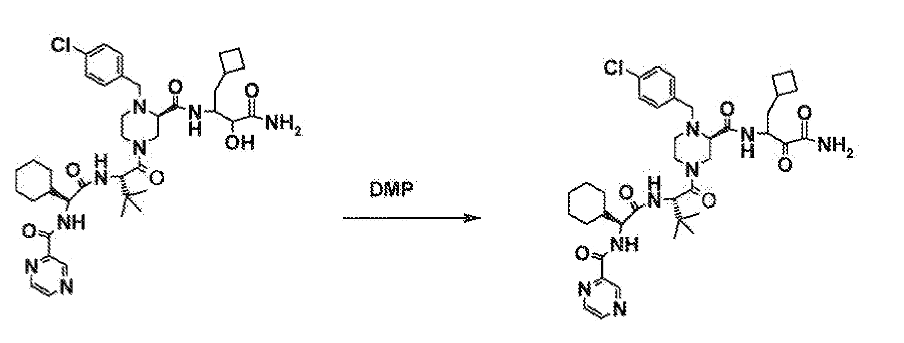

To a dichloromethane solution (5mL) of Pyrazine-2-carboxylic acid ((*S*)-{(*S*)-1-[(*R*)-3-(2-carbamoyl-1-cyclobutylmethyl-2-hydroxy-ethylcarbamoy l)-4-(4-chlorobenzyl)-piperazine-1-carbonyl]-2,2-dimethyl-propylcarbamoyl}-cyclohexyl-methyl)-amide (50mg, 0.065mmol) is added Dess-Martin periodinane (49.96mg, 0.12mmol). The reaction is stirred at room temperature for 1 hr and quenched with 10% NaSO₃(10ml) for 20 mins. The resulting mixture is extracted with EtOAc. The resulting residue is purified by Biotage 28%-100% EtOAc/Hexane, then 2%-20% MeOH/EtOAc. The product is obtained as a white solid (25.9mg). Found m/z ES+=764

### Example 50

### Pyrazine-2-carboxylic acid ((S)-{(S)-1-[(R)-3-(2-carbamoyl-1-cyclobutylmethyl-2-oxo-ethylcarbamoyl)-4-(4-chlorophenyl)-piperazine-1-carbonyl]-2,2-dimethyl-propylcarbamoyl}-cyclohexyl-methyl)-amide

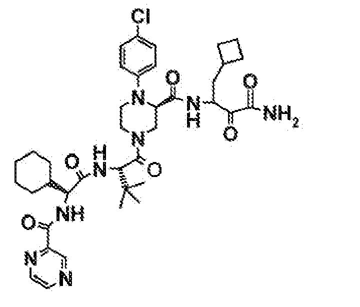

The title compound is prepared by the process of Example 49 in which (R)-1-(4-Chlorophenyl)-piperazine-2-carboxylic acid methyl ester as prepared below (after treatment with trifluoroacetic acid and basification with sodium carbonate) is used in place of (R)-1-(4-Chloro-benzyl)-piperazine-2-carboxylic acid methyl ester.

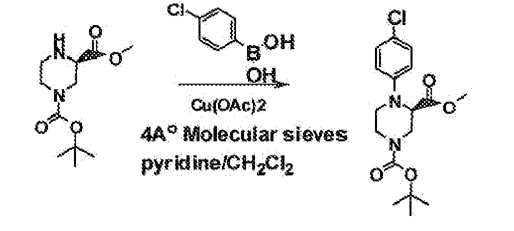

### (R)-1-(4-Chlorophenyl)-piperazin-2-carboxylic acid methyl ester

R-4N-Boc-piperazine 2-carboxylic acid methyl ester (4.0gms, 16.4mmol) and 4-chlorophenylbornic acid (5.0gms, 32.8mmol) are mixed in dichloromethane (50ml) followed by addition of cupric acetate (3.0gms, 16.4mmol), 4Ǻ molecular sieves (1 gm) and pyridine (3.28ml, 32.8mmol). The mixture is stirred at room temperature for 50 hr. The reaction mixture is concentrated directly in vacuo, diluted with ethyl acetate, and filtered through Celite. The organic filtrate is concentrated and the remaining residue is purified over silica gel column chromatography eluting with hexane and ethyl acetate to give 860 mg as a white solid.

### Example 51

### (3S,4R)-1-[(S)-2-(3-tert-Butyl-ureido)-3,3-dimethyl-butyryl]-4-(4-chloro-phenyl)-pyrrolidin-3-carboxylic acid (2-carbamoyl-1-cyclobutylmethyl-2-oxo-ethyl)-amid

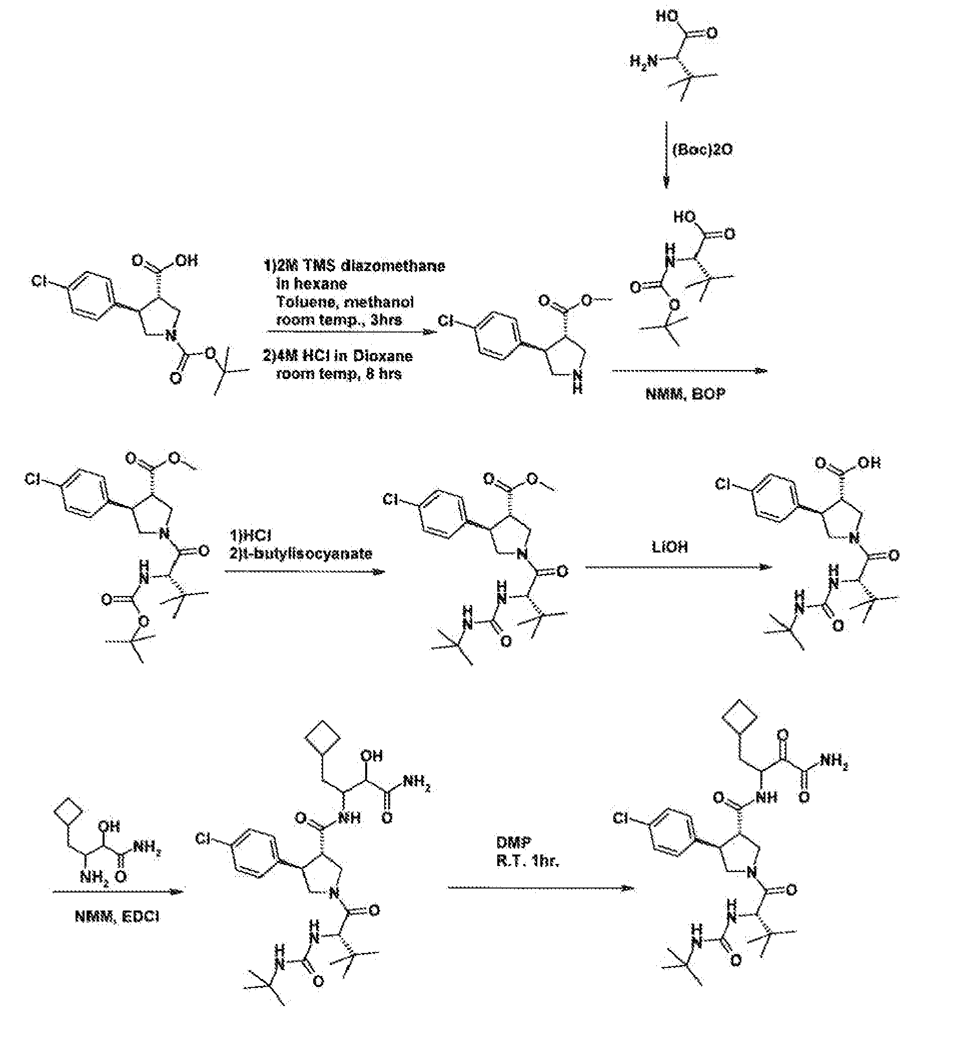

### Step A: (2S,3R)-3-(4-Chloro-phenyl)-2-methylaminomethyl-butyric acid methyl ester

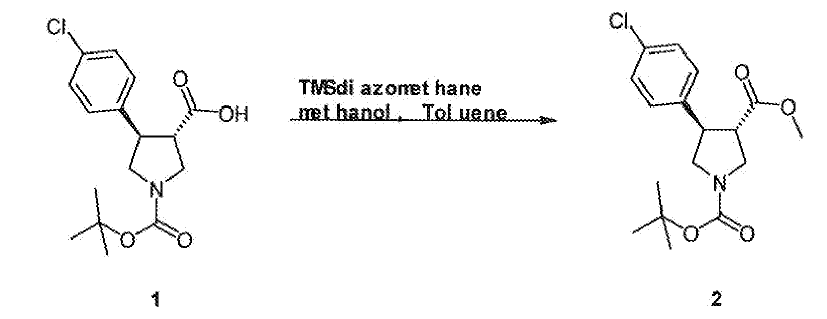

2M TMS diazomethane solution (370 µl, 0.75 mmol) is added to a solution of 1 (200 mg, 0.613mmol) in 2 ml toluene and 1 ml methanol at room temperature. Clear solution is observed. Reaction is stirred until the complete conversion of starting material by LCMS analysis is confirmed. The reaction is concentrated in vacuo to give desired product as a colorless oil. To the crude product 5 ml of 4M HCl in dioxane is added and stirred for 4 hrs at room temperature in a sealed reaction vial. Complete conversion of starting material is observed and reaction is concentrated in vacuo to afford 139 mg of the desired 2. as a white solids. found m/z in ES+ = 240.2

### Step B: (S)-2-tert-Butoxycarbonylamino-3,3-dimethyl-butyric acid

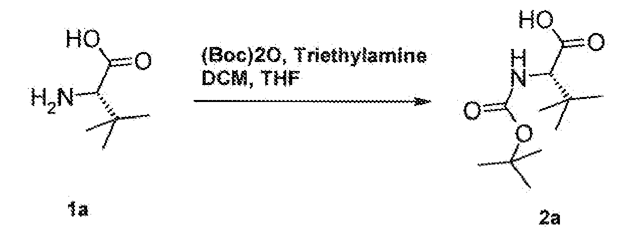

Boc anhydride (1.84 g, 8.38 mmol), triethylamine (1.2 g, 11.43 mmol) are added to the solution of la (1 g, 7.62 mmol) in 20 ml anhydrous CH₂Cl₂ at room temperature. Suspension of starting material is observed. After 30 min 5 ml anhydrous THF is added to the reaction mixture and reaction appeared clear solution. Reaction is stirred at room temperature for overnight. Complete conversion of starting material is observed by LCMS. Reaction is concentrated in vacuo and purified over silica gel column chromatography eluting with hexane and ethyl acetate to give 1.32 g of the desired 2a as a white crystals. found m/z in ES+ = 232.3, m/z in ES- = 230.3

### Step C: (3S,4R)-1-((S)-2-tert-Butoxycarbonylamino-3,3-dimethyl-butyryl)-4-(4-chloro-phenyl)-pyrroildine-3-carboxylic acid methyl ester

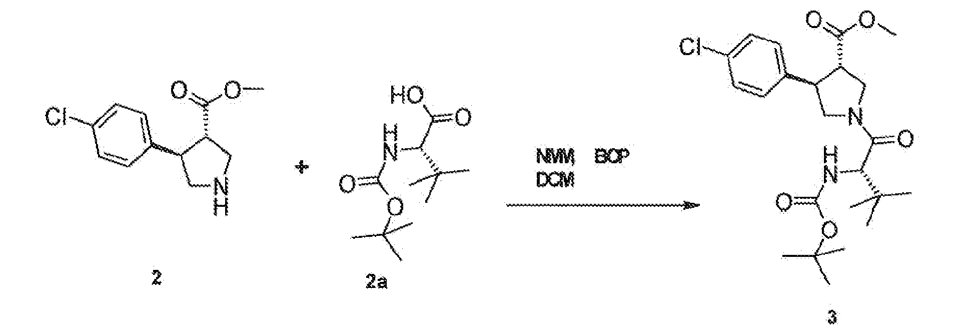

BOP reagent (210 mg, 0.476 mmol), 2 (131 mg, 0.476 mmol), 2a (100 mg, 0.432 mmol) and N-methyl morpholine (143 µl, 1.30 mmol) are added to I mL of anhydrous CH₂Cl₂ and 1 ml anhydrous DMF at 0°C under N₂ atmosphere. Clear solution is obtained after 5 minutes of stirring. Reaction is continued to stir for 4 hours. The reaction mixture is quenched with saturated NaHCO₃, extracted, dried over sodium sulphate, filtered, and concentrated in vacuo. The residue is purified over silica gel column chromatography eluting with hexane and ethyl acetate to give 118 mg of the desired 3 as a colorless oil. found m/z in ES+ = 453.0, m/x in ES- = 451.0

### Step D: (3S,4R)-1-[(S)-2-(3-tert-Butyl-ureido)-3,3-dimethyl-butyryl]-4-(4-chlorophenyl)-pyrrolidine-3-carboxylic acid methyl ester

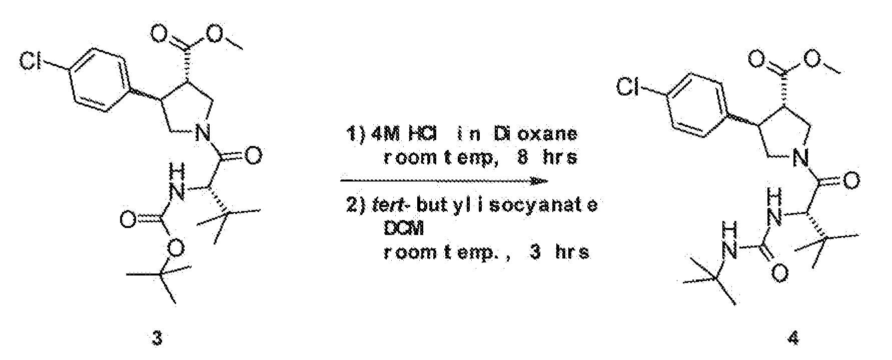

5 ml of 4M HCl in dioxane is added to the solution of 3 (118 mg, 0.261 mmol) in 2 ml anhydrous CH₂Cl₂ and stirred for 4 hrs at room temperature in a sealed reaction vial. Complete conversion of starting material is observed and reaction is concentrated in vacuo. To the crude product in 3 ml anhydrous CH₂Cl₂ *tert*-butylisocyanate (28.5 mg, 0.287 mmol), N-methyl morpholine (29.0 mg, 0.287 mmol) is added at 0°C and stirred for overnight. Reaction is concentrated in vacuo to afford 100 mg of the desired 4 as a colorless oil. found m/z in ES+ = 452.0

### Step E:(3S,4R)-1-[(S)-2-(3-tert-Butyl-ureido)-3,3-dimethyl-butyryl]-4-(4-chlorophenyl)-pyrrolidine-3-carboxylic acid

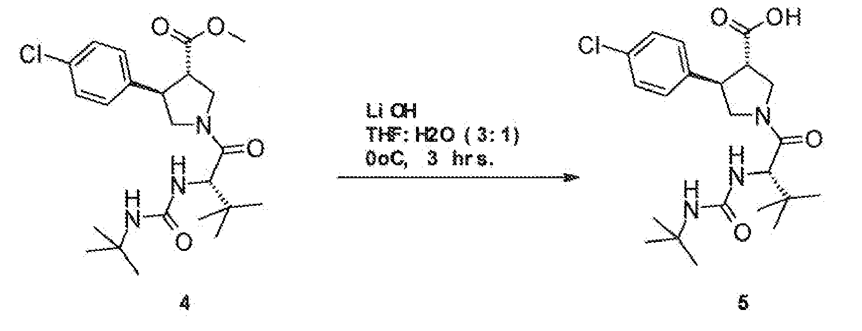

LiOH (12.3 mg, 0.55 mmol) is added to the solution of 4 (100 mg, 0.22 mmol) in 3ml THF and 1 ml H₂O at room temperature and stirred for 3 hrs. Complete conversion of starting material is observed. The reaction mixture is quenched with saturated ammonium chloride, extracted, dried over sodium sulphate, filtered, and concentrated in vacuo. The residue is purified over silica gel column chromatography eluting with hexane and ethyl acetate to give 50.2 mg of the desired 5 as a colorless oil. found m/z in ES+ = 438.3, m/z in ES- = 436.4

### Step F: (3S,4R)-1-[(S)-2-(3-tert-Butyl-ureido)-3,3-dimethyl-butyryl]-4-(4-chlorophenyl)-pyrrolidine-3-carboxylic acid (2-carbamoyl-1-cyclobutylmethyl-2-hydroxyethyl)-amide

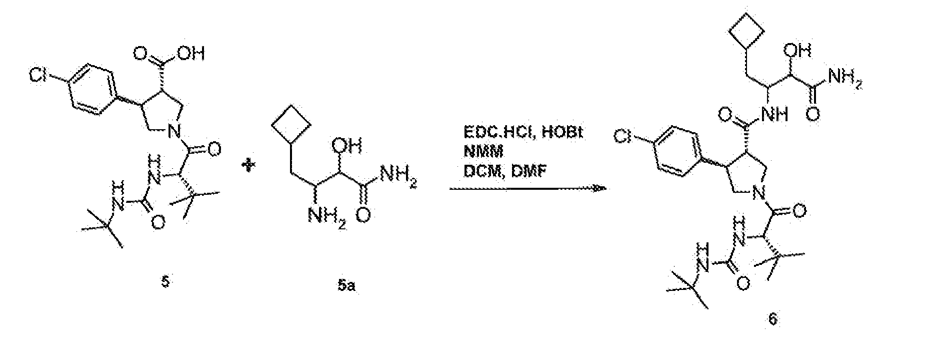

EDC.HCl (33.7 mg, 0.171 mmol), HOBt (23.4 mg, 0.171 mmol), 5 (50 mg, 0.114 mmol), 5a (23.6 mg, 0.126 mmol) and N-methyl morpholine (50.5 µl. 0.457 mmol) are added to 3 mL anhydrous CH₂Cl₂ and 2 mL of anhydrous DMF at 0°C under N₂ atmosphere. Clear solution is obtained after 5 min of stirring. Reaction is continued to stir for overnight. The reaction mixture is quenched with water, extracted, dried over sodium sulphate, filtered, and concentrated in vacuo to give 50.7 mg of the desired 6 as a colorless oil. found m/z in ES+ = 592.5, m/z in ES- = 591

### Step G:(3S,4R)-1-[(S)-2-(3-tert-Butyl-ureido)-3,3-dimethyl-butyryl]-4-(4-chlorophenyl)-pyrrolidine-3-carboxylic acid (2-carbamoyl-1-cyclobutylmethyl-2-oxoethyl)-amide

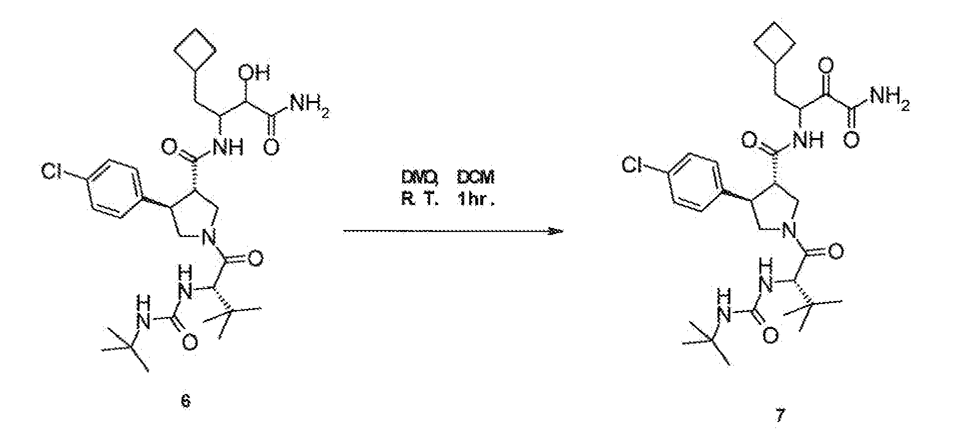

DMP reagent (54.6 mg, 0.1317 mmol) is added to the solution of 6 (50 mg, 0.084 mmol) in 5 ml anhydrous CH₂Cl₂ at 0°C and stirred for 1 hr. The reaction mixture is quenched with 10% sodium sulphite, extracted, dried over sodium sulphate, filtered, and concentrated in vacuo. The residue is purified over silica gel column chromatography eluting with hexane and ethyl acetate to give 20 mg of the desired 7 as a white solid. found m/z in ES+ =590.57, m/z in ES- = 588.62.

### Example 53: Generic procedure for the preparation of ketosulfonamide compounds of the invention.

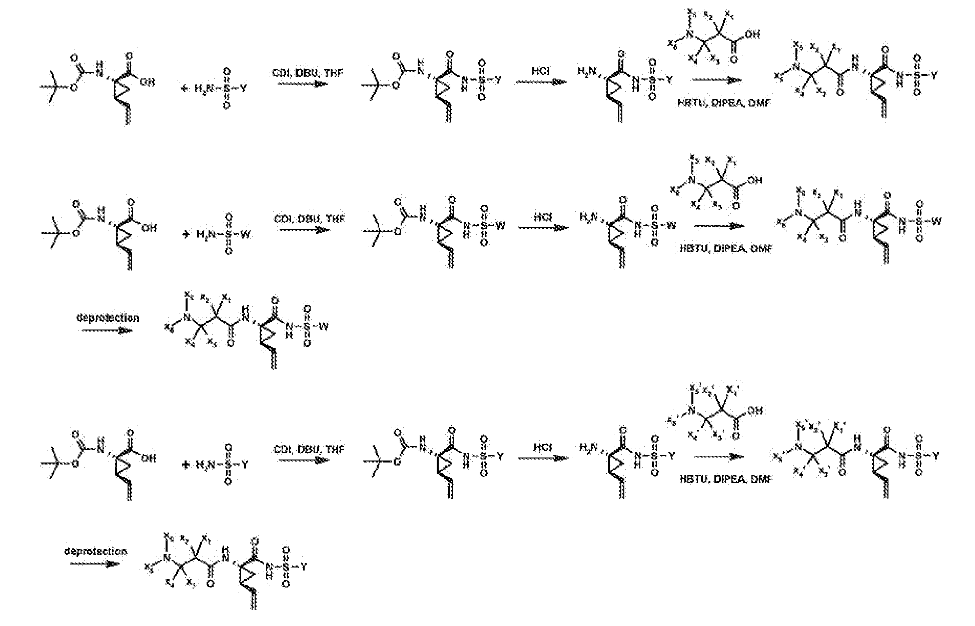

### Example 54:

### (S)-1-Quinolin-4-ylmethyl-pyrrolidine-3-carboxylic acid [(1R,2S)-1-(3-benzyloxy-benzenesulfonylaminocarbonyl)-2-vinyl-cyclopropyl]-amide

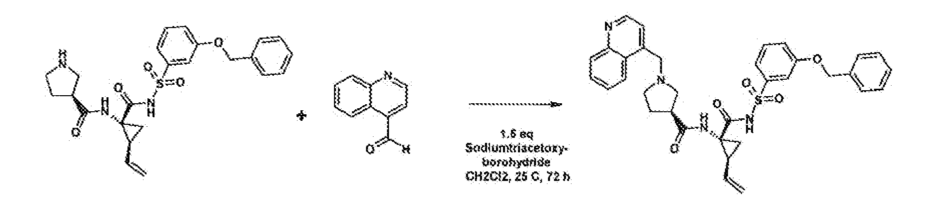

(S)-Pyrrolidine-3-carboxylic acid [(1R,2S)-1-(3-benzyloxy-benlenesulfonylaminocarbonyl)-2-vinyl-cyclopropyl]-amide (0.1 g) and quinoline-4-carboxaldehyde (0.033 g) in 2 mL of CH₂Cl₂ are treated with sodiumtriracetoxyborohydride (0.071 g) and stirred overnight at RT. More sodium triacetoxyborohydride (0.071 g) in THF is added together with 2 drops of acetic acid. The reaction mixture is stirred at RT for 72 hours, taken up in CH₂Cl₂, extracted with aqueous NaHCO₃, and concentrated to an oil. The residue is chromatographed on SiO₂ gel (eluent CH₂Cl₂ /MeOH 95:5 to 9:1) to give (S)-1-quinolin-4-ylmethyl-pyrrolidine-3-carboxylic acid [(1R,2S)-1-(3-benzyloxy-benzenesulfonylaminocarbonyl)-2-vinyl-cyclopropyl]-amide as a white powder. API-MS: M+1 = 611.

### Example 55:

### (S)-1-Naphthalen-1-ylmethyl-pyrrolidine-3-carboxylic acid [(1R,2S)-1-(3-benzyloxy-benzenesulfonylaminocarbonyl)-2-vinyl-cyclopropyl]-amide

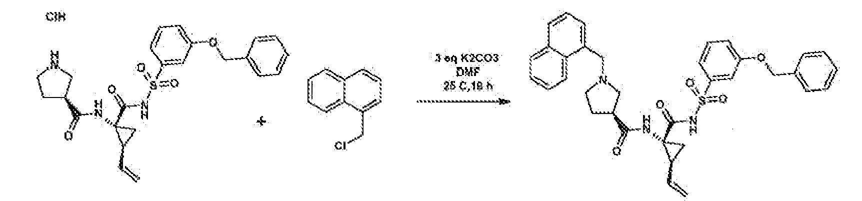

A solution of (S)-pyrrolidine-3-carboxylic acid [(1R,2S)-1-(3-benzyloxy-benzenesulfonylaminocarbonyl)-2-vinyl-cyclopropyl]-amide hydrochloride (0.08 g), 1-(chloromethyl)naphthalene (0.033 g), and K₂CO₃ (0.066 g) in 1 mL of DMF is stirred at RT overnight. The reaction mixture is taken up in 1N HCl, extracted with EtOAc, and concentrated. The residue is chromatographed by preparative reverse phase HPLC (CH₃CN, H₂O, TFA) to give (S)-1-naphthalen-1-ylmethyl-pyrrolidine-3-carboxylic acid [(1R,2S)-1-(3-benzyloxy-benzenesulfonylaminocarbonyl)-2-vinyl-cyclopropyl]-amide as a white powder. API-MS: M+1= 610.

### Example 56:

### (S)-pyrrolidine-3-carboxylic acid [(1R,2S)-1-(3-benxyloxy-benzenesulfonylaminocarbonyl)-2-vinyl-cyclopropyl]-amide hydrochloride

### Step A: (S)-Pyrolidine-3-carboxylic acid [(1R,2S)-1-(3-benzyloxy-benzenesulfonylaminocarbonyl)-2-vinyl-cyclopropyl]-amide is prepared as follows:

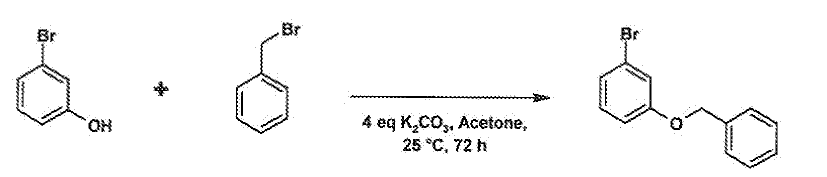

3-Bromophenol (19 g) and benzyl bromide (15.7 mL) in acetone (200 mL) are treated with potassium carbonate (60.1 g) and the reaction mixture is stirred at RT for 72 hours. The reaction is filtered and the filter cake is washed with acetone. The filtrate is concentrated and purified *via* chromatography on SiO₂ gel (eluent hexanes/EtOAc 96:4) to give 1-benzyloxy-3-bromobenzene as a white solid.

### Step B:

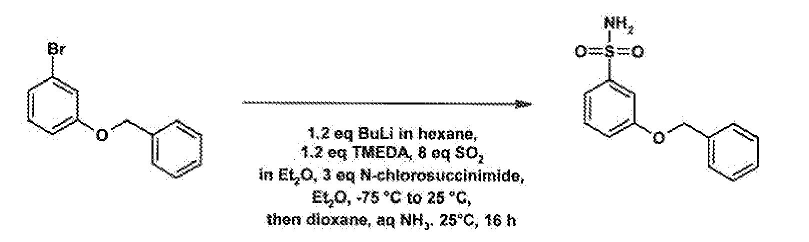

A solution of 1-benzyloxy-3-bromohenzene (28.3 g) in Et₂O (375 mL) is cooled to -70 °C and treated with TMEDA (19.2 mL) and *n*-BuLi in hexane (1.6 M, 79 mL). The solution is stirred at -70 °C for I h and transferred into a cooled solution (-70 °C) of SO₂ (54.4 g) in Et₂O (375 mL). The mixture is kept at -70°C for 15 minutes, then allowed to warm to RT over 1 h. The solvent is evaporated and the residue is suspended in aqueous sodium phosphate (1M, 750 mL, pH 6). EtOAc (500 mL) is added and the solution is cooled to 0°C. *N*-Chlorosuccinimide (43.5 g) is slowly added and the pH is readjusted to pH 6 by addition of Na₃PO₄. The reaction mixture is stirred vigorously for 1 h. The phases are separated and the aqueous phase is extracted twice with EtOAc. The combined organic phases are washed with H₂O and brine, dried and concentrated to give a yellowish oil. The residue is taken up in dioxane (400 mL) and NH₃ in H₂O (28%, 200 mL) is added. The reaction mixture is stirred for 12 h and then concentrated to dryness. The residue is chromatographed on SiO₂ gel (eluent hexanes/EtOAc 4:1 to 3:7) to give 3-benzyloxy-benzenesulfonamide as a white powder. API-MS: M-1 = 262.

### Step C:

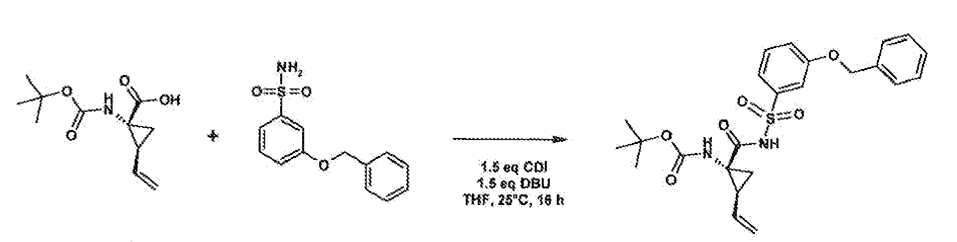

A solution of 0.7 g of (1*R*,2*S*)-1-*tert*-butoxycarbonylamino-2-vinyl-cyclopropanecarboxylic acid (prepared as described in Journal of Organic Chemistry, 2005, 5869-5879) in THF (10 mL) is treated with carbonyldiimidazole (0.789 g) and the reaction mixture is stirred at 65 °C for 30 min. The mixture is allowed to cool to RT and 3-benzyloxy-benzenesulfonamide (1.05 g) and DBU (0.697 ml) are added. The solution is stirred at RT for 12 h. The reaction mixture is taken up in EtOAc, washed with 0.1N aqueous HCl, aqueous NaHCO₃ and brine, dried with Na₂SO₄ and concentrated. The residue is chromatographed on SiO₂ gel (eluent hexanes/EtOAc 7:3 to EtOAc, then EtOAc/MeOH 9:1) to give [(1*R*,2*S*)-1-(3-benzyloxy-benzenesulfonylaminocarbonyl)-2-vinyl-cyclopropyl]-carbamic acid *tert*-butyl ester. API-MS: M+1 = 473.

### Step D:

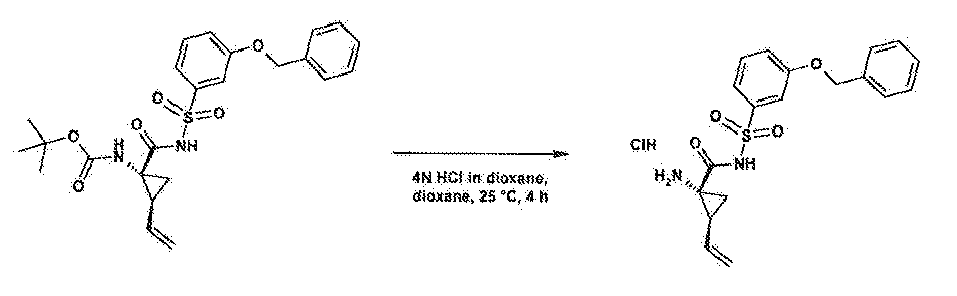

A solution of [(1*R*,2*S*)-1-(3-benzyloxy-benzenesulfonylaminocarbonyl)-2-vinyl-cyclopropyl]-carbamic acid *tert*-butyl ester (0.85 g) in dioxane (5 mL) is treated with HCl in dioxane (4N, 10 mL) and is stirred at RT for 4 h. The reaction mixture is evaporated to give *N*-((1*R*, 2*S*)-1-amino-2-vinyl-cyclopropanecarbonyl)-3-benzyloxy-benzenesulfonamide hydrochloride. API-MS: M+1 = 373.

### Step E:

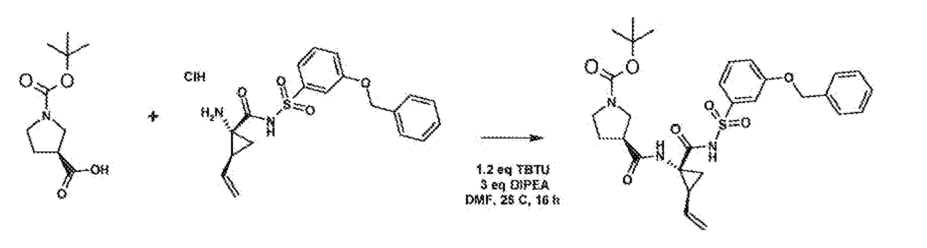

A solution of (S)-pyrrolidine-1,3-dicarboxylic acid-1-tert-t-butylester (0.35 g), *N-*((1*R*, 2*S*)-1-amino-2-vinyl-cyclopropanecarbonyl)-3-benzyloxy-benzenesulfonamide (0.665 g) and Hunig's base (0.852 mL) in 4 mL of DMF is treated with TBTU (0.627 g) and stirred at RT overnight. The reaction mixture is treated with 0.1 N HCl, extracted with EtOAc, washed with saturated aqueous NaHCO₃, brine, and concentrated in vacuo. The crude product is chromatographed by preparative reverse phase HPLC (CH₃CN, H₂O, TEA) to give (*S*)-3-[(1*R*,2*S*)-1-(3-Benzyloxy-benzenesulfonylaminocarbonyl)-2-vinyl-cyclopropylcarbamoyl]-pyrrolidine-1-carboxylic acid *tert*-butyl ester. API-MS: M-1 = 508.

### Step F:

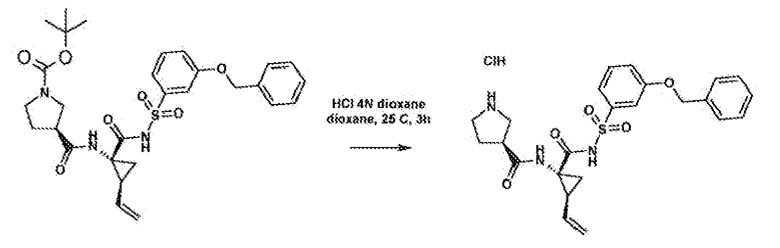

A suspension of (*S*)-3-[(1*R*,2*S*)-1-(3-Benzyloxy-benzenesulfonylaminocarbonyl)-2-vinyl-cyclopropylcarbamoyl]-pyrrolidtne-1-carboxylic acid *tert*-butyl ester (0,669 g) and 4N HCl in dioxane (5 mL) in 20 mL of dioxane is stirred at RT for 3 hours. The reaction mixture is concentrated in vacuo, treated with MeOH, and concentrated again to give (S)-pyrrolidine-3-carboxylic acid [(1R,2S)-1-(3-benzyloxy-benzenesulfonylaminocarbonyl)-2-vinyl-cyclopropyl]-amide hydrochloride. API-MS: M+1 = 470

### Example 57:

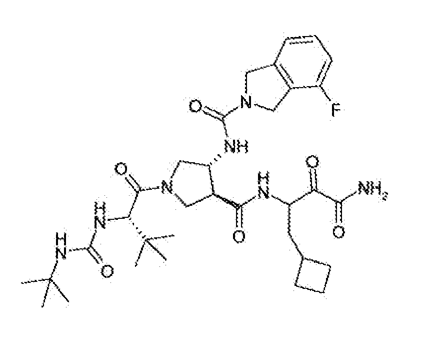

### Step 57A

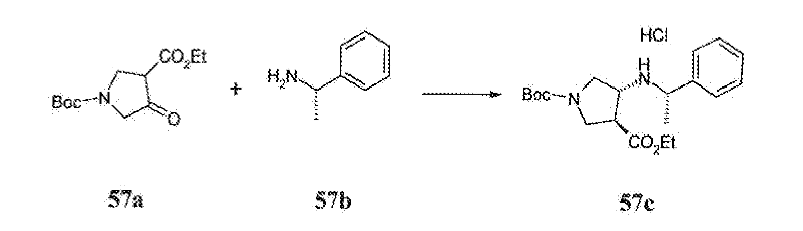

S-(-)-Methylbenxylamine (3 mL, 2.82 g, 23.3 mmol) and glacial acetic acid (1.33 mL, 1.39 g, 23.3 mmol) are added to a solution of **57a** (3.0 g, 0.11 mmol) in absolute ethanol (45 mL) and the mixture is stirred at room temperature for 3 hours. Sodium cyanoborohydride (2.93 g, 46,6 mmol) is added and the resultant mixture is stirred and heated at 75°C overnight. The mixture is concentrated *in vacuo* and the residue is diluted with water and extracted with diethyl ether, dried over Na₂SO₄ and filtered. The filtrate is evaporated to dryness and the residue is passed through a plug of silica eluting with a mixture of ethyl acetate and cyclohexane (1:2). The eluant is evaporated to dryness and the residue is dissolved in ethyl acetate and treated with a solution of hydrogen chloride in dioxane (4M, 3.1 mL). The mixture is cooled to 0°C and allowed to stand for 3 hours. The resultant precipitate is collected by filtration and washed with cold ethyl acetate to give the product **3c** as a white solid (1.17 g).
Found m/z ES+ = 363.

### Step 57B

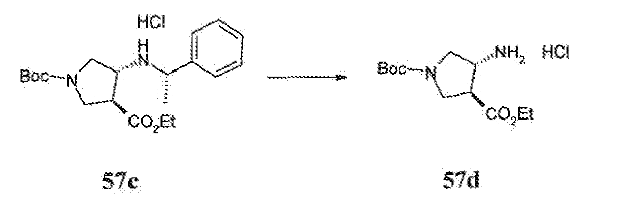

Palladium on carbon (10%, 1 g) is added to a solution of **57c** (1.4 g, 3.5 mmol) in ethanol (150 mL) and the solution is hydrogenated under a balloon of hydrogen for 24 hours. The mixture is filtered through Celite and the filtrate is evaporated to dryness to give the product **57d** as a white foam (965 mg).
Found m/z ES+ = 259.

### Step 57C

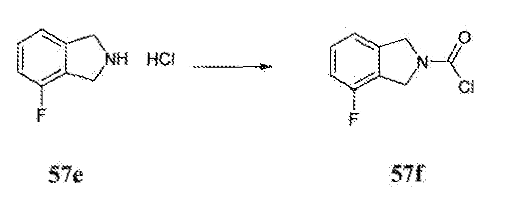

Triethylamine (3.66 mL, 2.66 g, 26.3 mmol) is added to a suspension of **57e** (1.2 g, 8.75 mmol) in toluene (46 mL) and the resultant mixture is stirred at room temperature for 5 minutes. A solution of triphosgene (2.86 g, 9.64 mmol) in toluene (4 mL) is added drop wise and the mixture is stirred at room temperature for 3 hours. The mixture is filtered and the filtrate is evaporated to dryness to give the crude product **57f**, which is used directly without further purification.

### Step 57D

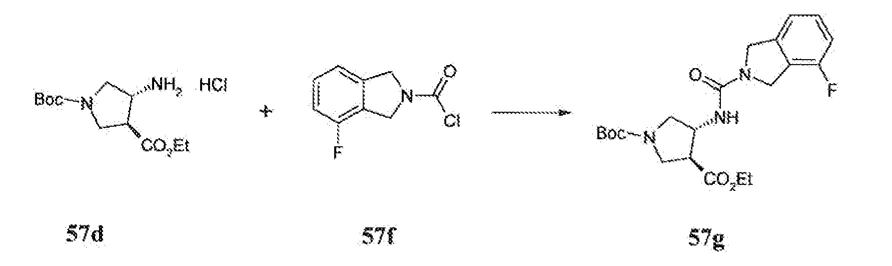

A solution of **57f** (654 mg, 3.52 mmol) in dry THF (6 mL) is added to a stirred mixture of **57d** (965 mg, 3.28 mmol) and triethylamine (1.37 mL, 993 mg, 0.98 mmol) in dry THF (9 mL). The resultant mixture is stirred at room temperature overnight. The solid is removed by filtration and the filtrate is evaporated to dryness. The residue is dissolved in ethyl acetate and washed with aqueous citric acid solution (10%), saturated aqueous sodium bicarbonate solution, and brine, dried over Na₂SO₄ and filtered. The filtrate is evaporated to dryness. The residue is purified by chromatography on silica (gradient: cyclohexane by a mixture of ethyl acetate and cyclohexane to 1:1) to give the product **57g** as a white foam (620 mg).
Found m/z ES+ = 422.

### Step 57F

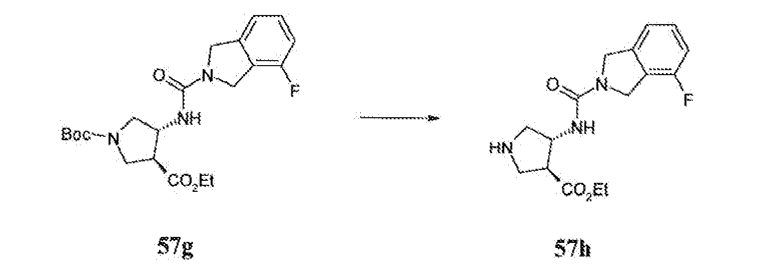

Trifluoroacetic acid (1 mL) is added to a solution of **57g** (610 mg, 1.45 mmol) in dichloromethane (3 mL). The resultant mixture is stirred at room temperature for 2 hours. The solution is passed through an lsolute® SCX-2 column eluting first with dichloromethane then methanol to remove any by-products and finally with a section of ammonia in methanol (2M) to give the product **57h** as a colourless oil (310 mg).
Found m/z ES+ = 322.

### Step 57F

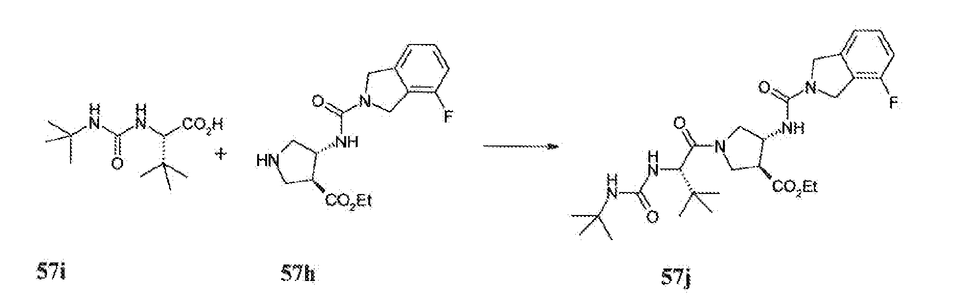

HATU (514 mg, 1,35 mmol), **57h** (310 mg, 0.965 mmol) and N-methylmorpholine (0.424 mL, 390 mg, 3.86 mmol) are added to a solution of **3i** (268 mg, L16 mmol) in a mixture of N,N-dimethylformamide (4 mL) and dichloromethane (4 mL) at 0°C. The resultant mixture is allowed to warm to room temperature and stirred overnight. The mixture is concentrated *in vacuo* and the residue is dissolved in ethyl acetate and washed with aqueous citric acid solution (10%), saturated aqueous sodium bicarbonate solution, and brine, dried over Na₂SO₄ and filtered. The filtrate is evaporated to dryness and the residue is purified by chromatography on silica (gradient: ethyl acetate and cyclohexane 1:4 to 2:3) to give the product **51j** as a colourless foam (500 mg).
Found m/z ES+ = 556 (+ Na).

### Step 57G

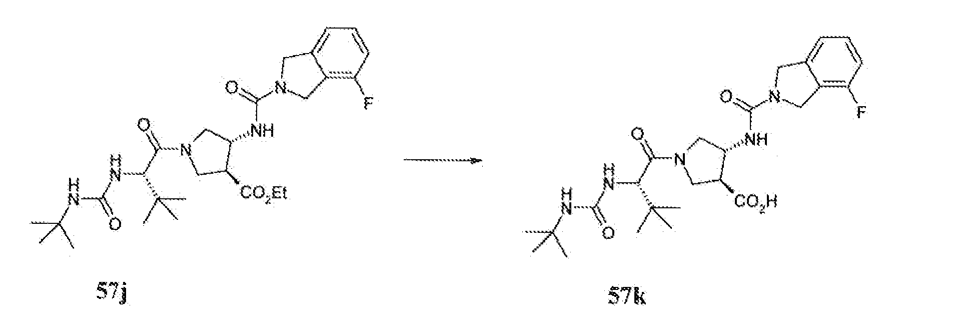

An aqueous solution of lithium hydroxide (L3M, 0.94 mL) is added to a solution of **57j** (500 mg, 0.94 mmol) in a mixture of THE (4 mL) and water (0.9 mL) at 0°C. The resultant mixture is allowed to to room temperature and stirred for 2 hours. The mixture is concentrated *in vacuo* and the residue is diluted with water and washed with diethyl ether. The aqueous layer is acidified to pH 2 by addition of hydrochloric acid (1M) and extracted with ethyl acetate, dried over Na₂SO₄ and filtered. The filtrate is evaporated to dryness to give the product **57k** as a white solid (400 mg).
Found m/z ES+ 506, ES- 504.

### Step 57H

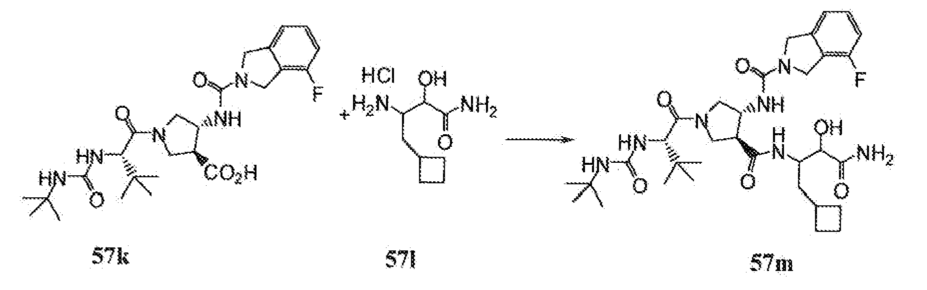

HATU (361 mg, 0.95 mmol), **57k** (400 mg, 0.79 mmol) and N-methylmorpholine (0.348 mL, 320 mg, 3.16 mmol) are added to a solution of **57l** (199 mg, 0.95 mmol) in a mixture of N,N-dimethylformamide (4 mL) and dichloromethane (4 mL) at 0°C under an atmosphere of nitrogen. The resultant mixture is allowed to warm to room temperature and stirred for 6 hours. The mixture is concentrated *in vacuo* and the residue is dissolved in ethyl acetate and washed with aqueous citric acid solution (10%), saturated aqueous sodium bicarbonate solution, and brine, dried over Na₂SO₄ and filtered. The filtrate is evaporated to dryness and purified by chromatography on silica (gradient: ethyl acetate and cyclohexane 1:1 to 100% ethyl acetate then methanol and ethyl acetate 1:99 to 3:7) collecting the product fraction. This was repurified by chromatography on silica (dichloromethane, methanol and ammonia 20:1:0.5) to give the product **57m** as a white solid (274 mg).
Found m/z ES+ 660.

### Step 57I

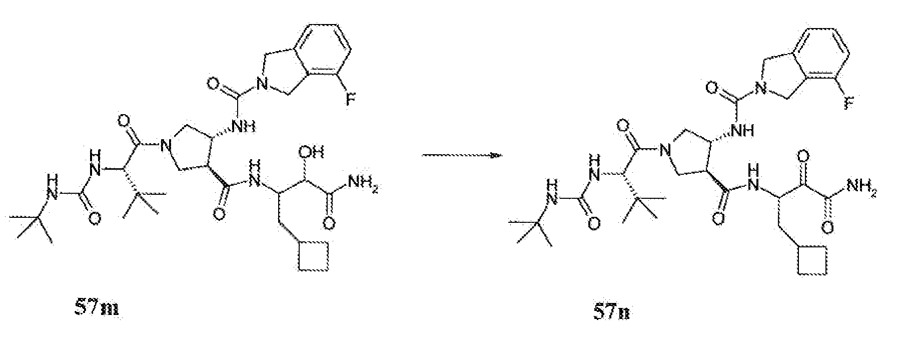

A solution of sulphur trioxide-pyridine complex (145 mg, 0.91 mmol) in dry DMSO (1.5 mL) is added to a solution of **57m** (86 mg, 0.13 mmol) and N,N-di-isopropyl-N-ethylamine (0.19 mL, 141 mg, 1.1 mmol) in dry DMSO (1.5 mL) under an atmosphere of nitrogen. The resultant mixture is stirred at room temperature for 2 hours. It is diluted with aqueous ammonium chloride solution and extracted with ethyl acetate, washed with water, dried over MgSO₄ and filtered. The filtrate is evaporated to dryness and the residue is purified, by chromatography on silica (gradient: dichloromethane followed by a mixture of acetone and dichloromethane to 2:3) to give the product **57n** as a white solid (28 mg).
Found m/z ES+ = 658.

### Example 58:

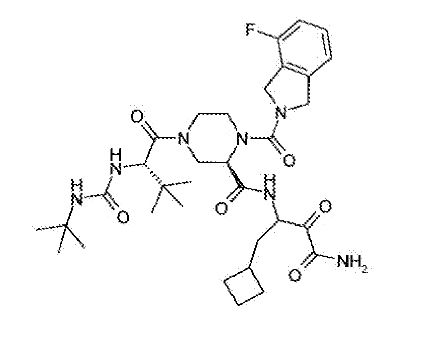

### Step 58-A

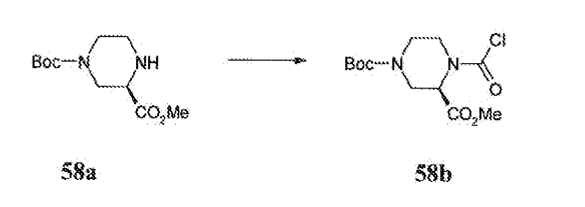

A solution of triphosgene (3.34 g, 11.2 mmol) in toluene (5 mL) is added to a stirred solution of **58a** (2.5 g, 1.02 mmol) and triethylamine (1.57 mL, 1.14 g, 11.3 mmol) in toluene (60 mL) at 0°C, under an atmosphere of nitrogen. The resultant mixture is allowed to warm to room temperature and stirred for 4 hours. The mixture is filtered and the filtrate is evaporated to dryness to give the product **58b** as a colourless oil (2.74 g).
¹H NMR (CDCl₃) □ 4.9 (m, 1H), 4.6 (m, 1H), 4.1 (br m, 2H), 3.7 (2s, 3H) 3.6 and 3.4 (2 br s, 1H), 3.15 (br m, 1H), 2.95 (br s, 1H), 1.45 (s, 9H).

### Step 58-B

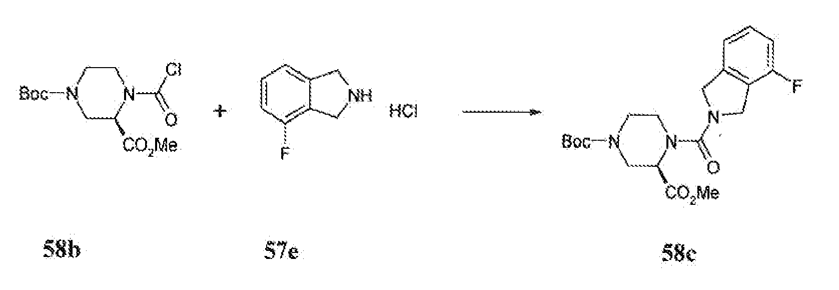

A solution of **58b** (2.74 g, 8.93 mmol) in dry THF (20 mL) is added to a suspension of **57e** (1.55 g, 8.93 mmol) and triethylamine (3.73 mL, 2.71 g, 26.8 mmol) in dry THF (10 mL). The resultant mixture is stirred at room temperature for 3 days. The solid is removed by filtration and the filtrate is evaporated to dryness. The residue is purified by chromatography on silica (gradient: cyclohexane to ethyl acetate and cyclohexane 2:3) to give the product **58c** as a colourless oil (2.94 g).
Found mj/z ES+ = 408.

### Step 58-C

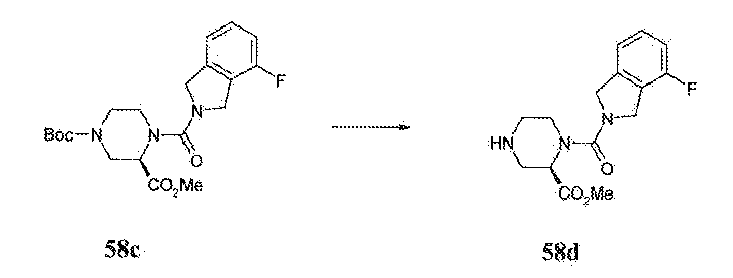

Trifluoroacetic acid (2 mL) is added to a solution of **58c** (1.0 g, 2.45. mmol) in dichloromethane (10 mL) and the resultant mixture is stirred at room temperature for I hour. The mixture is passed through an Isolute® SCX-2 column eluting first with dichloromethane, then methanol to remove the by-products and finally with a solution of ammonia in methanol (2M) to give the product **58d** as an orange oil (631 mg).
¹H NMR(CDCl₃) □ 725 (m, 1H), 7.05 (d, 1H), 6.95 (t, 1H), 4.85 (m, 4H), 4.6 (m, 1H), 3.8 (s, 3H), 3.5 (m, 4H), 3.05 (m, 2H), 2.85 (m, 1H).

### Step 58-D

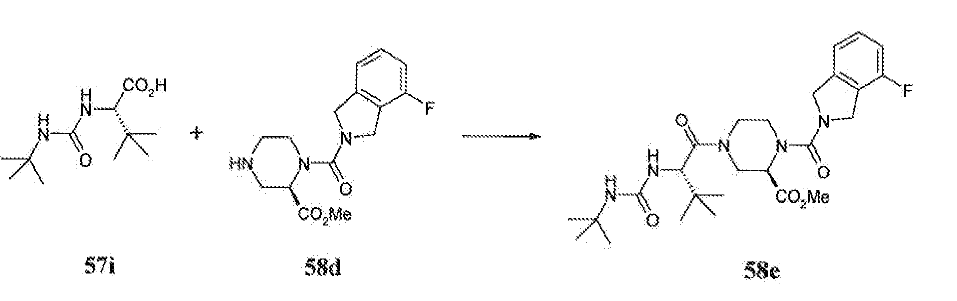

**58e** is prepared from **58d** and **57i** by proceeding in a manner similar to that used for the preparation of **57j (Step 57F).**
Found m/z ES+ = 520.

### Step 58-E

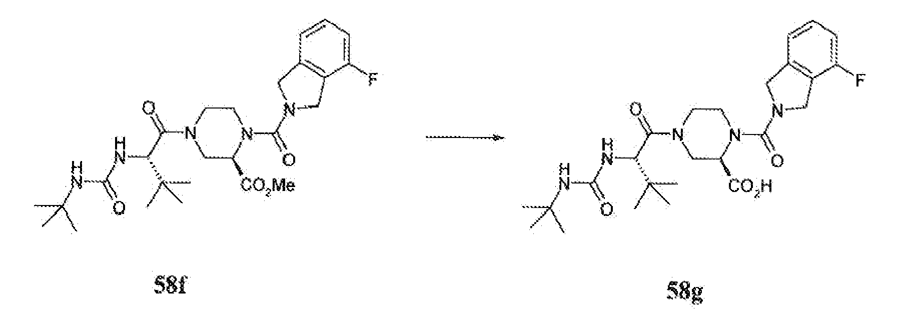

**58g** is prepared from **58f** by proceeding in a manner similar to that used for the preparation of **57k (Step 57G).**
Found m/z ES+ = 506.

### Step 58-F

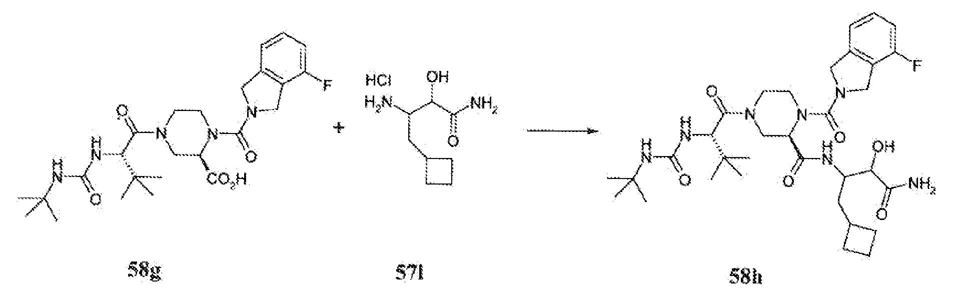

**58h** is prepared from **58g** and **571** by proceeding in a manner similar to that used for the preparation of **57m (Step 57H).**
Found m/z ES+ = 660.

### Step 58-G

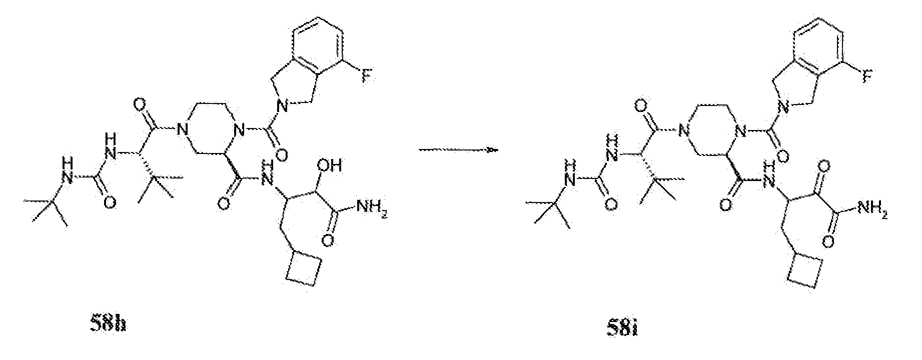

**58i** is prepared from **58h** by proceeding in a manner to that used for the preparation of **57n (Step 571).**
Found m/z ES+ = 658.

### BIOLOGICAL ACTIVITY

### Example 59: HCV NS3-4A protease assay

The inhibitory activity of certain compounds of Table A against HCV NS3-4A serine protease is determined in a homogenous assay using the full-length NS3-4A protein (genotype 1a, strain HCV-1) and a commercially available internally-quenched fluorogenic peptide substrate as described by Taliani, M., et al. 1996 Anal. Biochem. 240:60-67, which is incorporated by reference in its entirety.

### Example 60: Luciferase-based HCV replicon assay

The antiviral activity and cytotoxicity of certain compounds of Table A is determined using a subgenomic genotype 1b HCV replicon cell line (Huh-Luc/neo-ET) containing a luciferase reporter gene, the expression of which is under the control of HCV RNA replication and translation. Briefly, 5,000 replicon cells are seeded in each well of 96-well tissue culture plates and are allowed to attach in complete culture media without G418 overnight. On the next day, the culture media are replaced with media containing a serially diluted compound of Table A in the presence of 10% FBS and 0.5% DMSO. After a 48-h treatment with the compound of Table A, the remaining luciferase activities in the cells are determined using BriteLite reagent (Perkin Elmer, Wellesley, Massachusetts) with a LMaxII plate reader (Molecular Probe, Invitrogen). Each data point represents the average of four replicates in cell culture. IC₅₀ is the concentration of the at which the luciferase activity in the replicon cells is reduced by 50%. The cytotoxicity of the compound of Table A is evaluated using an MTS-based cell viability assay.

Compounds in Table A *supra* have been tested in at least one of the protease assay of Example 59 or the replicon assay of Example 60 and exhibit an IC₅₀ of less than about 10 µM or less in at least one of the assays recited in Example 59 and 60.

### Equivalents

Those skilled in the art will recognize, or be able to ascertain using no more than routine experimentation, many equivalents to the specific embodiments and methods described herein. Such equivalents are intended to be encompassed by the scope of the following claims.

### Incorporation by Reference

The entire contents of all patents, published patent applications and other references cited herein are hereby expressly incorporated herein in their entireties by reference. The entire contents of copending provisional patent applications U.S.S.N. 60/791,578, U.S.S.N. 60/791,318, and U.S.S.N. 60/791,320, each of which was filed on April 11, 2006, and U.S.S.N. 60/866,874, filed on November 22, 2006 and non-provistonal patent applications claiming the benefit therefrom are expressly incorporated herein, in their entirety, as applied to the compounds of the present invention.

### Embodiments of the invention

1. A compound of the formula I: 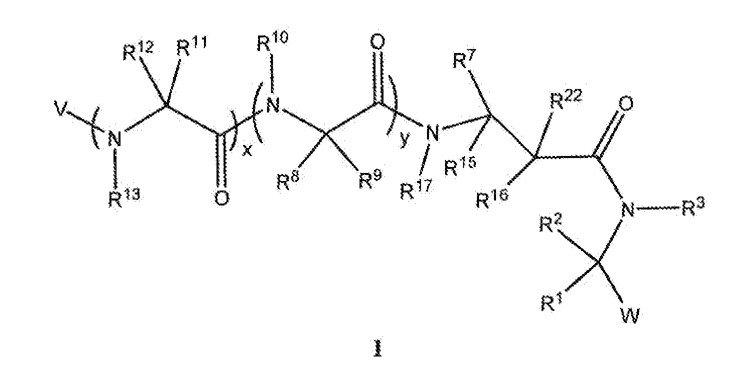 and pharmaceutically acceptable salts, enantiomers, stereoisomers, rotamers, tautomers, diastereomers, or racemates thereof;
   wherein
   x is 0 or 1,
   y is 0, 1 or 2;
   R¹, R², R³, R⁷, R⁸, R⁹, R¹⁰, R¹¹, R¹², R¹³ R¹⁶, R¹⁵, R¹⁷, R²², V and W are each, independently, hydrogen or selected from the group consisting of alkyl, alkyl-aryl, heteroalkyl, heterocyclyl, heteroaryl, aryl-heteroaryl, alkyl-heteroaryl, cycloalkyl, alkyloxy, alkyl-aryloxy, aryloxy, heteroaryloxy, heterocyclyloxy, cycloalkyloxy, amino, alkylamino, arylamino, alkyl-arylamino, arylamino, heteroarylamino, cycloalkylamino, carboxyalkylamino, arlylalkyloxy and heterocyclylamino; each of which may be further independently substituted one or more times with X¹ and X²; wherein X¹ is alkyl, alkenyl, alkynyl, cycloalkyl, cycloalkyl-alkyl, heterocyclyl, heterocyclylalkyl, aryl, alkylaryl, aralkyl, aryloxy, arylthio, arylheteroaryl, heteroaryl, heterocyclylamino, alkylheteroaryl, or heteroaralkyl; wherein X¹ can be independently substituted with one or more of X² moieties which can be the same or different and are independently selected; wherein X² is hydroxy, oxo, alkyl, cycloalkyl, heterocycloalkyl, aryl, heteroaryl, alkoxy, aryloxy, thio, alkylthio, amino, mono- and di-alkylamino, arylamino, alkylsulfonyl, arylsulfonyl, alkylsulfonamido, arylsulfonamido, carboxy, carbalkoxy, carboxamido, alkoxycarbonylamino, alkoxycarbonyl, alkoxycarbonyloxy, alkylureido, arylureido, halogen, cyano, or nitro; wherein each X₂ residue selected to be alkyl, alkoxy, and aryl can be unsubstituted of optionally independently substituted with one or more moieties which can be the same or different and are independently selected from alkyl, alkenyl, alkynyl, cycloalkyl, cycloalkyl-alkyl, heterocyclyl, heterocyclylalkyl, aryl, alkylaryl, aralkyl,arylneteroaryl, heteroaryl, heterocyclylamino, alkylheteroaryl and heteroaralkyl;
   W is also selected from the group consisting or C(O)OH, C(O)OR²⁴, C(O)-amine, C(O)-C(O)OH,C(=N-O-R²⁴)-C(O)-amine, C(O)N(H)S(O)₂R²⁴, C(O)-C(O)-amine, CON(H)SO₂-amine and C(O)-[C(O)]ₐ-heterocycle, wherein the heterocycle may be substituted or unsubstituted, wherein a is 0 or 1, wherein each R²⁴ is hydrogen or is independently selected from the group consisting C₁₋₄-alkyl, C₃₋₆-cycloalkylC₀₋₄alkyl, substituted or unsubstituted aryl and substituted or unsubstituted heterocycle, each of which may be independently substituted one or more times with a halogen atom or C₁₋₄-alkyl;
   V is also selected from the group consisting of -Q¹-Q², wherein Q¹ is absent, C(O), N(H), N(C₁₋₄-alkyl), C=N(CN), C=N(SO₂CH₃), or C=N-COH, and Q² is H or is selected from the group consisting of C₁₋₄alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
   or R²² and R¹⁶ may together form a 3, 4, 5, 6 or 7-membered ring that is aromatic or non-aromatic and may contain one or more heteroatoms, wherein the ring may be further substituted one or more times;
   or R⁷ and R¹⁵ may together form a 3, 4, 5, 6 or 7-membered ring that is aromatic or non-aromatic and may contain one or more heteroatoms, wherein the ring may be further substituted one or more times;
   or R¹⁵ and R¹⁷ may together form a 3, 4, 5, 6 or 7-membered ring that is aromatic or non-aromatic and may contain one or more heteroatoms, wherein the ring may be further substituted one or more times;
   or R¹⁵ and R¹⁶ may together form a 4, 5, 6 or 7-membered ring that is aromatic or non-aromatic and may contain one or more heteroatoms, wherein the ring may be further substituted one or more times;
   or R¹ and R² may together form a 3, 4, 5, 6 or 7-membered ring that is aromatic or non-aromatic arid may contain one or more heteroatoms, wherein the ring may be further substituted one or more times;
   or R¹⁷ and R¹⁶ may together form a 4, 5, 6, 7 or 8-membered ring of the formula III: 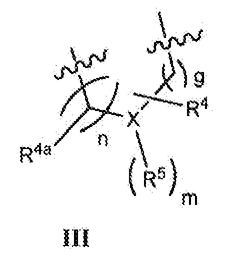 wherein
      n and g are each, independently, 0, 1 or 2;
      m is 0 or 1;
      x is O, N or C;
      R⁵, R⁴ and R^{4a} are each, independently, hydrogen or oxo or are selected from the group consisting of hydroxyl, C₁₋₈-alkyl, C₂₋₈-alkenyl, C₂₋₈-alkynyl, C₃₋₈-cycloalkyl-C₀₋₄-alkyl, aryl-C₀₋₄-alkyl, hetcrocycle-C₀₋₄-alkyl, heteroaryl-C₀₋₄-alkyl , C₃₋₈-cycloalkyloxy, aryloxy, N(R₂₃)₂, NR₂₃COR₂₃, CONR₂₃R₂₃, NR₂₃CONHR₂₃, OCONR₂₃R₂₃, NR₂₃COOR₂₃, OCOR₂₃, COOR₂₃, aryl-C(O)O, aryl-C(O)NR₂₃, heteroaryloxy, heteroaryl-C(O)O, heteroaryl-C(O)NR₂₃, each of which may be independently substituted one or more times with a halogen atom, aryl, heteroaryl, trihalomethyl, C₁₋₄-alkyl, or C₁₋₄-alkoxy;
      or R⁴ and R⁵ may together form a 4, 5, 6 or 7-membered ring that is aromatic or non-awmatic and may contain one or more heteroatoms, wherein the ring may be further substituted one or more times; and
      R₂₃ is independently selected at each occurrence from hydrogen or the group consisting of alkyl, alkenyl, alkynyl, cycloalkyl, cycloalkylalkyl, aryl, heteroaryl, heteroaralkyl and aralkyl, each of which is substituted with 0-2 substituents independently selected from halogen, alkyl, and alkoxy.
2. The compound of embodiment 1, wherein R¹⁵ and R¹⁶ together form a ring of the formula IV: 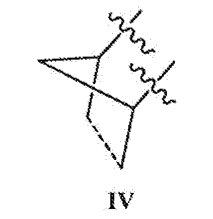 wherein
   the dashed line represents a single or double bond, wherein formula IV may be further substituted one or more times.
3. The compound of embodiment 1, wherein R¹⁵ and R¹⁶ together form a ring of the formula V: 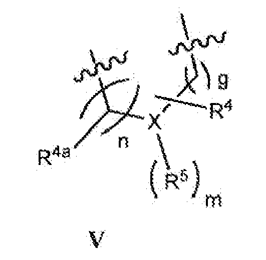 wherein
   n and gare each, independently, 0, 1, 2 or 3 (such that the sum of n an g is less than 5);
   m is 0 or 1;
   X is O, N or C;
   R⁵, R⁴ and R^{4a} are each, independently, hydrogen or oxo or are selected from the group consisting of hydroxyl, C₁₋₈-alkyl, C₂₋₈-alkenyl, C₂₋₈-alkynyl, C₃₋₈-cycloalkyl-C₀₋₄-alkyl, aryl-C₀₋₄-alkyl, heterocycle-C₀₋₄-alkyl, heteroaryl-C₀₋₄-alkyl, C₃₋₈-cycloalkyloxy, aryloxy, N(R₂₃)₂, NR₂₃COR₂₃, CONR₂₃R₂₃, NR₂₃CONHR₂₃, OCONR₂₃R₂₃, NR₂₃COOR₂₃, OCOR₂₃, COOR₂₃, aryl-C(O)O, aryl-C(O)NR₂₃, heteroaryloxy, heteroaryl-C(O)O, heteroaryl-C(O)NR₂₃, each of which may be independently substituted one or more times with a halogen atom, aryl, heteroaryl, trihalomethyl, C₁₋₄-alkyl, or C₁₋₄-alkoxy;
   or R⁴ and R⁵ may together form a 4, 5, 6 or 7-membered ring that is aromatic or non-aromatic and may contain one or more heteroatoms, wherein the ring may be further substituted one or more times;
   R₂₃ is independently selected at each occurrence from hydrogen or the group consisting of alkyl, alkenyl, alkynyl, cycloalkyl, cycloalkylalkyl, aryl, heteroaryl, heteroaralkyl and aralkyl, each of which is substituted with 0-2 substituents independently selected from halogen, alkyl, and alkoxy each of which;
   or R¹⁵ and R¹⁶ may together form a 4, 5, 6 or 7-membered ring that is aromatic or non-aromatic and may contain one or more heteroatoms, wherein the ring may be further substituted one or more times;
   or R¹ and R² may together form a 3, 4, 5, 6 or 7-membered ring that is aromatic or non-aromatic and may contain one or more heteroatoms, wherein the ring may be further substituted one or more times.
4. The compound of embodiment 1,
   wherein:
   R³ is selected from the group consisting of H, C₁₋₄-alkyl, and C₃₋₆-cycloalkylC₀₋₄alkyl;
   R⁸, R¹¹, R¹⁵ and R²² are selected from the group consisting of H, alkyl-aryl,C₁₋₄-alkyl, O-C₁₋₄-alkyl, N(H)-C₁₋₄-alkyl, and C₃₋₆-cycloalkylC₀₋₄alkyl;
   R¹⁶ and R¹⁷ are each, independently, selected from the group consisting of H, C₁₋₄-alkyl and C₃₋₆-cycloalkylC₀₋₄alkyl; and
   R¹³ is selected from the group consisting of -Q¹-Q², wherein Q¹ is absent, C(O), N(H), N(C₁₋₄-alkyl), C=N(CN), C=N(SC)₂CH₃), or C=N-COH, and Q² is H, C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyleach of which.
5. The compound of embodiment 1,
   wherein;
   y is 0, 1 or 2;
   R¹ and R² are each, independently, selected from the group consisting of H, C₁₋₄-alkyl, O-C₁₋₄-alkyl, N(H)-C₁₋₄-alkyl, and C₃₋₆-cycloalkylC₀₋₄alkyl;
   W is also selected from the group consisting of C(O)OH, C(O)O²⁴, C(O)-amine, C(O)-C(O)H, C(=N-O-R²⁴)-C(O)-amine, C(O)-C(O)-amine and C(O)-[C(O)]ₐ-heterocycle, wherein the heterocycle may be independently substituted one or more times with aryl, C₁₋₄-alkyl, C₁₋₄-alkyl substituted by one or more halogen atoms, and C₃₋₆-cycloalkyl, wherein a is 0 or 1, wherein each R²⁴ is hydrogen or is independently selected from the group consisting C₁₋₄-alkyl, C₃₋₆-cycloalkylC₀₋₄alkyl, substituted or unsubstituted aryl and substituted or unsubstituted heterocycle, each of whichmay be independently substituted one or more times with a halogen atom or C₁₋₄-alkyl;
   R³ is selected from the group consisting of H, C₁₋₄-alkyl, and C₃₋₆-cycloalkylC₀₋₄alkyl;
   R⁷ is hydrogen or is selected from the group consisting of C₁₋₄-alkyl, O-C₁₋₄-alkyl, N(H)-C₁₋₄-alkyl, C₃₋₆-cycloalkylC₀₋₄alkyl, aryl, CON(H)SO₂-amine and heterocycle, each of which may be independently substituted one or more times with a halogen atom, C₁₋₄-alkyl, C₁₋₄-alkyl substituted by one or more halogen atoms, or C₃₋₆-cycloalkyl;
   R⁸, R⁹, R¹¹, R¹², R¹⁵ and R¹⁶ are hydrogen or are independently selected from the group consisting C₁₋₄-alkyl, O-C₁₋₄-alkyl, N(H)-C₁₋₄-alkyl, and C₃₋₆-cycloalkylC₀₋₄alkyl;
   R¹⁰ and R¹⁷ are each, independently, selected from the group consisting, of H, C₁₋₄-alkyl and C₃₋₆-cycloalkylC₀₋₄alkyl;
   R¹³ is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-COC₁₋₄alkyl, and Q² is H or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cydoalkyl-C₀₋₄-alkyl, aryl, heteroaryl and heterocycle, each of which may be independently substituted one or more times with a halogen atom,C₁₋₄-alkyl, C₁₋₄-alkyl substituted by one or more halogen atoms, or C₃₋₆-cycloalkyl; each of which
   V is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N=COC₁₋₄alkyl, and Q² is H or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
   or R¹⁷ and R¹⁶ may together form a 5- or 6-membered ring of the formula III': 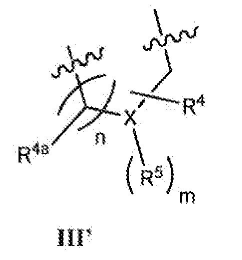 wherein
      m and n are each, independently, 0, 1 or 2;
      X is O, N or C;
      R⁵, R⁴ and R^{4a} are each, independently, hydrogen oroxo or are selected from the group consisting of hydroxyl, C₁₋₈-alkyl, C₂₋₈-alkenyl, C₂₋₈-alkynyl, C₂₋₈-cycloalkyl-C₀₋₄-alkyl, aryl-C₀₋₄-alkyl, heterocycle-C₀₋₄-alkyl, heteroaryl-C₀₋₄-alkyl, C₃₋₈-cycloalkyloxy, aryloxy, N(R₂₃)₂, NR₂₃COR₂₃, CONR₂₃R₂₃, NR₂₃CONHR₂₃, OCONR₂₃R₂₃, NR₂₃COOR₂₃, OCOR₂₃, COOR₂₃,aryl-C(O)O, aryl-C(O)NR₂₃, heteroaryloxy, heteroaryl-C(O)O, heteroaryl-C(O)NR₂₃, each of which may be independently substituted one or more times with a halogen atom, aryl, heteroaryl, trihalomethyl,C₁₋₄-alkyl, or C₁₋₄-alkoxy;
      or R⁴ and R⁵ may together form a 4, 5, 6 or 7-membered ring that is aromatic or non-aromatic and may contain one or more heteroatoms, wherein the ring may be further substituted one or more times;
      R₂₃ is independently selected at each occurrence from hydrogen or the group consisting of alkyl, alkenyl, alkynyl, cycloalkyl, cycloalkylalkyl, aryl, heteroaryl, heteroaralkyl and aralkyl, each of which is substituted with 0-2 substituents independently selected from halogen, alkyl, and alkoxy;
      R⁴ and R^{4a} are each, independently, hydrogen or are selected from the group consisting of C₁₋₄alkyl, O-C₁₋₄-alkyl, N(H)-C₁₋₄-alkyl, C₃₋₆-cycloalkylC₀₋₄alkyl, aryl and heterocycle, each of which may be independently substituted one or more times with a halogen atom or C₁₋₄-alkyl;
      R⁵ is selected from the group consisting of H, hydroxyl, oxo, C₁₋₄-alkyl, C₁₋₄-alkoxy, mono- and di-C₁₋₄alkylamino, C₃₋₆-cycloalkyl-C₀₋₄-alkyl, aryl-C₀₋₄-alkyl, heterocycle-C₀₋₄-alkyl, each of which may be independently substituted one or more times with a halogen atom, aryl, trihalomethyl, or C₁₋₄-alkyl;
      or R⁴ and R⁵ may together form a cycloalkyl or phenyl ring, either of which may be substituted with a halogen atom, aryl, trihalomethyl, or C₁₋₄-alkyl, or a dimethyl cyclopropyl ring such that formula III is a fused ring system;
      or R¹⁵ and R¹⁶ may together fore a ring of the formula IV: 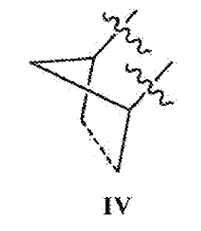 wherein
         the dashed line represents a single or double bond.
6. The compound of embodiment 1, wherein
   R¹ is selected from the group consisting of H and C₁₋₄-alkyl;
   R² is selected from the group consisting of C₁₋₄alkyl and C₃₋₆-cycloalkylC₀₋₄alkyl;
   W is selected from the group consisting of C(O)-C(O)-amine and C(O)-[C(O)]ₐ-heterocycle, wherein the heterocycle may be independently substituted one or more times with aryl, C₁₋₄-alkyl, C₁₋₄-alkyl substituted by one or more halogen atoms, or C₃₋₆-cycloalkyl, wherein a is 0 or 1;
   R³ is selected from the group consisting of H and C₁₋₄-alkyl;
   R¹³ is H;
   R⁸, R¹⁰ and R¹¹ are each, independently, selected from the group consisting of H and C₁₋₄-alkyl;
   R⁹ and R¹² are each, independently, selected from the group consisting of H, C₁₋₄-alkyl and C₃₋₆-cycloalkylC₀₋₄alkyl; and
   V is selected from the group consisting of -Q¹-Q², wherein Q¹ is absent, C(O), N(H), N(C₁₋₄-alkyl), C=N(CN), C=N(SO₂CH₃), or C=N-COH, and Q² is H, C₁₋₄-alkyl, O-C₁₋₄-alkyl, NH₂, N(H)-C₁₋₄-alkyl, N(C₁₋₄-alkyl)₂, C₃₋₆-cycloalkyl-C₀₋₄-alkyl, aryl, and heterocycle, each of which may be independently substituted one or more times with a halogen atom, C₁₋₄-alkyl, C₁₋₄-alkyl substituted by one or more halogen atoms, or C₃₋₆-cycloalkyl.
7. The compound of embodiment 1, wherein any of the C₃₋₆-cycloalkyl groups may be independently substituted one or more times with a halogen atom, aryl, trihalomethyl, or C₁₋₄-alkyl.
8. The compound of embodiment 1, wherein R¹⁷ is H and R¹⁵ and R¹⁶ together form the ring of formula IV, wherein the dashed line represents a double bond.
9. The compound of embodiment 1, wherein R¹⁷ and R¹⁶ together form a 5- or 6-membered ring of the formula III, wherein formula III is represented by the substituents selected from the group consisting of: 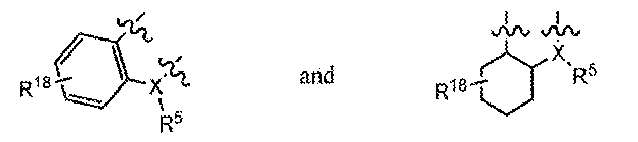 wherein R⁵ is (CH₂)₀₋₃-aryl or (CH₂)₀₋₃-heterocycle, wherein aryl and heterocycle may be independently substituted one or more times with a halogen atom, aryl, trihalomethyl, C₃₋₆-cycloalkyl or C₁₋₄-alkyl; and each R¹⁸ is independently selected from the group consisting of hydrogen, a halogen atom, aryl, trihalomethyl, or C₁₋₄-alkyl.
10. The compound of embodiment 1, wherein Formula I is represented by a compound of the Formula II: 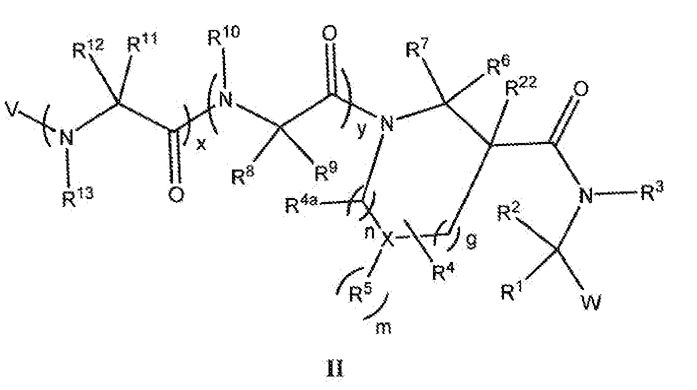 and pharmaceutically acceptable salts, enantiomers, stereoisomers, rotamers, tautomers, diastereomers, or racemates thereof;
   wherein
   x is 0 or 1;
   y is 0, 1 or 2;
   R¹ and R² are each, independently, selected from the group consisting of H, C₁₋₄-alkyl, O-C₁₋₄-alkyl, N(H)-C₁₋₄-alkyl, and C₃₋₆-cycloalkylC₀₋₄alkyl;
   W is selected from the group consisting of C(O)OH, C(O)OR²⁴, C(O)-amine, C(O)-C(O)OH, C(=N-O-R²⁴)-C(O)-amine, C(O)N(H)S(O)₂R²⁴, C(O)-C(O)-amine, SO₂-N(R²⁴)₂ and C(O)-[C(O)]ₐ-heterocycle, wherein the heterocycle may be substituted or unsubstituted, wherein a is 0 or 1, wherein each R²⁴ is independently selected from the group consisting of H, halogen, hydroxyl, formyl, carboxylate, amide, amino, substituted or unsubstituted-C₁₋₄-alkyl, substituted or unsubstituted-C₁₋₄-alkoxy, substituted or unsubstituted-C₁₋₄-alkanoyl, substituted or unsubstituted-C₁₋₄-alkoxycarbonyl, substituted or unsubstituted-C₁₋₄-alkanoyloxy, substituted or unsubstituted mono- and di-C₁₋₄-alkylamino, substituted or unsubstituted-C₃₋₆cycloalkyl-C₀₋₄alkyl, substituted or unsubstituted aryl-C₀₋₄alkyl, and substituted or unsubstituted heterocycle-C₀₋₄alkyl;
   R³ is selected from the group consisting of H, C₁₋₄-alkyl and C₃₋₆-cycloalkylC₀₋₄alkyl;
   R²² and R⁷ are each, independently, hydrogen or are selected from the group consisting of C₁₋₄-alkyl, O-C₁₋₄-alkyl, N(H)-C₁₋₄-alkyl, C₃₋₆-cycloalkylC₀₋₄alkyl, aryl and heterocycle, each of which may be independently substituted one or more times;
   n and g are each, independently, 0, 1 or 2;
   m is 0 or 1;
   X is 0, N or C;
   R⁴ and R^{4a} are each, independently, hydrogen or are selected from the group consisting of C₁₋₄-alkyl, O-C₁₋₄-alkyl, N(H)-C₁₋₄-alkyl, C₃₋₆-cycloalkylC₀₋₄alkyl, aryl, O-aryl and heterocycle, each of whichmay be further independently substituted;
   R⁵ is hydrogen or oxo or is selected from the group consisting of hydroxyl, C₁₋₄-alkyl, C₁₋₄-alkoxy, mono- and di-C₁₋₄alkylamino, C₃₋₆-cycoalkyl-C₀₋₄-alkyl, aryl-C₀₋₄-alkyl, heterocycle-C₀₋₄-alkyl, each of which may be furthe independently substituted;
   R⁶, R⁸, R⁹, R¹¹ and R¹² are each, independently, selected from the group consisting of H, C₁₋₄-alkyl, O-C₁₋₄-alkyl, N(H)-C₁₋₄-alkyl, and C₃₋₆-cycloalkylC₀₋₄alkyl;
   R¹⁰ is selected from the group consisting of H, C₁₋₄-alkyl and C₃₋₆-cycloalkylC₀₋₄alkyl;
   R¹³ is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-CO-C₁₋₄alkyl, and Q² is H or selected from the group consisting of C₁₋₄₋alkyl, O-C₁₋₄alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl; and
   V is selected from the group consisting of -O¹-Q², wherein Q¹ is absent, C(O), S(O)₂, N(H), N(C₁₋₄-alkyl), C=N(CN), C=N(SO₂CH₃), C=N-COH, or C=N-COC₁₋₄alkyl, and Q² is H or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alky, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
   or R⁴ and R⁵ may together form a 4, 5, 6 or 7-membered ring that is aromatic or non-aromatic and may contain one or more heteroatoms, wherein the ring may be further substituted one or more times.
11. The compound of embodiment 10, wherein R⁴ and R⁵ together form a phenyl ring, which may be substituted with a halogen atom, aryl, trihalomethyl, or C₁₋₄-alkyl, or a dimethyl cyclopropyl ring such that a fused ring system is formed.
12. The compound of embodiment 10, wherein one of g and n is 0.
13. The compound of embodiment 10, wherein
   R¹ is selected from the group consisting of H and C₁₋₄-alkyl;
   R² is selected from the group consisting of C₁₋₄-alkyl and C₃₋₆-cycloalkylC₀₋₄alkyl;
   W is selected from the group consisting of C(O)-C(O)-amine and C(O)-[C(O)]ₐ-heterocycle, wherein the heterocycle may be independently substituted one or more times with aryl, C₁₋₄-alkyl, C₁₋₄-alkyl substituted by one or more halogen atoms, and C₃₋₆-cycloalkyl, wherein a is 0 or 1, wherein R²⁴ is hydrogen or is selected from the group consisting of C₁₋₄-alkyl, C₃₋₆-cycloalkylC₀₋₄alkyl, aryl and heterocycle, each of which may be independently substituted one or more times with a halogen atom or C₁₋₄-alkyl;
   R³ is selected from the group consisting of H and C₁₋₄-alkyl;
   R⁷ is hydrogen or is selected from the group consisting of C₁₋₄-alkyl, C₃₋₆-cycloalkyl, aryl and heterocycle, each of which may be independently substituted one or more times with a halogen atom, C₁₋₄-alkyl, C₁₋₄-alkyl substituted by one or more halogen atoms, or C₃₋₆-cycloalkyl;
   R⁴ and R^{4a} are each, independently, hydrogen or are independently selected from the group consisting of C₁₋₄-alkyl, C₃₋₆-cycloalkyl, aryl and heterocycle, each of which may be independently substituted one or more times with a halogen atom or C₁₋₄-alkyl;
   R⁵ is hydrogen or is selected from the group consisting of hydroxyl, oxo, C₁₋₄-alkyl, C₁₋₄-alkoxy, mono- and di-C₁₋₄alkylamino, C₃₋₆-cycloalkyl-C₀₋₄-alkyl, aryl-C₀₋₄-alkyl, heterocycle-C₀₋₄alkyl, each of which may be independently substituted one or more limes with a halogen atom, aryl, trihalomethyl, or C₁₋₄-alkyl;
   R¹³ and R⁶ are H;
   R⁸, R¹⁰ and R¹¹ are each, independently, selected from the group consisting of H and C₁₋₄-alkyl;
   R⁹ and R¹² are each, independently, selected from the group consisting of H, C₁₋₄-alkyl and C₃₋₆-cycloalkyl; and
   V is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-COC₁₋₄alkyl, and Q² is H, C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
   or R⁴ and R⁵ may together form a phenyl ring, which may be substituted with a halogen atom, aryl, trihalomethyl, or C₁₋₄-alkyl, or a dimethyl cyclopropyl ring such that a fused ring system is formed.
14. The compound of embodiment 10, wherein R⁴ is H and R⁵ is (CH₂)₀₋₃-aryl, -O-heterocycle, or (CH₂)₀₋₃-heteirocycle, wherein aryl and heterocycle may be independently substituted one or more times with a halogen atom, aryl, trihalomethyl, C₃₋₆-cycloalkyl or C₁₋₄-alkyl.
15. The compound of embodiment 10, wherein n is 1, and R⁴ and R⁵ together form the following fused ring systems: 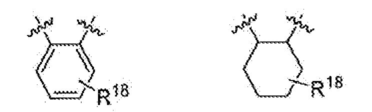 wherein each R¹⁸ is independently selected from the group consisting of hydrogen, a halogen atom, aryl, trihalomethyl, and C₁₋₄-alkyl.
16. The compound of embodiment 1, wherein Formula I is represented by a compound of the Formula VI: 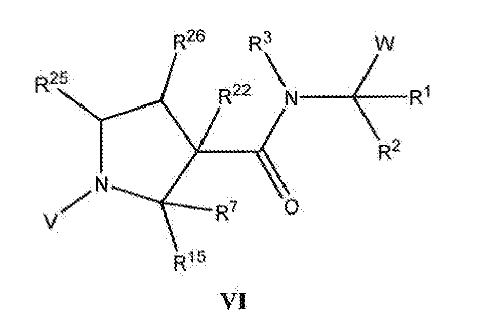 and pharmaceutically acceptable salts, enantiomers, stereoisomers, rotamers, tautomers, diastereomers, or racemates thereof;
   wherein
   R¹, R², R³, R¹⁵, R²², V and W have the meanings set forth for claim 1; and
   R²⁵ and R²⁶ are each, independently, selected from the group consisting of H, C₁₋₄-alkyl, O-C₁₋₄-alkyl, N(R²⁴)₂, C₃₋₆-cycloalkylC₀₋₄alkyl, substituted or unsubstituted aryl and substituted or unsubstituted heterocycle, wherein each R²⁴ is hydrogen or is independently selected from the group consisting of halogen, hydroxy, COOH, CONH₂, amino, mono- and di-C₁₋₄alkylamino, C₁₋₄-alkyl, C₁₋₄alkoxy, C₁₋₄alkanoyl, C₃₋₆-cycloalkylC₀₋₄alkyl, C₃₋₆-cycloalkylC₀₋₄alkoxy, aryl and heterocycle, each of which may be independently substituted one or more times with a halogen atom, C₁₋₄-alkyl, C₁₋₄-alkyl substituted by one or more halogen atoms, or C₃₋₆-cycloalkyl;
   or R²² or R²⁶ may together form a 3-membered ring that may or may not be substituted.
17. The compound of embodiment 16, wherein R²⁵ is H and R²⁶ is amine, substituted or unsubstituted phenyl, or substituted or unsubstituted benzyl.
18. The compound of embodiment 1, wherein Formula I is represented by a compound of the Formula VII: 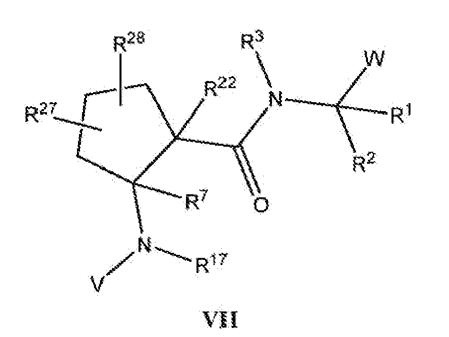 and pharmaceutically acceptable salts, enantiomers, stereoisomers, rotamers, tautomers, diastereomers, or racemates thereof:
   wherein
   R¹, R², R³, R⁷, R¹⁷, R²², V and W have the meanings set forth for claim 1; and
   R²⁷ and R²⁸ are each, independently, selected from the group consisting of hydrogen, C₁₋₄alkyl, C₁₋₄-alkoxy, N(R²⁴)₂, C₃₋₆-cycloalkylC₀₋₄alkyl, aryl, aryloxy, and heterocycle, each of which is substituted 0 to 5 times with halogen atom, C₁₋₄-alkyl, C₁₋₄-alkyl substituted by one or more halogen atoms, or C₃₋₆-cycloalkyl; wherein R²⁴ is hydrogen or is independently selected from the group consisting of, hydroxy, C(O)NH₂,, substituted or unsubstituted-C₁₋₄-alkyl, C₃₋₆-cycloalkylC₀₋₄alkyl, aryl and heterocycle, each of which is substituted 0 to 5 times with halogen atom, C₁₋₄-alkyl, C₁₋₄-alkyl substituted by one or more halogen atoms, or C₃₋₆-cycloalkyl.
19. The compound of embodiment 18, wherein Formula VII is represented by a compound of the formula: 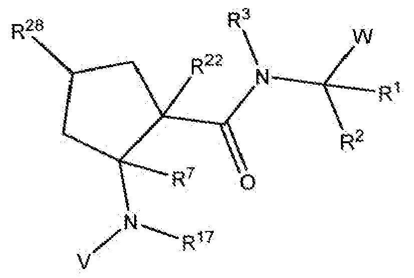 and pharmaceutically acceptable salts, enantiomers, stereoisomers, rotamers, tautomers, diastereomers, or racemates thereof; wherein
   R¹, R², R³, R⁷, R¹⁷, R²², V and W have the meanings set forth for claim 1; and
   R²⁸ is hydrogen or is selected from the group consisting of C₁₋₄alkyl, C₁₋₄-alkoxy, N(R²⁴)₂, C₃₋₆-cycloalkylC₀₋₄alkyl, aryl, aryloxy, and heterocycle, each of which is substituted 0 to 5 times with halogen atom, C₁₋₄-alkyl, C₁₋₄-alkyl substituted by one or more halogen atoms, or C₃₋₆-cycloalkyl; wherein R²⁴ is hydrogen independently selected from the group consisting hydroxy, C(O)NH₂,, substituted or unsubstituted-C₁₋₄-alkyy, C₃₋₆-cyclolalkylC₀₋₄alkyl, aryl and heterocycle, each of which is substituted 0 to 5 times with halogen atom, C₁₋₄-alkyl substituted by one or more halogen atoms, or C₃₋₆-cycloalkyl.
20. The compound of embodiment 19, wherein R²⁸ is quinoline, C₁₋₄-alkyl, O-C₁₋₄-alkyl, or O-quinoline, wherein the quinoline and O-quinoline substituents may be independently substituted one or more times with halogen, amino, O-C₁₋₄-alkyl, substituted or unsubstituted-C₁₋₄-alkyl, substituted or unsubstituted- C₃₋₆-cycloalkylC₀₋₄alkyl, substituted or unsubstituted aryl, substituted or unsubstituted O-aryl, and substituted or unsubstituted heterocycle.
21. The compound of embodiment 1, wherein Formula I is represented by a compound of the Formula VIII: 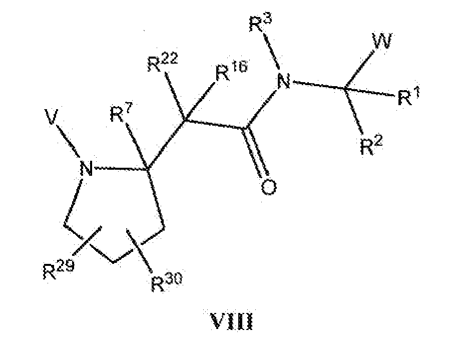 and pharmaceutically acceptable salts, enantiomers, stereoisomers, rotamers, tautomers, diastereomers, or racemates thereof;
   wherein
   R¹, R², R³, R⁷, R¹⁶, R²², V and W have the meanings set forth for claim 1; and
   R²⁹ and R³⁰ are hydrogen or are independently selected from the group consisting of C₁₋₄alkyl, C₁₋₄-alkoxy, N(R²⁴)₂, C₃₋₆-cycloalkylC₀₋₄alkyl, aryl, aryloxy, and heterocycle, each of which is substituted 0 to 5 times with halogen atom, C₁₋₄-alkyl, C₁₋₄-alkyl substituted by one or more halogen atoms, or C₃₋₆-cycloalkyl; wherein R²⁴ is hydrogen or independently selected from the group consisting hydroxy, C(O)NH₂, substituted or unsuhstituted-C₁₋₄-alkyl, C₃₋₆-cycloalkylC₀₋₄alkyl, aryl and heterocycle, each of which is substituted 0 to 5 times with halogen atom, C₁₋₄-alkyl, C₁₋₄-alkyl substituted by one or more halogen atoms, or C₃₋₆-cycloalkyl.
22. The compound of embodiment 21, wherein Formula VIII, is represented by a compound of the Formula IX: 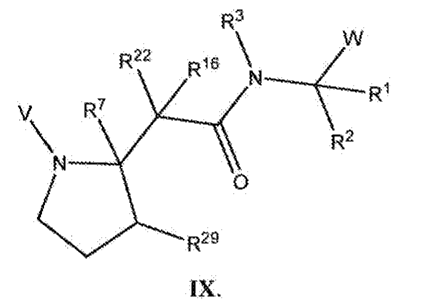 and pharmaceutically acceptable salts, enantiomers, stereoisomers, rotamers, tautomers, diastereomers, or racemates thereof;
   wherein R¹, R², R³, R⁷, R¹⁶, R²², R²⁹, V and W have the meanings set forth for claim 21.
23. The compound of embodiment 22, wherein R²⁹ is selected from the group consisting of O-phenyl and O-benzyl.
24. The compound of embodiment 1, wherein Formula I is represented by a compound of the Formula X: 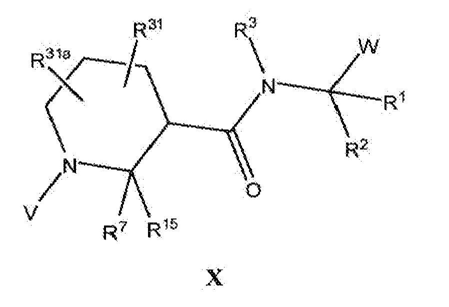 and pharmaceutically acceptable salts, enantiomers, stereoisomers, rotamers, tautomers, diastereomers, or racemates thereof;
   wherein
   R¹, R², R³, R⁷, R¹⁵, V and W have the meanings set forth for claim 1; and
   R³¹ and R^{31a} are hydrogen or are independently selected from the group consisting of C₁₋₄alkyl, C₁₋₄-alkoxy, N(R²⁴)₂, C₃₋₆-cycloalkylC₀₋₄alkyl, aryl, aryloxy, and heterocycle, each of which is substituted 0 to 5 times with halogen atom, C₁₋₄-alkyl, C₁₋₄-alkyl substituted by one or more halogen atoms, or C₃₋₆-cycloalkyl; wherein R²⁴ is hydrogen or is independently selected at each occurrence from the group consisting of hydroxy, C(O)NH₂, substituted or unsubstituted-C₁₋₄-alkyl, C₃₋₆-cycloalkylC₀₋₄alkyl, aryl and heterocycle, each of which is substituted 0 to 5 times with halogen atom, C₁₋₄-alkyl, C₁₋₄-alkyl substituted by one or more halogen atoms, or C₃₋₆-cycloalkyl;
   or R³³¹ and R^{31a} may together form a 3, 4, 5, 6 or 7-membered ring that is aromatic or non-aromatic and may contain one or more heteroatoms, wherein the ring may be further substituted one or more times.
25. The compound of embodiment 24, wherein Formula X is represented by a compound of the Formula XI: 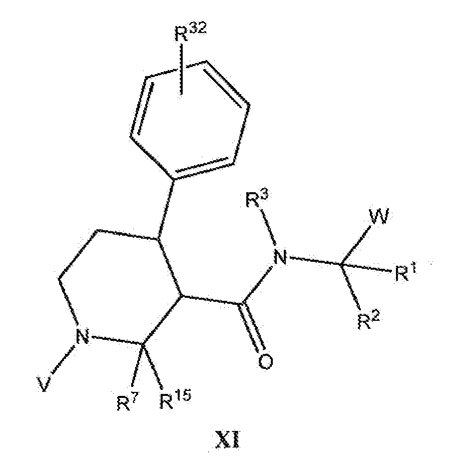 and pharmaceutically acceptable salts, enantiomers, stereoisomers, rotamers, tautomers, diastereomers, or racemates thereof;
   wherein
   R¹, R², R³, R⁷, R¹⁵, V and W have the meanings set forth for claim 19; and
   R³² is H, halogen, hydroxy, amino, C₁₋₄-alkyl, C₁₋₄alkoxy, mono- and di-C₁₋₄alkylamino, C₃₋₆-cycloalkylC₀₋₄alkyl, C₃₋₆-CycloalkylC₀₋₄alkoxy, aryl, aralkyl, heterocycleC₀₋₄alkyl, and heterocycleC₀₋₄alkoxy, each of which is substituted with 0 to 5 residues independently selected from halogen, hydroxy, amino, oxo, C₁₋₄-alkyl, C₁₋₄alkoxy, mono- and di-C₁₋₄alkylamino, C₃₋₆-cycloalkyl, aryl, and heterocycle.
26. The compound of embodiment 24, wherein Formula X is represented by a compound of the Formula XII: 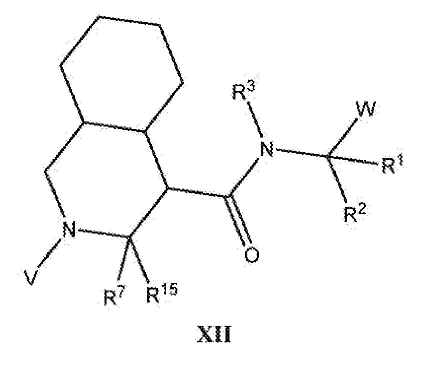 and pharmaceutically acceptable salts, enantiomers, stereoisomers, rotamers, tautomers, diastereomers, or racemates thereof;
   wherein
   R¹, R², R³, R⁷, R¹⁵, V and W have the meanings set forth for claim 24.
27. The compound of embodiment 1, wherein Formula I is represented by a compound of the Formula XIII: 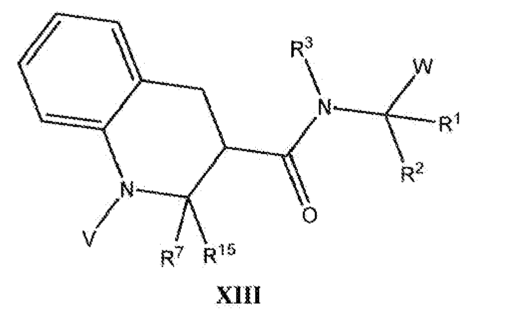 and pharmaceutically acceptable salts, enantiomers, stereoisomers, rotamers, tautomers, diastereomers, or racemates thereof;
   wherein
   R¹, R², R³, R⁷, R¹⁵, V and W have the meanings set forth for claim 1.
28. The compound of embodiment 1, wherein Formula I is represented by a compound of the Formula XIV: 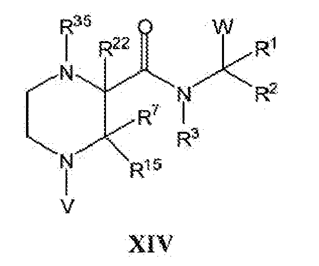 and pharmaceutically acceptable salts, enantiomers, stereoisomers, rotamers, tautomers, diastereomers, or racemates thereof;
   where
   R¹, R², R³, R⁷, R¹⁸, R²², V and W have the meanings set forth for claim 1; and
   R³⁵ is H, halogen, hydroxy, amino, C₁₋₄-alkyl, C₁₋₄alkoxy, mono- and di-C₁₋₄alkylamino, C₃₋₆-cycloalkylC₀₋₄alkyl, C₃₋₆-cycloalkylC₀₋₄alkoxy, aryl, aralkyl, heterocyclcC₀₋₄alkyl, and heterocycleC₀₋₄alkoxy, each of which is substituted with 0 to 5 residues independently selected from halogen, hydroxy, amino, oxo, C₁₋₄-alkyl, C₁₋₄alkoxy, mono- and di-C₁₋₄alkylamino, C₃₋₆-cycloalkyl, aryl, and heterocycle.
29. The compound of embodiment 28, wherein R²⁵ is phenyl, optionally substituted with chloro.
30. The compounds of any one of the above embodiment, wherein W, R¹ and R² form a substituent of the following formulas: 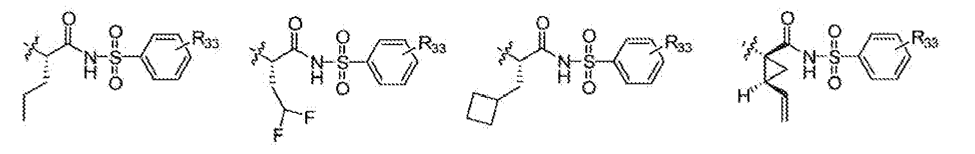 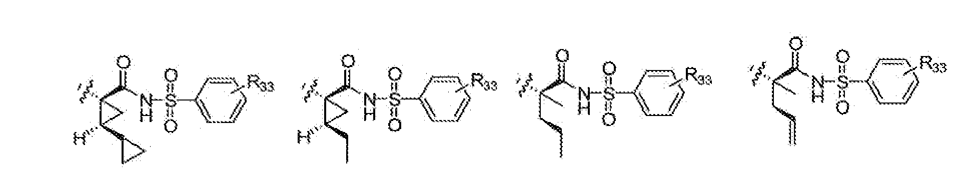 wherein R³³ is selected from the group consisting of H, phenyl, methyl, CF₃, tBu, NO₂, Cl, CN, NH₂, OH, NHCH₃, OCH₃, NHPh, OPh, NHCOCH, NHCOPh, OCH2Ph, COCH₃, CO₂Et, CO₂CH₃, CONHPh and CONHCH₃, or R³³ can be fused with the phenyl ring to form a naphthyl ring.
31. The compounds of any one of the above embodiments, wherein W, R¹ and R² form substituents selected from the group consisting of 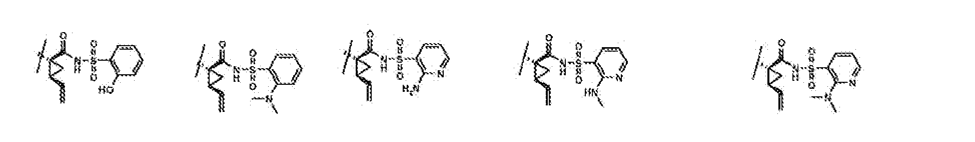 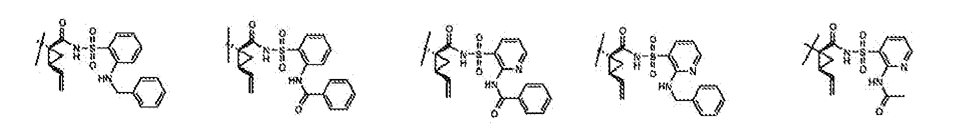 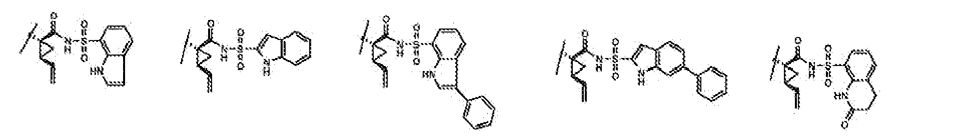 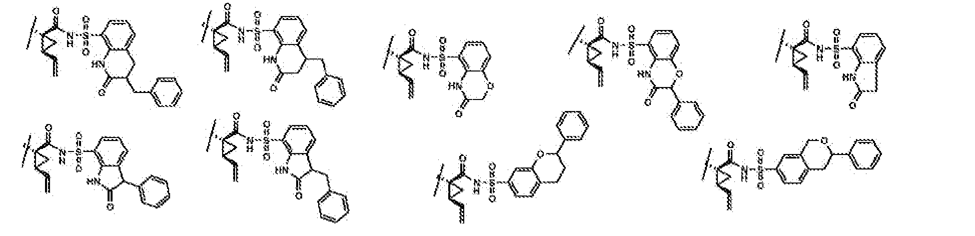 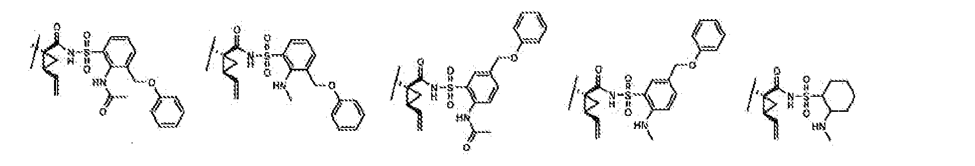 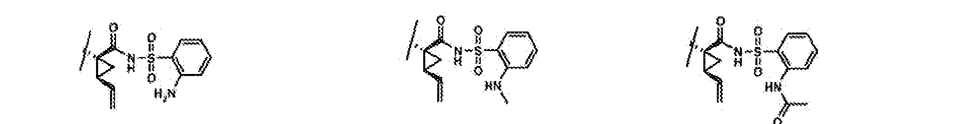 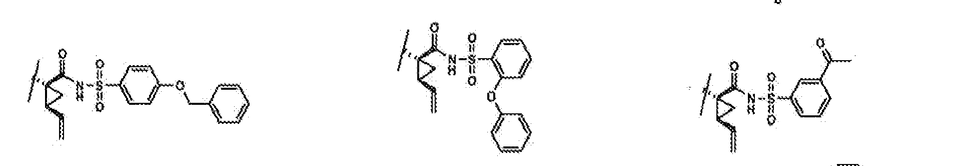 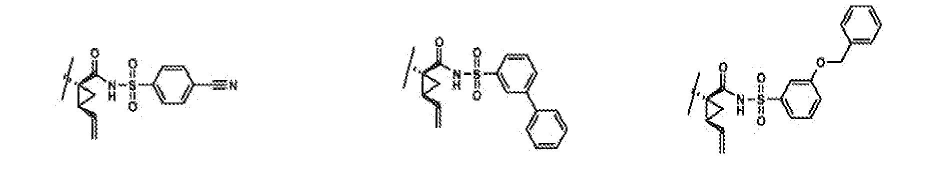 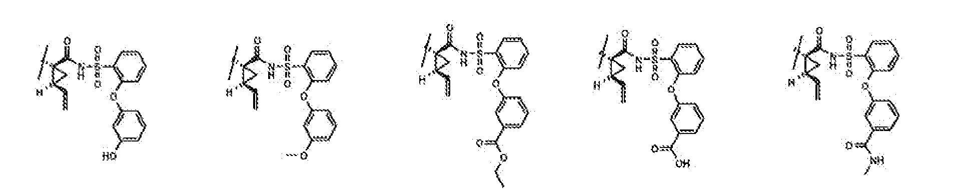 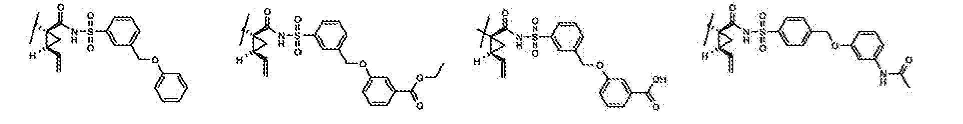 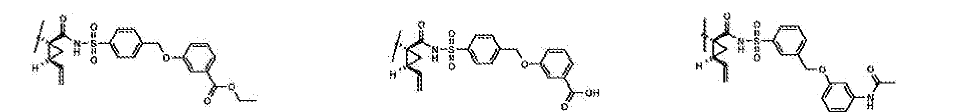 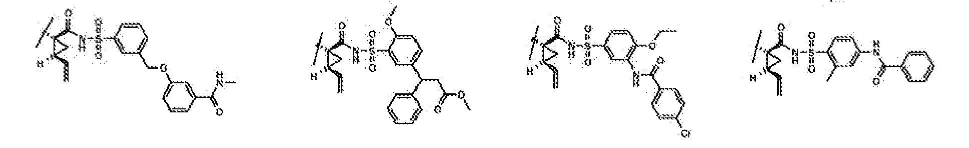 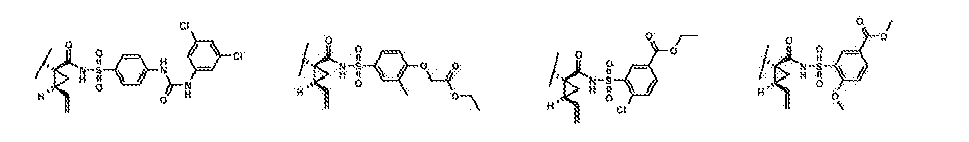 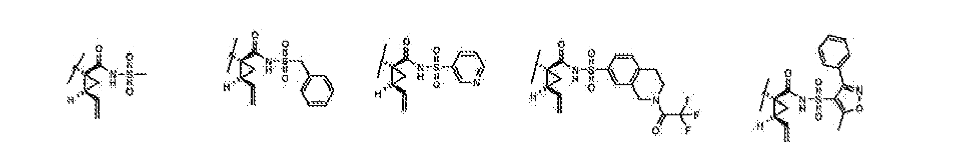 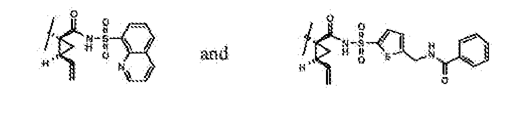
32. The compound of any one of the above embodiments, wherein any of the heterocycle groups are independently selected from the group consisting of aeridinyl, carbazolyl, cinnolinyl, quinoxalinyl, pyrrazolyl, indolyl, benzotriazolyl, furanyl, thienyl benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, tetrahydroquinoline, benzoimidazoly, benzofuranyl, benzofurazanyl, benzopyrazolyl, benzotriazolyl, benzothiophenyl, benzoxazolyl, carbazolyl, carbolinyl, cinnolinyl, furanyl, imidazolyl, indolinyl, indolyl, indolazinyl, indazolyl, isobenzofuranyl, isoindolyl, isoquinolyl,
   isothiazolyl, isoxazolyl, naphthpyridinyl, oxadiazolyl, oxazolyl, oxazoline, isoxazoline, oxetanyl, pyranyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrahydropyranyl, tetrazolyl, tetrazolopyridyl, thiadiazolyl, thiazolyl, thienyl, triazolyl, azetidinyl, 1,4-dioxanyl, hexahydmazepinyl, piperazinyl, piperidinyl, pyridin-2-onyl, pyrrolidinyl, morpholinyl, thiomorpholinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenzoyl, tetrahydrofuranyl, and tetrahydrothienyl, and N-oxides thereof, each of which may be independently further substituted one or more times with a halogen atom, C₁₋₄-alkyl, C₁₋₄-alkyl substituted by one or more halogen atoms, or C₃₋₆-cycloalkyl.
33. The compound of any one of the above embodiments, wherein W is C(O)-C(O)-N(H)-cyclopropyl or C(O)-C(O)-N(H)-NH₂.
34. The compound of any one of the above embodiments, wherein V is selected from the group consisting of C(O)R²⁴, C(O)N(H)R²⁴ and C(O)OR²⁴, wherein each R²⁴ is hydrogen or is independently selected from the group consisting of halogen, C₁₋₄-alkyl, amino, mono- and di-C₁₋₄alkylamino, C₁₋₄alkoxy, C₃₋₆-cycloalkylC₀₋₄alkyl, C₃₋₆-cycloalkylC₀₋₄alkoxy, aryl, aralkyl and heterocycleC₀₋₄alkyl, wherein each R²⁴ residue is further substituted with 0 to 5 groups selected from halogen, hydroxy, oxo, amino, C₁₋₄-alkyl, amino, mono- and di-C₁₋₄alkylamino, C₁₋₄alkoxy, C₃₋₆cycloalkyl, aryl, and heterocycle.
35. The compound of embodiment 34, wherein V is selected from the group consisting of benzyl, substituted benzyl, naphthyl, C₁₋₄-alkyl, and 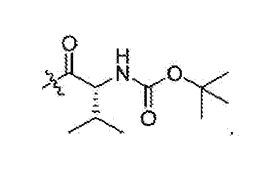
36. The compound of any one of the above embodiments, wherein any of the C₃₋₆-cycloalkyl groups may be independently substituted one or more times with a halogen atom, aryl, trihalomethyl, or C₁₋₄-alkyl.
37. The compound of embodiments 1 and 10, wherein R⁵ is selected from the group consisting of piperidine, phenyl, -O-pyridinyl and CH₂-pyridinyl, wherein the phenyl and pyridinyl groups may be independently substituted one or more times with a halogen atom or C₁₋₄-alkyl.
38. The compound of embodiment 37, wherein R⁵ is 5-chloro-pyridin-2-yl or 5-chloro-pyrodin-2-yloxy.
39. The compound of any one of the above embodiments, wherein W is selected from the group consisting of C(O)-C(O)N(R²³)₂, wherein R²³ is hydrogen or is independently selected from the group consisting of C₁₋₄-alkyl, C₃₋₆-cycloalkylC₀₋₄alkyl, aryl and heterocycle, each of which may be independently substituted one or more times with a halogen atom or C₁₋₄-alkyl.
40. The compound of any one of the above embodiments, wherein W is selected from the group consisting of C(O)-C(O)NH₂, C(O)-C(O)N(H)-cyclopropyl, C(O)-benzothiazole, C(O)-benzoimidazole, C(O)-oxazole, C(O)-imidazole, and C(O)-oxadiaxole, wherein the benzothiazole, benzoimidazole, oxazole and oxadiazole groups may be independently substituted one or more times with a halogen, atom, aryl, trihalomethyl, C₃₋₆cycloalkylC₀₋₄alkyl or C₁₋₄-alkyl.
41. The compound of any one of the above embodiments, wherein W is selected from the group consisting of 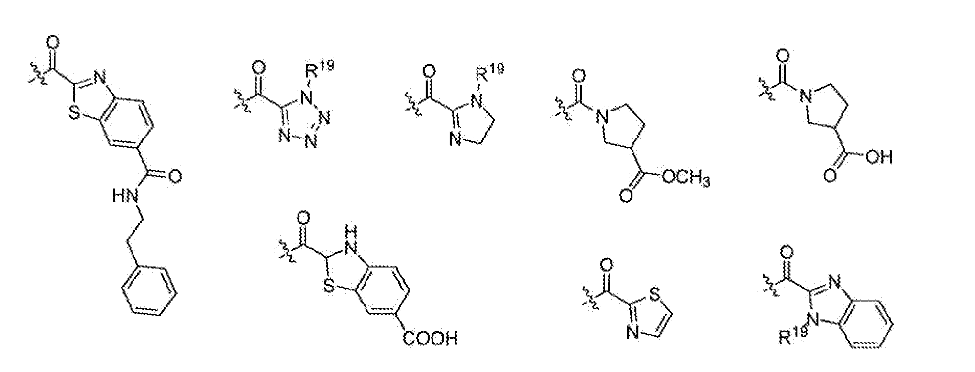 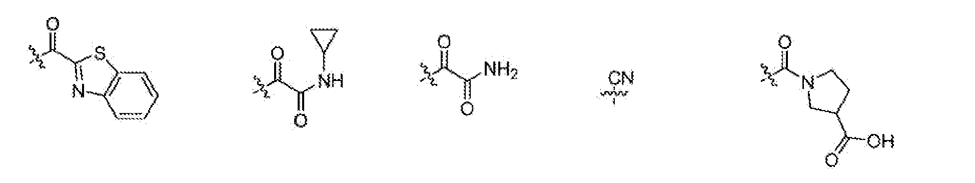 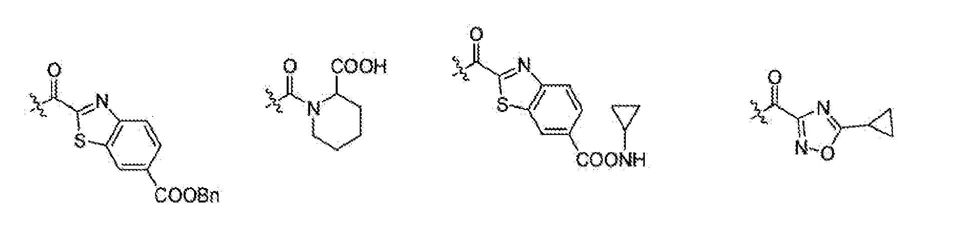 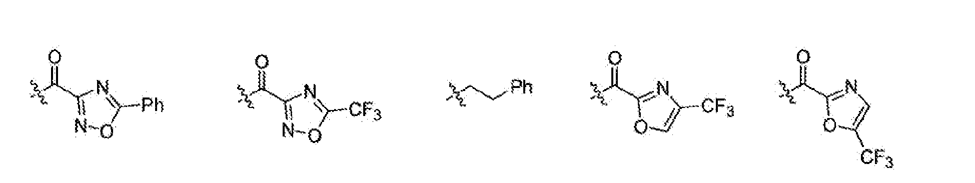 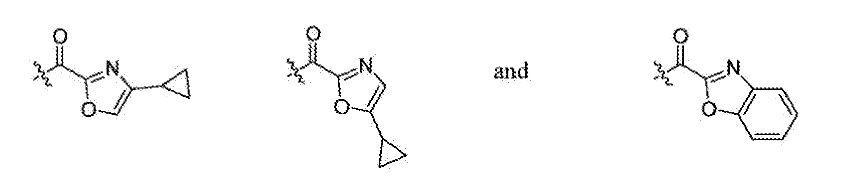 wherein R¹⁹ is selected from the group consisting of hydrogen, a halogen atom, aryl, trihalomethyl, and C₁₋₄-alkyl.
42. The compound of any one of the above embodiments, wherein R² is selected form the group consisting of 2,2-difluoroethyl, propyl, CH₂-cyclobutyl and (CH₂)₂-cyctobutyl.
43. The compound of any one of the above embodiments, wherein R¹¹ is H and R¹² is C₃₋₆-cycloalkyl.
44. The compound of any one of the above embodiments, wherein R¹² is cyclohexyl.
45. The compound of any one of the above embodiments, wherein V is selected from the group consisting of C(O)-N(H)-*t*-butyl.
46. The compound of any one of the above embodiments, wherein V is C(O)-R²⁰, wherein R²⁰ is selected from the group consisting of C₃₋₆-cycloalkyl, phenyl, pyrazine, benzooxazole, 4,4-dimethyl-4,5-dihydro-oxazole, benzoimidazole, pyrimidine, benzothiazole 1,1-dioxide and quinazoline, each of which may be further independently substituted with a halogen atom, CF₃, C₁₋₄-alkyl or C₃₋₆-cycloalkyl,
47. The compound of any one of the above embodiments, wherein V is C(O)-R²⁰, wherein is selected from the group consisting of 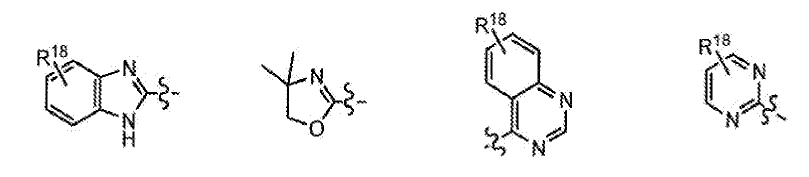 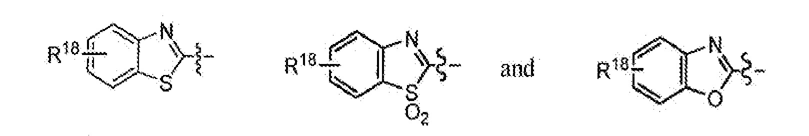 wherein R¹⁸ is selected from the group consisting of hydrogen, a halogen atom, aryl, trihalomethyl, and C₁₋₄-alkyl.
48. The compound of any one of the above embodiments, wherein V is C(O)-R²⁰, wherein R²⁰ is selected from the group consisting of 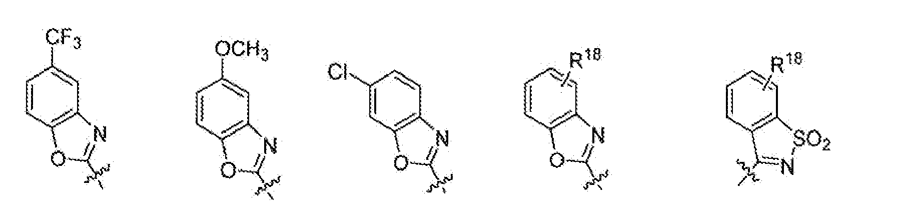 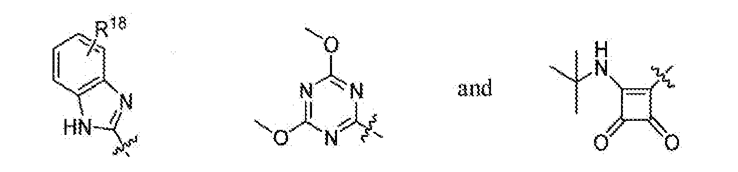 wherein R¹⁸ is selected from the group consisting of hydrogen, a halogen atom, aryl, trihalomethyl, and C₁₋₄-alkyl,
49. The compound of any one of the above embodiments, wherein V is selected from the group consisting of C₃₋₆-cycloalkyl, phenyl, pyrazine, benzooxazole, 4,4-dimethyl-4,5-dihydro-oxazole, benzoimidazole, pyrimidine, benzothiazole 1,1-dioxide and quinazoline, each of which may be further independently substituted with a halogen atom, CF₃, C₁₋₄-alkyl or C₃₋₆-cycloalkyl.
50. The compound of any one of the above embodiments, wherein V is selected from the group consisting of 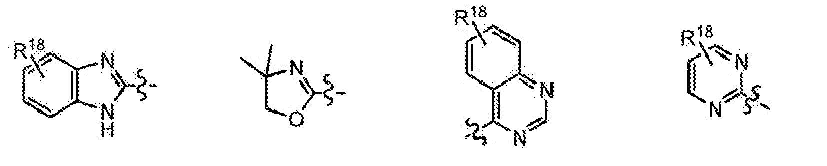 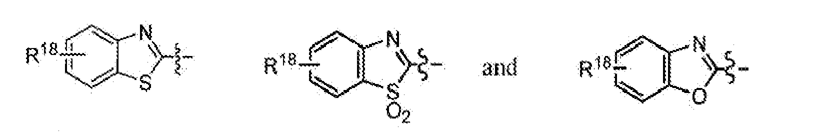 wherein R¹⁸ is selected from the group consisting of hydrogen, a halogen atom, aryl, trihalomethyl, and C₁₋₄-alkyl.
51. The compound of any one of the above embodiments, wherein V is selected from the group consisting of 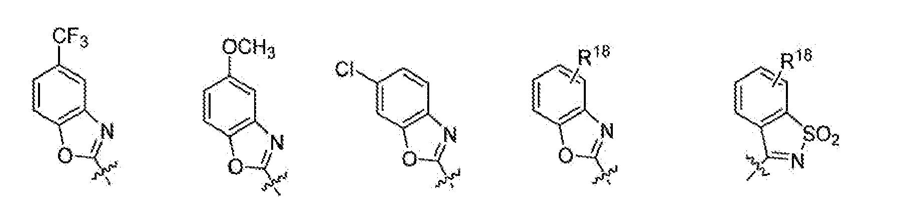 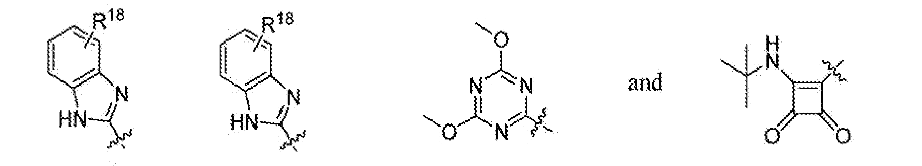 wherein R¹⁸ is selected from the group consisting of hydrogen, a halogen atom, aryl, trihalomethyl, and C₁₋₄alkyl.
52. The compound of any one of the above embodiments, wherein R⁵ is selected from the group consisting of 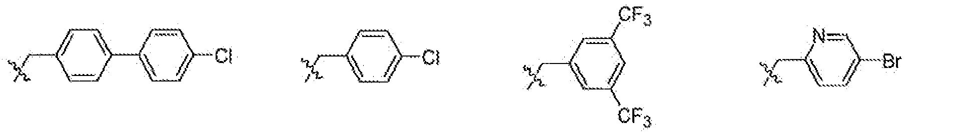 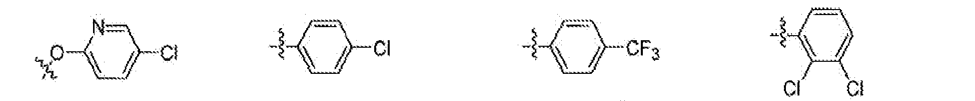 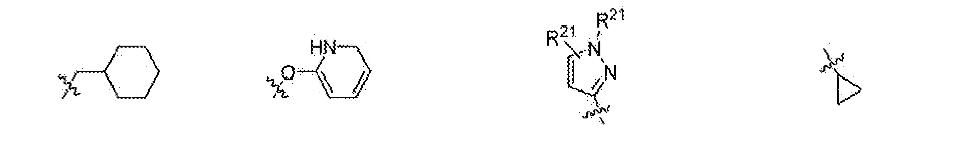 and 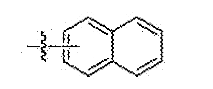 wherein R²¹ is independently selected from the group consisting of C₁₋₄-alkyl and aryl.
53. The compound of any one of the above embodiments, wherein W is C(O)-C(O)-amino.
54. The compound of embodiment 1, wherein or R¹⁷ and R¹⁶ together form a ring of the formula III, wherein n and g are each, independently, 0 or 1.
55. The compound of any one of the above embodiments, wherein R¹³ is H and V is selected from the group consisting of C=N(H)NH₂, C=N(CN)NH₂ and C(O)NH₂.
56. The compound of any one of the above embodiments, wherein W is C(O)N(H)S(O)₂R²⁴, wherein R²⁴ is hydrogen or is selected from the group consisting C₁₋₄-alkyl, C₃₋₆-cycloalkylC₀₋₄alkyl, substituted or unsubstituted aryl and substituted or unsubstituted heterocycle, each of which may be independently substituted one or more times with a halogen atom or C₁₋₄-alkyl.
57. The compound of any one of the above embodiments, wherein W is COOH, R¹ is H, and R² is selected from the group consisting of propyl, 2,2-difluoroethyl and CH₂-cyclobutyl, or R¹ and R² form together a cyclopropyl group that may be further substituted with a vinyl group.
58. The compound of any one of the above embodiments, wherein R⁵, R⁴ and R^{4a} are each, independently, selected from the group consisting of H, C₁₋₄alkoxy, aryloxy, heterocyclyl-oxy, aralkyloxy, C(O)N(R²⁴)₂, -N(R²⁴)C(O)R²⁴, C₁₋₄alkyl, aryl and aralkyl, wherein R²⁴ is hydrogen or halogen or is independently selected from the group consisting of C₁₋₄-alkyl, amino, mono- and di-C₁₋₄alkylamino, C₁₋₄alkoxy, C₃₋₆-cycloalkylC₀₋₄alkyl, C₃₋₆-cycloalkylyC₀₋₄alkoxy, aryl, aralkyl and heterocycleC₀₋₄alkyl, wherein each R²⁴ residue is further substituted with 0 to 5 groups independently selected from halogen, hydroxy, oxo, C₁₋₄alkyl, amino, mono- and di-C₁₋₄alkylamino, C₁₋₄alkoxy, C₃₋₆cycloalkyl, aryl, and heterocycle.
59. The compounds of any one of the above embodiments, wherein R¹ and R² form a substituent of the following formula: 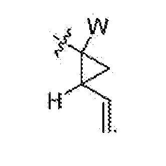
60. The compounds of any one of the above embodiments, wherein W, R¹ and R² form a substituent of the following formula: 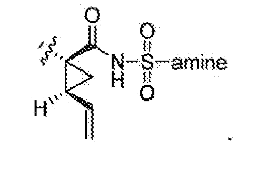
61. The compounds of any one of the above embodiments, wherein W, R¹ and R² form a substituent of the following formula: 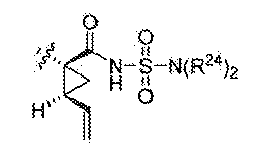 wherein each R²⁴ is independently selected from the group consisting of H, substituted or unsubstituted-C₁₋₄-alkyl, substituted or unsubstituted- C₃₋₆-cycloalkylC₀₋₄alkyl, substituted or unsubstituted aryl and substituted or unsubstituted heterocycle.
62. The compound of any one of the above embodiments, wherein R²⁴ is selected from the group consisting 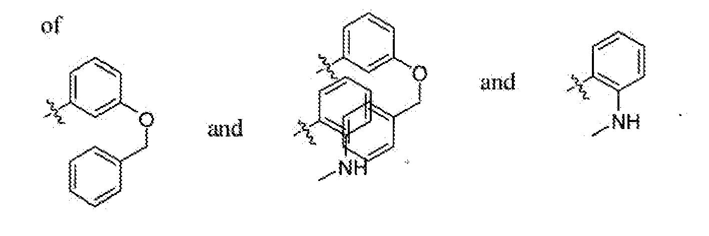
63. The compound of any one of the above embodiments, wherein W, R¹ and R² form a substituent selected from he group consisting of: 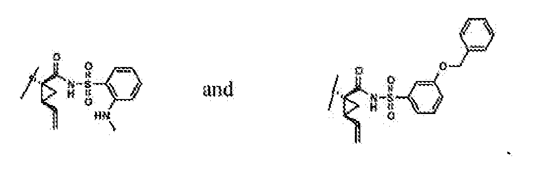
64. The compound of any one of the above embodiments, wherein V is selected from the group consisting of acyl, SO₂-R²⁴, C(O)N(R²⁴)₂, C(O)O(R²⁴)₂, and N(H)R²⁴, wherein each R²⁴ is hydrogen or is independently selected from the group consisting of C₁₋₄-alkyl, C₃₋₆-cycloalkylC₀₋₄alkyl, amino, mono-and diC₁₋₄ alkylamino, aryl, aralkyl, aryloxy, and heterocycle C₀₋₄alkyl, each of which is substituted with 0-5 groups independently selected from halogen, hydroxy, oxo, amino, C₁₋₄-alkyl, mono- and di-C₁₋₄alkylamino, C₁. ₄alkoxy, C₃₋₆cycloalkyl, aryl, and heterocycl.
65. A method of treating an HCV-associated disorder comprising administering to a subject in need thereof a pharmaceutically acceptable amount of a compound of formula I or II, such that the HCV-associated disorder is treated.
66. The method of embodiment 65, wherein the HCV-associated disorder is selected from the group consisting of HCV infection, liver cirrhosis, chronic liver disease, hepatocellular carcinoma, cryoglobulinaemia, non-Hodgkin's lymphoma, and a suppressed innate intracellular immune response.
67. A method of treating an HIV infection comprising administering to a subject in need thereof a pharmaceutically acceptable amount of a compound of formula 1 or 11.
68. A method of treating, inhibiting or preventing the activity of HCV in a subject in need thereof, comprising administering to the subject a pharmaceutically acceptable amount of a compound of Formula I, II, VI, VII, VIII, IX, X, XI, XII, XIII or XIV.
69. A method of inhibiting the activity of a serine protease, comprising the step of contacting said serine protease with a compound according to embodiment 68.
70. The method of embodiment 68, wherein the activity of the NS2 protease is inhibited.
71. The method of embodiment 68, wherein the activity of the NS3 protease is inhibited.
72. The method of embodiment 68, wherein the activity of the NS3 helicase is inhibited.
73. The method of embodiment 68, wherein the activity of the NS5a protein is inhibited.
74. The method of embodiment 68, wherein the activity of the NS5b polymerase is inhibited.
75. The method of embodiment 68, wherein the interaction between the NS3 protease and NS4A cofactor s disrupted.
76. The method of embodiment 68, wherein the severing of one or more of the NS4A-NS4B, NS4B-NS5A and NS5A-NS5B junctions of the HCV is prevented or altered.
77. The method of any one of embodiments 65-76, wherein an HCV-associated disorder is treated in a subject in need thereof.
78. The method of embodiment 77, wherein the HCV-associated disorder is selected from the group consisting of HCV infection, liver cirrhosis, chronic liver disease, hepatocellular carcinoma, cryoglobulinaemia, non-Hodgkin's lymphoma, and a suppressed innate intracellular immune response.
79. A method of treating, inhibiting or preventing the activity of HCV in a subject in need thereof, comprising administering to the subject a pharmaceutically acceptable amount of a compound of Formula I, II, VI, VII, VIII, IX, X, XI ,XII, XIII of XIV, wherein the compound interacts with any target in the HCV life cycle.
80. The method of embodiment 79, wherein the target is selected from the group consisting of NS2 protease, NS3 protease, NS3 helicase, NS5a protein andNS5b polymerase.
81. A method of decreasing the HCV RNA load in a subject in need thereof comprising administering to the subject a pharmaceutically acceptable amount of a compound of formula I or II, such that the HCV RNA load in the subject is decreased,
82. A compound exhibiting HCV protease activity, wherein the compound is of the Formula I, II, VI, VII, VIII, IX, X, XI, XII, XIII or XIV.
83. The compound of embodiment 82, wherein the compound is a HCV NS3-4A protease inhibitor.
84. A method of treating an HCV-associated disorder in a subject, comprising administering to a subject in need thereof a pharmaceutically acceptable amount of a compound of the Formula I, II, VI, VII, VIII, IX, X, XI, XII, XIII or XIV, and a pharmaceutically acceptable carrier, such that the HCV-associated disorder is treated.
85. A method of treating an HCV-associated disorder comprising administering to a subject in need thereof a pharmaceutically effective amount of a compound of the Formula I, 11, VI, VII, VIII, IX, X, XI, XII, XIII or XIV, in combination with a pharmaceutically effective amount of an additional HCV-modulating compound, such that the HCV-associated disorder is treated.
86. The method of embodiment 85, therein the additional HCV-moduialing compound is selected from the group consisting of Sch 503034 and VX-950.
87. The method of embodiment 85 wherein the additional HCV-modulating compound is interferon or derivatized interferon.
88. The method of embodiment 87, wherein the interferon is selected from the group consisting of interferon alpha 2B, pegylated interferon alpha, consensus interferon, interferon alpha 2A, lymphoblastoid interferon, and interferon tau; and said compound having anti-hepatitis C virus activity is selected from the group consisting of interleukin 2, interleukin 6, interleukin 12, a compound that enhances the development of a type I helper T cell response, double stranded RNA, double stranded RNA complexed with tobramycin, Imiquimod, ribavirin, an inosine 5'-monophosphate dehydrogenase inhibitor, amantadine, and rimantadine.
89. The method of embodiment 85 wherein the additional HCV-moduiating compound is a cytochrome P450 monooxygenase inhibitor.
90. The method of embodiment 89, wherein the cytochrome P450 inhibitor is selected from the group consisting of ritonavir, ketoconazole, troleandomycin, 4-methyl pyrazole, cyclosporin, and clomethiazole.
91. The method of embodiment 84 or 85, wherein the HCV-associated disorder is selected from the group consisting of HCV infection, liver cirrhosis, chronic liver disease, hepatocellular carcinoma, cryoglobulinaemia, non-Hodgkin's lymphoma, and a suppressed innate intracellular immune response.
92. A method of inhibiting hepatitis C virus replication in a cell, comprising contacting said cell with a compound of Formula I, II, VI, VII, VIII, IX, X, XI XII, XIII or XIV.
93. A packaged HCV-associated disorder treatment, comprising an HCV-modulating compound of the Formula I, II, VI, VII, VIII, IX, X, XI, XII, XIII or XIV, packaged with instructions for using an effective amount of the HCV-modulating compound to treat an HCV-associated disorder.
94. The treatment of embodiment 93, wherein the HCV-associated disorder is selected from the group consisting of HCV infection, liver cirrhosis, chronic liver disease, hepatocellular carcinoma, cryoglobulinaemia, non-Hodgkin's lymphoma, and a suppressed innate intracellular immune response.
95. A method of treating HCV infection, liver cirrhosis, chronic liver disease, hepatocellular carcinoma, cryoglobulinaemia, non-Hodgkin's lymphoma, and/or a suppressed innate intracellular immune response in subject in need thereof comprising administering to the subject a pharmaceutically acceptable amount of a compound of Formula I, II, VI, VII, VIII, IX, X, XI ,XII, XIII or XIV.
96. The method of embodiment 68, wherein the HCV is selected from any HCV genotype.
97. The method of embodiment 96, wherein the HCV is selected from HCV genotype 1, 2 and/or 3.

## Claims

1. A compound of the Formula II: 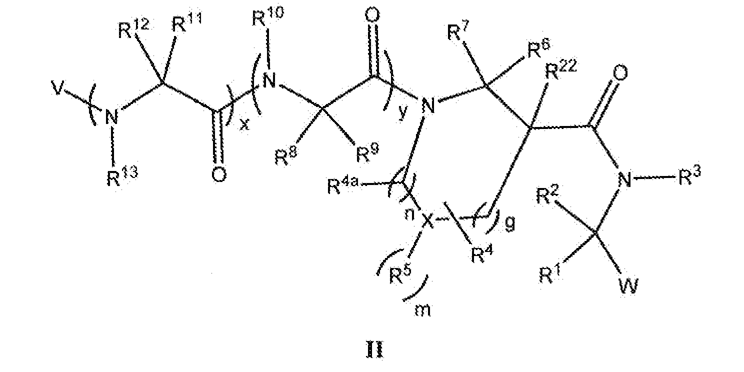 and pharmaceutically acceptable salts, enantiomers, stereoisomers, rotamers, tautomers, diastereomers, or racemates thereof;
wherein
x is 0 or 1;
y is 0, 1 or 2;
R¹ and R² are each, independently, selected from the group consisting of H, C₁₋₄-alkyl, O-C₁₋₄-alkyl,N(H)-C₁₋₄-alkyl, and C₃₋₆-cycloalkylC₀₋₄alkyl;
W is selected from the group consisting of C(O)OH, C(O)OR²⁴, C(O)-amine, C(O)-C(O)OH, C(=N-O-R²⁴)-C(O)-amine, C(O)N(H)S(O)₂R²⁴,C(O)-C(O)-amine, SO₂-N(R²⁴)₂ and C(O)-[C(O)]ₐ-heterocycle, wherein the heterocycle may be substituted or unsubstituted, wherein a is 0 or 1, wherein each R²⁴ is independently selected from the group consisting of H, halogen, hydroxyl, formyl, carboxylate, amide, amino, substituted or unsubstituted-C₁₋₄-alkyl, substituted or unsubstituted-C₁₋₄-alkoxy, substituted or unsubstituted-C₁₋₄-alkanoyl, substituted or utisubstituted-C₁₋₄-alkoxycarbonyl, substituted or unsubstituted-C₁₋₄-alkanoyloxy, substituted or unsubstituted mono- and di-C₁₋₄-alkylamino, substituted or unsubstituted-C₃₋₆cycloalkyl-C₀₋₄alkyl, substituted or unsubstituted aryl-C₀₋₄alkyl, and substituted or unsubstituted heterocycle-C₀₋₄alkyl;
R³ is selected from the group consisting of H, C₁₋₄-alkyl and C₃₋₆-cycloalkylC_{0- 4}alkyl;
R²² and R⁷ are each, independently, hydrogen or are selected from the group consisting of C₁₋₄-akyl, O-C₁₋₄-alkyl, N(H)-C₁₋₄-alkyl, C₃₋₆-cycloalkylC₀₋₄alkyl, aryl and heterocycle, each of which may be independently substituted one or more times;
n and g are each, independently, 0, 1 or 2;
m is 0 or 1;
X is O, N or C;
R⁴ and R^{4a} are each, independently, hydrogen or are selected from the group consisting of C₁₋₄-alkyl, O-C₁₋₄-alkyl, N(H)-C₁₋₄-alkyl, C₃₋₆-cycloalkylC₀₋₄alkyl, aryl, O-aryl and heterocycle, each of whichmay be further independently substituted;
R⁵ is hydrogen or oxo or is selected from the group consisting of hydroxyl, C₁₋₄-alkyl, C₁₋₄-alkoxy, and di-C₁₋₄alkylamino, C₃₋₆-cycloalkyl-C₀₋₄-alkyl, aryl-C₀₋₄-alkyl, heterocycle-C₀₋₄-alkyl, each of which may be further independently substituted;
R⁶, R⁸, R⁹, R¹¹ and R¹² are each, independently, selected from the group consisting or H, C₁₋₄-alkyl, O-C₁₋₄-alkyl, N(H)-C₁₋₄-alkyl, and C₃₋₆-cycloalkylC₀₋₄alkyl;
R¹⁰ is selected from the group consisting of H, C₁₋₄-alkyl and C₃₋₆-cycloalkylC₀₋₄alkyl;
R¹³ is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-CO-C₁₋₄alkyl, and Q² is H or selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl; and
V is selected from the group consisting of -Q¹-Q², wherein Q¹ is absent, C(O), S(O)₂, N(H), N(C₁₋₄-alkyl), C=N(CN), C=N(SO₂CH₃), C=N-COH, or C=N-COC₁₋₄alkyl, and Q² is H or is selected from the group consisting of C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
or R⁴ and R⁵ may together form a 4, 5, 6 or 7-membered ring that is aromatic or non-aromatic and may contain one or more heteroatoms, wherein the ring may be further substituted one or more times.

2. The compound of claim 1, wherein R⁴ and R⁵ together form a phenyl ring, which may be substituted with halogen atom, aryl, trihalomethyl, or C₁₋₄-alkyl, or a dimethyl cyclopropyl ring such that a fused ring system is formed.

3. The compound of claim 1, wherein one of and n is 0.

4. The compound of claim 1, wherein
R¹ is selected from the group consisting of H and C₁₋₄-alkyl;
R² is selected from the group consisting of C₁₋₄-alkyl and C₃₋₆-cycloalkylC₀₋₄alkyl;
W is selected from the group consisting of C(O)-C(O)-amine and C(O)-[C(O)]ₐ-heterocycle, wherein the heterocycle may be independently substituted one or more times with aryl, C₁₋₄-alkyl, C₁₋₄-alkyl substituted by one or more halogen atoms, and C₃₋₆-cycloalkyl, wherein a is 0 or 1, wherein R²⁴ is hydrogen or is selected from the group consisting of C₁₋₄-alkyl, C₃-₆-cydoalkyl C₀₋₄alkyl, aryl and heterocycle, each of which may be independently substituted one or more times with a halogen atom or C₁₋₄-alkyl;
R³ is selected from the group consisting of H and C₁₋₄-alkyl;
R⁷ is hydrogen or is selected from the group consisting of C₁₋₄-alkyl, C₃₋₆-cycloalkyl, aryl and heterocycle, each of which may be independently substituted one or more times with a halogen atom, C₁₋₄-alkyl, C₁₋₄-alkyl substituted by one of more halogen atoms, or C₃₋₆-cycoalkyl;
R⁴ and R^{4a} are each, independently, hydrogen or are independently selected from the group consisting of C₁₋₄-alkyl, C₃₋₆-cycloalkyl, aryl and heterocycle, each of which may be independently substituted one or more times with a halogen atom or C₁₋₄-alkyl,
R⁵ is hydrogen or is selected from the group consisting of hydroxyl, oxo, C₁₋₄-alkyl, C₁₋₄-alkoxy, mono- and di-C₁₋₄alkylamino, C₃₋₆-cycloalkyl-C₀₋₄-alkl, aryl-C₀₋₄-alkyl, heterocycle-C₀₋₄-alkyl each of which may be independently substituted one or more times with a halogen atom, aryl, trihalomethyl, or C₁₋₄-alkyl;
R¹³ and R⁶ are H;
R⁸, R¹⁰ and R¹¹ are each, independently, selected from the group consisting of H and C₁₋₄-alkyl;
R⁹ and R¹² are each, independently, selected from the group consisting of H, C₁₋₄-alkyl and C₃₋₆-cycloalkyl; and
V is selected from the group consisting of -Q¹-Q², wherein Q¹ is absent, C(O), S(O)₂, N(H), N(C₁₋₄-alkyl), C-N(CN), C=N(SO₂CH₃), C=N-COH, or C=N-COC₁₋₄alkyl, and Q² is H, C₁₋₄-alkyl, O-C₁₋₄-alkyl, NH₂, N(H)-C₁₋₄-alkyl, N(C₁₋₄-alkyl)₂, SO₂-aryl, SO₂-C₁₋₄-alkyl, C₃₋₆-cycloalkyl-C₀₋₄-alkyl, aryl, heteroaryl and heterocycle, each of which may be independently substituted one or more times with a halogen atom, C₁₋₄-alkyl, C₁₋₄-alkyl substituted by one or more halogen atoms, or C₃₋₆-cycloalkyl;
or R⁴ and R⁵ may together form a phenyl ring, which may be substituted with a halogen atom; aryl, trihalomethyl, or C₁₋₄-alkyl, or a dimethyl cyclopropyl ring such that a fused ring system is formed.

5. The compound of claim 1, wherein R⁴ is H and R⁵ is (CH₂)₀₋₃-aryl, -O-heterocycle, or (CH₂)₀₋₃-heterocyele, wherein aryl and heterocycle may be independently substituted one or more times with a halogen atom, aryl, trihalomethyl, C₃₋₆-cycloalkyl or C₁₋₄-alkyl.

6. The compound of claim 1, wherein n is 1, and R⁴ and R⁵ together form the following fused ring systems: 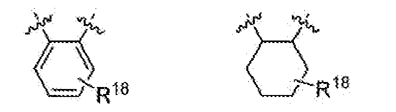 wherein each R¹⁸ is independently selected from the group consisting of hydrogen, a halogen atom, aryl, trihalomethyl, and C₁₋₄-alkyl.

7. A compound of formula II as claimed in any one of claims 1 to 6, for use in medicine.

8. A compound of formula II as claimed in any one of claims 1 to 6, for use in treating an HCV-associated disorder.

9. A compound of formula II as claimed in any one of claims 1 to 6, for use as claimed in claim 8, wherein the HCV-assoeiated disorder is selected from the group consisting of HCV infection, liver cirrhosis, chronic liver disease, hepatocellular carcinoma, cryoglobulinaemia, non-Hodgkin's lymphoma, and a suppressed innate intracellular immune response.

10. A compound of formula II as claimed in any one of claims 1 to 6, in combination With an additional HCV-modulating compound.

11. Use of a compound of formula II as claimed in any one of claims 1 to 6, in the manufacture of a medicament for the treatment of an HCV-associated disorder.

12. A pharmaceutically acceptabte composition comprising a compound of formula II as claimed in any one of claims 1 to 6, and a pharmaceutically acceptable excipient.
